(19) Europäisches Patentamt
European Patent Office
Office européen des brevets

(11) EP 1 354 882 A1

(12) **EUROPEAN PATENT APPLICATION**
published in accordance with Art. 158(3) EPC

(43) Date of publication:
**22.10.2003 Bulletin 2003/43**

(21) Application number: **01271892.0**

(22) Date of filing: **27.12.2001**

(51) Int Cl.[7]: **C07D 401/12**, C07D 417/12, C07D 401/14, C07D 403/12, C07D 405/12, C07D 207/16, C07D 417/14, A61K 31/4709, A61K 31/4725, A61K 31/428, A61K 31/4439, A61K 31/496, A61K 31/4545, A61K 31/497, A61K 31/498, A61K 31/506, A61K 31/433, A61K 31/502, A61K 31/501

(86) International application number:
**PCT/JP01/11578**

(87) International publication number:
**WO 02/051836 (04.07.2002 Gazette 2002/27)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **27.12.2000 JP 2000398441**
**30.08.2001 JP 2001261409**

(71) Applicant: **KYOWA HAKKO KOGYO CO., LTD.**
**Chiyoda-ku, Tokyo 100-8185 (JP)**

(72) Inventors:
- **MATSUNO, Kenji**
  **Tokyo 191-0062 (JP)**
- **UENO, K., c/o KYOWA HAKKO KOGYO CO., LTD**
  **Sunto-gun, Shizuoka 411-8731 (JP)**
- **IWATA, Yasuhiro**
  **Sapporo-shi, Hokkaido 007-0811 (JP)**
- **MATSUMOTO, Y.,**
  **c/o KYOWA HAKKO KOGYO CO., LTD**
  **Sunto-gun, Shizuoka 411-8731 (JP)**
- **NAKANISHI, S.,**
  **c/o KYOWA HAKKO KOGYO CO., LTD**
  **Tokyo 100-8185 (JP)**
- **TAKASAKI, K.,**
  **c/o KYOWA HAKKO KOGYO CO., LTD**
  **Sunto-gun, Shizuoka 411-8731 (JP)**
- **KUSAKA, H.,**
  **c/o KYOWA HAKKO KOGYO CO., LTD**
  **Sunto-gun, Shizuoka 411-8731 (JP)**
- **NOMOTO, Y.,**
  **c/o KYOWA HAKKO KOGYO CO., LTD**
  **Sunto-gun, Shizuoka 411-8731 (JP)**
- **OGAWA, A.,**
  **c/o KYOWA HAKKO KOGYO CO., LTD**
  **Sunto-gun, Shizuoka 411-8731 (JP)**

(74) Representative: **HOFFMANN - EITLE**
**Patent- und Rechtsanwälte**
**Arabellastrasse 4**
**81925 München (DE)**

(54) **DIPEPTIDYL PEPTIDASE IV INHIBITOR**

(57) A compound represented by general formula (I):

A-B-D

<wherein

A represents a substituted or unsubstituted 1-pyrrolidinyl group, a substituted or unsubstituted 3-thiazolidinyl group, a substituted or unsubstituted 1-oxo-3-thiazolidinyl group, or the like;
B represents a) a group represented by $-(C(R^1)(R^2))_kCO-$ (wherein k represents an integer of from 1 to 6, $R^1$ and $R^2$ may be the same or different and each represents a hydrogen atom, a hydroxyl group, a halogen atom, or the like) or the like;
D represents -U-V [wherein U represents a substi-

EP 1 354 882 A1

tuted or unsubstituted piperazinediyl group or the like, V represents -E-$R^7$ (wherein E represents a single bond, -CO-, -C(=O)O-, or -$SO_2$-; $R^7$ represents a hydrogen atom, a substituted or unsubstituted alkyl group, or the like)] > or a pharmacologically acceptable salt thereof.

## Description

Technical Field

The present invention relates to a compound which has an inhibitory action against dipeptidylpeptidase-IV (DPP-IV) and is useful for preventive and/or therapeutic treatment of Type II diabetes, for preventive and/or therapeutic treatment of complications accompanying said disease, or for therapeutic treatment for other pathologic conditions in which DPP-IV is involved, or a pharmacologically acceptable salt thereof.

Background Art

DPP-IV is known to be an enzyme which is involved in inactivation of glucagon-like peptide-1 (GLP-1). DPP-IV inhibitor is expected to be a medicament for Type II diabetes, because GLP-1 promotes secretion of insulin from pancreas in a manner depending on a glucose concentration. In several documents such as [Diabetologia, 42, 1324-1331 (1999), Diabetes, 47, 1663-1670 (1998), German Patent 19,616,486, and WO97/40832], the potentiality of the DPP-IV inhibitor as a medicament for Type II diabetes has been mentioned.

Physiologically and pathophysiologically in human, DPP-IV is known to play important roles such as (a) to (f) described below.

(a) DPP-IV is known to be involved in immune response.

Expression of DPP-IV in T-cell is increased by simulation with a mitogen or an antigen [Scand. J. Immunol., 33, 737 (1991)]. DPP-IV inhibitor and an antibody against DPP-IV suppress the proliferation of T-cells simulated with a mitogen or an antigen [Biol. Chem. Hoppe-Seyler, 305 (1991) and reference examples therein]. Various functions of T-lymphocytes, such as generation of cytokines, cell proliferation mediated by IL-2, and B-cell helper activities, are shown to depend on the activity of DPP-IV [Scand. J. Immunol., 29, 127 (1989)]. Boroproline derivatives as DPP-IV inhibitors described in the literature [Proc. Natl. Acad. Sci. USA, 88, 1556 (1991)] are shown to suppress the proliferation of lymphocyte simulated by antigen and generation of IL-2 in $CD4^+$ T-helper cell of mouse, in spite of their instability, thus to be effective for the suppression of the generation of antibody induced by immune challenge in mouse *in vivo* [Clin. Exp. Immunol., 89, 192 (1992)].

(b) In HIV infection, an inhibitor or an antibody against DPP-IV is reported to prevent virus invasion of cells. It is known that a selective reduction of CD26 expression is observed in T-cell derived from individual infected by HIV-1 [J. Immunol., 149, 3073 (1992)], and that HIV-1 Tat protein binds to DPP-IV [J. Immunol., 150, 2544 (1993)].

(c) Lung endothelial DPP-IV is shown to be an adhesion molecule for lung metastatic cancer cell of breast and prostate of rat [J. Cell. Biol., 121, 1423(1993)].

(d) High levels of DPP-IV expression are found in fibroblast cell of human skin of patients suffering from psoriasis, rheumatoid arthritis (RA), and lichen planus [J. Cell. Physiol., 151, 378 (1992)].

(e) High DPP-IV activity is observed in patients of benign prostate hypertrophy and in tissue homogenate of prostatosome [Eur. J. Clin. Chem. Clin. Biochem., 30, 333 (1992)].

(f) DPP-IV is shown to bind to an enzyme, i.e., adenosine deaminase (ADA), at the surface of T-cell [Science, 261, 466 (1993)]. Deficiency of ADA may cause severe combined immunodeficiency disease (SCID) in human.

Above described findings suggest that DPP-IV inhibitor is useful as a medicament for therapeutic treatment of diseases in which human DPP-IV is involved other than Type II diabetes. For example, the inhibitor is expected to be useful as (a) an immunosuppressant in tissue transplantation; for example, cytokine secretion suppressant for various autoimmune disease such as inflammatory bowel disease, encephalitis periaxialis scleroticans, rheumatoid arthritis (RA), (b) a medicament useful for preventing HIV invasion of T-cell and thereby useful for prevention and therapy of AIDS (acquired immunodeficiency syndrome), (c) a medicament for preventing metastasis, particularly preventing metastasis of breast and prostate cancer to lung, (d) a therapeutic medicament for dermatonosis such as psoriasis and lichen planus, (e) a medicament useful for benign prostate hypertrophy. Tetrahydroisoquinoline derivatives disclosed in Japanese Patent Unexamined Publication (KOKAI) No.10-182613 (1998), which is DPP-IV inhibitor, have been reported to practically suppress manifestation and evolution of adjuvant-induced arthritis.

As for the DPP-IV inhibitor, N-substituted pyrrolidine derivatives are reported in WO 95/34538, WO 98/19998, WO 00/34241, U.S. Patent No. 6,011,155, U.S. Patent No. 6,124,305, and U.S. Patent No. 5,462,928, N-substituted thiazole derivatives are reported in U.S. Patent No. 6,107,317 and U.S. Patent No. 6,110,949, heterocyclic compounds are reported in WO 95/15309, amino acid derivatives are reported in WO 99/67279, WO 99/61431, WO 99/67278, and U.S. Patent No. 6,090,786, phosphonate derivatives are reported in European Patent No. 1,050,540 and U.S. Patent No. 5,543,396 .

Disclosure of the Invention

An object of the present invention is to provide a novel compound which has an inhibitory activity against DPP-IV. Another object of the present invention is to provide a medicament which comprises as an active ingredient a compound having said activity or a pharmacologically acceptable salt thereof, and is useful for preventive and/or therapeutic treatment of Type II diabetes, for preventive and/or therapeutic treatment of complications accompanying said disease, or for therapeutic treatment for other pathological conditions in which DPP-IV is involved.
The inventors of the present invention conducted intensive studies to achieve the aforementioned objects. As a result, they achieved the aforementioned object by providing a compound represented by the following general formula (I).
The present invention thus relates to the following (I) to (XXV):

(I) A compound represented by general formula (I):

A-B-D

<wherein

A represents a substituted or unsubstituted 1-pyrrolidinyl group, a substituted or unsubstituted 3-thiazolidinyl group, a substituted or unsubstituted 1-oxo-3-thiazolidinyl group, a substituted or unsubstituted 1,1-dioxo-3-thiazolidinyl group, a substituted or unsubstituted 3-oxazolidinyl group, a substituted or unsubstituted 2,5-dihydro-1-pyrrolyl group, a substituted or unsubstituted 1-pyrrolyl group, a substituted or unsubstituted piperidino group, a substituted or unsubstituted 1-indolinyl group, a substituted or unsubstituted 1-indolyl group, a substituted or unsubstituted 1-octahydroindolyl group, a substituted or unsubstituted 1-tetrahydroquinolyl group, or a substituted or unsubstituted 1-decahydroquinolyl group;
B represents

a) a group represented by $-(C(R^1)(R^2))_kCO-$ (wherein k represents an integer of 1 to 6, $R^1$ and $R^2$ may be the same or different and each represents a hydrogen atom, a hydroxyl group, a halogen atom, a cyano group, a nitro group, a substituted or unsubstituted alkyl group, a substituted or unsubstituted alicyclic alkyl group, a substituted or unsubstituted alicyclic heterocyclic group, a substituted or unsubstituted alkenyl group, a substituted or unsubstituted alkynyl group, a substituted or unsubstituted aryl group, a substituted or unsubstituted aralkyl group, a substituted or unsubstituted heteroaryl group, or a substituted or unsubstituted heteroarylalkyl group, or $R^1$ and $R^2$, which attach to the same carbon atom, together with said carbon atom, or two $R^1$s, which attach to adjacent carbon atoms, respectively, together with the two carbon atoms when k is two or more, may combine to form a substituted or unsubstituted alicyclic alkyl group or a substituted or unsubstituted alicyclic heterocyclic group),
b) a group represented by $-CO(C(R^3)(R^4))_m-$ (wherein $R^3$ and $R^4$ may be the same or different and each has the same meaning as that defined above for $R^1$ and $R^2$, respectively, and m represents an integer of 1 to 6),
c) a group represented by $-(C(R^5)(R^6))_n-$ (wherein $R^5$ and $R^6$ may be the same or different and each has the same meaning as that defined above for $R^1$ and $R^2$, respectively, and n represents an integer of 2 to 7)
d) -CO-, or
e) $-SO_2$,

D represents

a group represented by -U-V [wherein U represents a substituted or unsubstituted piperazinediyl group or a homopiperazinediyl group; V represents $-E-R^7$ (wherein E represents a single bond, -CO-, -C(=O)O-, or $-SO_2-$; $R^7$ represents a hydrogen atom, a substituted or unsubstituted alkyl group, a substituted or unsubstituted alicyclic alkyl group, a substituted or unsubstituted alicyclic heterocyclic group, a substituted or unsubstituted alkenyl group, a substituted or unsubstituted alkynyl group, a substituted or unsubstituted aryl group, a substituted or unsubstituted aralkyl group,
a substituted or unsubstituted heteroaryl group, or a substituted or unsubstituted heteroarylalkyl group)]
or
a group represented by $-W^A-X^A-Y^A-Z^A$ {wherein

1) $W^A$ and $Y^A$ may be the same or different and each represents an oxygen atom, a sulfur atom, -SO-, $-SO_2-$, or $-N(R^{8A})-$ (wherein $R^{8A}$ has the same meaning as that defined above for $R^7$); $X^A$ represents a substituted or unsubstituted alicyclic alkylene group, a group formed by eliminating one hydrogen atom from a substituted or unsubstituted alicyclic heterocyclic group, a substituted or unsubstituted arylene group, a substituted or unsubstituted aralkylene group, a substituted or unsubstituted heteroarylene group, a substituted or unsubstituted heteroarylalkylene group, or $-(C(R^9)(R^{10}))q-$ [wherein q represents an integer of 1 to 6, $R^9$ and $R^{10}$ may be the same or different and each represents a hydrogen atom, a substituted or unsubstituted alkyl group, a substituted or unsubstituted alicyclic alkyl group, a substituted or unsubstituted alicyclic heterocyclic group, a substituted or unsubstituted alkenyl group, a substituted or unsubstituted alkynyl group, a substituted or unsubstituted aryl group, a substituted or unsubstituted aralkyl group, a substituted or unsubstituted heteroaryl group, or a substituted or unsubstituted heteroarylalkyl group, or $R^9$ and $R^{10}$, which attach to the same carbon atom, may combine together with said carbon atom to form a substituted or unsubstituted alicyclic alkyl group (provided that not all of the substituents $R^9$ and $R^{10}$ in the chain consisting of $(C(R^9)(R^{10}))q^-$ are hydrogen atoms)], or $X^A$ may combine together with one $-N(R^{8A})$-represented by $W^A$ or $Y^A$ to form a substituted or unsubstituted pyrrolidinediyl group, a substituted or unsubstituted piperidinediyl group, or a substituted or unsubstituted homopiperidinediyl group; $Z^A$ has the same meaning as that defined above for V (when $X^A$ combines with one $-N(R^{8A})-$ represented by $W^A$ or $Y^A$ to form a substituted or unsubstituted pyrrolidinediyl group or a substituted or unsubstituted piperidinediyl group, V is not a hydrogen atom or an aralkyl group ); or

2) $W^A$ and $Y^A$ may be the same or different and each represents an oxygen atom, a sulfur atom, -SO-, $-SO_2-$ or $-N(R^{8B})-$ (wherein $R^{8B}$ has the same meaning as that defined above for $R^7$); $X^A$ represents $-(CH_2)_r-$ (wherein r represents an integer of 1 to 6); $Z^A$ represents a substituted or unsubstituted alicyclic alkyl group, a substituted or unsubstituted alicyclic heterocyclic group, a substituted or unsubstituted alkenyl group, a substituted or unsubstituted alkynyl group, a substituted or unsubstituted aralkyl group, a substituted or unsubstituted heteroarylalkyl group, a pyridyl group substituted with $-SO_2-N(R^{15})(R^{16})$ [wherein $R^{15}$ and $R^{16}$ may be the same or different and each represents a hydrogen atom, a substituted or unsubstituted alkyl group, a substituted or unsubstituted aryl group, a substituted or unsubstituted aralkyl group, a substituted or unsubstituted heteroaryl group, or a substituted or unsubstituted heteroarylalkyl group, or $R^{15}$ and $R^{16}$, together with the adjacent nitrogen atom, may form a substituted or unsubstituted alicyclic heterocyclic group (said alicyclic heterocyclic group is selected from a pyrrolidinyl group, an oxazolidinyl group, a thiazolidinyl group, a piperidino group, a homopiperidino group, a piperazinyl group, a morpholino group, and a thiomorpholino group)], or a group selected from a trifluoromethylphenyl group, a methanesulfonylphenyl group, a nitrophenyl group, a cyanophenyl group, a naphthyl group, a pyrazinyl group, a pyridazinyl group, a triazinyl group, a thienyl group, a furyl group, a pyrrolyl group, an imidazolyl group, a pyrazolyl group, a triazolyl group, a tetrazolyl group, an oxazolyl group, a thiazolyl group, an isoxazolyl group, an isothiazolyl group, an oxadiazolyl group, a thiadiazolyl group, and a condensed heteroaryl group, each of which may be substituted or unsubstituted, or $G^A-R^{17}$ (wherein $G^A$ represents -CO-, -C(=O)O-, or $-SO_2-$, $R^{17}$ has the same meaning as that defined above for $R^7$)) (wherein when $W^A$ represents an oxygen atom, $Z^A$ may represent a pyridyl group substituted with a cyano group; when B represents -CO- or $-CH_2CO-$, $Z^A$ may represent a pyridyl group substituted with a nitro group or a cyano group)> or a pharmacologically acceptable salt thereof.

(II) The compound according to (I), wherein D represents $-W^A-X^A-Y^A-Z^A$, or a pharmacologically acceptable salt thereof.

(III) The compound according to (I) or (II), wherein B represents $-CO(C(R^3)(R^4))_{m-}$ (wherein $R^3$, $R^4$, and m have the same meanings as those defined above, respectively), or a pharmacologically acceptable salt thereof.

(IV) The compound according to (II), wherein A represents a substituted or unsubstituted 1-pyrrolidinyl group or a substituted or unsubstituted 3-thiazolidinyl group;

B represents $-CO(C(R^3)(R^4))_m-$ (wherein $R^3$ , $R^4$, and m have the same meanings as those defined above, respectively);

D represents $-W^c-X^c-Y^c-Z^c$ {wherein $W^c$ and $Y^c$ represent $-N(R^{8C})-$ (wherein $R^{8C}$ has the same meaning as that defined above for $R^7$); $X^C$ represents a substituted or unsubstituted alicyclic alkylene group, a group formed by eliminating one hydrogen atom from a substituted or unsubstituted alicyclic heterocyclic group, a substituted or unsubstituted arylene group, a substituted or unsubstituted aralkylene group, a substituted or unsubstituted heteroarylene group, a substituted or unsubstituted heteroarylalkylene group, or $-(C(R^9)(R^{10}))_{q-}$

[wherein q represents an integer of 1 to 6, $R^9$ and $R^{10}$ may be the same or different and each represents a hydrogen atom, a substituted or unsubstituted alkyl group, a substituted or unsubstituted alicyclic alkyl group, a substituted or unsubstituted alicyclic heterocyclic group, a substituted or unsubstituted alkenyl group, a substituted or unsubstituted alkynyl group, a substituted or unsubstituted aryl group, a substituted or unsubstituted aralkyl group, a substituted or unsubstituted heteroaryl group, or a substituted or unsubstituted heteroarylalkyl group, or $R^9$ and $R^{10}$, which attach to the same carbon atom, may combine together with said carbon atom to form a substituted or unsubstituted alicyclic alkyl group (provided that not all of the substituents $R^9$ and $R^{10}$ in the chain consisting of $-(C(R^9)(R^{10}))q^-$ are hydrogen atoms)], or $X^c$ combines with one $-N(R^{8c})$-represented by $W^c$ or $Y^c$ to form a substituted or unsubstituted pyrrolidinediyl group, a substituted or unsubstituted piperidinediyl group, or a substituted or unsubstituted homopiperidinediyl group; and $Z^c$ has the same meaning as that defined above for V}, or a pharmacologically acceptable salt thereof.

(V) The compound according to (II), wherein A represents a substituted or unsubstituted 1-pyrrolidinyl group or a substituted or unsubstituted 3-thiazolidinyl group;

B represents $-CO(C(R^3)(R^4))_m-$ (wherein $R^3$, $R^4$, and m have the same meanings as those defined above, respectively);

D represents $-W^D-X^D-Y^D-Z^D$ {wherein $X^D$ represents $-(CH_2)_r-$ (wherein r represents an integer of 1 to 6), $W^D$ and $Y^D$ represents $-N(R^{8D})-$ (wherein $R^{8D}$ has the same meaning as that defined above for $R^7$), $Z^D$ represents a substituted or unsubstituted alicyclic alkyl group, a substituted or unsubstituted alicyclic heterocyclic group, a substituted or unsubstituted alkenyl group, a substituted or unsubstituted alkynyl group, a substituted or unsubstituted aralkyl group, a substituted or unsubstituted heteroarylalkyl group, a pyridyl group substituted with $-SO_2-N(R^{15})(R^{16})$ [wherein $R^{15}$ and $R^{16}$ may be the same or different and each represents a hydrogen atom, a substituted or unsubstituted alkyl group, a substituted or unsubstituted aryl group, a substituted or unsubstituted aralkyl group, a substituted or unsubstituted heteroaryl group, or a substituted or unsubstituted heteroarylalkyl group, or $R^{15}$ and $R^{16}$ combine together with the adjacent nitrogen atom to form a substituted or unsubstituted alicyclic heterocyclic group (said alicyclic heterocyclic group is selected from a pyrrolidinyl group, an oxazolidinyl group, a thiazolidinyl group, a piperidino group, a homopiperidino group, a piperazinyl group, a morpholino group, and a thiomorpholino group)], or a group selected from a trifluoromethylphenyl group, a methanesulfonylphenyl group, a nitrophenyl group, a cyanophenyl group, a naphthyl group, a pyrazinyl group, a pyridazinyl group, a triazinyl group, a thienyl group, a furyl group, a pyrrolyl group, an imidazolyl group, a pyrazolyl group, a triazolyl group, a tetrazolyl group, an oxazolyl group, a thiazolyl group, an isoxazolyl group, an isothiazolyl group, an oxadiazolyl group, a thiadiazolyl group, and a condensed heteroaryl group, each of which may be substituted or unsubstituted, or $G^A-R^{17}$ (wherein $G^A$ represents -CO-, -C(=O)O-, or $-SO_2-$, and $R^{17}$ has the same meaning as that defined above for $R^7$), or a pharmacologically acceptable salt thereof.

(VI) The compound according to (IV) or (V), wherein $Z^C$ or $Z^D$ is a substituted or unsubstituted heteroaryl group, or a pharmacologically acceptable salt thereof.
(VII) The compound according to (VI), wherein the heteroaryl group is a 6-membered heteroaryl ring, or a 10-membered condensed heteroaryl ring wherein a benzene ring is fused, or a pharmacologically acceptable salt thereof.
(VIII) The compound according to (VI) or (VII), wherein the substituent of the substituted heteroaryl group is a cyano group, a halogen atom, an alkoxy group, a heteroaryl group, or $-SO_2-R^{18}$ (wherein $R^{18}$ represents an alkyl group; a trifluoromethyl group; an alicyclic alkyl group; an alicyclic heterocyclic group; an alkenyl group; an alkynyl group; an aryl group; an aralkyl group; a heteroaryl group; a heteroarylalkyl group; an alkoxy group; an alicyclic alkoxy group; an O-(alicyclic heterocycle)-substituted hydroxyl group; an alkenyloxy group; an alkynyloxy group; an aryloxy group; an aralkyloxy group; a heteroaryloxy group; a heteroarylalkoxy group; an amino group; an alkylamino group; a dialkylamino group; an alicyclic alkylamino group; an N-(alicyclic heterocycle)-substituted amino group; an alkenylamino group; an alkynylamino group; an arylamino group; an aralkylamino group; a heteroarylamino group; or a heteroarylalkylamino group), or a pharmacologically acceptable salt thereof.
(IX) The compound according to any one of (II) to (VIII), wherein $-W^A-X^A-Y^A-$, $-W^C-X^C-Y^C-$, or $-W^D-X^D-Y^D-$ has two nitrogen atoms, and said two nitrogen atoms are separated by 2 to 6 carbon atoms linked to each other, or a pharmacologically acceptable salt thereof.
(X) The compound according to (IX), wherein the 2 to 6 carbon atoms linked to each other has 1 to 3 alkyl groups as substituents, or a pharmacologically acceptable salt thereof.
(XI) The compound according to (IX), wherein the 2 to 6 carbon atoms linked to each other has an alicyclic alkyl group formed together with one of said carbon atoms as a substituent, or a pharmacologically acceptable salt thereof.

(XII) The compound according to (VIII), wherein the substituent of the substituted heteroaryl group is -$SO_2$-$R^{18}$ (wherein $R^{18}$ has the same meaning as that defined above), or a pharmacologically acceptable salt thereof.
(XIII) The compound according to (VI) or (VII), wherein $X^A$ combines with one - N($R^{8A}$)- represented by $W^A$ or $Y^A$ to form a substituted or unsubstituted piperidinediyl group, or a pharmacologically acceptable salt thereof.
(XIV) The compound according to any one of (I) to (XIII), wherein A is a 2-cyano-1-pyrrolidinyl group, or a pharmacologically acceptable salt thereof.
(XV) A medicament which comprises as an active ingredient the compound according to any one of (I) to (XIV) or a pharmacologically acceptable salt thereof.
(XVI) The medicament according to (XV) used for therapeutic treatment of a pathological condition in which dipeptidylpeptidase-IV is involved.
(XVII) The medicament according to (XV) used for preventive and/or therapeutic treatment of Type II diabetes.
(XVIII) The medicament according to (XV) used for preventive and/or therapeutic treatment of a complication accompanying Type II diabetes.
(XIX) A medicament for therapeutic treatment of Type II diabetes which comprises as an active ingredient the compound according to any one of (I) to (XIV) or a pharmacologically acceptable salt thereof.
(XX) A medicament for therapeutic treatment of a complication accompanying Type II diabetes which comprises as an active ingredient the compound according to any one of (I) to (XIV) or a pharmacologically acceptable salt thereof.
(XXI) A dipeptidylpeptidase-IV inhibitor which comprises as an active ingredient the compound according to any one of (I) to (XIV) or a pharmacologically acceptable salt thereof.
(XXII) A combination use for preventive and/or therapeutic treatment of Type II diabetes of the dipeptidylpeptidase-IV inhibitor according to any one of (I) to (XIV) and a medicament for therapeutic treatment of diabetes other than a the dipeptidylpeptidase-IV inhibitor.
(XXIII) A combination use for preventive and/or therapeutic treatment of Type II diabetes of the dipeptidylpeptidase-IV inhibitor according to any one of (I) to (XIV) and one to three medicaments for therapeutic treatment of diabetes selected from a biguanide agent, a sulfonylurea agent, an $\alpha$ -glucosidase inhibitor, a PPAR $\gamma$ agonist, a PPAR $\alpha / \gamma$ dual agonist, a SGLT2 inhibitor, an aP2 inhibitor, a glycogen phosphorylase inhibitor, an insulin sensitivity potentiator, a glucagon-like peptide-1 (GLP-1) or the analogues thereof, Insulin, and Meglinitide.
(XXIV) A combination use for preventive and/or therapeutic treatment of Type II diabetes of the dipeptidylpeptidase-IV inhibitor according to any one of (I) to (XIV) together with one to three medicaments for therapeutic treatment of diabetes selected from Metformin, Tolbutamide, Glibenclamide, Glyburide, Glimepiride, Glipiride, Glipizide, Chloropropamide, Gliclazid, Acarbose, Voglibose, Miglitol, Pioglitazone, Troglitazone, Rosiglitazone, Insulin, GI-262570, Isaglitazone, JTT-501, NN-2344, L895645, YM-440, R-119702, AJ9677, Repaglinide, Nateglinide, KAD1229, AR-HO39242, GW-409544, KRP297, AC2993, T-1095, Exendin-4, LY307161, NN2211, and LY315902.
(XXV) A method for therapeutic treatment of a disease selected from the group consisting of Type II diabetes, hyperlipemia, syndrome X, diabetes complications, hyperglycemia, hyperinsulinism, arteriosclerosis, impaired glucose tolerance, infertility; polycystic ovary syndrome, a growth defect, arthritis, rejection against allotransplantation, autoimmune disease, acquired immunodeficiency disease (AIDS), enterocolitis, anorexia, and osteoporosis, comprising administration of a therapeutically effective amount of the compound according to (I) to (XIV) to a human.

The present invention also provides a use of the aforementioned compound or a pharmacologically acceptable salt thereof for manufacturing of the aforementioned medicaments; A method for preventive and/or therapeutic treatment of Type II diabetes which comprises the step of administering a preventively and/or therapeutically effective amount of the aforementioned compound or a pharmacologically acceptable salt thereof to a mammal including human; a method for preventive and /or therapeutic treatment of a complication accompanying Type II diabetes; a method for preventive and /or therapeutic treatment of a pathological condition in which DPP-IV is involved, which comprises the step of administering a preventively and/or therapeutically effective amount of the aforementioned compound or a pharmacologically acceptable salt thereof to a mammal including a human.
In the definitions of each groups in the general formula (I), an alkyl group may be either linear alkyl group or branched alkyl group, and represents an alkyl group having 1 to 12 carbons, unless otherwise mentioned. The same may be applied to an alkyl moiety of other substitutes containing an alkyl moiety. More specifically, examples of the alkyl group include a methyl group, an ethyl group, a propyl group, an isopropyl group, a butyl group, an isobutyl group, a sec-butyl group, a tert-butyl group, a pentyl group, an isopentyl group, a neopentyl group, a hexyl group, a heptyl group, an octyl group, a nonyl group, a decyl group, an undecyl group, a dodecyl group, or the like.
The alicyclic alkyl group represents an alicyclic alkyl group having 3 to 12 carbons, unless otherwise mentioned. Examples of alicyclic alkyl group include monocyclic alkyl groups such as a cyclopropyl group, a cyclobutyl group, a cyclopentyl group, a cyclohexyl group, a cycloheptyl group, a cyclooctyl group, a cyclododecyl group, or the like, and polycyclic alkyl groups such as a pinanyl group, an adamantyl group, a bicyclo [3.3.1] octyl group, a bicyclo

[3.1.1] heptyl group, a bicyclo [2.1.1] hexyl group, or the like. The alicyclic alkyl group may be a group in which a cycloalkyl group and the above alkyl group are combined. Examples of such group include a cyclopropylmethyl group, a cyclobutylmethyl group, or the like.

The types and numbers of the heteroatoms contained in the alicyclic heterocyclic group are not particularly limited. For example, the alicyclic heterocyclic group may contain one or more hetero atoms selected from the group consisting of a nitrogen atom, an oxygen atom, and a sulfur atom. Examples of the alicyclic heterocyclic group include a tetrahydrofuryl group, a tetrahydropyranyl group, a pyrrolidinyl group, an oxazolidinyl group, a thiazolidinyl group, a piperidinyl group, a homopiperidinyl group, a piperazinyl group (the said piperazinyl group may be substituted with an alkyl group, or the like), a morpholinyl group, a thiomorpholinyl group, a 3-pyrrolinyl group, a tetrahydroquinolyl group, a tetrahydroisoquinolyl group, or the like.

The alkenyl group may be linear alkenyl group or branched alkenyl group, and represents an alkenyl group having 2 to 12 carbons, unless otherwise mentioned. Examples of the alkenyl group include a vinyl group, an allyl group, a 1-propenyl group, an isopropenyl group, a methacryl group, a butenyl group, a crotyl group, a pentenyl group, a hexenyl group, a heptenyl group, a decenyl group, a dodecenyl group, or the like.

The alkynyl group may be linear alkynyl group or branched alkynyl group, and represents an alkynyl group having 2 to 12 carbons, unless otherwise mentioned. Examples of the alkynyl group include an ethynyl group, a propargyl group, a butynyl group, a pentynyl group, a hexynyl group, a heptynyl group, a decynyl group, a dodecynyl group, or the like.

The aryl group may be a monocyclic aryl group or a condensed aryl group, and a 6- to 14-membered aryl group may be used. More specifically, examples of aryl group include a phenyl group, a naphthyl group, an anthryl group, a pyrenyl group, or the like. Aryl moieties of other substituents containing the aryl moieties have the similar meaning. Examples of the aralkyl group include a group in which the above alkyl group and the above aryl group are combined, and an aralkyl group having 7 to 15 carbons may be used. Examples of an aralkyl group include a benzyl group, a phenethyl group, a phenylpropyl group, a phenylbutyl group, a benzhydryl group, a trityl group, a naphthylmethyl group, a naphthylethyl group, a phenylcyclopropyl group, or the like.

The types and numbers of the heteroatoms contained in the heteroaryl group are not particularly limited. For example, the heterocyclic group may contain one or more hetero atoms selected from the group consisting of a nitrogen atom, an oxygen atom, and a sulfur atom, and may be either a monocyclic heteroaryl group or a condensed heteroaryl group. More specifically, examples of the monocyclic heteroaryl group include a pyridyl group, a pyrimidinyl group, a pyrazinyl group, a pyridazinyl group, a triazinyl group, a thienyl group, a furyl group, a pyrrolyl group, an imidazolyl group, a pyrazolyl group, a triazolyl group, a tetrazolyl group, an oxazolyl group, a thiazolyl group, a thiadiazolyl group, or the like, and examples of the condensed heteroaryl group include a quinolyl group, an isoquinolyl group, a quinazolinyl group, a phthalazinyl group, a quinoxalinyl group, a naphthylidinyl group, a cinnolinyl group, a pyridopyrimidinyl group, a pyrimidopyrimidinyl group, a pyridopyrazinyl group, a pyridopyridazinyl group, a pyrazinopyrimidinyl group, a pyridazinopyrimidinyl group, a pyrazinopyrazinyl group, a pyrazinopyridazino group, a pyridazinopyridazinyl group, a benzothienyl group, a benzofuryl group, an indolyl group, an indazolyl group, a benzimidazolyl group, a benzotriazolyl group, a benzoxazolyl group, a benzothiazolyl group, a purinyl group, an imidazopyridyl group, or the like. An example of the heteroarylalkyl group includes a group in which the above alkyl group and the above heteroaryl group are combined.

In the specification, the halogen atom means a fluorine atom, a chlorine atom, a bromine atom, or an iodine atom.

Examples of the condensed heteroaryl group include a quinolyl group, an isoquinolyl group, a quinazolinyl group, a phthalazinyl group, a quinoxalinyl group, a naphthylidinyl group, a cinnolinyl group, a pyridopyrimidinyl group, a pyrimidopyrimidinyl group, a pyridopyrazinyl group, a pyridopyridazinyl group, a pyrazinopyrimidinyl group, a pyridazinopyrimidinyl group, a pyrazinopyrazinyl group, a pyrazinopyridazino group, a pyridazinopyridazinyl group, a benzothienyl group, a benzofuryl group, an indolyl group, an indazolyl group, a benzimidazolyl group, a benzotriazolyl group, a benzoxazolyl group, a benzothiazolyl group, a purinyl group, an imidazopyridyl group, or the like.

The alkylene group may be either linear alkylene group or branched alkylene group, and may preferably be a linear alkylene group. More specifically, examples of alkylene group include a methylene group, an ethylene group, a propylene group, a butylene group, or the like.

The alicyclic alkylene group may be an alkylene group in which an alkyl group and a cycloalkyl group are combined. Examples of the alicyclic alkylene group include a cyclopentylene group, a cyclohexylene group, a cyclohexylmethyl group, or the like.

As the arylene group, an aryldiyl group comprising the aryl ring constituting the above aryl group may be used. More specifically, examples include a phenylene group, a naphthalenediyl group, a biphenyldiyl group, and a stilbenediyl group.

As the aralkylene group, an aralkylene group consisting of an alkyl-substituted aryl group, which is composed of the aryl ring constituting the above aryl group and the above alkyl group, may be used. More specifically, examples

of the aralkylene group include an α -toluenediyl group, an α, α '-xylenediyl group, or the like.

As the heteroarylene group, a heteroaryldiyl group consisting of a heteroaryl ring constituting the above heteroaryl group may be used. More specifically, examples of the heteroarylene group include a pyridinediyl group, a pyrimidinediyl group, a pyrazinediyl group, a pyridazinediyl group, a triazinediyl group, a quinolinediyl group, a isoquinolinediyl group, a quinazolinediyl group, a phthalazinediyl group, a quinoxalinediyl group, a naphthylidinediyl group, a cinnolinediyl group, or the like. As the heteroarylalkylene group, a heteroaryldiyl group consisting of an alkyl-substituted heteroaryl, which is composed of a heteroaryl ring constituting the above heteroaryl group and the above alkyl group, may be used. An examples of the heteroarylalkylene group includes an α , α '-lutidinediyl group, or the like. A pyridopyrimidinyl group, a pyrimidopyrimidinyl group, a triazolyl group, a tetrazolyl group, an oxadiazolyl group, a thiadiazolyl group, a benzothienyl group, a benzofuryl group, a benzimidazolyl group, a benzotriazolyl group, a pyridopyrazinyl group, a pyridopyridazinyl group, a pyrazinopyrimidinyl group, a pyridazinopyrimidinyl group, a pyrazinopyrazinyl group, a pyrazinpyridazino group, and a pyridazinopyridazinyl group may be any of possible regioisomer.

The types, numbers, and positions of substituents are not particularly limited for a substituted alkyl group, a substituted alicyclic alkyl group, a substituted alicyclic heterocyclic group, a substituted alkenyl group, a substituted alkynyl group, a substituted aryl group, a substituted aralkyl group, a substituted heteroaryl group, a substituted heteroarylalkyl group, a substituted 1-pyrrolidinyl group, a substituted pyrrolidinediyl group, a substituted 3-thiazolidinyl group, a substituted 1-oxo-3-thiazolidinyl group, a substituted 1,1-dioxo-3-thiazolidinyl group, a substituted 3-oxazolidinyl group, a substituted 2,5-dihydro-1-pyrrolyl group, a substituted 1-pyrrolyl group, a substituted piperidino group, a substituted piperidinediyl group, a substituted 1-indolinyl group, a substituted 1-indolyl group, a substituted 1-octahydroindolyl group, a substituted 1-tetrahydroquinolyl group, a substituted 1-decahydroquinolyl group, a substituted piperazinyl group, a substituted piperazinediyl group, a substituted homopiperazinyl group, a substituted alkylene group, a substituted alicyclic alkylene group, a substituted arylene group, a substituted aralkylene group, a substituted heteroarylene group, a substituted heteroarylalkylene group, a substituted pyrrolidinyl group, a substituted piperidyl group, a substituted homopiperidyl group, a substituted homopiperidinediyl group, a substituted phenyl group, a substituted naphthyl group, a substituted trifluoromethylphenyl group, a substituted methanesulfonylphenyl group, a substituted nitrophenyl group, a substituted cyanophenyl group, a substituted pyridyl group, a substituted pyrimidinyl group, a substituted pyrazinyl group, a substituted pyridazinyl group, a substituted triazinyl group, a substituted quinolyl group, a substituted isoquinolyl group, a substituted quinazolinyl group, a substituted phthalazinyl group, a substituted quinoxalinyl group, a substituted naphthylidinyl group, a substituted cinnolinyl group, a substituted pyridopyrimidinyl group, a substituted pyrimidopyrimidinyl group, a substituted pteridinyl group, a substituted thienyl group, a substituted furyl group, a substituted pyrrolyl group, a substituted imidazolyl group, a substituted pyrazolyl group, a substituted triazolyl group, a substituted tetrazolyl group, a substituted oxazolyl group, a substituted thiazolyl group, a substituted isoxazolyl group, substituted isothiazolyl group, a substituted oxadiazolyl group, a substituted thiadiazolyl group, a substituted benzothienyl group, a substituted benzofuryl group, a substituted indolyl group, a substituted indazolyl group, a substituted benzimidazolyl group, a substituted benzotriazolyl group, a substituted benzoxazolyl group, a substituted benzothiazolyl group, a substituted purinyl group, a substituted condensed heteroaryl group, and other substituted functional groups defined in this specification. When two or more substituents are present on one functional group, they may be the same or different.

Examples of the substituents include a nitro group; a cyano group; a hydroxy group; an oxo group; a halogen atom; an alicyclic alkyl group; an aryl group; an alicyclic heterocyclic group; a carboxyl group; a formyl group; a group represented by $R^{19}$-CO-J- (wherein, J represents a single bond or an oxygen atom, $R^{19}$ represents an alkyl group; an alicyclic alkyl group; an alicyclic heterocyclic group; an alkenyl group; an alkynyl group; an aryl group; an aralkyl group; a heteroaryl group; a heteroarylalkyl group; an alkoxy group; a trifluoromethyl group; a trifluoromethoxy group; an alicyclic alkoxy group; an O-(alicyclic heterocycle)-substituted hydroxyl group; an alkenyloxy group; an alkynyloxy group; an aryloxy group; an aralkyloxy group; a heteroaryloxy group; a heteroarylalkoxy group; an amino group; an alkylamino group; a dialkylamino group; an alicyclic alkylamino group; an N-(alicyclic heterocycle)-substituted amino group; an alkenylamino group; an alkynylamino group; an arylamino group; an aralkyl amino group; a heteroarylamino group; or a heteroarylalkylamino group); a group represented by $N(R^{20})(R^{21})$ (wherein, $R^{20}$ and $R^{21}$ may be the same or different and each represents a hydrogen atom; an alkyl group; an alicyclic alkyl group; an alicyclic heterocyclic group; an alkenyl group; an alkynyl group; an aryl group; an aralkyl group; a heteroaryl group; a heteroarylalkyl group; an alkanoyl group; an alicyclic alkanoyl group; an alicyclic heterocycliccarbonyl group; an alkenoyl group; an alkynoyl group; an aroyl group; an aralkylcarbonyl group; a heteroarylcarbonyl group; a heteroarylalkylcarbonyl group; an alkoxycarbonyl group; an alicyclic alkoxycarbonyl group; an O-(alicyclic heterocycle)-substituted hydroxycarbonyl group; an alkenyloxycarbonyl group; an alkynyloxycarbonyl group; an aryloxycarbonyl group; an aralkyloxycarbonyl group; a heteroaryloxycarbonyl group; a heteroarylalkoxycarbonyl group; an alkylsulfonyl group; an alicyclic alkylsulfonyl group; an alicyclic heterocyclic sulfonyl group; an alkenylsulfonyl group; an alkynylsulfonyl group; an arylsulfonyl group; an aralkylsulfonyl group; a heteroarylsulfonyl group; or a heteroarylalkylsulfonyl group); an ureido group; a thioureido group; an alkoxycarbonylamino group; an alicyclic alkoxycarbonylamino group; an O-(alicyclic heterocycle)-substituted

hydroxycarbonylamino group; an alkenyloxycarbonylamino group; an alkynyloxycarbonylamino group; an aryloxycarbonylamino group; an aralkyloxycarbonylamino group; a heteroaryloxycarbonylamino group; a heteroarylalkoxycarbonylamino group; an alkoxy group; an alicyclic alkoxy group; an O-(alicyclic heterocycle)-substituted hydroxyl group; an alkenyloxy group; an alkynyloxy group; an aryloxy group; an aralkyloxy group; a heteroaryloxy group; a heteroarylalkoxy group; a sulfo group; a trifluoromethylsulfinyl group; an alkylsulfinyl group; an alicyclic alkylsulfinyl group; an alicyclic heterocyclic sulfinyl group; an alkenylsulfinyl group; an alkynylsulfinyl group; an arylsulfinyl group; an aralkylsulfinyl group; an heteroarylsulfinyl group; an heteroarylalkylsulfinyl group; a group represented by $-SO_2R^{22}$ (wherein, $R^{22}$ represents an alkyl group; a trifluoromethyl group; an alicyclic alkyl group; an alicyclic heterocyclic group; an alkenyl group; an alkyny group; an aryl group; an aralkyl group; a heteroaryl group; a heteroarylalkyl group; an alkoxy group; an alicyclic alkoxy group; an O-(alicyclic heterocycle)-substituted hydroxyl group; an alkenyloxy group; an alkynyloxy group; an aryloxy group; an aralkyloxy group; a heteroaryloxy group; a heteroarylalkoxy group; an amino group; an alkylamino group; a dialkylamino group; an alicyclic alkylamino group; an N-alkyl-N-alicyclicalkylamino group; an N-(alicyclic heterocycle)-substituted amino group; an alkenylamino group; an N-alkyl-N-alkenylamino group; an alkynylamino group; an arylamino group; an N-alkyl-N-arylamino group; an aralkylamino group; an N-alkyl-N-aralkylamino group; an N-alkyl-N-alkoxyamino group; a heteroarylamino group; a heteroarylalkylamino group; a tetrahydroisoquinolyl group; a tetrahydroquinolyl group; an 1-indolinyl group; or 1-isoindolinyl group); an alkylsulfonyloxy group; an alicyclic alkylsulfonyloxy group; an alicyclic heterocyclicsulfonyloxy group; an alkenylsulfonyloxy group; an alkynylsulfonyloxy group; an arylsulfonyloxy group; an aralkylsulfonyloxy group; a heteroarylsulfonyloxy group; a heteroarylalkylsulfonyloxy group; a mercapto group; or $SR^{23}$ (wherein, $R^{23}$ represents a trifluoromethyl group; an alkyl group; an alicyclic alkyl group; an alicyclic heterocyclic group; an alkenyl group; an alkynyl group; an aryl group; an aralkyl group; a heteroaryl group, or a heteroaryalkyl group), or the like. Further examples of the substituents on the substituted aryl group and the substituted heteroaryl group, other than the above substituents, include an alkyl group, a trifluoromethyl group, an aryl group, a heteroaryl group, or the like. A further example of the substituents on the substituted piperidinediyl group, other than the above substituents, includes an alkyl group, or the like. One or more substituents may be present on the substituents exemplified above. Examples of such substituents include, but not limited thereto, a hydroxyalkyl group, a halogenated alkyl group, a cyanoalkyl group, an alkoxyalkyl group, a halogenated aryl group, an alkoxyaryl group, or the like. More specific examples include, but not limited thereto, a hydroxyalkyl group, a cyanoalkyl group, or an alkoxyalkyl group which substitutes for an alkyl group of the alkylamino group or dialkylamino group in the above exemplified substituents.

In the above definitions of the substituents, the alkyl group and alkyl moieties of the substituents having the alkyl moieties (for example, an alkoxy group; an alkylamino group; an alkanoyl group; an alkylsulfonyl group; an alkoxycarbonyl group; an alkoxycarbonylamino group; an alkylsulfinyl group; an alkylsulfonyloxy group or the like) have the same meaning as that defined above for alkyl group. The alicyclic alkyl group and an alicyclic alkyl moieties of the substituents having the alicyclic alkyl moieties (for example, an alicyclic alkoxy group; an alicyclic alkylamino group; an alicyclic alkanoyl group; an alicyclic alkylsulfonyl group; an alicyclic alkoxycarbonyl group; an alicyclic alkoxycarbonylamino group; an alicyclic alkylsulfinyl group; and an alicyclic alkylsulfonyloxy group, or the like) have the same meaning as that defined above for alicyclic alkyl group. The alicyclic heterocyclic group and an alicyclic heterocyclic moieties of the substituents having the alicyclic heterocyclic moieties (for example, an O-(alicyclic heterocycle)-substituted hydroxyl group; an N-(alicyclic heterocycle)-substituted amino group; an alicyclic heterocyclic carbonyl group; an alicyclic heterocyclic sulfonyl group; an O-(alicyclic heterocycle)-substituted hydroxycarbonyl group; an O-(alicyclic heterocycle)-substituted hydroxycarbonylamino group; an alicyclic heterocyclic sulfinyl group; and an alicyclic heterocyclic sulfonyloxy group) have the same meaning as that defined above for alicyclic heterocyclic group. The alkenyl group and alkenyl moieties of the substituents having the alkenyl moieties (for example, an alkenyloxy group; an alkenylamino group; an alkenoyl group; an alkenylsulfonyl group; alkenyloxycarbonyl group; an alkenyloxycarbonylamino group; an alkenylsulfinyl group; an alkenylsulfonyloxy group, or the like) have the same meaning as that defined above for alkenyl group. The alkynyl group and alkynyl moieties of the substituents having the alkynyl moieties (for example, an alkynyloxy group; an alkynylamino group; an alkynoyl group; an alkynylsulfonyl group; an alkynyloxycarbonyl group; an alkynyloxycarbonylamino group; an alkynylsulfinyl group; an alkynylsulfonyloxy group, and the like) have the same meaning as that defined above for alkynyl group. The aryl group and aryl moieties of the substituents having the aryl moieties (for example, an aryloxy group; an arylamino group; an aroyl group; an arylsulfonyl group; an aryloxycarbonyl group; an aryloxycarbonylamino group; an arylsulfinyl group; an arylsulfonyloxy group; an arylazo group, and so on) have the same meaning as that defined above for aryl group. The aralkyl group and aralkyl moieties of the substituents having the aralkyl moieties (for example, an aralkyloxy group; an aralkylamino group; an aralkylcarbonyl group; an aralkylsulfonyl group; an aralkyloxy carbonyl group; an aralkyloxycarbonylamino group; an aralkylsulfinyl group; an aralkylsulfonyloxy group, or the like) have the same meaning as that defined above for aralkyl group. The heteroaryl group and heteroaryl moieties of the substituents having the heteroaryl moieties (for example, a heteroaryloxy group; a heteroarylamino group; a heteroarylcarbonyl group; a heteroarylsulfonyl group; a heteroaryloxycarbonyl group; a heteroaryloxycarbonylamino group; a heteroarylsulfinyl group; a heteroarylsulfonyloxy group; a heteroarylazo group,

or the like) have the same meaning as that defined above for heteroaryl group. The heteroarylalkyl group and heteroarylalkyl moieties of the substituents having the heteroarylalkyl moieties (for example, a heteroarylalkoxy group; a heteroarylalkylamino group; a heteroarylalkylcarbonyl group; a heteroarylalkylsulfonyl group; a heteroarylalkoxycarbonyl group; a heteroarylalkoxycarbonylamino group; a heteroarylalkylsulfinyl group; a heteroarylalkylsulfonyloxy group, or the like) are the same as the aforementioned heteroarylalkyl group. A halogen atom means a fluorine atom, a chlorine atom, a bromine atom, an iodine atom.

The compound represented by the aforementioned general formula (I) may be present as a form of a salt, which falls within the scope of the present invention. As a salt, a pharmacologically acceptable salt is preferred. Examples of the pharmacologically acceptable salt include an acid addition salt, a metal salt, an ammonium salt, an organic amine addition salt, an amino acid addition salt, or the like.

More specifically, examples of the pharmacologically acceptable acid addition salts include an inorganic acid salt such as a hydrochloride, a sulfate, and a phosphate, or an organic acid salts such as a acetate, a maleate, a fumarate, a tartrate, a citrate, and a methanesulfonate. Examples of the pharmacologically acceptable metal salt include alkali metal salts such as a sodium salt and a potassium salt, alkaline earth metal salts such as a magnesium salt and calcium salt, an alminium salt, and a zinc salt. Examples of the pharmacologically acceptable ammonium salt include an ammonium and a tetramethylammonium. Examples of the pharmacologically acceptable organic amine addition salt include addition salts of morpholine, piperidine, or the like. Examples of the pharmacologically acceptable amino acid addition salt include addition salts of lysine, glycine, phenylalanine, or the like.

The compound represented by the aforementioned general formula (I) or a pharmacologically acceptable salt thereof may be present as a hydrate or a solvate. A solvent which forms a solvate is not particularly limited. Examples of the solvent include ethanol, acetone, or the like. The compound represented by the aforementioned general formula (I) may have one or more asymmetric carbons, and any opticalisomers or diastereoisomers in a pure form, any mixtures in any ratio of the isomers, racemates and the like fall within the scope of the present invention.

When the compound represented by the aforementioned general formula (I) has an olefinic double bond, the configuration may be either Z or E, and a mixture in any ratio of Z and E falls within the scope of the present invention. Further, some of the compound represented by the general formula (I) may present as tautomers, which are obvious to those skilled in the art. Any one of two or more tautomers or a mixture thereof in any ratio falls within the scope of the present invention.

Examples of the preferred compounds of the present invention are shown below. However, the scope of the present invention is not limited to the following compounds.

Table 1

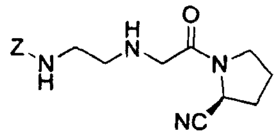

(I-1)

| Compound number | Z |
|---|---|
| 101 | 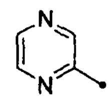 |
| 102 | 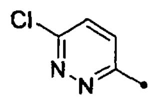 |
| 103 | 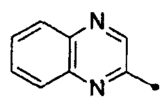 |
| 104 | 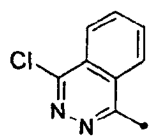 |
| 105 | 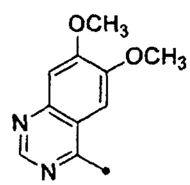 |
| 106 | 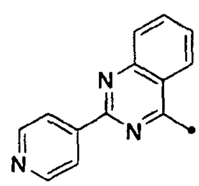 |
| 107 | 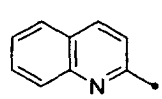 |

Table 1 continued

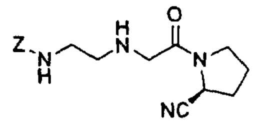

(I-1)

| Compound number | Z |
| --- | --- |
| 108 | 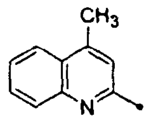 |
| 109 | 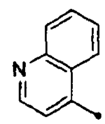 |
| 110 | 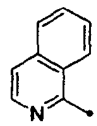 |
| 111 | 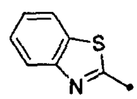 |
| 112 | 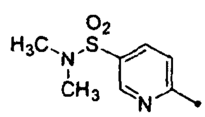 |

Table 1 continued

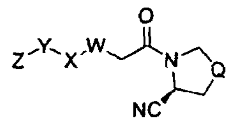

(I-2)

| Compound number | Z | —Y—X—W— | Q |
|---|---|---|---|
| 201 | 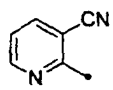 | 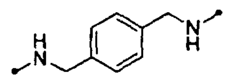 | $CH_2$ |
| 202 | 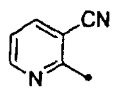 | 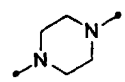 | $CH_2$ |
| 203 | 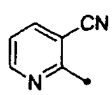 | 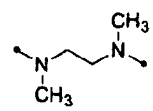 | $CH_2$ |
| 204 | 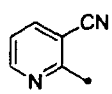 | 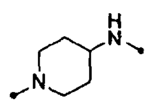 | S |
| 205 | 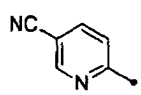 | 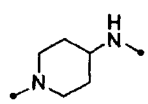 | $CH_2$ |
| 206 | 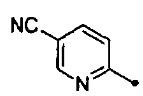 | 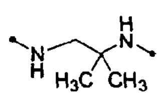 | $CH_2$ |
| 207 | 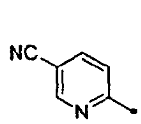 | 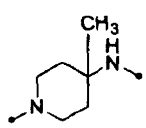 | $CH_2$ |
| 208 | 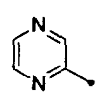 | 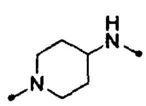 | $CH_2$ |

Table 1 continued

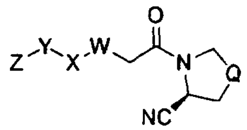

(I-2)

| Compound number | Z | —Y—X—W— | Q |
|---|---|---|---|
| 209 | 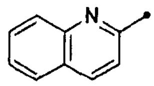 | 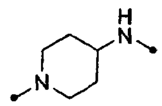 | $CH_2$ |
| 210 | 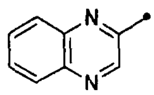 | 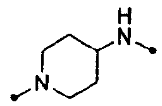 | $CH_2$ |
| 211 | 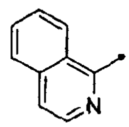 | 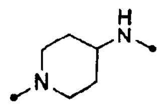 | $CH_2$ |
| 212 | 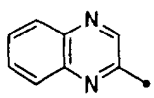 | 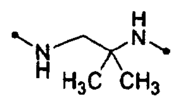 | $CH_2$ |
| 213 | 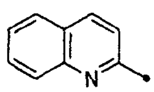 | 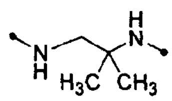 | $CH_2$ |
| 214 | 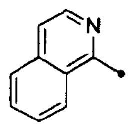 | 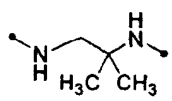 | $CH_2$ |
| 215 | 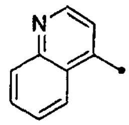 | 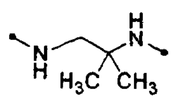 | $CH_2$ |
| 216 | 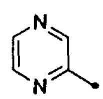 | 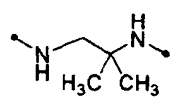 | $CH_2$ |
| 217 | 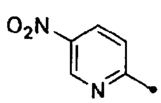 | 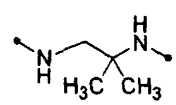 | $CH_2$ |
| 218 | 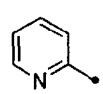 | 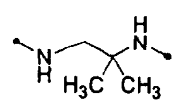 | $CH_2$ |

Table 1 continued

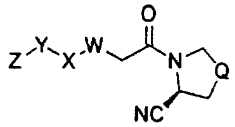

(I-2)

| Compound number | Z | —Y—X—W— | Q |
|---|---|---|---|
| 219 | 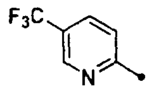 | 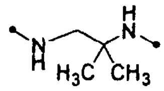 | $CH_2$ |
| 220 | 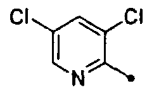 | 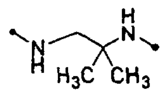 | $CH_2$ |
| 221 | 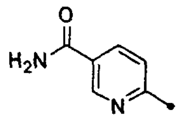 | 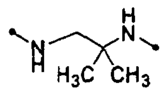 | $CH_2$ |
| 222 | 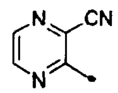 | 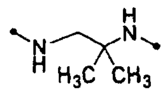 | $CH_2$ |
| 223 | 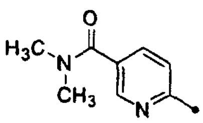 | 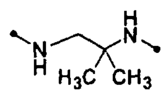 | $CH_2$ |
| 224 | 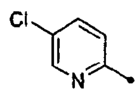 | 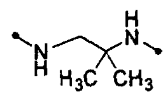 | $CH_2$ |
| 225 | 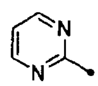 | 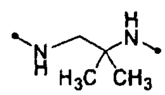 | $CH_2$ |
| 226 | 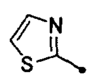 | 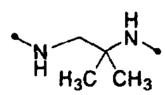 | $CH_2$ |
| 227 | 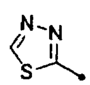 | 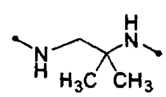 | $CH_2$ |
| 228 | 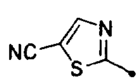 | 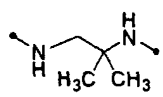 | $CH_2$ |
| 229 | 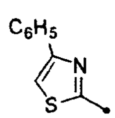 | 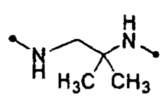 | $CH_2$ |
| 230 | 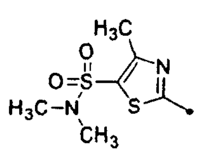 | 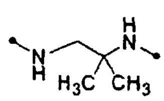 | $CH_2$ |

Table 1 continued

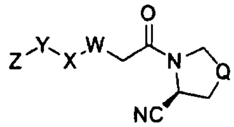

(I-2)

| Compound number | Z | —Y—X—W— | Q |
|---|---|---|---|
| 231 | 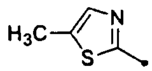 | 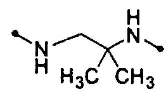 | $CH_2$ |
| 232 | 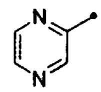 | 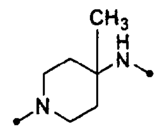 | $CH_2$ |
| 233 | 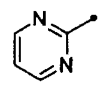 | 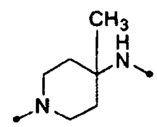 | $CH_2$ |
| 234 | 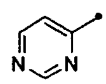 | 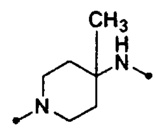 | $CH_2$ |
| 235 | 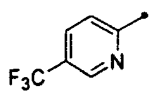 | 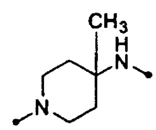 | $CH_2$ |
| 236 | 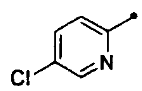 | 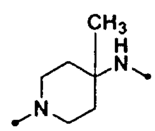 | $CH_2$ |
| 237 | 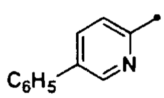 | 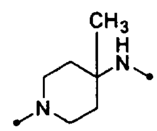 | $CH_2$ |
| 238 | 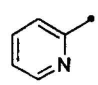 | 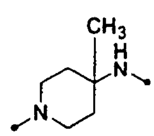 | $CH_2$ |
| 239 | 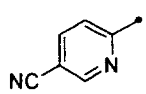 | 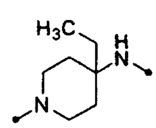 | $CH_2$ |
| 240 | 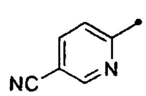 | 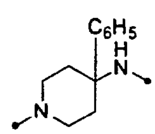 | $CH_2$ |

Table 1 continued

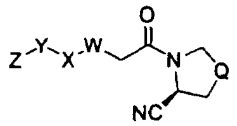

(I-2)

| Compound number | Z | —Y—X—W— | Q |
|---|---|---|---|
| 241 | 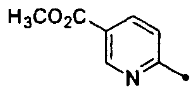 | 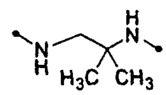 | $CH_2$ |
| 242 | 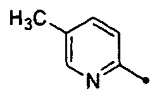 | 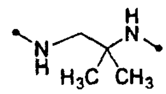 | $CH_2$ |
| 243 | 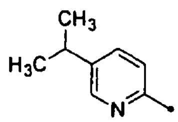 | 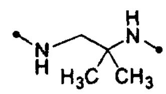 | $CH_2$ |
| 244 | 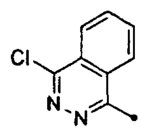 | 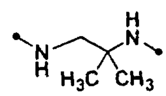 | $CH_2$ |
| 245 | 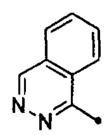 | 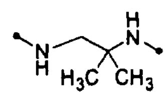 | $CH_2$ |
| 246 | 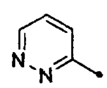 | 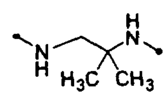 | $CH_2$ |
| 247 | 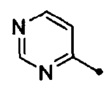 | 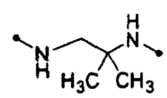 | $CH_2$ |
| 248 | 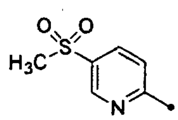 | 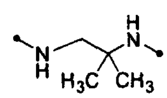 | $CH_2$ |
| 249 | 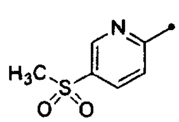 | 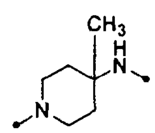 | $CH_2$ |
| 250 | 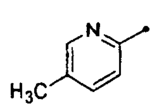 | 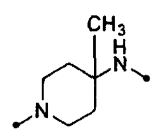 | $CH_2$ |

Table 1 continued

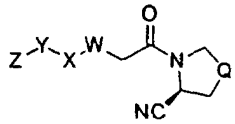

(I-2)

| Compound number | Z | —Y—X—W— | Q |
|---|---|---|---|
| 251 | 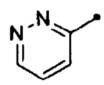 | 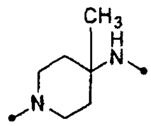 | $CH_2$ |
| 252 | 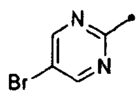 | 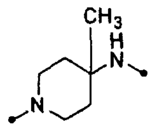 | $CH_2$ |

Table 1 continued

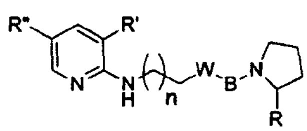

(I-3)

| Compound number | B | W | n | R | R' | R" |
|---|---|---|---|---|---|---|
| 301 | 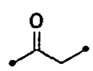 | NH | 1 | (*S*)-CN | H | $NO_2$ |
| 302 | 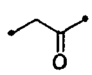 | O | 1 | (*S*)-CN | CN | H |
| 303 | 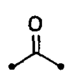 | NH | 1 | (*S*)-CN | H | $NO_2$ |
| 304 | 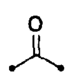 | NH | 1 | (*S*)-CN | CN | H |
| 305 | 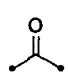 | NH | 1 | H | CN | H |
| 306 | 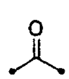 | NH | 2 | (*S*)-CN | CN | H |

Table 1 continued

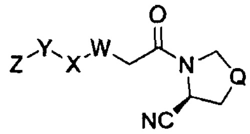

(I-4)

| Compound number | Z | —Y—X—W— | Q |
|---|---|---|---|
| 401 | 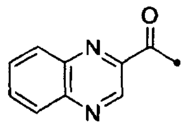 | 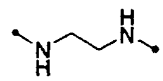 | $CH_2$ |
| 402 | 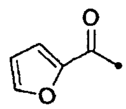 | 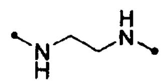 | $CH_2$ |
| 403 | 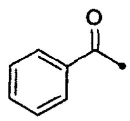 | 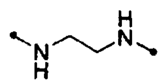 | $CH_2$ |
| 404 | 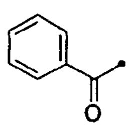 | 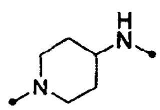 | $CH_2$ |
| 405 | 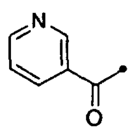 | 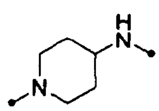 | $CH_2$ |
| 406 | 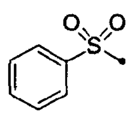 | 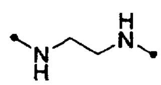 | $CH_2$ |
| 407 | 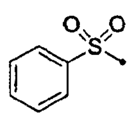 | 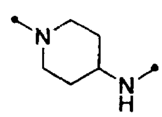 | $CH_2$ |

Table 1 continued

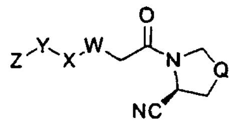

(I-5)

| Compound number | Z | —Y—X—W— | Q |
|---|---|---|---|
| 501 | 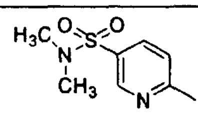 | 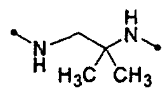 | $CH_2$ |
| 502 | 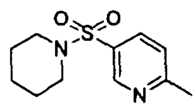 | 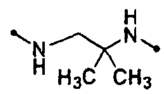 | $CH_2$ |
| 503 | 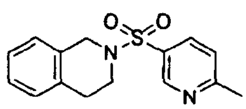 | 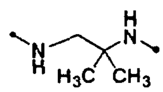 | $CH_2$ |
| 504 | 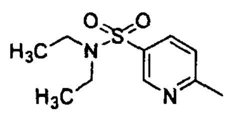 | 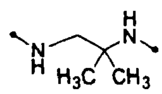 | $CH_2$ |
| 505 | 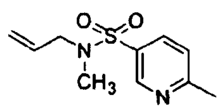 | 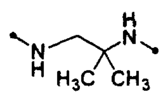 | $CH_2$ |
| 506 | 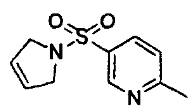 | 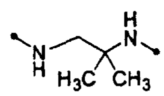 | $CH_2$ |
| 507 | 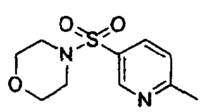 | 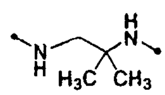 | $CH_2$ |
| 508 | 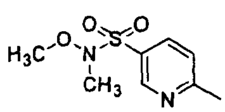 | 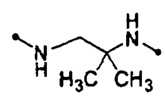 | $CH_2$ |
| 509 | 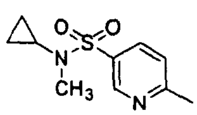 | 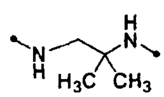 | $CH_2$ |
| 510 | 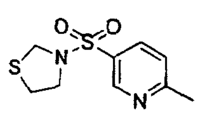 | 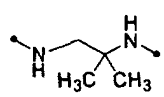 | $CH_2$ |

Table 1 continued

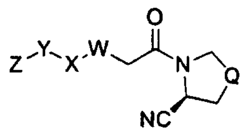

(I-5)

| Compound number | Z | —Y—X—W— | Q |
|---|---|---|---|
| 511 | 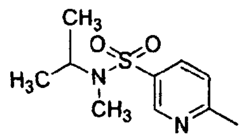 | 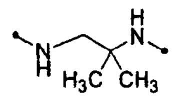 | $CH_2$ |
| 512 | 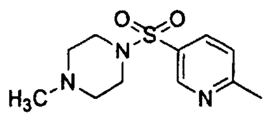 | 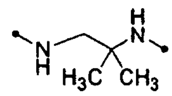 | $CH_2$ |
| 513 | 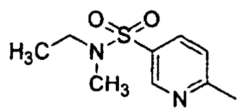 | 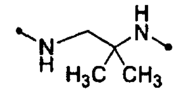 | $CH_2$ |
| 514 | 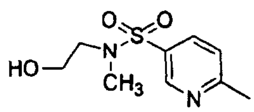 | 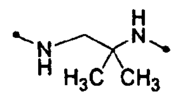 | $CH_2$ |
| 515 | 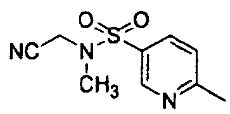 | 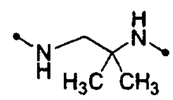 | $CH_2$ |
| 516 | 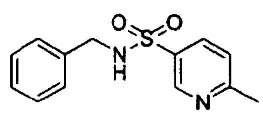 | 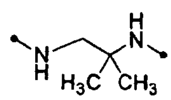 | $CH_2$ |
| 517 | 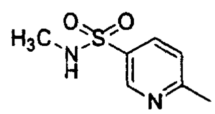 | 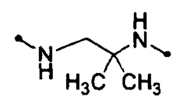 | $CH_2$ |
| 518 | 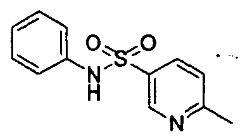 | 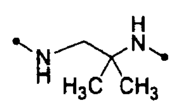 | $CH_2$ |
| 519 | 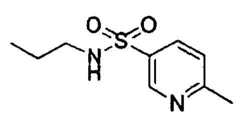 | 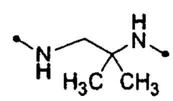 | $CH_2$ |
| 520 | 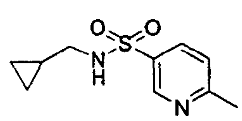 | 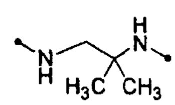 | $CH_2$ |

Table 1 continued

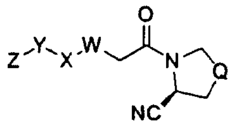

(I-5)

| Compound number | Z | —Y—X—W— | Q |
|---|---|---|---|
| 521 | 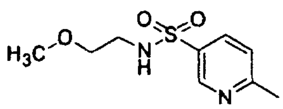 | 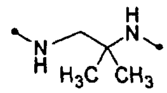 | $CH_2$ |
| 522 | 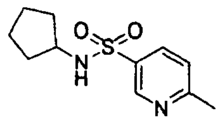 | 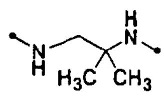 | $CH_2$ |
| 523 | 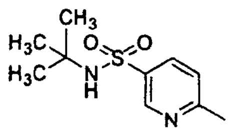 | 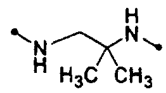 | $CH_2$ |
| 524 | 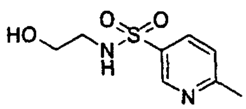 | 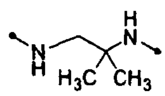 | $CH_2$ |
| 525 | 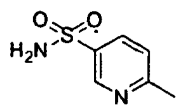 | 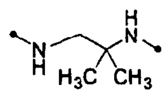 | $CH_2$ |
| 526 | 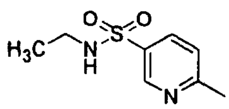 | 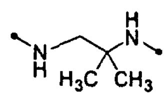 | $CH_2$ |
| 527 | 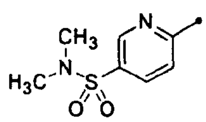 | 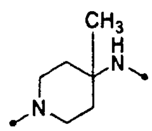 | $CH_2$ |
| 528 | 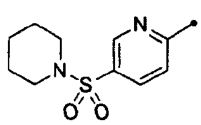 | 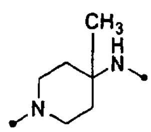 | $CH_2$ |
| 529 | 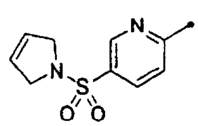 | 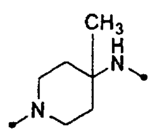 | $CH_2$ |

Table 1 continued

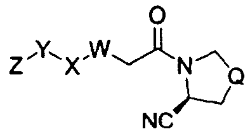

(I-5)

| Compound number | Z | —Y—X—W— | Q |
|---|---|---|---|
| 530 | 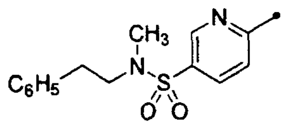 | 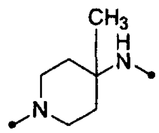 | $CH_2$ |
| 531 | 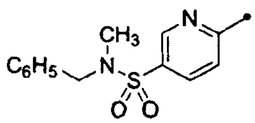 | 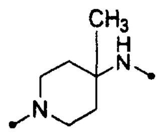 | $CH_2$ |
| 532 | 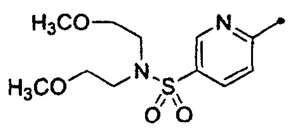 | 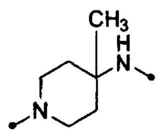 | $CH_2$ |
| 533 | 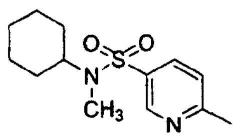 | 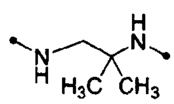 | $CH_2$ |
| 534 | 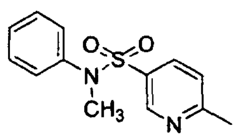 | 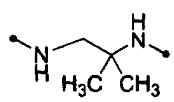 | $CH_2$ |
| 535 | 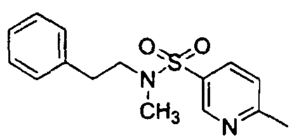 | 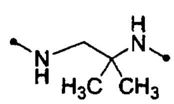 | $CH_2$ |
| 536 | 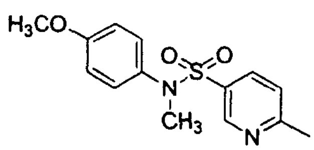 | 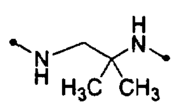 | $CH_2$ |
| 537 | 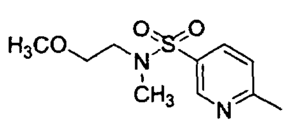 | 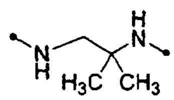 | $CH_2$ |

Table 1 continued

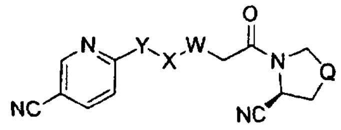

(I-6)

| Compound number | —Y—X—W— | Q |
|---|---|---|
| 601 | 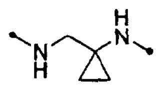 | $CH_2$ |
| 602 | 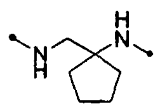 | $CH_2$ |
| 603 | 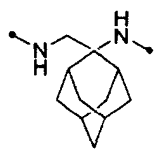 | $CH_2$ |
| 604 | 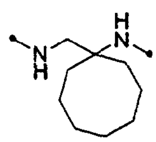 | $CH_2$ |
| 605 | 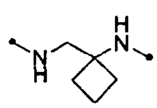 | $CH_2$ |
| 606 | 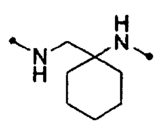 | $CH_2$ |

Table 1 continued

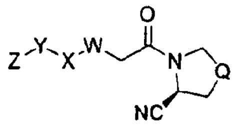

(I-7)

| Compound number | Z | —Y—X—W— | Q |
|---|---|---|---|
| 701 | 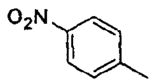 | 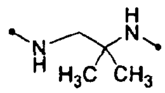 | $CH_2$ |
| 702 | 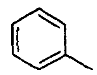 | 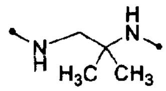 | $CH_2$ |
| 703 | 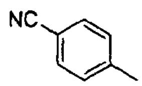 | 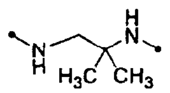 | $CH_2$ |
| 704 | 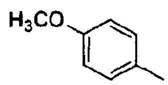 | 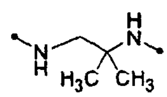 | $CH_2$ |
| 705 | 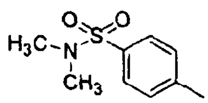 | 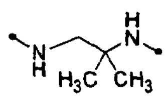 | $CH_2$ |
| 706 | 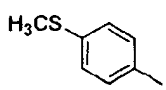 | 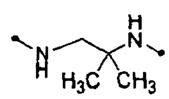 | $CH_2$ |
| 707 | 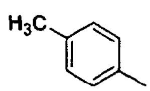 | 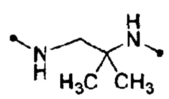 | $CH_2$ |
| 708 | 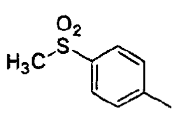 | 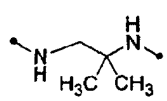 | $CH_2$ |
| 709 | 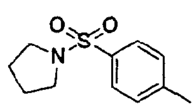 | 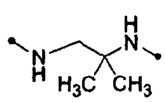 | $CH_2$ |
| 710 | 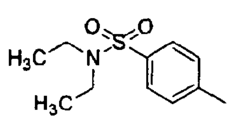 | 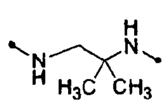 | $CH_2$ |

Table 1 continued

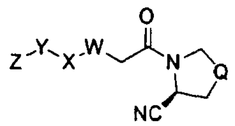

(1-7)

| Compound number | Z | —Y—X—W— | Q |
| --- | --- | --- | --- |
| 711 | 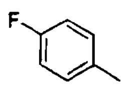 | 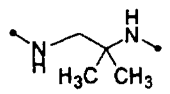 | $CH_2$ |
| 712 | 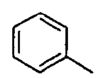 | 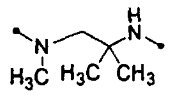 | $CH_2$ |

In the general formula (1-1) in the above table, Z is preferably a substituted or unsubstituted heteroaryl group, and is more preferably a substituted or unsubstituted nitrogen-containing heteroaryl group. A heteroaryl ring constituting said heteroaryl group is preferred to contain one or two nitrogen atoms as ring constituting atoms, and a 6-membered heteroaryl ring or a 10-membered heteroaryl ring condensed with a benzene ring is preferred. When the heteroaryl ring has substituent(s), the substituent is preferably a cyano group, a halogen atom, an alkoxy group, or a heteroaryl group. A most preferred Z is a 6-membered heteroaryl ring or a 10-membered condensed heteroaryl ring condensed with a benzene ring (wherein, the condensed heteroaryl ring binds at the ring containing nitrogen atoms, and the heteroaryl ring or the condensed heteroaryl ring may have one or more substituents selected from the group consisting of a cyano group, a halogen atom, an alkoxy group, and a heteroaryl group).

In the general formula (1-2) in the above table, Z is preferably a substituted or unsubstituted heteroaryl group, and is more preferably a substituted or unsubstituted nitrogen-containing heteroaryl group. A heteroaryl ring constituting said heteroaryl group is preferred to contain one or two nitrogen atom as ring constituting atoms, and a 6-membered heteroaryl ring or a 10-membered condensed heteroaryl ring condensed with a benzene ring is preferred. When the heteroaryl ring has substituent(s), the substituent is preferably a cyano group, a halogen atom, an alkoxy group, or a heteroaryl group. A most preferred Z is a 6-membered heteroaryl ring or a 10-membered condensed heteroaryl ring condensed with a benzene ring (wherein, the condensed heteroaryl ring binds at the ring containing nitrogen atoms, and the heteroaryl ring or the condensed heteroaryl ring may have one or more substituents selected from the group consisting of a cyano group, a halogen atom, an alkoxy group, or a heteroaryl group). In the general formula (1-2), -W-X-Y- contains two nitrogen atoms and functions as a linking group binding at said nitrogen atoms, and the two nitrogen atoms are preferred to be separated by 2 to 6 carbon atoms linked to each other (when a ring structure is present, a number of carbon atoms constituting the shortest carbon chain, among two or more partial structure of the carbon chains from one nitrogen atom toward the other nitrogen atom, is preferably 2 to 6 ). When -W-X-Y- contains two nitrogen atoms, one to three alkyl groups preferably stand on the linkage of 2 to 6 carbon atoms between the two nitrogen atoms, and the compound in which two alkyl groups bind to the same carbon atom is more preferred. When a ring structure is present, one to three alkyl groups preferably stand on the ring. A compound is also preferred in which the substituents on the carbon atom form an alicyclic alkyl group together with said carbon atom.

In the general formula (1-5) in the above table, Z is preferably a substituted or unsubstituted heteroaryl group, and is more preferably a substituted or unsubstituted nitrogen-containing heteroaryl group. A heteroaryl ring constituting said heteroaryl group is preferred to contain one or two nitrogen atoms as ring constituting atoms, and a 6-membered heteroaryl ring or a 10-membered condensed heteroaryl ring condensed with a benzene ring is preferred. The 6-membered heteroaryl ring is more preferred. When the heteroaryl ring has any substituents, the substituent is preferably $-SO_2-R^{22}$, and $R^{22}$ is preferably a dialkylamino group, an N-(alicyclic heterocycle)-substituted amino group, tetrahydroisoquinolyl group, or benzyl group. A most preferred Z is a 6-membered heteroaryl ring having $-SO_2-R^{22}$ (wherein, $R^{22}$ is a dialkylamino group or an N-(alicyclic heterocycle)-substituted amino group) as the substituent. In the general

formula (I-5), -W-X-Y- contains two nitrogen atoms and functions as a linking group binding at said nitrogen atoms, and the two nitrogen atoms are preferred to be separated by 2 to 6 carbon atoms linked to each other (when a ring structure is present, a number of carbon atoms constituting the shortest carbon chain, among two or more partial structure of the carbon chains from one nitrogen atom toward the other nitrogen atom, is preferably 2 to 6 ). When -W-X-Y- contains two nitrogen atoms, one to three alkyl groups preferably stand on the linkage of 2 to 6 carbon atoms between the two nitrogen atoms, and the compound in which two alkyl groups bind to the same carbon atom is more preferred. When a ring structure is present, one to three alkyl groups preferably stand on the ring. A compound is also preferred in which the substituents on the carbon atom form an alicyclic alkyl group together with said carbon atom.

Methods for preparation of the compound represented by the general formula (I) are not particularly limited. Generally, the compounds can be prepared by the following methods (The compound represented by the general formula (I) will be referred to as "Compound (I)" hereinafter in the explanations of the methods for preparation. Compounds of other formula numbers will be similarly referred to.).

<Preparation Method 1>

Compound (I) can be prepared according to the following reaction process.

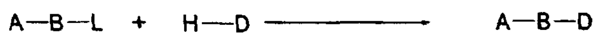

(II) (III) (I)

(wherein L represents a leaving group, each of A, B, and D has the same meaning as that defined above, respectively)

Examples of the leaving groups represented by L include a halogen atom, a substituted or unsubstituted alkoxy group, a substituted or unsubstituted aryloxy group, a substituted or unsubstituted alkylthio group, a substituted or unsubstituted alkylsulfinyl group, a substituted or unsubstituted alkylsulfonyl group, a substituted or unsubstituted alkylsulfonyloxy group, a substituted or unsubstituted arylsulfonyloxy group (each of a halogen atom, an alkoxy group, an aryloxy group, an alkylthio group, an alkylsulfinyl group, an alkylsulfonyl group, an alkylsulfonyloxy group, and a arylsulfonyloxy group has the same meaning as that defined above, and examples of the substituents include a halogen atom, an alkyl group, a nitro group, or the like), or the like.

Compound (I) can be obtained by the reaction of Compound (II) with Compound (III), in the presence of a base if necessary, in a suitable inert solvent, for example, a halogenated hydrocarbon such as chloroform and dichloromethane, an aromatic hydrocarbon such as benzene and toluene, an ether-type solvent such as diethylether, tetrahydrofuran (THF) and 1,4-dioxane, a lower alcohol such as methanol, ethanol, and 2-propanol, an aprotic polar solvent such as N,N-dimethylformamide, N-methylpyrrolidone, and dimethyl sulfoxide, or a mixture thereof, at a temperature between -78°C and the boiling point of a used solvent , for 5 minutes to 48 hours.

Examples of the base include organic bases such as triethylamine and pyridine, inorganic bases such as potassium carbonate, potassium hydrogencarbonate, sodium hydroxide, sodium hydride, cesium hydroxide, a metal alkoxides such as sodium methoxide, potassium tert-butoxide, and potassium fluoride.

Compound (II) can be obtained by a method described in the literature such as WO 98/19998 or in a similar manner thereto.

Compound (III) can be obtained by a method described in the literature such as WO 98/19998, WO 98/14431, and WO 99/51582, a method described in the reference examples, or in a similar manner thereto.

<Preparation method 2>

The compound (I-a) in which D is -$W^A$-$X^A$-$Y^A$-$Z^A$ can be prepared according to the following reaction process.

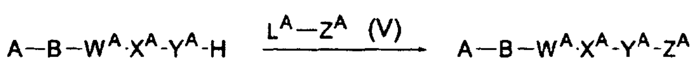


(IV) (I-a)

(wherein $L^A$ has the same meaning as that of the aforementioned L, and each of A, B, $W^A$, $X^A$, $Y^A$, and $Z^A$ has the same meaning as that defined above, respectively)

Compound (I-a) can be obtained by the reaction of Compound (IV) with Compound (V) , in the presence of a base if necessary, in a suitable inert solvent, for example, a halogenated hydrocarbon such as chloroform and dichloromethane, an aromatic hydrocarbon such as benzene and toluene, an ether-type solvent such as diethylether, THF, and 1,4-dioxane, a lower alcohol such as methanol, ethanol, and 2-propanol, an aprotic polar solvent such as N,N-dimethylformamide, N-methylpyrrolidone, and dimethyl sulfoxide, or a mixture thereof, at a temperature between -78°C and the boiling point of a used solvent, for 5 minutes to 48 hours.

Examples of the base include organic bases such as triethylamine and pyridine, inorganic bases such as potassium carbonate, potassium hydrogencarbonate, sodium hydroxide, and sodium hydride, and metal alkoxides such as sodium methoxide and potassium tert-butoxide.

Compound (IV) can be obtained by a method described in the literature such as WO 98/19988 or in a similar manner thereto.

Compound (V) is a commercially available. Compound(V) can also be obtained by methods described in the literature such as J. Chem. Soc., 890-899 (1947); J. Chem. Soc., 561-572 (1962); J. Chem. Soc., B, 449-454 (1967); J. Indian Chem. Soc., 36, 787-791 (1959); J. Org. Chem., 17, 1571-1575 (1952); J. Med. Chem., 14, 1060-1066 (1971); French Patent 1,388,756 (1965); J. Am. Chem. Soc., 68, 1204-1208 (1946); Japanese Patent Unexamined Publication (KOKAI) No. 60-120872; J. Med. Chem., 39, 918-928 (1996); South African Patent 67/06512 (1968) or in a similar manner thereto.

<Preparation method 3>

The compound (I-b) in which D is -U-V can be prepared according to the following reaction process.

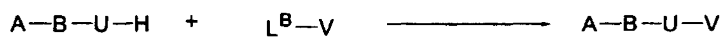

(VI) (VII) (I-b)

(wherein $L^B$ has the same meaning as that of the aforementioned L, and each of A, B, U, and V has the same meaning as that defined above, respectively)

Compound (I-b) can be obtained by the reaction of Compound (VI) with Compound (VII) , in the presence of a base if necessary, in a suitable inert solvent, for example, a halogenated hydrocarbon such as chloroform and dichloromethane, an aromatic hydrocarbon such as benzene and toluene, an ether-type solvent such as diethylether, THF, and 1,4-dioxane, a lower alcohol such as methanol, ethanol, and 2-propanol, an aprotic polar solvent such as N,N-dimethylformamide, N-methylpyrrolidone, and dimethyl sulfoxide, or a mixture thereof, at a temperature between -78°C and the boiling point of a used solvent, for 5 minutes to 48 hours.

Examples of the base include organic bases such as triethylamine and pyridine, inorganic bases such as potassium carbonate, potassium hydrogencarbonate, sodium hydroxide, and sodium hydride, and a metal alkoxides such as sodium methoxide and potassium tert-butoxide.

Compound (VI) can be obtained by a method described in the literature such as WO 98/19988 or in a similar manner thereto.

Compound (VII) as the starting material is a commercially available. Compound (VII) can also be obtained by a method described in the literature such as J. Chem. Soc., 890-899 (1947); J. Chem. Soc., 561-572 (1962); J. Chem. Soc., B, 449-454 (1967); J. Indian Chem. Soc., 36, 787-791 (1959); J. Org. Chem., 17, 1571-1575 (1952); J. Med. Chem., 14, 1060-1066 (1971); French Patent No. 1,388,756 (1965); J. Am. Chem. Soc., 68, 1204-1208 (1946); Japanese Patent Unexamined Publication (KOKAI) No. 60-120872; J. Med. Chem., 39, 918-928 (1996); South African Patent No. 67/06512 (1968) or in a similar manner thereto.

<Preparation method 4>

The compound (I-c) in which B is $-CO(C(R^3)(R^4))_{m-1}CH(R^{3A})-$ and D is $-N(R^{8A})-X^A-Y^A-Z^A$ can be prepared according to the following reaction process.

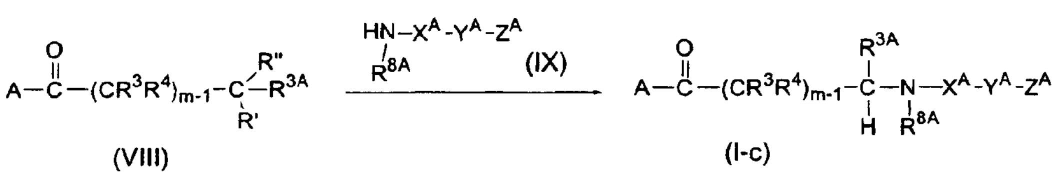

(IX)

(VIII) (I-c)

(wherein R' and R" may be the same or different and each represents a substituted or unsubstituted alkoxyl group or a substituted or unsubstituted alkylthio group, or R' and R" combine together to form an oxo group; $R^{3A}$ has the same meaning as that of the aforementioned $R^3$; and each of A, B, $X^A$, $Y^A$, $Z^A$, $R^3$, $R^4$, $R^{8A}$, and m has the same meaning as that defined above, respectively)

Compound (I-c) can be obtained by the reaction of Compound (VIII) with Compound (IX) , under an acidic condition if necessary, in a suitable inert solvent, for example, a halogenated hydrocarbon such as chloroform and dichloromethane, an aromatic hydrocarbon such as benzene and toluene, an ether-type solvent such as diethylether, THF, and 1,4-dioxane, a lower alcohol such as methanol, ethanol, and 2-propanol, an aprotic polar solvent such as N, N-dimethylformamide, N-methylpyrrolidone, and dimethyl sulfoxide, or a mixture thereof, at a temperature between -78°C and the boiling point of a used solvent, for 5 minutes to 48 hours, and by conducting a successive reduction under a suitable condition.

Examples of the reduction method include a method using a reducing agent such as sodium borohydride ($NaBH_4$), sodium cyano borohydride ($Na(CN)BH_3$), sodium triacetoxy borohydride ($NaH(OCOCH_3)_3$), and aluminium lithium hydride ($LiAlH_4$), and a method by a hydrogenation in the presence of a catalyst such as palladium on carbon and platinum. Compound (I-c) can also be obtained by the reaction of Compound (VIII) with Compound (IX) under a suitable reductive condition.

Compound (VIII) as the starting material can be obtained by a method described in the literature such as U. S. Patent No. 4,794,185, and Eur. J. Org. Chem, 1, 329-333(1999), or in a similar manner thereto.

Compound (IX) can be obtained by a method described in the literature such as WO 98/19988, a method described in the reference examples, or in a similar manner thereto.

<Preparation method 5>

Compound (I-d) in which D is -NH-$X^A$-$Y^A$-$Z^A$ can be prepared according to the following reaction process.

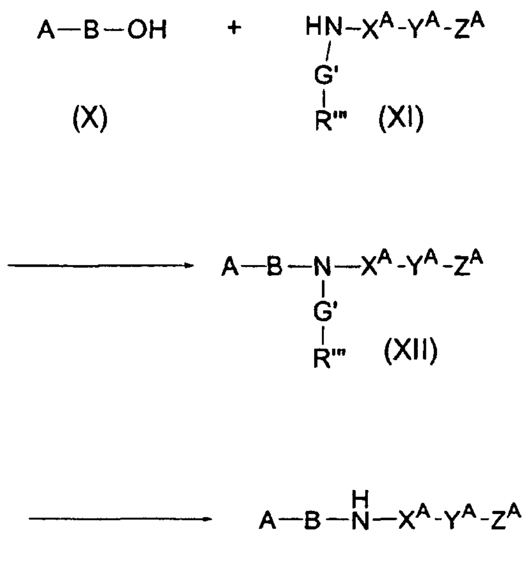

(X) (XI) (XII)

(I-d)

(wherein R"' has the same meaning as that of the aforementioned $R^5$; G' has the same meaning as that of the aforementioned $G^A$; and each of A, B, $X^A$, $Y^A$, and $Z^A$ has the same meaning as that defined above, respectively)

Compound (I-d) can be obtained by the reaction of Compound (X) with Compound (XI) in a suitable inert solvent, for example, a halogenated hydrocarbon such as chloroform and dichloromethane, an aromatic hydrocarbon such as benzene and toluene, an ether-type solvent such as diethylether, THF, and 1,4-dioxane, a lower alcohol such as methanol, ethanol, and 2-propanol, an aprotic polar solvent such as N,N-dimethylformamide, N-methylpyrrolidone, and dimethyl sulfoxide, or a mixture thereof, under a condition of the Mitsunobu reaction at a temperature between -78°C and the boiling point of a solvent used for 5 minutes to 48 hours to give Compound (XII), and by conducting a successive deprotection of this compound.

Deprotection of a protective group can be conducted under a condition according to a method ordinarily used in the synthetic organic chemistry [for example, see, Protective Groups in Organic Synthesis by T. W. Greene, John

Wiley & Sons Inc.(1981)], or in a similar manner thereto.

Compound (X) can be obtained by a method described in the literature such as Tetrahedron, 45, 5787-5790 (1989), or in a similar manner thereto.

Compound (XII) can be obtained by a method described in the reference examples, or in a similar manner thereto.

<Preparation method 6>

The compound (I-e) in which B is -CO- and D is -NH-$X^A$-$Y^A$-$Z^A$ can be prepared according to the following reaction process.

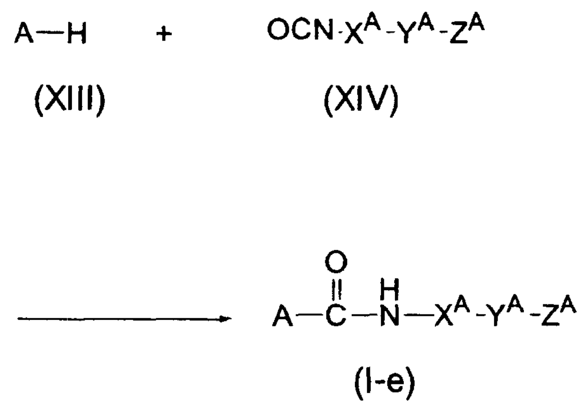

(XIII) (XIV)

(I-e)

(wherein each of A, $X^A$, $Y^A$, and $Z^A$ has the same meaning as that defined above, respectively)

Compound (I-e) can be obtained by the reaction of Compound (XIII) with Compound (XIV) , in the presence of a base if necessary, in a suitable inert solvent, for example, a halogenated hydrocarbon such as chloroform and dichloromethane, an aromatic hydrocarbon such as benzene and toluene, an ether-type solvent such as diethylether, THF, and 1,4-dioxane, a lower alcohol such as methanol, ethanol, and 2-propanol, an aprotic polar solvent such as N, N-dimethylformamide, N-methylpyrrolidone, and dimethyl sulfoxide, or a mixture thereof, at a temperature between -78°C and the boiling point of a used solvent for 5 minutes to 48 hours.

Examples of the base include organic bases such as triethylamine and pyridine, inorganic bases such as potassium carbonate, potassium hydrogencarbonate, sodium hydroxide, and sodium hydride, and a metal alkoxides such as sodium methoxide and potassium tert-butoxide.

Compound (XIII) can be obtained by a method described in the literature such as Bioorganic & Medicinal Chemistry Letters, 6, 1163-1166 (1996), or in a similar manner thereto.

Compound (XIV) can be obtained by a method described in the examples, or in a similar manner thereto.

<Preparation method 7>

Compound (I) can also be prepared according to the following reaction process.

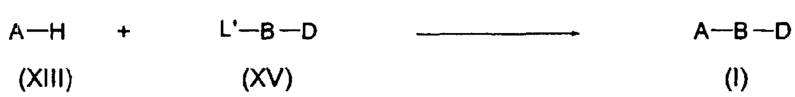

(XIII) (XV) (I)

(wherein L' represents a hydroxy group or has the same meaning as that of the aforementioned L, and each of A, B, and D has the same meaning as that defined above, respectively)

Compound (I) can be obtained by the reaction of Compound (XIII) with Compound (XV) , in the presence of a base or a condensing agent if necessary, in a suitable inert solvent, for example, a halogenated hydrocarbon such as chloroform and dichloromethane, an aromatic hydrocarbon such as benzene and toluene, an ether-type solvent such as diethylether, THF, and 1,4-dioxane, a lower alcohol such as methanol, ethanol, and 2-propanol, an aprotic polar solvent such as N,N-dimethylformamide, N-methylpyrrolidone, and dimethyl sulfoxide, or a mixture thereof, at a temperature between -78°C and the boiling point of a used solvent for 5 minutes to 48 hours.

Examples of the base include organic bases such as triethylamine and pyridine, inorganic bases such as

potassium carbonate, potassium hydrogencarbonate, sodium hydroxide, and sodium hydride, and a metal alkoxides such as sodium methoxide and potassium tert-butoxide. Examples of the condensing agent include 1,3-dicyclohexyl carbodiimide and 1-ethyl-3-(3-dimethylaminopropyl)carbodiimide.

Compound (XV) as the starting material can be obtained by a method described in the specification of the U. S. Patent No. 6,110,949 or the like, or a similar method thereto.

In the aforementioned methods for preparation, when the defined groups are changed under conditions of the methods to be conducted, or are not suitable for conducting the method, the desired compounds can be obtained by employing methods for introducing and eliminating a protective group which are ordinarily used in the synthetic organic chemistry [for example, see, Protective Groups in Organic Synthesis by T. W. Greene, John Wiley & Sons Inc. (1981)]. Conversion of functional groups contained in each of the substituents can be also carried out by known methods other than the aforementioned preparation methods [for example, Comprehensive Organic Transformations by R.C. Larock (1989)], and some of Compound (I) can be used as intermediates and converted to others of Compound (I) as novel derivatives.

For the purpose of improving pharmacodynamics (absorption, duration and the like), and stability, the compounds may be converted in prodrugs. The conversion of the compounds into the prodrugs can be carried out by method ordinarily used in the field of medicinal chemistry. To functional groups that can be modified for the synthesis of the prodrug from the compounds, treatments such as an acylation (the acyl groups used for the acylation may include acyl groups derived from natural amino acids), an aroylation, an acyloxymethyleneoxycarbonylation, a methoxycarbonylation, an ethoxycarbonylation, and a benzyloxycarbonylation can be conducted to obtain compounds converted into prodrugs.

The intermediates and the target compound can be isolated and purified by applying methods ordinarily used in the synthetic organic chemistry, such as a neutralization, a filtration, an extraction, a washing, a drying, a condensation, recrystallization, and various chromatographies. Intermediates can be applied to the subsequent reaction without a particular purification.

When a salt of Compound (I) is desired, Compound (I) obtained as a salt form may be purified, per se. When the compound is obtained as a free form, the compound may be dissolved or suspended in a suitable organic solvent to form salt with addition of an acid or a base according to an ordinary procedure.

The compound of the present invention represented by the general formula (I) has an inhibitory activity against dipeptidylpeptidase-IV (DPP-IV), and can be used as an active ingredient of a medicament for preventive and/or therapeutic treatment of Type II diabetes, and for preventive and/or therapeutic treatment of complications accompanying Type II diabetes. Typical examples of the complications accompanying Type II diabetes include, but not limited thereto, retinopathies, nephropathies, and neuropathies. The compound can also be used as an active ingredient of a medicament useful for therapeutic treatment of other pathologic conditions in which DPP-IV is involved.

As the medicament of the present invention, the compound represented by the general formula (I) or a pharmacologically acceptable salt thereof, per se, can be administered. Generally, it is preferred to formulate a pharmaceutical composition together with one or more of pharmaceutical additives and then administer the same. The medicament of the present invention can be administered to humans or mammals other than human. As the active ingredient of the medicament of the present invention, two or more of the compounds represented by the general formula (I) or pharmacologically acceptable salts thereof may be used in combination. As the active ingredient of the medicament of the present invention, the hydrates and the solvates may be used, as well as the compounds represented by the general formula (I) and pharmacologically acceptable salts thereof.

Route of administration of the medicament of the present invention is not particularly limited, and the medicament may be orally or parenterally administered. It is preferred to choose the most effective route of administration for therapeutic treatment.

Examples of forms of formulations suitable for oral administration include tablets, granules, powders, and syrups. Examples of forms of formulations suitable for parenteral administration include injections (such as those for intravenous administration). However, forms of formulations are not limited to these examples.

Liquid formulations suitable for oral administration (for example, syrups) can be prepared by using water, sugars such as sucrose, sorbitol, and fructose, glycols such as polyethylene glycol and propylene glycol, oils such as sesame oil, olive oil, and soybean oil, antiseptics such as p-hydroxybenzoic ester, flavors such as strawberry flavor and peppermint. Tablets, granules, powders and the like can be prepared by using excipients such as lactose, glucose, sucrose, and mannitol, disintegrants such as starch and sodium alginate, lubricants such as magnesium stearate and talc, binders such as polyvinylalcohol, hydroxypropylcellulose and gelatin, surfactants such as fatty acid esters, plasticizers such as glycerol.

Formulations suitable for parenteral administrations are preferably comprising aqueous sterilized formulations which is isotonic with blood of a recipient and comprises the active compound. For example, for preparation of injections, a solution for injection can be prepared by using carriers comprising a salt solution, a glucose solution, or a mixture of saline and a glucose solution. One or more of supplemental components selected from those exemplified for oral

pharmaceuticals, such as glycols, oils, flavors, antiseptics (including antioxidant), excipients, disintegrants, lubricants, binders, surfactants, and plasticizers, can also be added to these parenteral formulations.

Dose and frequency of administration of the medicament of the present invention depend on administration forms, the age and body weight of a patient, a nature of a disease to be treated, severity of the disease and the like, and preferably, they may be appropriately increased or decreased. Generally, a dose may be 0.01 to 1,000 mg, preferably 5 to 500 mg, per day for an adult, and the above dose may be administered once a day or twice or more a day as divided portions.

Best Mode for Carrying out the Invention

The present invention will be explained more specifically with reference to examples. However, the scope of the present invention is not limited to the following examples.

Example 1: (S)-1-[2-(2-Pyrazinylamino)ethylamino]acetyl-2-pyrrolidinecarbonitrile dimethanesulfonate (Compound 101)

(1) To a solution of 2-(2-pyrazinylamino)ethylamine (415 mg, 3.00 mmol) obtained in Reference example 2 in THF (2 mL) was added a solution of (S)-1-bromoacetyl-2-pyrrolidinecarbonitrile (217 mg, 1.00 mmol) described in the U.S. Patent No. 6,011,155 in THF (4 mL) under ice-cooling, and the mixture was stirred at the same temperature for one hour. After the reaction mixture was concentrated, the obtained residue was purified by preparative thin layer chromatography (chloroform/methanol=6/1) to obtain a free form of the title compound (114 mg, 0.420 mmol).
(2) To a solution of the free form obtained in (1) (225 mg, 0.820 mmol) in THF (2.5 mL) was added methanesulfonic acid (160 μ L, 2.46 mmol), and the mixture was stirred at room temperature for 10 minutes. The THF and the methanesulfonic acid was evaporated under reduced pressure. The obtained residue was crystallized from 2-propanol-ethanol to obtain the title compound (239 mg, 0.870 mmol) as colorless crystals.
yield: 26%
$^1$H NMR (DMSO-$d_6$) δ(ppm): 8.99 (1H, br s), 8.00-7.97 (2H, m), 7.75 (1H, d, J = 5.4 Hz), 4.84 (1H, dd, J = 5.9, 5.4 Hz), 4.11-4.00 (2H, m), 3.65-3.37 (4H, m), 3.22-3.04 (2H, m), 2.35 (6H, s), 2.24-1.96 (4H, m).
APCIMS (m/z): 275 $(M + H)^+$
Elemental analysis: Calcd. for $C_{13}H_{18}SN_6O$ $2CH_4O_3S$ $1.5H_2O$: C, 36.49; H, 5.93; N, 17.02. Found C, 36.64; H, 5.66; N, 16.75.

Example 2: (S)-1-[2-(6-Chloro-3-pyridazinylamino)ethylamino]acetyl-2-pyrrolidinecarbonitrile dimethanesulfonate (Compound 102)

The title compound was obtained in a similar manner to that of Example 1 by using 2-(6-chloro-3-pyridazinylamino)ethylamine obtained in Reference example 3 instead of 2-(2-pyrazinylamino)ethylamine, and by using potassium carbonate as a base in the process of condensation reaction with (S)-1-bromoacetyl-2 pyrrolidinecarbonitrile.
yield: 3%
APCIMS (m/z): 309 $(^{35}ClM + H)^+$, 311 $(^{37}ClM + H)^+$

Example 3: (S)-1-[2-(2-Quinoxalinylamino)ethylamino]acetyl-2-pyrrolidinecarbonitrile dihydrochloride (Compound 103)

The title compound was obtained in a similar manner to that of Example 1 by using 2-(2-quinoxalinylamino) ethylamine obtained in Reference example 4 instead of 2-(2-pyrazinylamino)ethylamine, and by using potassium carbonate as a base in the process of condensation reaction with (S)-1-bromoacetyl-2- pyrrolidinecarbonitrile, and by using a solution of hydrogen chloride in ethyl acetate instead of methanesulfonic acid in the preparation of a salt.
yield: 16%
$^1$H NMR (DMSO-$d_6$) δ (ppm) : 8.40 (1H, s), 7.83-7.53 (3H, m), 7.43-7.36 (1H, m), 4.84 (1H, dd, J = 5.9, 5.4 Hz), 4.21-3.42 (6H, m), 3.32-3.11 (2H, m), 2.25-2.02 (4H, m).
APCIMS (m/z): 324 $(M + H)^+$

Example 4: (S)-1-[2-(4-Chloro-1-phthalazinylamino)ethylamino]acetyl-2-pyrrolidinecarbonitrile dimethanesulfonate (Compound 104)

The title compound was obtained in a similar manner to that of Example 1 by using 2-(4-chloro-1-phthalazinylamino)ethylamine obtained in Reference example 5 instead of 2-(2-pyrazinylamino)ethylamine, and by using potassium carbonate as a base in the process of condensation reaction with (S)-1-bromoacetyl-2-pyrrolidinecarbonitrile.
yield: 18%
$^1$H NMR (DMSO-$d_6$) δ (ppm): 8.16-8.05 (4H, m), 4.84 (1H, dd, J = 6.2, 5.1 Hz), 4.15-4.11 (2H, m), 3.91-3.87 (2H, m), 3.65-3.55 (2H, m), 3.44-3.20 (2H, m), 2.31 (6H, s), 2.25-1.99 (4H, m).
APCIMS (m/z): 357 ($^{35}$ClM - H)-, 359 ($^{37}$ClM - H)-

Example 5: (S)-1-[2-(6,7-Dimethoxy-4-quinazolinylamino)ethylamino]acetyl-2-pyrrolidinecarbonitrile dimethanesulfonate (Compound 105)

The title compound was obtained in a similar manner to that of Example 1 by using 2-(6,7-dimethoxy-4-quinazolinylamino)ethylamine obtained in Reference example 6 instead of 2-(2-pyrazinylamino)ethylamine, and by using potassium carbonate as a base in the process of condensation reaction with (S)-1-bromoacetyl-2-pyrrolidinecarbonitrile.
yield: 12%
$^1$H NMR (DMSO-$d_6$) δ (ppm): 8.88 (1H, s), 7.84 (1H, s), 7.29 (1H, s), 4.84 (1H, dd, J = 6.2, 4.9 Hz), 4.20-3.93 (4H, m), 3.99 (3H, s), 3.97 (3H, s), 3.66-3.56 (1H, m), 3.53-3.20 (3H, m), 2.55-2.04 (4H, m), 2.34 (6H, s).
APCIMS (m/z): 383 (M - H)$^-$

Example 6: (S)-1-{2-[2-(4-Pyridyl)-4-quinazolinylamino]ethylamino)acetyl-2-pyrrolidinecarbonitrile dimethanesulfonate (Compound 106)

The title compound was obtained in a similar manner to that of Example 1 by using 2-[2-(4-pyridyl)-4-quinazolinylamino]ethylamine obtained in Reference example 7 instead of 2-(2-pyrazinylamino)ethylamine, and by using potassium carbonate as a base in the process of condensation reaction with (S)-1-bromoacetyl-2-pyrrolidinecarbonitrile.
yield: 17%
$^1$H NMR (free form, $CDCl_3$) δ (ppm): 8.74-8.72 (2H, m), 8.50-8.30 (3H, m), 7.93-7.87 (1H, m), 7.77-7.72 (1H, m), 7.58-7.51 (1H, m), 5.00 and 4.77 (1H, m), 3.86-3.58 (4H, m), 3.50-3.09 (4H, m), 2.38-2.00 (4H, m).
APCIMS (m/z): 402 (M + H)$^+$

Example 7: (S)- 1-[2-(2-Quinolylamino)ethylamino]acetyl-2-pyrrolidinecarbonitrile dihydrochloride (Compound 107)

The title compound was obtained in a similar manner to that of Example 1 by using 2-(2-quinolylamino)ethylamine obtained by the method described in Reference example 1 instead of 2-(2-pyrazinylamino)ethylamine, and by using potassium carbonate as a base in the process of condensation reaction with (S)-1-bromoacetyl-2-pyrrolidinecarbonitrile.
yield: 20%
$^1$H NMR (DMSO-$d_6$) δ (ppm): 10.2 (1H, br s), 9.43 (1H, br s), 8.45-8.16 (2H, m), 7.90 (1H, d, J = 7.6 Hz), 7.75 (1H, dd, J = 7.6, 7.6 Hz), 7.48 (1H, dd, J = 7.6, 7.6 Hz), 7.21 (1H, d, J = 6.8 Hz), 4.82 (1H, dd, J = 6.9, 4.2 Hz), 4.29-3.93 (4H, m), 3.62-3.47 (4H, m), 2.23-1.86 (4H, m).
APCIMS (m/z): 324 (M + H)$^+$

Example 8: (S)-1-[2-(4-Methyl-2-quinolylamino)ethylamino]acetyl-2-pyrrolidinecarbonitrile dihydrochloride (Compound 108)

The title compound was obtained in a similar manner to that of Example 1 by using 2-(4-methyl-2-quinolylamino)ethylamine obtained in Reference example 8 instead of 2-(2-pyrazinylamino)ethylamine, and by using potassium carbonate as a base in the process of condensation reaction with (S)-1-bromoacetyl-2-pyrrolidinecarbonitrile.
yield: 26%
$^1$H NMR (DMSO-$d_6$) δ (ppm): 9.26-9.19 (1H, m), 8.21-7.94 (2H, m), 7.86-7.74 (1H, m), 7.61-7.40 (1H, m), 7.06-6.92 (1H, m), 4.78-4.67 (1H, m), 4.54-4.16 (1H, m), 4.10-3.82 (6H, m), 3.42-3.34 (2H, m), 2.58 (3H, s), 2.01-1.81 (4H, m).
APCIMS (m/z): 338 (M + H)$^+$

Example 9: (S)- 1-[2-(4-Quinolylamino)ethylamino]acetyl-2-pyrrolidinecarbonitrile dihydrochloride (Compound 109)

The title compound was obtained in a similar manner to that of Example 1 by using 2-(4-quinolylamino)ethylamine obtained in Reference example 9 instead of 2-(2-pyrazinylamino)ethylamine, and by using potassium carbonate as a base in the process of condensation reaction with (S)-1-bromoacetyl-2-pyrrolidinecarbonitrile.
yield: 28%
$^1$H NMR (DMSO-$d_6$) δ (ppm): 9.69 (1H, br s), 9.62-9.30 (1H, m), 8.72-8.64 (2H, m), 7.99-7.94 (2H, m), 7.76-7.71 (1H, m), 7.00 (1H, d, J = 7.0 Hz), 4.84 (1H, dd, J = 5.5, 5.5 Hz), 4.30-4.10 (2H, m), 4.02-3.89 (2H, m), 3.64-3.06 (4H, m), 2.20-1.91 (4H, m).
APCIMS (m/z): 324 $(M + H)^+$

Example 10: (S)-1-[2-(1-Isoquinolylamino)ethylamino]acetyl-2-pyrrolidinecarbonitrile dihydrochloride (Compound 110)

The title compound was obtained in a similar manner to that of Example 1 by using 2-(1-isoquinolylamino) ethylamine obtained in Reference example 10 instead of 2-(2-pyrazinylamino)ethylamine, and by using potassium carbonate as a base in the process of condensation reaction with (S)- 1-bromoacetyl-2-pyrrolidinecarbonitrile.
yield: 3%
$^1$H NMR (DMSO-$d_6$) δ (ppm) : 10.30 (1H, br s), 9.38 (1H, br s), 8.86-8.83 (1H, m), 7.96-7.95 (2H, m), 7.81-7.65 (2H, m), 7.28 (1H, d, J = 6.8 Hz), 4.83 (1H, dd, J = 5.4, 5.4 Hz), 4.35-4.04 (4H, m), 3.70-3.52 (2H, m), 3.46-3.36 (2H, m), 2.22-1.93 (4H, m).
APCIMS (m/z): 324 $(M + H)^+$

Example 11: (S)-1-[2-(2-Benzothiazolylamino)ethylamino]acetyl-2-pyrrolidinecarbonitrile dihydrochloride (Compound 111)

The title compound was obtained in a similar manner to that of Example 1 by using 2-(2-benzothiazolylamino) ethylamine obtained in Reference example 11 instead of 2-(2-pyrazinylamino)ethylamine, and by using potassium carbonate as a base in the process of condensation reaction with (S)-1-bromoacetyl-2- pyrrolidinecarbonitrile.
yield: 18%
$^1$H NMR (DMSO-$d_6$) δ (ppm): 9.45-9.23 (1H, m), 8.59-8.49 (1H, m), 7.72 (1H, d, J = 6.8 Hz), 7.49 (1H, dd, J = 6.8 Hz), 7.27 (1H, dd, J = 6.8, 6.8 Hz), 7.08 (1H, dd, J = 6.8, 6.8 Hz), 4.86 (1H, dd, J = 6.5, 4.6 Hz), 4.20-4.08 (2H, m), 3.77-3.20 (6H, m), 2.26-1.94 (4H, m).
APCIMS (m/z): 330 $(M + H)^+$

Example 12: (S)-1-[2-(5-N,N-Dimethylaminosulfonyl-2-pyridylamino)ethylamino] acetyl-2-pyrrolidinecarbonitrile dihydrochloride (Compound 112)

The title compound was obtained in a similar manner to that of Example 1 by using 2-[5-(N,N-dimethylaminosulfonyl)-2-pyridylamino]ethylamine obtained by the method described in Reference example 12 instead of 2-(2-pyrazinylamino)ethylamine, and by using a 4 mol/L solution of hydrogen chloride in 1,4-dioxane in stead of methanesulfonic acid in the preparation of a salt.
yield: 27%
$^1$H NMR (DMSO-$d_6$) δ (ppm): 8.30 (1H, d, J = 2.4 Hz), 7.68 (1H, dd, J = 8.9, 2.4 Hz), 6.70 (1H, d, J = 8.9 Hz), 4.84 (1H, m), 3.68-3.41 (6H, m), 3.16 (2H, br s), 2.58 (6H, s), 2.17-1.76 (4H, m).
APCIMS (m/z): 381 $(M + H)^+$

Example 13: (S)-1-[4-[(3-Cyano-2-pyridyl)aminomethyl]benzylamino}acetyl-2-pyrrolidinecarbonitrile oxalate (Compound 201)

The title compound was obtained in a similar manner to that of Example 1 by using 4-[(3-cyano-2-pyridyl) aminomethyl]benzylamine obtained by the method described in Reference example 15 instead of 2-(2-pyrazinylamino) ethylamine, by using potassium carbonate as a base in the process of condensation reaction with (S)-1-bromoacetyl-2-pyrrolidinecarbonitrile, and by using oxalic acid instead of methanesulfonic acid in the preparation of a salt.
yield: 56%
$^1$H NMR (free form, $CDCl_3$) δ (ppm): 8.30 (1H, dd, J = 4.9, 1.9 Hz), 7.66 (1H, dd, J = 7.6, 1.9 Hz), 7.31 (4H, s), 6.62 (1H, dd, J = 7.6, 4.9 Hz), 5.59 (1H, br), 4.75 (1H, m), 4.69 (2H, d, J = 5.6 Hz), 3.82 (2H, s), 3.55-3.30 (2H, m), 3.37 (2H, s), 2.56 (1H, br), 2.32-2.04 (4H, m).
APCIMS (m/z): 373 $(M - H)^-$

Example 14: (S)-1-[4-(3-Cyano-2-pyridyl)-1-piperazinyl]acetyl-2-pyrrolidinecarbonitrile (Compound 202)

The title compound was obtained in a similar manner to that of Example 1 (1) by using 1-(3-cyano-2-pyridyl) piperazine dihydrochloride obtained in Reference example 16 instead of 2-(2-pyrazinylamino)ethylamine, and by using potassium carbonate as a base in the process of condensation reaction with (S)-1-bromoacetyl-2 pyrrolidinecarbonitrile.
yield: 60%
$^1$H NMR ($CDCl_3$) δ (ppm): 8.34 (1H, dd, J = 4.8, 1.9 Hz), 7.77 (1H, dd, J = 7.6, 1.9 Hz), 6.77 (1H, dd, J = 7.6, 4.8 Hz), 5.17 and 4.76 (1H, m), 3.82-3.72 (4H, m), 3.71-3.50 (2H, m), 2.96 and 2.88 (2H, s), 2.75-2.68 (4H, m), 2.31-2.04 (4H, m).
APCIMS (m/z): 325 $(M + H)^+$

Example 15: (S)-1-(2-[(3-Cyano-2-pyridyl)-N-methylamino]ethyl-N-methylamino] acetyl-2-pyrrolidinecarbonitrile (Compound 203)

The title compound was obtained in a similar manner to that of Example 1 (1) by using 2-[(3-cyano-2-pyridyl) -N-methylamino]ethyl-N-methylamine obtained by the method described in Reference example 17 instead of 2-(2-pyrazinylamino)ethylamine, and by using potassium carbonate as a base in the process of condensation reaction with (S)-1-bromoacetyl-2-pyrrolidinecarbonitrile.
yield: 79%
$^1$H NMR (free form, $CDCl_3$) δ (ppm): 8.27 (1H, dd, J = 4.8, 2.0 Hz), 7.72 (1H, dd, J = 7.6, 2.0 Hz), 6.62 (1H, dd, J = 7.6, 4.8 Hz), 5.26 and 4.71 (1H, m), 3.82 (2H, m), 3.66 (1H, m), 3.50 (1H, m), 3.30 (3H, s), 3.28 (2H, s), 2.80 (2H, m), 2.41 and 2.37 (3H, s), 2.29-2.01 (4H, m).
APCIMS (m/z): 327 $(M + H)^+$

Example 16: (R)-3-[1-(3-Cyano-2-pyridyl)-4-piperidyl]aminoacetyl-4-thiazolinecarbonitrile dihydrochloride (Compound 204)

(R)-3-[N-tert-Butoxycarbonyl-1-(3-cyano-2-pyridyl)-4-piperidylamino]acetyl-4-thiazolinecarbonitrile (21 mg, 0.046 mmol) obtained by the method described in Reference example 19 was dissolved in methylene chloride (0.5 mL), added with trifluoroacetic acid (0.5 mL) under ice-cooling, and stirred for 15 minutes. Then, the solution was further stirred for 1 hour without ice bath. The reaction mixture was concentrated. To the obtained residue was added ethanol (3 mL). To the mixture was then added BioRad AG (registered trademark) 1X-8 ion-exchange resin until the pH reached to 8. The resin was removed by filtration and the filtrate was concentrated under reduced pressure.
The obtained residue was separated and purified by HPLC (YMC Pack SIL SH-043-5 2X25cm YMC Co.,Ltd, chloroform/methanol=96/4, flow rate 10 mL/minute) to obtain the free form of the title compound (8 mg, 0.02 mmol).
The free form was suspended in THF (0.5 mL). To the suspension was added a 4 mol/L solution of hydrogen chloride in 1,4-dioxane (17 μ L, 0.068 mmol), and the mixture was stirred for 30 minutes. The solvent was evaporated under reduced pressure to obtain the title compound (10 mg, 0.02 mmol).
yield: 50%
$^1$H NMR (DMSO-$d_6$) δ (ppm): 9.20 (2H, br s), 8.42 (1H, dd, J = 4.8, 2.0 Hz), 8.09 (1H, dd, J = 7.7, 2.0 Hz), 6.95 (1H, dd, J = 7.7, 4.8 Hz), 5.37-5.36 (1H, m), 4.80 (1H, d, J = 8.5 Hz), 4.62 (1H, d, J = 8.5 Hz), 4.31-4.15 (4H, m), 3.3-3.5 (3H, m), 3.18-2.99 (2H, m), 2.20-2.16 (2H, m), 1.76-1.68 (2H, m).
APCIMS (m/z): 357 $(M + H)^+$

Example 17: (S)-1-(1-(5-Cyano-2-pyridyl)-4-piperidylamino]acetyl-2-pyrrolidinecarbonitrile dihydrochloride (Compound 205)

A solution of 4-amino-1-(5-cyano-2-pyridyl)piperidine (0.606 g, 3.00 mmol) obtained in Reference example 26 in THF (9 mL) was cooled by ice and stirred for one hour. The solution was added with (S)-1-bromoacetyl-2-pyrrolidinecarbonitrile (217 mg, 1.00 mmol) described in the U.S. Patent No. 6,011,155 at the same temperature, and stirred for 3 hours. The solvent was evaporated under reduced pressure. The obtained residue was added with ethyl acetate and water, which were then separated. The obtained organic layer was dried over anhydrous magnesium sulfate. The solvent was evaporated under reduced pressure and the obtained residue was purified by silica gel column chromatography (chloroform/methanol=100/0 to 95/5) to obtain the free form of the title compound.
The resulting oil was dissolved in THF (12mL), to which a 4 mol/L solution of hydrogen chloride in 1,4-dioxane (0.9mL) was added dropwise. The solvent was evaporated under reduced pressure and the obtained residue was added with THF, filtered, dried under reduced pressure to obtain the title compound (317 mg, 77%).
yield: 77%

$^1H$ NMR (DMSO-$d_6$) δ (ppm): 9.22 (2H, br s), 8.49 (1H, d, J = 2.3 Hz), 7.86 (1H, dd, J = 8.9, 2.3 Hz), 7.00 (1H, d, J = 8.9 Hz), 4.85 (1H, dd, J = 6.8, 4.5 Hz), 4.58-4.53 (2H, br d), 3.71-3.38 (4H, m), 3.00-2.91 (2H, m), 2.26-1.99 (6H, m), 1.59-1.53 (2H, m).
FABMS (m/z): 339 $(M + H)^+$
Elemental analysis: Calcd. for $C_{18}H_{23}N_6O \cdot 2HCl \cdot H_2O$(430.36): C, 50.24; H, 6.32; N, 19.53. Found: C, 50.51; H, 6.42; N, 19.24.

Example 18: (S)-1 -[3 -(5-Cyano-2-pyridyl)amino-2-methyl-2-propylamino]acetyl-2-pyrrolidinecarbonitrile dihydrochloride (Compound 206)

(1) To a solution of 2-amino-N-(5-cyano-2-pyridyl)-2-methylpropylamine (856 mg, 4.50 mmol) obtained by the method described in Reference example 13 in THF (10 mL) was added a solution of (S)-1-bromoacetyl-2-pyrrolidinecarbonitrile (326 mg, 1.50 mmol) described in the U.S. Patent No. 6,011,155 in THF (5 mL) under ice-cooling, and the mixture was stirred at the same temperature for 10 minutes, and then stirred at room temperature for 70 minutes. After addition of chloroform to the reaction mixture, the organic layer was washed with water and saturated brine, and dried over anhydrous magnesium sulfate. The solvent was evaporated under reduced pressure. The obtained residue was purified by silica gel column chromatography (chloroform/methanol=8/1) to obtain a free form of the title compound (481 mg, 1.47 mmol).
(2) To a solution of the free form (481 mg, 1.47 mmol) obtained in (1) in methanol (3 mL) was added a 4 mol/L solution of hydrogen chloride in 1,4-dioxane (1.1 mL, 4.41 mmol). The solvent was evaporated under reduced pressure. The obtained residue was crystallized from 2-propanol-ethanol to obtain the title compound (110 mg, 0.32 mmol) as colorless crystals.
yield: 22%
$^1H$ NMR (DMSO-$d_6$) δ (ppm): 8.98 (1H, br s), 8.38 (1H, dd, J = 2.2 Hz), 8.05-7.95 (1H, m), 7.74 (1H, dd, J = 8.5, 2.2 Hz), 6.71 (1H, d, J = 8.5 Hz), 4.84 (1H, dd, J = 6.9, 4.5 Hz), 3.74-3.48 (4H, m), 3.64 (2H, d, J = 6.5 Hz), 2.25-2.00 (4H, m), 1.30 (6H, s).
APCIMS (m/z): 327 $(M + H)^+$

Example 19:

(S)- 1-[1-(5-Cyano-2-pyridyl)-4-methyl-4-piperidylamino]acetyl-2-pyrrolidinecarbonitril e dihydrochloride (Compound 207)

(1) 4-tert-butoxycarbonylamino-4-methylpiperidine described in European Patent No. 647,639 (1.07 g, 5.00 mmol) was suspended in 1,4-dioxane (20 mL), and added with potassium carbonate (1.04 g, 7.50 mmol) and 2-chloro-5-cyanopyridine (1.04 g, 7.50 mmol). The mixture was refluxed overnight, and then allowed to stand for cooling. Then to the mixture was added water, and the mixture was extracted with chloroform three times. The combined organic layer was washed with saturated aqueous sodium hydrogencarbonate solution and then with saturated brine, dried over anhydrous magnesium sulfate and concentrated. The obtained residue was purified by silica gel column chromatography to obtain 4-tert-butoxycarbonylamino- 1-(5-cyano-2-pyridyl)-4-methylpiperidine (0.78 g, 2.5 mmol).
$^1H$ NMR ($CDCl_3$) δ (ppm): 8.39 (1H, d, J = 2.4Hz), 7.59 (1H, dd, J = 8.9, 2.4Hz), 6.61 (1H, d, J = 8.9 Hz), 4.43 (1H, br s), 3.96 (2H, ddd, J = 13.5, 8.9, 4.6Hz), 3.39 (2H, ddd, J = 13.5, 10.5, 3.0Hz), 2.15-2.05 (2H, m), 1.70-1.50 (2H, m), 1.44 (9H, s), 1.39 (3H, s).
APCIMS (m/z): 317 $(M + H)^+$
(2) To a solution of
4-tert-butoxycarbonylamino-1-(5-cyano-2-pyridyl)-4-methylpiperidine (0.78 g, 2.5 mmol) in 1,4-dioxane (5 mL) was added a 4 mol/L solution of hydrogen chloride in 1,4-dioxane (50 mL) under ice-cooling. The reaction mixture was stirred at room temperature for one hour. The solvent was evaporated under reduced pressure, and azeotropically distilled with toluene. The residue was dissolved in ethanol (100 mL). To the solution was added BioRad AG (registered trademark) 1-X8 ion-exchange resin (40 g), and the mixture was stirred for 5 minutes. After the reaction mixture was filtered, the filtrate was concentrated to obtain 4-amino-1-(5-cyano-2-pyridyl)-4-methylpiperidine (0.53 g, 2.5 mmol).
$^1H$ NMR ($CDCl_3$) δ (ppm) : 8.38 (1H, d, J = 2.4Hz), 7.57 (1H, dd, J = 9.2, 2.4Hz), 6.61 (1H, d, J = 9.2 Hz), 3.95-3.70

(2H, m), 3.70-3.55 (2H, m), 1.65-1.50 (4H, m), 1.33 (2H, br s), 1.19 (3H, s).
APCIMS (m/z): 217 $(M + H)^+$
(3) To a solution of 4-amino-1-(5-cyano-2-pyridyl)-4-methylpiperidine (0.53 g, 2.5 mmol) obtained in (2) in methanol (10 mL) was added (S)-1-bromoacetyl-2-pyrrolidinecarbonitrile (174 mg, 0.80 mmol) described in the U.S. Patent No. 6,011,155 under ice-cooling, and the mixture was stirred at room temperature for 4 hours. The reaction mixture was concentrated. To the solution was added water, and the mixture was extracted three times with chloroform. The combined organic layer was washed with aqueous saturated sodium hydrogencarbonate solution and then with saturated brine, dried over anhydrous magnesium sulfate, and concentrated. The obtained residue was purified by silica gel column chromatography to obtain a free form of the title compound (272 mg, 0.770 mmol).
(4) To a solution of the free form (272 mg, 0.770 mmol) obtained in (3) in 1,4-dioxane (10 mL) was added a 4 mol/L solution of hydrogen chloride (0.5 mL) in 1,4-dioxane under ice-cooling, and the mixture was stirred at room temperature for 10 minutes. The deposited precipitate was collected by filtration to obtain the title compound (185 mg, 0.440 mmol) as colorless crystals.
yield: 57%
$^1H$ NMR (DMSO-$d_6$) δ (ppm): 9.05 (2H, br s), 8.49 (1H, d, J = 1.9Hz), 7.87 (1H, dd, J = 9.2, 1.9Hz), 6.99 (1H, d, J = 1.9 Hz), 4.83 (1H, dd, J = 6.8, 4.1 Hz), 4.38 (2H, m), 4.15-3.88 (2H, m), 3.80-3.70 (1H, m), 3.70-3.50 (2H, m), 3.20-3.05 (2H, m), 2.25-2.10 (2H, m), 2.10-1.95 (2H, m), 1.95-1.80 (4H, m), 1.45 (3H, s).
APCIMS (m/z): 353 $(M + H)^+$

Example 20: (S)-1-[1-(2-Pyrazinyl)-4-piperidylaminoacetyl-2-pyrrolidinecarbonitrile dihydrochloride (Compound 208)

(1) To a solution of 4-amino-1-(2-pyrazinyl) piperidine (1.07 g, 6.00 mmol) obtained by the method described in Reference example 22 in N,N-dimethylformamide (15 mL) was added a solution of (S)-1-bromoacetyl-2-pyrrolidinecarbonitrile (434 mg, 2.00 mmol) described in the U.S. Patent No. 6,011,155 in N,N-dimethylformamide (5 mL) at room temperature, and the mixture was stirred at the same temperature for 3 hours. After the evaporation of the solvent, the residue was added with chloroform. The organic layer was washed with water and saturated brine, and dried over anhydrous magnesium sulfate. Then the solvent was evaporated under reduced pressure. The obtained residue was purified by column chromatography (chloroform/methanol=8/1) to obtain a free form of the title compound (127 mg, 0.400 mmol).
(2) To a solution of the free form (127 mg, 0.400 mmol) obtained in (1) in methanol (1.5 mL) was added a 4 mol/L solution of hydrogen chloride in 1,4-dioxane (0.30 mL, 1.2 mmol). The solvent was evaporated under reduced pressure and the obtained residue was crystallized from 2-propanol/ethanol to obtain the title compound (35 mg, 0.09 mmol) as colorless crystals.
yield: 5%
$^1H$ NMR (DMSO-$d_6$) δ (ppm) : 8.40 (1H, s), 8.15 (1H, d, J = 2.2 Hz), 7.84 (1H, d, J = 2.2 Hz), 4.82-(1H, dd, J = 6.9, 3.9 Hz), 4.51-4.29 (3H, m), 4.19-4.00 (2H, m), 3.69-3.26 (3H, m), 2.94-2.84 (2H, m), 2.33-1.82 (6H, m), 1.71-1.50 (2H, m).

APCIMS (m/z): 315 $(M + H)^+$

Example 21: (S)-1-[1-(2-Quinolyl)-4-piperidylamino]acetyl-2-pyrrolidinecarbonitrile dihydrochloride (Compound 209)

The title compound was obtained in a similar manner to that of Example 20 by using 4-amino-1-(2-quinolyl) piperidine obtained in Reference example 23 instead of 4-amino-1-(2-pyrazinyl)piperidine.
yield: 3%
$^1H$ NMR (DMSO-$d_6$) δ (ppm): 9.38-9.18 (1H, m), 8.45-8.27 (2H, m), 7.92-7.8 (1H, m), 7.79-7.70 (1H, m), 7.62-7.36 (2H, m), 4.85 (1H, dd, J = 6.2, 5.1 Hz), 4.76-4.65 (2H, m), 4.22-4.01 (2H, m), 3.72-3.36 (5H, m), 2.23-1.98 (6H, m), 1.83-1.65 (2H, m).
APCIMS (m/z): 364 $(M + H)^+$

Example 22: (S)-1-[1-(2-Quinoxalinyl)-4-piperidylamino]acetyl-2-pyrrolidinecarbonitrile dihydrochloride (Compound 210)

The title compound was obtained in a similar manner to that of Example 20 by using 4-amino-1-(2-quinoxalinyl) piperidine obtained in Reference example 24 instead of 4- amino- 1 -(2-pyrazinyl)piperidine.
yield: 28%

$^1$H NMR (DMSO-$d_6$) δ (ppm): 9.30-9.01 (1H, m), 8.88 (1H, s), 7.83 (1H, d, J = 8.1 Hz), 7.65-7.57 (2H, m), 7.44-7.38 (1H, m), 4.84 (1H, dd, J = 7.0, 4.3 Hz), 4.73-4.68 (2H, m), 4.19-3.99 (2H, m), 3.63-3.34 (3H, m), 3.07-2.98 (2H, m), 2.28-1.89 (6H, m), 1.76-1.55 (2H, m).
APCIMS (m/z) : 365 $(M + H)^+$

Example 23: (S)-1-[1-(1-Isoquinolyl)-4-piperidylamino]acetyl-2-pyrrolidinecarbonitrile dihydrochloride (Compound 211)

The title compound was obtained in a similar manner to that of Example 20 by using 4-amino-1-(1-isoquinolyl) piperidine obtained in Reference example 25 instead of 4-amino-1-(2-pyrazinyl)piperidine, and by using cesium hydroxide monohydrate as a base in the process of condensation reaction with (S)-1-bromoacetyl-2-pyrrolidinecarbonitrile.
yield: 9%
$^1$H NMR (DMSO-$d_6$) δ (ppm): 9.45 (1H, br s), 8.15 (1H, d, J = 7.7 Hz), 8.06-7.93 (3H, m), 7.77 (1H, dd, J = 7.8, 7.0 Hz), 7.56 (1H, d, J = 7.7 Hz), 4.88 (1H, dd, J = 4.5, 4.5 Hz), 4.31-3.98 (2H, m), 3.66-3.23 (7H, m), 2.32-1.87 (8H, m).
APCIMS (m/z): 364 $(M + H)^+$

Example 24: (S)-1-[2-Methyl-1-(2-quinoxalinylamino)-2-propylamino]acetyl-2-pyrrolidinecarbonitrile dihydrochloride (Compound 212)

(1) To a solution of 2-amino-2-methyl-N- (2-quinoxalinyl)propylamine (865 mg, 4.00 mmol) obtained by the method described in Reference example 27 in N,N-dimethylformamide (20 mL) was added cesium hydroxide monohydrate (504 mg, 3.00 mmol) at room temperature, and the mixture was stirred at the same temperature for 5 minutes. To the mixture was added (S)-1-bromoacetyl-2-pyrrolidinecarbonitrile (434 mg, 2.00 mmol) described in the U.S. Patent No. 6,011,155 and the mixture was stirred at the same temperature for 7 hours. After chloroform was added to the reaction mixture, the organic layer was washed with water, and dried over anhydrous magnesium sulfate. The solvent was evaporated under reduced pressure. The obtained residue was purified by silica gel column chromatography (chloroform/methanol= 100/0 to 90/10) to obtain a free form of the title compound.

(2) To a solution of the free form obtained in (1) in methanol was added a 4 mol/L solution of hydrogen chloride in 1,4-dioxane. The solvent was evaporated under reduced pressure and the obtained residue was crystallized from 2-propanol-ethanol to obtain the title compound (93.0 mg, 0.219 mmol) as colorless crystals.
yield: 11%
$^1$H NMR (DMSO-$d_6$) δ (ppm): 9.00 (1H, br s), 8.41 (1H, s), 8.10-8.01 (1H, m), 7.81-7.78 (1H, m), 7.59-7.54 (1H, m), 7.41-7.32 (1H, m), 4.79 (1H, dd, J = 5.7, 4.3 Hz), 4.28-4.13 (3H, m), 3.84-3.47 (4H, m), 2.26-1.98 (4H, m), 1.38 (6H, s).
APCIMS (m/z): 353 $(M + H)^+$

Example 25: (S)-1-[2-Methyl-1-(2-quinolylamino)-2-propylamino]acetyl-2-pyrrolidinecarbonitrile fumarate (Compound 213)

The title compound (85.0 mg, 0.182 mmol) was obtained in a similar manner to that of Example 24 by using 2-amino-2-methyl-N-(2-quinolyl)propylamine (860 mg, 4.00 mmol) obtained by the method described in Reference example 28 instead of 2-amino-2-methyl-N-(2-quinoxalinyl)propylamine, and by using fumaric acid instead of a 4 mol/L solution of hydrogen chloride in 1,4-dioxane.
yield: 9%
$^1$H NMR (DMSO-$d_6$) δ (ppm): 8.03-7.65 (4H, m), 7.36-7.12-(3H, m), 6.57 (2H, s), 4.86 (1H, dd, J = 6.8, 4.3 Hz), 4.27-3.16 (7H, m), 2.29-1.96 (4H, m), 1.43 (6H, s).
APCIMS (m/z): 352 $(M + H)^+$

Example 26: (S)-1-[ 1-( 1-Isoquinolylamino)-2-methyl-2-propylamino]acetyl-2-pyrrolidinecarbonitrile dihydrochloride (Compound 214)

The title compound (136 mg, 0.320 mmol) was obtained in a similar manner to that of Example 24 by using 2-amino-N-(1-isoquinolyl)-2-methylpropylamine (860 mg, 4.00 mmol) obtained by the method described in Reference example 29 instead of 2-amino-2-methyl-N-(2-quinoxalinyl)propylamine.
yield: 16%
$^1$H NMR (DMSO-$d_6$) δ (ppm): 8.01-7.68 (4H, m), 7.33-7.07 (3H, m), 4.87 (1H, dd, J = 6.2, 4.6 Hz), 4.34-3.16 (7H, m),

2.29-1.95 (4H, m), 1.41 (6H, s).
APCIMS (m/z): 352 $(M + H)^+$

Example 27: (S)-1-[2-Methyl- 1 (4-quinolylamino)-2-propylamino]acetyl-2-pyrrolidinecarbonitrile fumarate (Compound 215)

The title compound (270 mg, 0.578 mmol) was obtained in a similar manner to that of Example 24 by using 2-amino-2-methyl-N-(4-quinolyl)propylamine (860 mg, 4.00 mmol) obtained by the method described in Reference example 30 instead of 2-amino-2-methyl-N-(2-quinoxalinyl)propylamine, and by using fumaric acid instead of a 4 mol/L solution of hydrogen chloride in 1,4-dioxane.
yield: 29%
$^1H$ NMR (DMSO-$d_6$) δ (ppm): 8.39 (1H, d, J = 5.8 Hz), 8.27 (1H, d, J = 8.4 Hz), 7.80 (1H, d, J = 6.8 Hz), 7.67 (1H, dd, J = 7.3, 6.8 Hz), 7.48 (1H, dd, J = 8.4, 7.3 Hz), 6.66 (1H, d, J = 5.8 Hz), 6.55 (2H, s), 4.75 (1H, dd, J = 6.5, 4.3 Hz), 3.83-3.02 (8H, m), 2.27-1.90 (4H, m), 1.12 (6H, s).
APCIMS (m/z): 352 $(M + H)^+$

Example 28: (S)-1-[2-Methyl-1-(2-pyrazinylamino)-2-propylamino]acetyl-2-pyrrolidinecarbonitrile fumarate (Compound 216)

The title compound (217 mg, 0.519 mmol) was obtained in a similar manner to that of Example 24 by using 2-amino-2-methyl-N-(2-pyrazinyl)propylamine (664 mg, 4.00 mmol) obtained by the method described in Reference example 31 instead of 2-amino-2-methyl-N-(2-quinoxalinyl)propylamine, and by using fumaric acid instead of a 4 mol/L solution of hydrogen chloride in 1,4-dioxane.
yield: 26%
$^1H$ NMR (DMSO-$d_6$) δ (ppm): 8.00 (1H, d, J = 1.4 Hz), 7.86 (1H, dd, J = 2.8, 1.4 Hz), 7.61 (1H, d, J = 2.8 Hz), 6.94 (1H, t, J = 6.6 Hz), 6.58 (2H, s), 4.72 (1H, dd, J = 6.9, 4.2 Hz), 3.83-3.29 (7H, m), 2.26-1.90 (4H, m), 1.07 (6H, s).
APCIMS (m/z): 303 $(M + H)^+$

Example 29: (S)-1-[2-Methyl-1-(5-nitro-2-pyridylamino)-2-propylamino]acetyl-2-pyrrolidinecarbonitrile L-tartrate (Compound 217)

(1) To a solution of 2-amino-2-methyl-N-(5-nitro-2-pyridyl) propylamine (1.26 g, 6.00 mmol) obtained in the method described in Reference example 32 in THF (14 mL) was added (S)-1-bromoacetyl-2-pyrrolidinecarbonitrile (434 mg, 2.00 mmol) described in the U.S. Patent No. 6,011,155 at room temperature, and the mixture was stirred at the same temperature for 4 hours. After the reaction mixture was added with chloroform, the organic layer was washed with water and dried over anhydrous magnesium sulfate. The solvent was evaporated under reduced pressure. The obtained residue was purified by silica gel column chromatography (chloroform/methanol= 100/0 to 90/10) to obtain a free form of the title compound (489 mg, 1.41 mmol).
(2) To a solution of the free form (489 mg, 1.41 mmol) obtained in (1) in methanol (3 mL) was added L-tartaric acid (212 mg, 1.41 mmol). The methanol was evaporated under reduced pressure. The obtained residue was washed with 2-propanol to obtain the title compound (606 mg, 1.22 mmol) as green crystals.
yield: 61%
$^1H$ NMR (DMSO-$d_6$) δ (ppm) : 8.87 (1H, d, J = 3.0 Hz), 8.18-8.03 (2H, m), 6.71 (1H, d, J = 9.2 Hz), 4.75 (1H, dd, J = 6.8, 4.3 Hz), 4.16 (2H, s), 3.66-3.40 (7H, m), 2.25-1.91 (4H, m), 1.16 (6H, s).
APCIMS (m/z): 347 $(M + H)^+$

Example 30: (S)-1-[2-Methyl-1-(2-pyridylamino)-2-propylamino]acetyl-2-pyrrolidinecarbonitrile fumarate (Compound 218)

The title compound (268 mg, 0.642 mmol) was obtained in a similar manner to that of Example 29 by using 2-amino-2-methyl-N-(2-pyridyl)propylamine (744 mg, 4.50 mmol)) obtained by the method described in Reference example 33 instead of 2-amino-2-methyl-N-(5-nitro-2-pyridyl)propylamine, and by using fumaric acid instead of L-tartaric acid.
yield: 43%
$^1H$ NMR (DMSO-$d_6$) δ (ppm): 7.91 (1H, d, J = 5.1 Hz), 7.35 (1H, dd, J = 7.0, 6.5 Hz), 6.56-6.43 (3H, m), 6.56 (2H, s),

4.74 (1H, dd, J = 6.7, 4.4 Hz), 3.55-3.28 (5H, m), 3.28 (2H, d, J = 5.2 Hz), 2.24-1.87 (4H, m), 1.10 (6H, s).
APCIMS (m/z): 302 $(M + H)^+$

Example 31: (S)- 1-[2-Methyl- 1-(5-trifluoromethyl-2-pyridylamino)-2-propylamino]acetyl-2-pyrrolidinecarbonitrile fumarate (Compound 219)

The title compound (548 mg, 1.33 mmol) was obtained in a similar manner to that of Example 29 by using 2-amino-2-methyl-N-(5-trifluoromethyl-2-pyridyl) propylamine (1.05 g, 4.50 mmol) obtained by the method described in Reference example 34 instead of 2-amino-2-methyl-N-(5-nitro-2-pyridyl)propylamine, and by using fumaric acid instead of L-tartaric acid.
yield: 89%
$^1$H NMR (DMSO-$d_6$) δ (ppm): 8.24 (1H, d, J = 2.7 Hz), 7.62 (1H, dd, J = 9.0, 2.7 Hz), 7.43 (1H, t, J = 5.7 Hz), 6.70 (1H, d, J = 9.0 Hz), 6.58 (2H, s), 4.73 (1H, dd, J = 6.2, 4.9 Hz), 3.61-3.25 (5H, m), 3.37 (2H, d, J = 5.7 Hz), 2.17-1.95 (4H, m), 1.09 (6H, s).
APCIMS (m/z): 370 (M + H)+

Example 32: (S)- 1-[1-(3,5-Dichloro-2-pyridylamino)-2-methyl-2-propylamino]acetyl-2-pyrrolidinecarbonitrile fumarate (Compound 220)

The title compound (475 mg, 0.977 mmol) was obtained in a similar manner to that of Example 29 by using 2-amino-N-(3,5-dichloro-2-pyridyl)-2-methylpropylamine (1.05 g, 4.50 mmol) obtained by the method described in Reference example 35 instead of 2-amino-2-methyl-N-(5-nitro-2-pyridyl)propylamine, and by using fumaric acid instead of L-tartaric acid.
yield: 65%
$^1$H NMR (DMSO-$d_6$) δ (ppm): 7.98 (1H, d, J = 2.4 Hz), 7.83 (1H, d, J = 2.4 Hz), 6.58 (2H, s), 6.48 (1H, t, J = 5.4 Hz), 4.77 (1H, dd, J = 6.5, 4.5 Hz), 3.68-3.21 (5H, m), 3.40 (2H, d, J = 5.4 Hz), 2.25-2.01 (4H, m), 1.10 (6H, s).
APCIMS (m/z): 370 $(^{35}Cl^{35}ClM + H)^+$, 372 $(^{35}Cl^{37}ClM + H)^+$, 374 $(^{37}Cl^{37}ClM + H)^+$

Example 33: (S)-1-[1-(5-Carbamoyl-2-pyridylamino)-2-methyl-2-propylamino]acetyl-2-pyrrolidinecarbonitrile (Compound 221)

The title compound was obtained in a similar manner to that of Example 29 (1) by using 2-amino-N-(5-carbamoyl-2-pyridyl)-2-methylpropylamine obtained by the method described in Reference example 36 instead of 2-amino-2-methyl-N-(5-nitro-2-pyridyl)propylamine.
yield: 43%
$^1$H NMR (DMSO-$d_6$) δ (ppm) : 8.47 (1H, d, J = 2.3 Hz), 7.80 (1H, dd, J = 8.9, 2.3 Hz), 7.08 (1H, br s), 7.01 (1H, br s), 6.53 (2H, s), 4.73 (1H, m), 3.82-3.27 (6H, m), 2.19-1.81 (4H, m), 1.12 (6H, s).
APCIMS (m/z): 345 $(M + H)^+$

Example 34: (S)-1-[1-(3-Cyano-2-pyrazinylamino)-2-methyl-2-propylamino]acetyl-2-pyrrolidinecarbonitrile fumarate (Compound 222)

The title compound (145 mg, 0.327 mmol) was obtained in a similar manner to that of Example 29 by using 2-amino-N-(3-cyano-2-pyrazinyl)-2-methylpropylamine (860 mg, 4.50 mmol) obtained by the method described in Reference example 37 instead of 2-amino-2-methyl-N-(5-nitro-2-pyridyl)propylamine, and by using fumaric acid instead of L-tartaric acid.
yield: 22%
$^1$H NMR (DMSO-$d_6$) δ (ppm): 8.29 (1H, d, J = 2.4 Hz), 7.90 (1H, d, J = 2.4 Hz), 7.21 (1H, br s), 6.60 (2H, s), 4.75 (1H, dd, J = 6.8, 4.3 Hz), 3.66-3.17 (7H, m), 2.24-2.04 (4H, m), 1.09 (6H, s).
APCIMS (m/z): 328 $(M + H)^+$

Example 35: (S)-1-{1-[5-(N,N-Dimethylaminocarbonyl)-2-pyridylamino]-2-methyl-2-propylamino}acetyl-2-pyrrolidinecarbonitrile fumarate (Compound 223)

(1) To a solution of 2-amino-N-[5-(N,N-dimethylaminocarbonyl)-2-pyridyl]-2-methylpropylamine (532 mg, 2.25 mmol) obtained by the

method described in Reference example 38 and basic almina (Merck, Aluminium oxide 90 active basic 0.063-0.200 mm, 326 mg) in N,N-dimethylformamide (10 mL) was added (S)-1-bromoacetyl-2-pyrrolidinecarbonitrile (326 mg, 1.50 mmol) described in the U.S. Patent No. 6,011,155 at 0 °C, and the mixture was stirred at room temperature for 1.5 hours. After the reaction mixture was filtered using Celite as filtration aid, the reaction mixture was added with ethyl acetate. The organic layer was washed with water and dried over anhydrous magnesium sulfate. The solvent was evaporated under reduced pressure. The residue obtained was purified by silica gel column chromatography (chloroform/methanol=100/0 to 90/10) to obtain a free form of the title compound (37.0 mg, 0.0993 mmol).
(2) To a solution of the free compound (37.0 mg, 0.0993 mmol) obtained in (1) in methanol (1 mL) was added fumaric acid (11.5 mg, 0.0993 mmol). The methanol was evaporated under reduced pressure to obtain the title compound (23.0 mg, 0.0471 mmol) as colorless crystals.
yield: 3%
$^1$H NMR (DMSO-$d_6$) δ (ppm): 8.08 (1H, d, J = 2.2 Hz), 7.49 (1H, dd, J = 8.6, 2.2 Hz), 7.09 (1H, t, J = 6.2 Hz), 6.63 (1H, d, J = 8.6 Hz), 6.57 (2H, s), 4.77 (1H, dd, J = 7.0, 4.3 Hz), 3.80-3.20 (5H, m), 3.43 (2H, d, J = 6.2 Hz), 2.97 (6H, s), 2.25-1.86 (4H, m), 1.18 (6H, s).
APCIMS (m/z): 373 $(M + H)^+$

Example 36: (S)-1-[1-(5-Chloro-2-pyridylamino)-2-methyl-2-propylamino]acetyl-2-pyrrolidinecarbonitrile fumarate (Compound 224)

The title compound (60.0 mg, 0.133 mmol) was obtained in a similar manner to that of Example 35 by using 2-amino-N-(5-chloro-2-pyridyl)-2-methylpropylamine (163 mg, 0.816 mmol) obtained by the method described in Reference example 39 instead of 2-amino-N-[5-(N,N-dimethylaminocarbonyl)-2-pyridyl]-2-methylpropylamine.
yield: 24%
$^1$H NMR (DMSO-$d_6$) δ (ppm) : 7.92 (1H, d, J = 2.7 Hz), 7.45 (1H, dd, J = 8.9 Hz, 2.7 Hz), 6.99 (1H, t, J = 5.1 Hz), 6.62 (1H, d, J = 8.9 Hz), 6.56 (2H, s), 4.78 (1H, dd, J = 6.2, 4.6 Hz), 3.82-3.25 (5H, m), 3.40 (2H, d, J = 5.1 Hz), 2.25-1.90 (4H, m), 1.18 (6H, s)
APCIMS (m/z): 336 $(^{35}ClM + H)^+$, 338 $(^{37}ClM + H)^+$

Example 37: (S)-1-[2-Methyl-1-(2-pyrimidinylamino)-2-propylamino]acetyl-2-Pyrrolidinecarbonitrile fumarate (Compound 225)

(1) To solution of 2-amino-2-methyl-N- (2-pyrimidinyl)propylamine (374 mg, 2.25 mmol) obtained by the method described in Reference example 40 and potassium fluoride (50 weight % on Celite, 872 mg, 7.50 mmol) in N,N-dimethylformamide (6 mL) was added (S)-1-bromoacetyl-2-pyrrolidinecarbonitrile (326 mg, 1.50 mmol) described in the U.S. Patent No. 6,011,155 at 0 °C, and the mixture was stirred at the same temperature for 3 hours. After the reaction mixture was filtered using Celite as a filtration aid, the reaction mixture was added with ethyl acetate. The organic layer was washed with aqueous saturated sodium hydrogencarbonate solution, and dried over anhydrous magnesium sulfate. The solvent was evaporated under reduced pressure. The obtained residue was purified by silica gel column chromatography (chloroform/methanol= 100/0 to 90/10) to obtain a free form of the title compound (347 mg, 1.96 mmol).
(2) To a solution of the free compound (347 mg, 1.96 mmol) obtained in (1) in methanol (3 mL) was added fumaric acid (227 mg, 1.96 mmol). The methanol was evaporated under reduced pressure to obtain the title compound (359 mg, 0.858 mmol) as colorless crystals.
yield: 57%
$^1$H NMR (DMSO-$d_6$) δ (ppm): 7.35 (1H, t, J = 6.4 Hz), 6.56 (2H, s), 6.44 (2H, d, J = 6.4 Hz), 4.74 (1H, dd, J = 6.8, 4.4 Hz), 3.65-3.16 (8H, m), 2.24-1.87 (4H, m), 1.10 (6H, s).
APCIMS (m/z): 303 $(M + H)^+$

Example 41: (S)-1-[2-Methyl-1-(2-thiazolylamino)-2-propylamino]acetyl-2-pyrrolidinecarbonitrile fumarate (Compound 226)

The title compound (157 mg, 0.371 mmol) was obtained as a white solid in a similar manner to that of Example 37 from 2-amino-2-methyl-N-(2-thiazolyl) propylamine (410 mg, 2.40 mmol) obtained in Reference example 41.
yield: 15%
$^1$H NMR (DMSO-$d_6$) δ (ppm): 6.98 (1H, d, J = 3.8 Hz), 6.61 (1H, d, J = 3.8 Hz), 6.56 (2H, s), 4.78 (1H, dd, J = 6.5, 1.9 Hz), 3.79-3.33 (6H, m), 2.24-1.99 (4H, m), 1.18 (6H, s).

APCIMS (m/z) : 308 $(M + H)^+$

Example 42: (S)-1-[2-Methyl-1-(1,3,4-thiadiazol-2-ylamino)-2-propylamino]acetyl-2-pyrrolidinecarbonitrile fumarate (Compound 227)

The title compound (730 mg, 1.72 mmol) was obtained as a white solid in a similar manner to that of Example 37 from 2-amino-2-methyl-N-(1,3,4-thiadiazol-2-yl) propylamine (632 mg, 3.67 mmol) obtained in Reference example 42.
yield: 47%
$^1H$ NMR (DMSO-$d_6$) δ (ppm): 8.60 (1H, s), 6.57 (2H, s), 4.76 (1H, dd, J = 6.8, 4.3 Hz), 3.76-3.42 (6H, m), 2.22-2.02 (4H, m), 1.24 (6H, s).
APCIMS (m/z): 309 $(M + H)^+$

Example 43: (S)-1-[1-(5-Cyano-2-thiazolylamino)-2-methyl-2-propylamino]acetyl-2-pyrrolidinecarbonitrile fumarate (Compound 228)

The title compound (872 mg, 1.95 mmol) was obtained as a white solid in a similar manner to that of Example 37 from 2-amino-N-(5-cyano-2-thiazolyl)-2-methylpropylamine (581 mg, 2.96 mmol) obtained in Reference example 43.
yield: 66%
$^1H$ NMR (DMSO-$d_6$) δ (ppm): 7.86 (1H, d, J = 2.7 Hz), 6.59 (2H, s), 4.74 (1H, dd, J = 6.8, 4.6 Hz), 3.61-3.37 (6H, m), 2.20-2.01 (4H, m), 1.09 (6H, s).
APCIMS (m/z): 333 $(M + H)^+$

Example 44: (S)-1-[2-Methyl-1-(4-phenyl-2-thiazolylamino)-2-propylamino]acetyl-2-pyrrolidinecarbonitrile fumarate (Compound 229)

The title compound (1.28 g, 0.257 mmol) was obtained as a white solid in a similar manner to that of Example 37 from 2-amino-2-methyl-N-(4-phenyl-2-thiazolyl) propylamine (741 mg, 3.00 mmol) obtained in Reference example 44.
yield: 86%
$^1H$ NMR (DMSO-$d_6$) δ (ppm): 7.82 (2H, d, J = 7.6 Hz), 7.56 (1H, m), 7.36 (2H, d, J = 7.6 Hz), 7.25 (1H, m), 7.01 (1H, s), 6.58 (2H, s), 4.66 (1H, t, J = 5.4 Hz), 3.62-3.27 (6H, m), 2.09-1.94 (4H, m), 1.12 (6H, s).
APCIMS (m/z): 384 $(M + H)^+$

Example 45: (S)-1-{ 1-[5-(N,N-Dimethylaminosulfonyl)-4-methyl-2-thiazolylamino]-2-methyl-2-propylamino)acetyl-2-pyrrolidinecarbonitrile fumarate (Compound 230)

The title compound (745 mg, 1.37 mmol) was obtained as a white solid in a similar manner to that of Example 37 from 2-amino-N-[5-(N,N-dimethyl--4-methyl-2-thiazolyl-2-methylpropylamine (645 mg, 2.21 mmol) obtained in Reference example 45.
yield: 62%
$^1H$ NMR (DMSO-$d_6$) δ (ppm): 8.43 (1H, br s), 6.58 (2H, s), 4.76 (1H, dd, J = 5.7, 4.3 Hz), 3.67-3.30 (6H, m), 2.67 (6H, s), 2.33 (3H, s), 2.07-1.98 (4H, m), 1.14 (6H, s).
APCIMS (m/z): 429 $(M + H)^+$

Example 46: (S)-1-[2-Methyl- 1-(5-methyl-2-thiazolylamino)-2-propylamino]acetyl-2-pyrrolidinecarbonitrile fumarate (Compound 231)

The title compound (378 mg, 0.865 mmol) was obtained as a white solid in a similar manner to that of Example 37 from 2-amino-2-methyl-N-(5-methyl-2-thiazolyl) propylamine (500 mg, 2.70 mmol) obtained in Reference example 46.
yield: 32%
$^1H$ NMR (DMSO-$d_6$) δ (ppm): 6.63 (1H, d, J = 1.6 Hz), 6.56 (2H, s), 4.77 (1H, dd, J = 6.5, 4.6 Hz), 3.72-3.25 (6H, m), 2.19-2.01 (4H, m), 2.19 (3H, s), 1.14 (6H, s).
APCIMS (m/z): 322 $(M + H)^+$

Example 47: (S)- 1-[4-Methyl- 1-(2-pyrazinyl)-4-piperidylamino]acetyl-2-pyrrolidinecarbonitrile dihydrochloride (Compound 232)

The title compound was obtained in a similar manner to that of Example 54 described later by using 4-amino-4-methyl-1-{2-pyrazinyl)piperidine obtained in Reference example 47 instead of 4-amino-1-(5-cyano-2-pyridyl)-4-ethylpiperidine.
yield: 25%
$^1$H NMR (DMSO-$d_6$) δ (ppm): 9.13 (1H, br s), 9.05 (1H, br s), 8.40 (1H, d, J = 1.4 Hz), 8.16 (1H, dd, J = 2.7, 1.4 Hz), 7.84 (1H, d, J = 1.4 Hz), 4.83 (1H, dd, J = 7.0, 4.3 Hz), 4.30 (2H, d, J = 13.5 Hz), 4.10-3.90 (2H, m), 3.70-3.60 (1H, m), 3.53 (2H, m), 3.15-3.00 (2H, m), 2.25-2.10 (2H, m), 2.10-1.95 (2H, m), 1.95-1.75 (4H, m), 1.44 (3H, s).
APCIMS (m/z): 329 $(M + H)^+$

Example 48: (S)-1-[4-Methyl-1-(2-pyrimidinyl)-4-piperidylamino]acetyl-2-pyrrolidinecarbonitrile (Compound 233)

The title compound was obtained in a similar manner to that of Example 52 described later by using 4-amino-4-methyl-1-(2-pyrimidinyl)piperidine obtained in Reference example 48 instead of 4-amino-4-methyl-1-(5-phenyl-2-pyridyl)piperidine.
yield: 29%
$^1$H NMR ($CDCl_3$) δ (ppm) : 8.28 (2H, d, J = 4.6 Hz), 6.44 (1H, t, J = 4.6 Hz), 4.78 (1H, d, J = 7.0 Hz), 3.95-3.75 (4H, m), 3.75-3.60 (1H, m), 3.60-3.40 (1H, m), 3.40 (2H, d, J = 1.1 Hz), 2.40-2.05 (4H, m), 1.70-1.50 (5H, m), 1.15 (3H, s).
APCIMS (m/z): 329 $(M + H)^+$

Example 49: (S)-1-[4-Methyl-1-(4-pyrimidinyl)-4-piperidylamino]acetyl-2-pyrrolidinecarbonitrile (Compound 234)

The title compound was obtained in a similar manner to that of Example 52 described later by using 4-amino-4-methyl-1-(4-pyrimidinyl)piperidine obtained in Reference example 49 instead of 4-amino-4-methyl-1-(5-phenyl-2-pyridyl)piperidine.
yield: 8%
$^1$H NMR ($CDCl_3$) δ (ppm): 8.57 (1H, d, J = 1.1 Hz), 8.16 (1H, d, J = 6.5 Hz), 6.49 (1H, dd, J = 6.5, 1.1 Hz), 4.78 (1H, d, J = 6.8 Hz), 3.85-3.40 (6H, m), 3.37 (2H, d, J = 1.6 Hz), 2.30-2.10 (4H, m), 1.60-1.40 (5H, m), 1.14 (3H, s).
APCIMS (m/z): 329 $(M + H)^+$

Example 50: (S)-1-[4-Methyl-1-(5-trifluoromethyl-2-pyridyl)-4-piperidylamino]acetyl - 2-pyrrolidinecarbonitrile dihydrochloride (Compound 235)

The title compound was obtained in a similar manner to that of Example 19 (3) and (4) by using 4-amino-4-methyl-1-(5-trifluoromethyl-2-pyridyl)piperidine obtained in Reference example 50 instead of 4-amino-1-(5-cyano-2-pyridyl)-4-methylpiperidine, and by using N,N-dimethylformamide as a solvent in the process of condensation with (S)-1-bromoacetyl-2-pyrrolidinecarbonitrile.
$^1$H NMR (DMSO-$d_6$) δ (ppm): 9.09 (2H, br s), 8.41 (1H, d, J = 2.7 Hz), 7.83 (1H, dd, J = 9.2, 2.7 Hz), 7.04 (1H, d, J = 9.2 Hz), 4.83 (1H, dd, J = 6.6, 4.2 Hz), 4.37 (2H, m), 4.15-3.90 (2H, m), 3.70-3.60 (1H, m), 3.60-3.40 (2H, m), 3.15-2.95 (2H, m), 2.25-2.10 (2H, m), 2.05-1.95 (2H, m), 1.90-1.75 (4H, m), 1.45 (3H, s).
APCIMS (m/z): 395 $(M + H)^+$

Example 51: (S)-1-[1-(5-Chloro-2-pyridyl)-4-methyl-4-piperidylamino]acetyl-2-pyrrolidinecarbonitrile dihydrochloride (Compound 236)

(1) To solution of 4-amino-1-(5-chloro-2-pyridyl)- 4-methylpiperidine (310 mg, 1.37 mmol) obtained in Reference example 51 in acetonitrile (10 mL) were added potassium fluoride (50 weight % on Celite, 580 mg, 5.00 mmol) and (S)-1-bromoacetyl-2-pyrrolidinecarbonitrile (217 mg, 1.00 mmol) described in the U.S. Patent No. 6,011,155 under ice-cooling, and the mixture was stirred at room temperature overnight. The reaction mixture was filtered and the filtrate was concentrated. The obtained residue was purified by silica gel column chromatography to obtain a free form of the title compound (362 mg, 1.00 mmol).
(2) To a solution of the free form obtained in (1) in 1,4-dioxane (10 mL) was added a 4 mol/L solution of hydrogen chloride in 1,4-dioxane (5 mL) under ice-cooling, and the mixture was stirred at room temperature for 10 minutes. The solvent was evaporated under reduced pressure. Crystallization from ethanol-diethyl ether gave the title compound (330 mg, 0.759 mmol) as colorless crystals.
yield: 55%
$^1$H NMR (DMSO-$d_6$) δ (ppm): 9.06 (2H, br s), 8.11 (1H, d, J = 2.7 Hz), 7.65 (1H, dd, J = 9.2, 2.7 Hz), 6.97 (1H, d, J = 9.2 Hz), 4.83 (1H, dd, J = 7.0, 4.6 Hz), 4.21 (2H, m), 4.10-3.90 (2H, m), 3.75-3.65 (1H, m), 3.65-3.50 (2H, m), 3.05-2.95 (2H, m), 2.24-2.10 (2H, m), 2.05-1.95 (2H, m), 1.90-1.75 (4H, m), 1.42 (3H, s).
APCIMS (m/z): 362 ($^{35}$ClM + H)$^+$, 364 ($^{37}$ClM + H)$^+$

Example 52: (S)-1-[4-Methyl-1-(5-phenyl-2-pyridyl)-4-piperidylamino]acetyl-2-pyrrolidinecarbonitrile (Compound 237)

To a solution of 4-amino-4-methyl-1-(5-phenyl-2-pyridyl)piperidine (1.25 g, 4.68 mmol) obtained in Reference example 53 in N,N-dimethylformamide (15 mL) was added (S)-1-bromoacetyl-2-pyrrolidinecarbonitrile (326 mg, 1.50 mmol) described in the U.S. Patent No. 6,011,155, and the mixture was stirred at room temperature overnight. After the reaction mixture was concentrated, the obtained residue was purified by silica gel column chromatography. Crystallization from hexane-ethyl acetate gave the title compound (556 mg, 1.38 mmol).
yield: 29%
$^1$H NMR ($CDCl_3$) δ (ppm): 8.43 (1H, d, J = 2.7 Hz), 7.70 (1H, dd, J = 8.9, 2.7 Hz), 7.53 (2H, d, J = 7.6 Hz), 7.41 (2H, dd, J = 7.6, 7.6 Hz), 7.30-7.27 (1H, m), 6.73 (1H, d, J = 8.9 Hz), 4.90 (1H, d, J = 8.1 Hz), 3.80-3.50 (6H, m), 3.50-3.30 (2H, m), 2.40-2.10 (4H, m), 1.80-1.50 (5H, m), 1.16 (3H, s).
APCIMS (m/z): 404 (M + H)$^+$

Example 53: (S)-1-[4-Methyl- 1 -(2-pyridyl)-4-piperidylamino]acetyl-2-pyrrolidinecarbonitrile dihydrochloride (Compound 238)

The title compound was obtained in a similar manner to that of Example 51 by using 4-amino-4-methyl-1-(2-pyridyl)piperidine obtained in Reference example 52 instead of 4-amino-1-(5-chloro-2-pyridyl)-4-methylpiperidine, and by using N,N-dimethylformamide as a solvent in the process of condensation reaction with (S)-1-bromoacetyl-2-pyrrolidinecarbonitrile.
yield: 48%
$^1$H NMR (DMSO-$d_6$) δ (ppm): 9.26 (1H, br s), 9.15 (1H, br s), 8.10-7.90 (2H, m), 7.42 (1H, d, J = 8.9 Hz), 6.97 (1H, dd, J = 6.8, 6.8 Hz), 4.83 (1H, dd, J = 7.0, 4.3 Hz), 4.38 (2H, d, J = 13.2 Hz), 4.15-3.88 (2H, m), 3.80-3.70 (1H, m), 3.70-3.50 (2H, m), 3.20-3.05 (2H, m), 2.25-2.10 (2H, m), 2.10-1.95 (2H, m), 1.95-1.80 (4H, m), 1.45 (3H, s).
APCIMS (m/z): 328 (M + H)$^+$

Example 54: (S)- 1 -[1 -(5-Cyano-2-pyridyl)-4-ethyl-4-piperidylamino]acetyl-2-pyrrolidinecarbonitrile dihydrochloride (Compound 239)

(1) To a solution of 4-amino-1-(5-cyano-2- pyridyl)-4-ethylpiperidine (485 mg, 2.11 mmol) obtained in Reference example 54 in N,N-dimethylformamide (10 mL) were added cesium hydroxide monohydrate (177 mg, 1.05 mmol) and (S)-1-bromoacetyl-2-pyrrolidinecarbonitrile (152 mg, 0.700 mmol) described in the U.S. Patent No. 6,011,155, and the mixture was stirred at room temperature for 4 hours. After the reaction mixture was concentrated, water was added to the reaction mixture, and the mixture was extracted with chloroform three times. The combined organic layer was washed with saturated brine, dried over anhydrous magnesium sulfate, and concentrated. The obtained residue was purified by silica gel column chromatography to obtain a free form of the title compound (256 mg, 0.700 mmol).
(2) To a solution of the free form (256 mg, 0.700 mmol) obtained in (1) in THF (30 mL) was added a 4 mol/L solution of hydrogen chloride in 1,4-dioxane (2 mL) under ice-cooling, and the mixture was stirred at room temperature for 10 minutes. Diethylether was added to the reaction mixture, and the deposited precipitate was collected by filtration to obtain the title compound (300 mg, 0.683 mmol) as colorless crystals.
yield: 32%
$^1$H NMR (DMSO-$d_6$) δ (ppm): 8.85 (2H, br s), 8.50 (1H, d, J = 2.2 Hz), 7.87 (1H, dd, J = 8.9, 2,2 Hz), 6.97 (1H, d, J = 8.9 Hz), 4.83 (1H, dd, J = 7.0, 4.9 Hz), 4.27 (2H, d, J = 14.0 Hz), 4,05-3.85 (2H, m), 3.80-3.70 (1H, m), 3.70-3.50 (2H, m), 3.25-3.05 (2H, m), 2.18 (2H, dd, J = 9.5, 6.5 Hz), 2.10-2.00 (2H, m), 1.95-1.75 (6H, m), 0.91 (3H, t, J = 7.2 Hz).
APCIMS (m/z): 367 (M + H)$^+$

Example 55: (S)- 1-[1-(5-Cyano-2-pyridyl)-4-phenyl-4-piperidylamino]acetyl-2-pyrrolidinecarbonitrile dihydrochloride (Compound 240)

The title compound was obtained in a similar manner to that of Example 54 by using 4-amino-1-(5-cyano-2-pyridyl)-4-phenylpiperidine obtained in Reference example 55 instead of 4-amino-1-(5-cyano-2-pyridyl)-4-ethylpiperidine.
$^1$H NMR (DMSO-$d_6$) δ (ppm): 9.51 (2H, br s), 8.49 (1H, d, J = 2.2 Hz), 7.87 (1H, dd, J = 9.2, 2.2 Hz), 7.75 (2H, d, J = 7.3 Hz), 7.55-7.40 (3H, m), 6.98 (1H, d, J = 9.2 Hz), 4.68 (1H, dd, J = 6.2, 4.1 Hz), 4.44 (2H, d, J = 12.4 Hz), 3.75-3.65 (2H, m), 3.60-3.40 (2H, m), 3.27 (1H, m), 2.95-2.65 (4H, m), 2.30-2.15 (2H, m), 2.10-2.05 (2H, m), 2.00-1.80 (2H, m).
FABMS (m/z): 416 $(M + 2H)^+$

Example 56: (S)-1-[1-(5-Methoxycarbonyl-2-pyridylamino)-2-methyl-2-propylamino] acetyl-2-pyrrolidinecarbonitrile fumarate (Compound 241)

(1) To a solution of 2-amino-N-(5-methoxycarbonyl-2-pyridyl)-2-methylpropylamine (335 mg, 1.50 mmol) obtained in Reference example 56 and potassium fluoride (spray dried) (174 mg, 3.00 mmol) in N,N-dimethylformamide (4 mL) was added (S)-1-bromoacetyl-2-pyrrolidinecarbonitrile (217 mg, 1.00 mmol) described in the U.S. Patent No. 6,011,155 at 0°C, and the mixture was stirred at the same temperature for 4 hours. After the reaction mixture was filtered with Celite as a filtration aid, ethyl acetate was added to the reaction mixture, and the organic layer was washed with aqueous saturated sodium hydrogencarbonate solution and dried over anhydrous magnesium sulfate. The solvent was evaporated under reduced pressure, and the obtained residue was purified by silica gel column chromatography (chloroform/methanol= 100/0 to 90/10) to obtain a free form of the title compound (301 mg, 0.837 mmol).

(2) To a solution of the free form (301 mg, 0.837 mmol) obtained in (1) in methanol (3 mL) was added fumaric acid (97.0 mg, 0.837 mmol). The methanol was evaporated under reduced pressure to obtain the title compound (332 mg, 0.698 mmol) as colorless crystals.
yield: 70%
$^1$H NMR (DMSO-$d_6$) δ (ppm): 8.52 (1H, d, J = 2.2 Hz), 7.80 (1H, dd, J = 8.8, 2.2 Hz), 7.32 (1H, br s), 6.62 (1H, d, J = 8.8 Hz), 6.58 (2H, s), 4.72 (1H, dd, J = 6.2 Hz, 4.6 Hz), 3.76 (3H, s), 3.68-3.33 (7H, m), 2.27-1.93 (4H, m), 1.09 (6H, s).

APCIMS (m/z): 360 $(M + H)^+$

Example 57: (S)-1-[2-Methyl-1-(5-methyl-2-pyridylamino)-2-propylamino]acetyl-2-pyrrolidinecarbonitrile fumarate (Compound 242)

The title compound (265 mg, 0.614 mmol) was obtained in a similar manner to that of Example 56 by using 2-amino-2-methyl-N-(5-methyl-2-pyridyl)propylamine (269 mg, 1.50 mmol) obtained in Reference example 57 instead of 2-amino-N-(5-methoxycarbonyl-2-pyridyl)-2-methylpropylamine.
yield: 61%
$^1$H NMR (DMSO-$d_6$) δ (ppm): 7.75 (1H, d, J = 2.2 Hz), 7.22 (1H, dd, J = 8.6, 2.2 Hz), 6.55 (2H, s), 6.50 (1H, d, J = 8.6 Hz), 6.30 (1H, t, J = 5.4 Hz), 4.75 (1H, dd, J = 6.5, 4.6 Hz), 3.66-3.36 (5H, m), 3.27 (2H, d, J = 5.4 Hz), 2.18-1.98 (4H, m), 2.08 (3H, s), 1.10 (6H, s).
APCIMS (m/z): 316 $(M + H)^+$

Example 58: (S)-1-[1-(5-Isopropyl-2-pyridylamino)-2-methyl-2-propylamino]acetyl-2-pyrrolidinecarbonitrile fumarate (Compound 243)

The title compound (66.0 mg, 0.142 mmol) was obtained in a similar manner to that of Example 56 by using 2-amino-N-(5-isopropyl-2-pyridyl)-2-methylpropylamine (74.0 mg, 0.357 mmol) obtained in Reference example 58 instead of 2-amino-N-(5-methoxycarbonyl-2-pyridyl)-2-methylpropylamine.
yield: 60%
$^1$H NMR (DMSO-$d_6$) δ (ppm): 7.80 (1H, d, J = 2.3 Hz), 7.32 (1H, dd, J = 8.6, 2.3 Hz), 6.55 (2H, s), 6.54 (1H, d, J = 8.6 Hz), 6.43 (1H, br s), 4.77 (1H, dd, J = 6.8, 4.6 Hz), 3.74-3.37 (5H, m), 3.30 (2H, d, J = 3.2 Hz), 2.78-2.68 (1H, m), 2.20-1.94 (4H, m), 1.15 (6H, d, J = 6.8 Hz), 1.14 (6H, s).
APCIMS (m/z): 344 $(M + H)^+$

Example 59: (S)-2-(2-Cyano-1-pyrrolidinyl)-N-[2-(5-nitro-2-pyridylamino)ethyl] acetamide (Compound 301)

To a solution of (S)-2-pyrrolidinecarbonitrile hydrochloride (0.10 g, 0.75 mmol) known in the literature [Bull. Chem. Soc. Jpn., 50, 1956-1960 (1977)] in 1,4-dioxane (10 mL) were added potassium carbonate (0.22 g, 1.66 mmol), potassium iodide (0.25 g, 1.51 mmol), and 2-chloro-N-[2-(5-nitro-2-pyridylamino)ethyl]acetamide (0.23 g, 0.89 mmol) obtained in Reference example 18, and the mixture was heated with stirring at 80°C for 4 hours. After the reaction mixture was stand for cooling, saturated brine was added to the reaction mixture, and the mixture was extracted with chloroform, which was then dried over anhydrous sodium sulfate.

After the solvent was evaporated, the residue was purified by preparative thin layer chromatography to obtain the title compound (34.9 mg, 0.11 mmol).
yield: 15%
$^1$H NMR ($CDCl_3$) δ (ppm): 9.00 (1H, d, J = 2.6 Hz), 8.13 (1H, dd, J = 9.2, 2.6 Hz), 6.41 (1H, d, J = 9.2 Hz), 6.36 (1H, br), 4.89 (1H, m), 3.79-3.68 (2H, m), 3.66-3.55 (3H, m), 3.49-3.37 (1H, m), 3.31-3.19 (2H, m), 2.59 (1H, m), 2.19-2.06 (2H, m), 1.94-1.75 (2H, m). APCIMS (m/z) : 317 (M - H)$^+$

Example 60: (S)-1-[2-(3-Cyano-2-pyridylamino)ethoxy]acetyl-2-pyrrolidinecarbonitrile (Compound 302)

To a solution of 2-(3-cyano-2-pyridylamino)ethanol (0.20 g, 1.23 mmol) obtained by the method described in Reference example 14 in THF (10 mL) was added sodium hydride (60% dispersion in mineral oil, 98.2 mg, 2.46 mmol) under ice-cooling, and the mixture was stirred at the same temperature for one hour. To the reaction mixture added dropwise a solution of (S)-bromoacetyl-2-pyrrolidinecarbonitrile (265 mg, 1.23 mmol) in THF (5 mL), and the mixture was stirred at the same temperature for 3 hours. A small amount of water was added to the reaction mixture, which was then extracted with methylene chloride, and dried over anhydrous sodium sulfate. The solvent was evaporated and the residue was purified by silica gel column chromatography to obtain the title compound (172 mg, 0.58 mmol).
yield: 47%
$^1$H NMR ($CDCl_3$) δ (ppm): 8.26 (1H, dd, J = 4.9, 1.8 Hz), 7.65 (1H, dd, J = 7.6, 1.8 Hz), 6.60 (1H, dd, J = 7.6, 4.9 Hz), 5.94 and 5.87 (1H, br), 4.89 and 4.79 (1H, m), 4.29 and 4.19 (2H, s), 3.83-3.71 (4H, m), 3.63 and 3.48 (2H, m), 2.39-2.02 (4H, m).
APCIMS (m/z): 300 (M + H)$^+$

Example 61: (S)-2-Cyano-N-[2-(5-nitro-2-pyridylamino)ethyl]- 1-pyrrolidinecarboxamide (Compound 303)

To a solution of triphosgene (81 mg, 0.27 mmol) in methylene chloride (5 mL) was added a solution of (S)-2-pyrrolidinecarbonitrile hydrochloride (73 mg, 0.55 mmol) described above in methylene chloride (5 mL), then triethylamine (0.15 mL, 1.08 mmol). The mixture was stirred at the same temperature for 2.5 hours. To the reaction mixture was added commercially available 2-(2-aminoethylamino)-5-nitropyridine (100 mg, 0.55 mmol) and triethylamine (0.15 mL, 1.08 mmol), and the mixture was stirred at room temperature for 4 hours. To the reaction mixture was added methanol and the solvent was evaporated. The residue was purified by silica gel column chromatography to obtain the title compound (148 mg, 0.49 mmol).
yield: 89%
$^1$H NMR ($CDCl_3$ + methanol-$d_4$) δ (ppm): 8.94 (1H, d, J = 2.6 Hz), 8.14 (1H, dd, J = 9.2, 2.6 Hz), 6.52 (1H, d, J = 9.2 Hz), 4.68 (1H, m), 3.55 (2H, t, J = 5.9 Hz), 3.46-3.35 (3H, m), 3.26 (1H, m), 2.28-2.14 (4H, m).
APCIMS (m/z): 305 (M + H)$^+$
Elemental analysis : Calcd. for $C_{13}H_{16}N_6O_3$ (304.308): C, 51.31; H, 5.30; N, 27.62. Found: C, 51.07; H, 5.33; N, 27.47.

Example 62: (S)-2-Cyano-N-[2-(3-cyano-2-pyridylamino)ethyl]-1-pyrrolidinecarboxamide (Compound 304)

2-(3-Cyano-2-pyridyl)aminoethylamine dihydrochloride was obtained by the treatment described in Reference example 20 (2) with 3-cyano-2-(2-butoxycarbonylaminoethyl)aminopyridine (112 mg, 0.43 mmol) obtained in Reference example 20 (1) as a starting material. To a suspension of the obtained 2-(3-cyano-2-pyridyl)aminoethylamine dihydrochloride in methylene chloride (10 mL) were added triethylamine (0.29 mL, 2.08 mmol) and N,N-carbonyldiimidazole (55.2 mg, 0.34 mmol) under ice-cooling. After stirring at the same temperature for 1.5 hours, the mixture was added dropwise to a mixed-solution of (S)-2-pyrrolidinecarbonitrilehydrochloride (37.6 mg, 0.28 mmol) in methylene chloride (5 mL) and triethylamine (0.12 mL, 0.86 mmol) under ice-cooling. The solution was stirred at room temperature overnight. After the reaction mixture was concentrated, the residue was purified by silica gel column chromatography to obtain the title compound (23.3 mg, 0.08 mmol).
yield: 29%
$^1$H NMR ($CDCl_3$) δ (ppm): 8.24 (1H, dd, J = 4.9, 2.0 Hz), 7.67 (1H, dd, J = 7.6, 2.0 Hz), 6.63 (1H, dd, J = 7.6, 4.9 Hz),

5.85 (1H, br), 5.51 (1H, br), 4.75 (1H, m), 3.70 (2H, m), 3.55-3.51 (2H, m), 3.39 (1H, m), 3.24 (1H, m), 2.27-2.11 (4H, m).
APCIMS (m/z): 285 $(M + H)^+$

Example 63: N-[2-(3-Cyano-2-pyridylamino)ethyl]-1-pyrolidinecarboxamide (Compound 305)

The title compound was obtained in a similar manner to that of Example 62 by using pyrrolidine instead of (S)-2-pyrrolidinecarbonitrile hydrochloride.
yield: 11%
$^1$H NMR ($CDCl_3$) δ (ppm): 8.25 (1H, dd, J = 5.0, 2.0 Hz), 7.67 (1H, dd, J = 7.6, 2.0 Hz), 6.61 (1H, dd, J = 7.6, 5.0 Hz), 3.67 (2H, m), 3.54-3.29 (6H, m), 1.95-1.87 (4H, m).
APCIMS (m/z): 260 $(M + H)^+$

Example 64: (S)-2-Cyano-N-[3-(3-cyano-2-pyridylamino)propyl]-1-pyrrolidinecarboxamide (Compound 306)

The title compound was obtained in a similar manner to that of Example 62 by using 3-(3-cyano-2-pyridylamino)propylamine dihydrochloride obtained by the method described in Reference example 21 instead of 2-(3-cyano-2-pyridyl)aminoethylamine dihydrochloride.
yield: 82%
$^1$H NMR ($CDCl_3$) δ (ppm) : 8.23 (1H, dd, J = 4.9, 1.8 Hz), 7.64 (1H, dd, J = 7.6, 1.8 Hz), 6.59 (1H, dd, J = 7.6, 4.9 Hz), 5.77 (1H, t, J = 5.9 Hz), 5.63 (1H, t, J = 5.6 Hz), 4.78 (1H. m), 3.64 (2H, dt, J = 6.1, 5.9 Hz), 3.52 (1H, m), 3.40-3.24 (3H, m), 2.32-2.15 (4H, m), 1.80 (2H, tt, J = 6.1, 6.1 Hz).

Example 65: (S)- 1-[2-(2-Quinoxalinecarboxamide)ethylamino]acetyl-2-pyrrolidinecarbonitrile dihydrochloride (Compound 401)

(1) To a solution of N-(2-aminoethyl)-2-quinoxalinecarboxamide (860 mg, 3.98 mmol) obtained by the method described in Reference example 59 in N,N-dimethylformamide (10 mL) was added a solution of (S)-1-bromoacetyl-2-pyrrolidinecarbonitrile (345 mg, 1.59 mmol) described in the U.S. Patent No. 6,011,155 in N,N-dimethylformamide (5 mL) at room temperature, and the mixture was stirred at the same temperature for 3 hours. After the solvent was evaporated under reduced pressure, chloroform was added to the residue. The organic layer was washed with water and saturated brine, and dried over anhydrous magnesium sulfate. The solvent was evaporated under reduced pressure, and the obtained residue was purified by column chromatography (chloroform/methanol=8/1) to obtain a free form of the title compound (322 mg, 0.910 mmol).
(2) To a solution of the free form (322 mg, 0.91 mmol) obtained in (1) in methanol (3 mL) was added a 4 mol/L solution of hydrogen chloride in 1,4-dioxane (0.68 mL, 2.73 mmol). The solvent was evaporated under reduced pressure, and the obtained residue was recrystallized from 2-propanol/ethanol to obtain the title compound (145 mg, 0.340 mmol) as colorless crystals.
yield: 21%
$^1$H NMR (DMSO-$d_6$) δ (ppm): 9.51 (1H, s), 9.38-9.30 (1H, m), 8.24-8.20 (2H, m), 8.05-7.98 (2H, m), 4.85 (1H, dd, J = 5.7, 5.7 Hz), 4.17-4.07 (2H, m), 3.78-3.18 (6H, m), 2.26-1.96 (4H, m).
APCIMS (m/z): 353 $(M + H)^+$

Example 66: (S)-1-[2-(2-Furancarboxamide)ethylamino]acetyl-2-pyrrolidinecarbonitrile hydrochloride (Compound 402)

The title compound was obtained in a similar manner to that of Example 65 by using N-(2-aminoethyl)-2-furancarboxamide obtained in Reference example 60 instead of N-(2-aminoethyl)-2-quinoxalinecarboxamide.
yield: 3%
$^1$H NMR (DMSO-$d_6$) δ (ppm): 9.16 (1H, br s), 8.68 (1H, t, J = 5.8 Hz), 7.86 (1H, d, J = 1.4 Hz), 7.17 (1H, d, J = 3.2 Hz), 6.64 (1H, dd, J = 3.2, 1.4 Hz), 4.84 (1H, dd, J = 6.2, 4.9 Hz), 4.16-4.02 (2H, m), 3.65-3.41 (2H, m), 3.39-3.24 (2H, m), 3.21-3.04 (2H, m), 2.23-1.88 (4H, m).
APCIMS (m/z): 291 $(M + H)^+$

Example 67: (S)-1-(2-Benzamidoethylamino)acetyl-2-pyrrolidinecarbonitrile hydrochloride (Compound 403)

The title compound was obtained in a similar manner to that of Example 65 by using N-(2-aminoethyl)benzamide obtained in Reference example 61 instead of N-(2-aminoethyl)-2-quinoxalinecarboxamide.
yield: 21%
$^1$H NMR (DMSO-$d_6$) δ (ppm): 9.09 (1H, br s), 8.75-8.67 (1H, m), 7.89-7.86 (2H, m), 7.55-7.45 (3H, m), 4.84 (1H, dd, J = 5.5, 5.5 Hz), 4.17-4.01 (2H, m), 3.65-3.06 (6H, m), 2.25-1.94 (4H, m).
APCIMS (m/z): 301 $(M + H)^+$

Example 68: (S)-1-( 1-Benzoyl-4-piperidylamino)acetyl-2-pyrrolidinecarbonitrile hydrochloride (Compound 404)

(1) To a solution of 4-amino-1-benzoylpiperidine (1.20 g, 6.00 mmol) obtained by the method described in Reference example 63 in N,N-dimethylformamide (15 mL) was added cesium hydroxide monohydrate (504 mg, 3.00 mmol) at room temperature, and the mixture was stirred at the same temperature for 5 minutes. Then, a solution of (S)-1-bromoacetyl-2-pyrrolidinecarbonitrile (434 mg, 2.00 mmol) described in the U.S. Patent No. 6,011,155 in N,N-dimethylformamide (5 mL) was added to the mixture, which was stirred at the same temperature for 3 hours. After the solvent was evaporated under reduced pressure, chloroform was added to the residue. The organic layer was washed with water and saturated brine, and dried over anhydrous magnesium sulfate. The solvent was evaporated under reduced pressure, and the obtained residue was purified by column chromatography (chloroform/methanol=8/1) to obtain a free form of the title compound (680 mg, 2.00 mmol).

(2) To a solution of the free form (680 mg, 2.00 mmol) obtained in (1) in methanol (6 mL) was added a 4 mol/L solution of hydrogen chloride in 1,4-dioxane (1.50 mL, 6.00 mmol). The solvent was evaporated under reduced pressure, and the obtained residue was crystallized from 2-propanol/ethanol to obtain the title compound (180 mg, 0.48 mmol) as colorless crystals.
yield: 24%
$^1$H NMR (DMSO-$d_6$) δ (ppm): 9.22 (1H, br s), 7.47-7.43 (3H, m), 7.38-7.33 (2H, m), 4.84 (1H, dd, J = 6.8, 4.6 Hz), 4.01-3.99 (2H, m), 3.63-3.24 (7H, m), 2.24-1.95 (6H, m), 1.58-1.34 (2H, m).

APCIMS (m/z): 341 $(M + H)^+$

Example 69: (S)-1-(1-Nicotinoyl-4-piperidylamino)acetyl-2-pyrrolidinecarbonitrile hydrochloride (Compound 405)

The title compound was obtained in a similar manner to that of Example 68 by using 4-amino-1-nicotinoylpiperidine obtained in Reference example 64 instead of 4-amino-1-benzoylpiperidine.
yield: 69%
$^1$H NMR (DMSO-$d_6$) δ (ppm): 9.34 (1H, br s), 8.78 (1H, dd, J = 5.1, 1.6 Hz), 8.74 (1H, d, J = 1.6 Hz), 8.07 (1H, ddd, J = 7.8, 1.6, 1.6 Hz), 7.72 (1H, dd, J = 7.8, 5.1 Hz), 4.85 (1H, dd, J = 7.6, 5.1 Hz), 4.17-3.98 (2H, m), 3.65-3.36 (6H, m), 3.24-3.06 (1H, m), 2.27-1.94 (6H, m), 1.74-1.51 (2H, m).
APCIMS (m/z): 342 $(M + H)^+$

Example 70: (S)-N-[2-(2-Cyano- 1 -pyrrolidinylcarbonylmethylamino)ethyl] benzenesulfonamide methanesulfonate (Compound 406)

(1) To a solution of N-(2-aminoethyl)benzenesulfonamide (1.20 g, 6.00 mmol) obtained by the method described in Reference example 62 in N,N-dimethylformamide (20 mL) was added (S)-1-bromoacetyl-2-pyrrolidinecarbonitrile (434 mg, 2.00 mmol) described in U.S. Patent No. 6,011,155 at room temperature, and the mixture was stirred at the same temperature for 24 hours. After the solvent was evaporated under reduced pressure, chloroform was added to the residue. The organic layer was washed with water and then with saturated brine, and dried over anhydrous magnesium sulfate. The solvent was evaporated under reduced pressure, and the obtained residue was purified by preparative thin layer chromatography (chloroform/methanol=8/1) to obtain a free form of the title compound (480 mg, 1.43 mmol).

(2) To a solution of the free form obtained in (1) (480 mg, 1.43 mmol) in methanol (3 mL) was added methanesulfonic acid (138 mL, 2.15 mmol). The methanol was evaporated and the obtained residue was crystallized from THF and water to obtain the title compound (460 mg, 1.06 mmol) as colorless crystals.
yield: 53%
$^1$H NMR (DMSO-$d_6$) δ (ppm) : 7.83-7.80 (2H, m), 7.69-7.60 (3H, m), 4.83 (1H, dd, J = 5.9, 5.9 Hz), 4.01-3.85 (2H, m), 3.63-3.29 (5H, m), 3.09-2.89 (2H, m), 2.59-2.49 (1H, m), 2.29 (3H, s), 2:25-1.93 (4H, m).

APCIMS (m/z): 337 $(M + H)^+$

Example 71: (S)-1-(1-Benzenesulfonyl-4-piperidylamino)acetyl-2-carbonitrile methanesulfonate (Compound 407)

(1) To a solution of 4-amino-1-benzenesulfonylpiperidine (1.44 g, 6.00 mmol) obtained in Reference example 65 in N,N-dimethylformamide (20 mL) was added (S)-1-bromoacetyl-2-pyrrolidinecarbonitrile (434 mg, 2.00 mmol) described in the U.S. Patent No. 6,011,155 at room temperature, and the mixture was stirred at the same temperature for 24 hours. After the solvent was evaporated under reduced pressure, chloroform was added to the residue. The organic layer was washed with water and saturated brine, and dried over anhydrous magnesium sulfate. The solvent was evaporated under reduced pressure and the obtained residue was purified by preparative thin layer chromatography (chloroform/methanol=8/1) to obtain a free form of the title compound (670 mg, 1.78 mmol).
(2) To a solution of the free compound obtained in (1) (480 mg, 1.78 mmol) in methanol (3 mL) was added methanesulfonic acid (173 mL, 2.67 mmol). The methanol was evaporated and the obtained residue was crystallized from 2-propanol and ethanol to obtain the title compound (444 mg, 0.930 mmol) as colorless crystals.
yield: 47%
$^1$H NMR (DMSO-$d_6$) δ (ppm): 9.01 (1H, br s), 7.77-7.63 (5H, m), 4.83 (1H, dd, J = 5.9, 4.9 Hz), 3.99-3.94 (2H, m), 3.75-3.61 (4H, m), 3.45-3.32 (2H, m), 3.14-2.97 (1H, m), 2.34-2.02 (6H, m), 2.29 (3H, s), 1.70-1.56 (2H, m).
APCIMS (m/z): 377 $(M + H)^+$

Example 72: (S)-1-{1-[5-(N,N-Dimethylaminosulfonyl)-2-pyridylamino]-2-methyl-2-propylamino}acetyl-2-pyrrolidinecarbonitrile (Compound 501)

The title compound was obtained in a similar manner to that of Example 1 by using 2-amino-N-[5-(N,N-dimethylaminosulfonyl)-2-pyridyl]-2-methylpropylamine obtained by the method described in Reference example 66 instead of 2-(2-pyrazinylamino)ethylamine.
yield: 71%
$^1$H NMR ($CDCl_3$) δ (ppm): 8.42 (1H, d, J = 2.4 Hz), 7.64 (1H, dd, J = 8.9, 2.4 Hz), 6.49 (1H, d, J = 8.9 Hz), 6.01 (1H, br s), 4.75 (1H, d, J = 5.9 Hz), 3.62-3.56 (1H, m), 4.72-4.44 (1H, m), 3.39 (2H, d, J = 4.0 Hz), 2.76 (2H, s), 2.70 (6H, s), 2.31-2.20 (4H, m), 1.96 (1H, br s), 1.17 (6H, s).
APCIMS (m/z): 409 $(M + H)^+$

Example 73: (S)-1-[2-Methyl-1-(5-piperidinosulfonyl-2-pyridylamino)-2-propylamino] acetyl-2-pyrrolidinecarbonitrile (Compound 502)

The title compound was obtained in a similar manner to that of Example 1 by using 2-amino-2-methyl-N-[5-(piperidinosulfonyl)-2-pyridyl]propylamine obtained by the method described in Reference example 67 instead of 2-(2-pyrazinylamino)ethylamine.
yield: 79%
$^1$H NMR ($CDCl_3$) δ (ppm): 8.41 (1H, d, J = 2.4 Hz), 7.63 (1H, dd, J = 8.9, 2.4 Hz), 6.46 (1H, d, J = 8.9 Hz), 5.90 (1H, br s), 4.76-4.60 (1H, m), 3.62-3.56 (1H, m), 3.45-3.43 (1H, m), 3.38 (2H, d, J = 3.3 Hz), 3.31 (2H, br s), 3.00-2.96 (4H, m), 2.32-2.15 (4H, m), 1.82 (1H, br s), 1.69-1.61 (4H, m), 1.47-1.43 (2H, m), 1.17 (6H, s).
APCIMS (m/z): 449 $(M + H)^+$

Example 74: (S)-1-{2-Methyl-1-[5-(1,2,3,4-tetrahydroisoquinolin-2-ylsulfonyl)-2-pyridylamino]-2-propylamino}acetyl-2-pyrrolidinecarbonitrile fumarate (Compound 503)

The title compound was obtained in a similar manner to that of Example 1 by using 2-amino-2-methyl-N-[5-(1,2,3,4-tetrahydroisoquinolin-2-ylsulfonyl)-2-pyridyl] propylamine obtained by the method described in Reference example 68 instead of 2-(2-pyrazinylamino)ethylamine.
yield: 50%
$^1$H NMR (DMSO-$d_6$) δ (ppm) : 8.31 (1H, d, J = 2.3 Hz), 7.98 (1H, dd, J = 8.9, 2.3 Hz), 7.6,7-7.13 (4H, m), 6.98 (1H, d, J = 8.9 Hz), 6.61 (2H, s), 4.72 (1H, m), 4.12 (2H, s), 3.68-3.21 (6H, m), 2.84 (2H, t, J = 5.6 Hz), 2.21-1.96 (4H, m), 1.15 (6H, s).
APCIMS (m/z): 497 $(M + H)^+$

Example 75-1 to 16:

Compounds 504, 505, 506, 511, 512, 513, 519, 520, 521, 522, 523, and 533 to 537 were obtained by the following method.

(1) In a reaction bottle, 2-chloropyridine-5-sulfonylchloride (252 mg, 1.2 mmol) described in WO 98/40332, triethylamine (167 μ L, 1.2 mmol), a corresponding amine (1.8 mmol) were mixed with THF (3 mL), and the mixture was stirred at room temperature for 2 days. To the reaction mixture was added BioRad (registered trademark) AG 1-X8 resin (522 mg), and the mixture was stirred for 5 minutes. After filtration, the resin was rinsed with chloroform, and the solvent was evaporated. Chloroform (4.8 mL) was added to the obtained residue, then polystyrenecarbonylchloride (2 to 3 mmol/g, 276 mg) and poly(4-vinylpyridine) (264 mg) was added. The mixture was stirred overnight. After consumption of the starting material was confirmed by thin layer chromatography (TLC), the resin was collected by filtration and the resulting resin [poly(4-vinylpyridine)] was rinsed with methanol (2.4 mL) and chloroform (2.4 mL). The solvent was evaporated to obtain the corresponding 2-chloro-5-substituted aminosulfonylpyridine derivative.

(2) To the pyridine derivative obtained above were added 1,4-dioxane (5.04 mL), 1,2-diamino2-methylpropane (2.4 mL, 1.4 mmol), and potassium carbonate (783 mg, 1.2 mmol), and the mixture was stirred at 100 °C for 2 days. After a consumption of the starting material was confirmed by TLC, insoluble solids were filtered and rinsed with methanol (3.6 mL) and chloroform (3.6 mL). Then the solvent was evaporated. Chloroform (4.8 mL), then formylpolystyrene (1 to 2 mmol/g, 465 mg) was added, and the mixture was stirred two overnights. The resin was removed by filtration and the solution was concentrated to obtain an amine as a yellow solid or oil.

(3) The amine obtained above was weighed and dissolved in N,N-dimethylformamide (0.25 mol/L). To the solution was added (S)-1-bromoacetyl-2-pyrrolidinecarbonitrile (0.5 equivalent) described in the U.S. Patent No. 6,011,155 under ice-cooling, and the mixture was stirred at the same temperature for 1 hour. After the reaction mixture was concentrated, the obtained residue was purified by silica gel column chromatography (chloroform/methanol=95/5) to obtain each of Compounds 504, 505, 506, 511, 513, 519, and 533 to 537 or each of free forms of Compounds 512, 520, 521, 522, and 523.

Compound 512, 520, 521, 522, 523

(4) To a solution of the free form of each Compounds obtained in (3) in THF (2-3 mL) was added fumaric acid (1 equivalent), and the mixture was stirred at room temperature for 10 minutes. The solvent was evaporated under reduced pressure, and the obtained residue was crystallized from ethanol-ethyl acetate to obtain
Compound 512, 520, 521, 522, and 523.

| Compound number | Compound name | yield (%) |
|---|---|---|
| 504 | (S)-1-{1-[5-(N,N-Diethylaminosulfonyl)-2-pyridylamino]-2-methyl -2-propylamino}acetyl-2-pyrrolidinecarbonitrile | 32 |
| 505 | (S)- 1-[1-(5-N-Allyl-N-methylsulfonyl-2-pyridylamino)-2-methyl-2-propylamino] acetyl-2-pyrrolidinecarbonitrile | 81 |
| 506 | (S)-1-{2-Methyl-1-[5-(3-pyrrolin-1-ylsulfonyl)-2-pyridylamino]-2-propylamino} acetyl-2-pyrrolidinecarbonitrile | 30 |
| 511 | (S)-1-{1-[5-(N-Isopropyl-N-methylaminosulfonyl)-2-pyridylamino]-2-methyl-2-propylamino}acetyl-2-pyrrolidinecarbonitrile | 76 |
| 512 | (S)-1-{2-Methyl-1-[5-(4-methylpiperarazin-1-ylsulfonyl)-2-pyridylamino]-2-propylamino}acetyl-2-pyrrolidinecarbonitrile fumarate | 51 |
| 513 | (S)-1-{ 1-[5-(N-Ethyl-N-methylaminosulfonyl)-2-pyridylamino]-2-methyl-2-propylamino}acetyl-2-pyrrolidinecarbonitrile | 94 |
| 519 | (S)-1-{2-Methyl-1-[5-(N-propylaminosulfonyl)-2-pyridylamino]-2-propylamino} acetyl-2-pyrrolidinecarbonitrile | 19 |
| 520 | (S)-1-{1-[5-(N-Cyclopropylmethylaminosulfonyl)-2-pyridylamino]-2-methyl-2-propylamino}acetyl-2-pyrrolidinecarbonitrile fumarate | 57 |

(continued)

| Compound number | Compound name | yield (%) |
|---|---|---|
| 521 | (S)-1-{1-{5-[N-(2-Methoxyethyl)aminosulfonyl]-2-pyridylamino)-2-methyl-2-propylamino}acetyl-2-pyrrolidinecarbonitrile fumarate | 40 |
| 522 | (S)-1-{1 -[5-(N-Cyclopentylaminosulfonyl)-2-pyridylamino]-2-methyl-2-propylamino}acetyl-2-pyrrolidinecarbonitrile fumarate | 61 |
| 523 | (S)- 1-{1-[5-(N-tert-Butylaminosulfonyl)-2-pyridylamino]-2-methyl-2-propylamino}acetyl-2-pyrrolidinecarbonitrile fumarate | 91 |
| 533 | (S)-1-[1-(5-N-Cyclohexyl-N-methylsulfonyl-2-pyridylamino)-2-methyl-2-propylamino]acetyl-2-pyrrolidinecarbonitrile | 27 |
| 534 | (S)-1-[2-Methyl- 1-(5-N-methyl-N-phenylsulfonyl-2-pyridylamino)-2-propylamino]acetyl-2-pyrrolidinecarbonitrile | 45 |
| 535 | (S)-1-[2-Methyl-1-(5-N-methyl-N-phenethylsulfonyl-2-pyridylamino)-2-propylamino]acetyl-2-pyrrolidinecarbonitrile | 53 |
| 536 | (S)- 1-{2-Methyl- 1-[5-N-(4-methoxyphenyl)-N-methylsulfonyl-2-pyridylamino]-2-propylamino}acetyl-2-pyrrolidinecarbonitrile | 22 |
| 537 | (S)-1-{2-Methyl-1-[5-N-(2-methoxyethyl)-N-methylsulfonyl-2-pyridylamino]-2-propylamino}acetyl-2-pyrrolidinecarbonitrile | 78 |

| Compound Data | | |
|---|---|---|
| Compound number | MS | NMR |
| 504 | APCIMS (m/z): 437 $(M + H)^+$ | $^1H$ NMR ($CDCl_3$) δ (ppm) : 8.47 (1H, d, J = 2.3 Hz), 7.67 (1H, dd, J = 8.9, 2.3 Hz), 6.52 (1H, d, J = 8.9 Hz), 6.08 (1H, br s), 4.71 (1H, m), 3.64-3.41 (6H, m), 3.21 (4H, q, J = 7.2 Hz), 2.31-2.04 (4H, m), 1.24 (6H, s), 1.15 (6H, t, J = 7.2 Hz). |
| 505 | APCIMS (m/z): 435 $(M + H)^+$ | $^1H$ NMR ($CDCl_3$) δ (ppm): 8.44 (1H, d, J = 2.3 Hz), 7.65 (1H, dd, J = 8.9, 2.3 Hz), 6.45 (1H, d, J = 8.9 Hz), 5.91 (1H, br s), 5.91-5.67 (1H, m), 5.22 (1H, dd, J = 6.2, 1.3 Hz), 5.18 (1H, t, J = 1.3 Hz), 4.77-4.74 (1H, m), 3.62 (2H, d, J = 6.3 Hz), 3.61-3.28 (6H, m), 2.66 (3H, s), 2.32-2.18 (4H, m), 1.16 (6H, s). |
| 506 | APCIMS (m/z): 433 $(M + H)^+$ | $^1H$ NMR ($CDCl_3$) δ (ppm) : 8.49 (1H, d, J = 2.3 Hz), 7.70 (1H, dd, J = 8.9, 2.3 Hz), 6.47 (1H, d, J = 8.9 Hz), 5.91 (1H, br s), 5.67 (2H, s), 4.73 (1H, m), 3.61-3.19 (6H, m), 2.32-2.14 (4H, m), 1.81-1.79 (4H,m), 1.16 (6H, s). |
| 511 | FABMS (m/z): 437 $(M\ ^1H + H)^+$ | NMR ($CDCl_3$) δ (ppm) : 8.46 (1H, d, J = 2.3 Hz), 7.66 (1H, dd, J = 8.9, 2.3 Hz), 6.45 (1H, d, J = 8.9 Hz), 5.84 (1H, m), 4.75 (1H, m), 4.19 (1H, m), 3.61-3.28 (6H, m), 2.68 (3H, s), 2.32-2.15 (4H, m), 1.16 (6H, s), 1.04 (3H, s), 1.01 (3H, s). |
| 512 | FABMS (m/z): 464 $(M\ ^1H + H)^+$ | NMR (DMSO-$d_6$) δ (ppm): 8.21 (1H, d, J = 2.3 Hz), 7.58 (1H, dd, J = 8.9, 2.3 Hz), 6.70 (1H, d, J = 8.9 Hz), 6.04 (2H, s), 4.73 (1H, m), 3.62-3.44 (6H, m), 2.35 (4H, br s), 2.35 (4H, br s), 1.65-1.53 (m, 7H), 1.13 (6H, s). |

(continued)

| Compound Data | | |
|---|---|---|
| Compound number | MS | NMR |
| 513 | APCIMS (m/z): 423 $(M + H)^+$ | $^1H$ NMR ($CDCl_3$) δ (ppm) : 8.44 (1H, d, J = 2.3 Hz), 7.65 (1H, dd, J = 8.9, 2.3 Hz), 6.45 (1H, d, J = 8.9 Hz), 5.85 (1H, br s), 4.75 (1H, m), 3.64-3.29 (6H, m), 3.08 (2H, q, J = 7.2), 2.71 (3H, s), 2.33-2.18 (4H, m), 1.16 (6H, s), 1.15 (3H, t, J = 7.2 Hz). |
| 519 | APCIMS (m/z): 423 $(M + H)^+$ | $^1H$ NMR ($CDCl_3$) δ (ppm) : 8.50 (1H, d, J = 2.3 Hz), 7.71 (1H, dd, J = 8.9, 2.3 Hz), 6.51 (1H, d, J = 8.9 Hz), 6.01 (1H, br s), 4.74 (1H, m), 3.64-3.33 (6H, m), 2.9.1 (2H, q, J = 6.9 Hz), 2.32-2.17 (4H, m), 1.51 (2H, q, J = 7.2 Hz), 1.22 (6H, s), 0.89 (3H, t, J = 7.2 Hz). |
| 520 | APCIMS (m/z): 435 $(M + H)^+$ | $^1H$ NMR (DMSO-$d_6$) δ (ppm) : 8.26 (1H, d, J = 2.3 Hz), 7.66 (1H, dd, J = 8.9, 2.3 Hz), 7.47 (2H, m), 6.66 (1H, d, J = 8.9 Hz), 6.52 (2H, s), 4.71 (1H, m), 3.47-3.38 (6H, m), 2.59 (2H, m, J = 6.3 Hz), 2.14-2.00 (4H, m), 1.18 (6H, s), 0.77 (1H, m), 0.33 (2H, m), 0.05 (2H, m). |
| 521 | FABMS (m/z): 439 $(M + H)^+$ | $^1H$ NMR (DMSO-$d_6$) δ (ppm) : 8.32 (1H, d, J = 2.3 Hz), 7.67 (1H, dd, J = 8.9, 2.3 Hz), 7.44 (1H, m), 7.25 (1H, br s), 6.73 (1H, d, J = 8.9 Hz), 6.56 (2H, s), 4.77 (1H, m), 3.68-3.23 (8H, m), 2.95 (3H, s), 2.91 (2H, m), 2.25-2.06 (4H, m), 1.10 (6H, s). |
| 522 | FABMS (m/z) : 449 $(M+ H)^+$ | $^1H$ NMR (free form, DMSO-$d_6$) δ (ppm): 8.49 (1H, d, J = 2.3 Hz), 7.70 (1H, dd, J = 8.9, 2.3 Hz), 7.29 (1H, m), 7.16 (1H, br s), 6.57 (1H, d, J = 8.9 Hz), 6.02 (1H, br s), 4.77 (1H, br s), 4.47 (1H, s), 3.78-3.44 (6H, m), 2.32-2.12 (4H, m), 1.85-1.33 (8H, m), 1.36 (3H, s), 1.32 (3H, s). |
| 523 | FABMS (m/z): 437 $(M+H)^+$ | $^1H$ NMR (DMSO-$d_6$) δ (ppm): 8.29 (1H, d, J = 2.3 Hz), 7.65 (1H, dd, J = 8.9, 2.3 Hz), 7.44 (1H, br s), 6.65 (1H, d, J = 8.9 Hz), 6.55 (2H, s), 4.74 (1H, m), 3.72-3.35 (6H, m), 2.25-1.97 (4H, m), 1.15 (6H, s), 1.11 (9H, s). |
| 533 | APCIMS (m/z): 477 $(M + H)^+$ | $^1H$ NMR ($CDCl_3$) δ (ppm): 8.45 (1H, d, J = 2.3 Hz), 7.65 (1H, dd, J = 8.9, 2.3 Hz), 6.45 (1H, d, J = 8.9 Hz), 5.89 (1H, br s), 4.74 (1H, m), 3.73-3.26 (6H, m), 2.71 (3H, s), 2.33-2.10 (4H, m), 2.00 (1H, br s), 1.75-1.22 (10H, m), 1.16 (6H, s). |
| 534 | APCIMS (m/z): 471 $(M + H)^+$ | $^1H$ NMR ($CDCl_3$) δ (ppm): 8.26 (1H, d, J = 2.3 Hz), 7.35-7.15 (6H, m), 6.35 (1H, d, J = 8.5 Hz), 5.88 (1H, br s), 4.75 (1H, m), 3.61-3.27 (6H, m), 2.67 (3H, s), 3.33-2.16 (4H, m), 1.16 (6H, s). |
| 535 | APCIMS (m/z): 499 $(M + H)^+$ | $^1H$ NMR ($CDCl_3$) δ (ppm): 8.42 (1H, d, J = 2.3 Hz), 7.57 (1H, dd, J = 8.9, 2.3 Hz), 7.32-7.56 (5H, m), 6.43 (1H, d, J = 8.9 Hz), 5.96 (1H, br s), 4.73 (1H, m), 3.60-3.21 (8H, m), 2.85 (2H, m), 2.74 (3H, s), 2.30-2.07 (4H, m), 1.15 (6H, s). |
| 536 | APCIMS (m/z): 501 $(M + H)^+$ | $^1H$ NMR ($CDCl_3$) δ (ppm): 8.22 (1H, s), 7.34 (1H, dd, J = 8.9, 2.3 Hz), 7.05 (2H, d, J = 9.0 Hz), 6.81 (2H, d, J = 9.0 Hz), 6.39 (1H, d, J = 8.9 Hz), 6.11 (1H, br s), 4.73 (1H, m), 3.79 (3H, s), 3.74-3.27 (6H, m), 3.12 (3H, s), 2.30-2.17 (4H, m), 1.16 (6H, s). |

(continued)

| Compound Data | | |
|---|---|---|
| Compound number | MS | NMR |
| 537 | APCIMS (m/z): 453 (M+ H)$^+$ | $^1$H NMR ($CDCl_3$) δ (ppm): 8.44 (1H, d, J = 2.3 Hz), 7.65 (1H, dd, J = 8.9, 2.3 Hz), 6.45 (1H, d, J = 8.9 Hz), 5.87 (1H, br s), 4.75 (1H, m), 3.55 (2H, t, J = 5.6 Hz), 3.64-3.28 (6H, m), 3.33 (3H, s), 3.20 (2H, t, J = 5.6 Hz), 2.82 (3H, s), 2.33-2.20 (4H, m), 1.16 (6H, s). |

Example 76: (S)- 1-[2-Methyl-1-(5-morpholinosulfonyl-2-pyridylamino)-2-propylamino] acetyl-2-pyrrolidinecarbonitrile (Compound 507)

The title compound (296 mg) was obtained in a similar manner to that of Example 37 by using 2-amino-2-methyl-N-(5-morpholinosulfonyl-2-pyridyl) propylamine (285mg, 0.908 mmol) obtained in Reference example 69-1 instead of 2-amino-2-methyl-N-(2-pyrimidyl)propylamine.
yield: 72%
$^1$H NMR ($CDCl_3$) δ (ppm): 8.39 (1H, d, J = 2.6 Hz), 7.60 (1H, dd, J = 8.9, 2.6 Hz), 6.52 (1H, d, J = 8.9 Hz), 6.21 (1H, br t, J = 5.3 Hz), 4.75-4.73 (1H, m), 3.76-3.72 (4H, m), 3.69-3.32 (6H, m), 3.00-2.97 (4H, m), 2.31-2.17 (4H, m), 1.17 (6H, s).
APCIMS (m/z): 451 (M + H)$^+$

Example 77: (S)- 1-{2-Methyl- 1-[5-(N-methyl-O-methylhydroxyaminosulfonyl)-2-pyridylamino]-2-propylamino)acetyl-2-pyrrolidinecarbonitrile (Compound 508)

The title compound (180 mg) was obtained in a similar manner to that of Example 37 by using 2-amino-2-methyl-N-[5-(N-methyl-O-methyl-sulfonyl)-2-pyridyl]propylamine (160mg, 0.556 mmol) obtained in Reference example 70 instead of 2-amino-2-methyl-N-(2-pyrimidyl)propylamine.
yield: 77%
$^1$H NMR ($CDCl_3$) δ (ppm): 8.44 (1H, d, J = 2.3 Hz), 7.69 (1H, dd, J = 8.9, 2.3 Hz), 6.53 (1H, d, J = 8.9 Hz), 6.36 (1H, br s), 4.75-4.72 (1H, m), 3.78 (3H, s), 3.59-3.32 (6H, m), 2.78 (3H, s), 2.30-2.19 (4H, m), 1.17 (6H, s).
APCIMS (m/z) : 425 (M + H)$^+$

Example 78: (S)-1-{1-[5-(N-cyclopropyl-N-methylaminosulfonyl)-2-pyridylamino]-2-methyl-2-propylamino)acetyl-2-pyrrolidinecarbonitrile (Compound 509)

The title compound (290 mg) was obtained in a similar manner to that of Example 37 by using 2-amino-N-[5-(N-cyclopropyl-N-methylaminosulfonyl)-2-pyridyl]-2-methylpropylamine (270 mg, 0.906 mmol) obtained in Reference example 71 instead of 2-amino-2-methyl-N-(2-pyrimidyl)propylamine.
yield: 74%
$^1$H NMR ($CDCl_3$) δ (ppm): 8.47 (1H, d, J = 2.3 Hz), 7.69 (1H, dd, J = 8.9, 2.3 Hz), 6.53 (1H, d, J = 8.9 Hz), 6.22 (1H, br s), 4.75-4.72 (1H, m), 3.59-3.34 (6H, m), 2.74 (3H, s), 2.27-2.18 (4H, m), 1.87-1.82 (1H, m), 1.17 (6H, s), 0.88-0.82 (2H, m), 0.73-0.66 (2H, m).
APCIMS (m/z): 435 (M + H)$^+$

Example 79: (S)-1-{2-Methyl-1-[5-(1,3-thiazolidin-3-ylsulfonyl)-2-pyridylamino]-2-propylamino}acetyl-2-pyrrolidinecarbonitrile (Compound 510)

The title compound (88 mg) was obtained in a similar manner to that of Example 37 by using 2-amino-2-methyl-N-[5-(1,3-thiazolidin-3-ylsulfonyl)-2-pyridyl] propylamine (165 mg, 0.522 mmol) obtained in Reference example 69-2 instead of 2-amino-2-methyl-N-(2-pyrimidyl)propylamine.
yield: 37%
$^1$H NMR ($CDCl_3$) δ (ppm): 8.49 (1H, d, J = 2.5 Hz), 7.69 (1H, dd, J = 8.9, 2.5 Hz), 6.49 (1H, d, J = 8.9 Hz), 6.14 (1H, br s), 4.75-4.73 (1H, m), 4.42 (2H, s), 3.61 (2H, t, J = 6.3 Hz), 3.48-3.32 (6H, m), 2.80 (2H, t, J = 6.3 Hz), 2.32-2.01 (4H, m), 1.18 (6H, s).
APCIMS (m/z): 453 (M + H)$^+$

Example 80: (S)-1-{1-(5-[N-(2-Hydroxyethyl)-N-methylaminosulfonyl]-2-pyridylamino) -2-methyl-2-propylamino} acetyl-2-pyrrolidinecarbonitrile (Compound 514)

The title compound (180 mg) was obtained in a similar manner to that of Example 37 by using 2-amino-N-{5-[N-(2-hydroxyethyl)-N-methylaminosulfonyl]-2-pyridyl)-2-methylpropylamine (300 mg, 0.993 mmol) obtained in Reference example 72 instead of 2-amino-2-methyl-N-(2-pyrimidyl)propylamine.
yield: 42%
$^1$H NMR, ($CDCl_3$) δ (ppm): 8.43 (1H, d, J = 2.0 Hz), 7.65 (1H, dd, J = 8.9, 2.0 Hz), 6.52 (1H, d, J = 8.9 Hz), 6.15 (1H, br t, J = 5.1 Hz), 4.75-4.73 (1H, m), 3.74 (2H, t, J = 5.3 Hz), 3.64-3.33 (6H, m), 3.14 (2H, t, J = 5.3 Hz), 2.80 (3H, s), 2.36-2.10 (4H, m), 1.18 (6H, s).
APCIMS (m/z): 439 $(M + H)^+$

Example 81: (S)- 1-{1-[5-(N-Cyanomethyl-N-methylaminosulfonyl)-2-pyridylamino]-2-methyl-2-propylamino}acetyl-2-pyrrolidinecarbonitrile (Compound 515)

The title compound (180 mg) was obtained in a similar manner to that of Example 37 by using 2-amino-N-[5-(N-cyanomethyl-N-methyl aminosulfonyl)-2-pyridyl]-2-methylpropylamine (300 mg, 0.993 mmol) obtained in Reference example 73 instead of 2-amino-2-methyl-N-(2-pyrimidyl)propylamine.
yield: 55%
$^1$H NMR ($CDCl_3$) δ (ppm) : 8.48 (1H, d, J = 2.6 Hz), 7.66 (1H, dd, J = 8.9, 2.6 Hz), 6.54 (1H, d, J = 8.9 Hz), 6.25 (1H, br t, J = 5.0 Hz), 4.75-4.73 (1H, m), 4.17 (2H, s), 3.62-3.56 (1H, m), 3.48-3.35 (5H, m), 2.88 (3H, s), 2.33-2.12 (4H, m), 1.18 (6H, s).
FABMS (m/z) : 434 $(M + H)^+$

Example 82: (S)-1-{1-[5-(N-Benzylaminosulfonyl)-2-pyridylamino]-2-methyl-2-propylamino}acetyl-2-pyrrolidinecarbonitrile fumarate (Compound 516)

The title compound was obtained in a similar manner to that of Example 1 by using 2-amino-N-[5-(N-benzylaminosulfonyl)-2-pyridyl]-2-methylpropylamine obtained by the method described in Reference example 74 instead of 2-(2-pyrazinylamino)ethylamine, and by using fumaric acid instead of methanesulfonic acid in the preparation of a salt.
yield: 77%
$^1$H NMR (DMSO-$d_6$) δ (ppm) : 8.38 (1H, d, J = 2.3 Hz), 7.90 (1H, br s), 7.65 (1H, dd, J = 8.9, 2.3 Hz), 7.46 (1H, br s), 7.26 (5H, m), 6.65 (1H, d, J = 8.9 Hz), 6.56 (2H, s), 4.74 (1H, m), 3.94 (2H, s), 3.72-3.52 (6H, m), 2.16-2.00 (4H, m), 1.17 (6H, s).
APCIMS (m/z): 471 $(M + H)^+$

Example 83: (S)-1-{2-Methyl-1-[5-(N-methylaminosulfonyl)-2-pyridylamino]-2-propylamino}acetyl-2-pyrrolidinecarbonitrile fumarate (Compound 517)

The title compound was obtained in a similar manner to that of Example 1 by using 2-amino-2-methyl-N-[5-(N-methylaminosulfonyl)-2-pyridyl]propylamine obtained by the method described in Reference example 75 instead of 2-(2-pyrazinylamino)ethylamine, and by using fumaric acid instead of methanesulfonic acid in the preparation of a salt.
yield: 93%
$^1$H NMR (DMSO-$d_6$) δ (ppm) : 8.24 (1H, d, J = 2.3 Hz), 7.59 (1H, dd, J = 8.9, 2.3 Hz), 7.29 (1H, br s), 7.12 (1H, m), 6.68 (1H, d, J = 8.9 Hz), 6.62 (2H, s), 4.70 (1H, m), 3.64-3.26 (6H, m), 2.36 (3H, d, J = 2.4 Hz), 2.20-1.99 (4 H, m), 1.07 (6H, s).
APCIMS (m/z): 395 $(M + H)^+$

Example 84: (S)-1-{2-Methyl-1-[5-(N-phenylaminosulfonyl)-2-pyridylamino]-2-propylamino}acetyl-2-pyrrolidinecarbonitrile fumarate (Compound 518)

The title compound was obtained in a similar manner to that of Example 1 by using 2-amino-2-methyl-N-[5-(N-phenylaminosulfonyl)-2-pyridyl)propylamine obtained by the method described in Reference example 76 instead of 2-(2-pyrazinylamino)ethylamine, and by using fumaric acid instead of methanesulfonic acid in the preparation of a salt.
yield: 70%
$^1$H NMR (DMSO-$d_6$) δ (ppm) : 8.22 (1H, d, J = 2.3 Hz), 7.56 (1H, dd, J = 8.9, 2.3 Hz), 7.40 (1H, br s), 7.25-6.97 (6H, m), 6.59 (2H, s), 4.70 (1H, m), 3.62-3.25 (6H, m), 2.20-1.96 (4H, m), 1.76 (1H, m), 1.06 (6H, s).

APCIMS (m/z): 457 (M + H)+

Example 85: (S)- 1-{1-{5-[N-(2-Hydroxyethyl)aminosulfonyl]-2-pyridylamino}-2-methyl-2-propylamino}acetyl-2-pyrrolidinecarbonitrile (Compound 524)

The title compound was obtained in a similar manner to that of Example 1 (1) by using 2-amino-N-(5-[N-(2-hydroxyethyl)aminosulfonyl]-2-pyridyl}-2-methyl-amine obtained by the method described in Reference example 77 instead of 2-(2-pyrazinylamino)ethylamine.
yield: 92%
$^1$H NMR ($CDCl_3$) δ (ppm): 8.49 (1H, d, J = 2.3 Hz), 7.70 (1H, dd, J = 8.9, 2.3 Hz), 6.46 (1H, d, J = 8.9 Hz), 5.87 (1H, br s), 4.75 (1H, m), 4.48 (1H, br s), 3.64-3.30 (6H, m), 2.32-2.15 (4H, m), 1.50-1.25 (4H, m), 1.16 (6H, s).
FABMS (m/z): 425 (M + H)+

Example 86: (S)-1-[2-Methyl-1-(5-sulfamoyl-2-pyridylamino)-2-propylamino]acetyl-2-pyrrolidinecarbonitrile fumarate (Compound 525)

The title compound was obtained in a similar manner to that of Example 1 by using 2-amino-2-methyl-N-(5-sulfamoyl-2-pyridyl)propylamine obtained by the method described in Reference example 78 instead of 2-(2-pyrazinylamino)ethylamine, and by using fumaric acid instead of methanesulfonic acid in the preparation of a salt.
yield: 24%
$^1$H NMR (DMSO-$d_6$) δ (ppm): 8.30 (1H, d, J = 2.3 Hz), 7.68 (1H, dd, J = 8.9, 2.3 Hz), 7.40 (1H, br s), 6.86 (1H, d, J = 8.9 Hz), 6.64 (2H, s), 4.74 (1H, m), 3.77-3.37 (6H, m), 2.26-1.98 (4H, m), 1.15 (3H, s), 1.14 (3H, s).
FABMS (m/z): 381 (M + H)+

Example 87: (S)-1-{1-[5-(N-Ethylaminosulfonyl)-2-pyridylamino]-2-methyl-2-amino)acetyl-2-pyrrolidinecarbonitrile (Compound 526)

The title compound (272 mg) was obtained in a similar manner to that of Example 37 by using 2-amino-N-[5-(N-ethylaminosulfonyl)-2-pyridyl]-2-methyl-amine (310 mg, 1.14 mmol) obtained in Reference example 79 instead of 2-amino-2-methyl-N-(2-pyrimidyl)propylamine,
yield: 64%
$^1$H NMR ($CDCl_3$) δ (ppm): 8.48 (1H, d, J = 2.5 Hz), 7.70 (1H, dd, J = 8.9, 2.5 Hz), 6.48 (1H, d, J = 8.9 Hz), 6.01 (1H, br s), 4.86 (1H, br t, J = 5.9 Hz), 4.75-4.73 (1H, m), 3.59-3.56 (1H, m), 3.48-3.40 (5H, m), 3.03-2.93 (2H, m), 2.31-2.18 (4H, m), 1.17 (6H, s), 1.09 (3H, t, J = 7.3 Hz).
APCIMS (m/z): 409 (M + H)+

Example 88:

Compound 527 to 532 were prepared in the following method.

To the chloropyridine derivative (1.20 mmol) prepared in a similar manner to that of Reference example 66 (1) was added 1,4-dioxane (500 μ L), 4-tert-butoxycarbonylamino-4-methylpiperidine (308 mg, 1.44 mmol), and potassium carbonate (166 mg, 1.44 mmol), and the mixture was stirred at 100°C overnight. The reaction mixture was filtered, and the filtrate was concentrated under reduced pressure. To the obtained residue were added chloroform (9.6 mL), polystyrenecarbonylchloride (2 to 3 mmol/g, 276 mg), and poly(4-vinylpyridine) (264 mg), and the mixture was stirred at room temperature overnight. The resin was filtered and the solvent was evaporated to obtain an amine.
The obtained amine was weighed and dissolved in N,N-dimethylformamide (2 mL). To the solution was added (S)-1-bromoacetyl-2-pyrrolidinecarbonitrile (0.4 equivalent) described in the U.S. Patent No. 6,011,155, and the mixture was stirred at room temperature for 1 hour. The solvent was evaporated, and ethyl acetate and saturated brine was added to the residue. The solution was separated and the resulting organic layer was dried over anhydrous magnesium sulfate. The solvent was evaporated and the obtained residue was purified by silica gel column chromatography (chloroform to chloroform/methanol=95/5) to obtain each compounds shown in the following table.

| Compound number | Compound name | yield (%) |
|---|---|---|
| 527 | (S)-1-{1-[5-(N,N-Dimethylaminosulfonyl)-2-pyridyl]-4-methyl-4-piperidylamino} acetyl-2-pyrrolidinecarbonitrile | 31 |

(continued)

| Compound number | Compound name | yield (%) |
|---|---|---|
| 528 | (S)-1-[4-Methyl-1-(5-pyperidinosulfonyl-2-pyridyl)-4-piperidylamino]acetyl-2-pyrrolidinecarbonitrile | 97 |
| 529 | (S)-1-(4-Methyl-1-[5-(3-pyrrolin-1-ylsulfonyl)-2-pyridyl]-4-piperidylamino}acetyl-2-pyrrolidinecarbonitrile | 81 |
| 530 | (S)-1-{4-Methyl-1-[5-(N-methyl-N-phenethylaminosulfonyl)-2-pyridyl]-4-piperidylamino}acetyl-2-pyrrolidinecarbonitrile | 63 |
| 531 | (S)-1-{1-[5-(N-Benzyl-N-methylaminosulfonyl)-2-pyridyl]-4-methyl-4-piperidylamino}acetyl-2-pyrrolidinecarbonitrile | 82 |
| 532 | (S)-1-{1-{5-[N,N-Bis(2-methoxyethyl)aminosulfonyl]-2-pyridyl}-4-methyl-4-piperidylamino}acetyl-2-pyrrolidinecarbonitrile | 40 |

| Compound Data | | |
|---|---|---|
| Compound number | MS APCIMS (m/z): $(M + H)^+$ | NMR |
| 527 | 435 | $^1$H NMR (free form, $CDCl_3$) δ (ppm) : 8.49 (1H, d, J = 2.3 Hz), 8.01 (1H, s), 7.77-7.70 (2H, m), 6.64 (1H, d, J = 8.9 Hz), 4.76 (1H, m), 3.75-3.36 (6H, m), 2.96 (3H. s), 2.88 (3H, m), 2.35 (4H, m), 2.35-2.16 (4H, m), 1.75-1.64 (4H, m), 1.20 (3H, s). |
| 528 | 475 | $^1$H NMR ($CDCl_3$) δ (ppm) : 8.42 (1H, d, J = 2.6 Hz), 7.65 (1H, dd, J = 9.2, 2.6 Hz), 6.60 (1H, d, J = 9.2 Hz), 4.75-4.73 (1H, m), 3.76-3.54 (4H, m), 3.49-3.32 (4H, m), 2.94 (4H, t, J = 5.3 Hz), 2.31-2.01 (6H, m), 1.68-1.51 (6H, m), 1.44-1.40 (m, 2H), 1.14 (3H, s). |
| 529 | 459 | $^1$H-NMR ($CDCl_3$) δ (ppm): 8.53 (1H, d, J = 2.3 Hz), 7.77 (1H, dd, J = 9.2, 2.5 Hz), 6.64 (1H, d, J = 9.2 Hz), 5.67 (2H, s), 4.78-4.76 (1H, m), 4.10 (4H, s), 3.74-3.49 (5H, m), 3.47-3.40 (3H, m), 2.32-2.12 (4H, m), 1.81-1.63 (4H, m), 1.17 (3H, s). |
| 530 | 525 | $^1$H NMR ($CDCl_3$) δ (ppm): 8.48 (1H, d, J = 2.6 Hz), 7.66 (1H, dd, J = 9.2, 2.6 Hz), 7.31-7.18 (5H, m), 6.59 (1H, d, J = 9.2 Hz), 4.77-4.75 (1H, m), 3.72-3.62 (5H, m), 3.58-3.39 (3H, m), 3.23 (2H, t, J = 7.9 Hz), 2.86 (2H, t, J = 7.6 Hz), 2.74 (3H, s), 2.30-2.04 (4H, m), 1.69-1.53 (4H, m), 1.15 (3H, s). |
| 531 | 511 | $^1$H NMR ($CDCl_3$) δ (ppm): 8.55 (1H, d, J = 2.4 Hz), 7.76 (1H, dd, J = 9.1, 2.4 Hz), 7.36-7.26 (5H, m), 6.66 (1H, d, J = 9.1 Hz), 4.78-4.75 (1H, m), 4.12 (2H, s), 3.76-3.57 (5H, m), 3.51-3.41 (3H, m), 2.58 (3H, s), 2.32-2.20 (4H, m), 1.66-1.61 (4H, m), 1.17 (3H, s). |
| 532 | 523 | $^1$H NMR ($CDCl_3$) δ (ppm): 8.52 (1H, d, J = 2.5 Hz), 7.76 (1H, dd, J = 9.2, 2.5 Hz), 6.61 (1H, d, J = 9.2 Hz), 4.78-4.76 (1H, m), 3.74-3.67 (4H, m), 3.54 (4H, t, J = 5.9 Hz), 3.47 (6H, s), 3.44-3.31 (4H, m), 3.35 (4H, t, J = 5.9 Hz), 2.27-2.18 (4H, m), 1.68-1.60 (4H, m), 1.17 (3H, s). |

Example 89: 1-[(5-Cyanopyridin-2-ylamino)methyl]-1-[(S)-2-cyanopyrrolidinylacetylamino]cyclopropane fumarate (Compound 601)

(1) 1-[(5-Cyanopyridin-2-ylamino)methyl]cyclopropylamine (264 mg, 1.40 mmol) obtained in Reference example

80 and cesium hydroxide hydrate (261 mg, 1.55 mmol) were dissolved in THF (10 mL). To the solution was added a solution of (S)-1-bromoacetyl-2-pyrrolidinecarbonitrile (304 mg, 1.40 mmol) in THF (4 mL) under ice-cooling, and the mixture was stirred at room temperature for two days. Water was added to the reaction mixture, and the mixture was extracted twice with chloroform. The organic layer was dried over anhydrous magnesium sulfate. The solvent was evaporated and the obtained residue was purified by silica gel column chromatography (chloroform/methanol=100/1) to obtain a free form of the title compound (133 mg, 0.410 mmol).

(2) To a fraction of the free form obtained in (1) (127 mg, 0.392 mmol) was added fumaric acid (45.0 mg, 0.390 mmol), which was then dissolved in methanol. The methanol was evaporated to obtain the title compound (137 mg, 0.311 mmol) as a white solid.

yield: 22%

$^1$H NMR (DMSO-$d_6$) δ (ppm): 8.33 (1H, d, J = 2.2 Hz), 7.65-7.62 (2H, m), 6.61 (2H, s), 6.55 (1H, m), 4.69 (1H, t, J = 5.1 Hz), 3.60-3.36 (6H, m), 2.17-1.99 (4H, m), 0.59-0.52 (4H, m).

APCIMS (m/z): 325 $(M + H)^+$

Example 90: 1-[(5-Cyanopyridin-2-ylamino)methyl]- 1- [(S)-2-cyanopyrrolidinylacetyl amino]cyclopentane fumarate (Compound 602)

The title compound (362 mg, 0.774 mmol) was obtained in a similar manner to that of Example 94 from 1-[(5-cyanopyridin-2-ylamino)methyl]cyclopentylamine(432 mg, 2.00 mmol) obtained in Reference example 81 as a white solid.

yield: 39%

$^1$H NMR (DMSO-$d_6$) δ (ppm): 8.34 (1H, d, J = 2.2 Hz), 7.65 (1H, dd, J = 8.4, 2.2 Hz), 7.50 (1H, m), 6.65 (1H, d, J = 8.4 Hz), 6.59 (2H, s), 4.73 (1H, dd, J = 6.2, 4.6 Hz), 3.64-3.37 (6H, m), 2.19-2.00 (4H, m), 1.66-1.57 (8H, m).

APCIMS (m/z): 353 $(M + H)^+$

Example 91: 2-[(5-Cyanopyridin-2-ylamino)methyl]-2-[(S)-2-cyanopyrrolidinylacetyl amino]adamantane fumarate (Compound 603)

The title compound (555 mg, 1.04 mmol) was obtained in a similar manner to that of Example 94 from 2-[(5-cyanopyridin-2-ylamino)methyl]adamantan-2-ylamine (425 mg, 1.51 mmol) obtained in Reference example 82 as a white solid.

yield: 69%

$^1$H NMR (DMSO-$d_6$) δ (ppm): 8.33 (1H, d, J = 2.4 Hz), 7.61 (1H, dd, J = 8.9, 2.4 Hz), 7.17 (1H, m), 6.68 (1H, d, J = 8.9 Hz), 6.63 (2H, s), 4.70 (1H, t, J = 6.2 Hz), 3.68-3.36 (6H, m), 2.17-1.46 (18H, m).

APCIMS (m/z): 417 $(M - H)^-$

Example 92: 1-[(5-Cyanopyridin-2-ylamino)methyl)-1-[(S)-2-amino]cyclooctane fumarate (Compound 604)

The title compound (617 mg, 1.21 mmol) was obtained in a similar manner to that of Example 94 from 1-[(5-cyanopyridin-2-ylamino)methyl]cyclooctylamine (516 mg, 2.00 mmol) obtained in Reference example 83 as a white solid.

yield: 60%

$^1$H NMR (DMSO-$d_6$) δ (ppm): 8.32 (1H, d, J = 2.2 Hz), 7.63 (1H, dd, J = 8.9, 2.2 Hz), 7.30 (1H, m), 6.65 (1H, d, J = 8.9 Hz), 6.61 (2H, s), 4.70 (1H, t, J = 7.6 Hz), 3.60-3.26 (6H, m), 2.23-2.00 (4H, m), 1.53-1.50 (14H, m).

APCIMS (m/z): 395 $(M + H)^+$

Example 93: 1-[(5-Cyanopyridin-2-ylamino)methyl]-1-[(S)-2-cyanopyrrolidinylacetyl amino]cyclobutane fumarate (Compound 605)

The title compound (494 mg, 1.09 mmol) was obtained in a similar manner to that of Example 94 from 1-[(5-cyanopyridin-2-ylamino)methyl]cyclobutylamine (460 mg, 2.28 mmol) obtained in Reference example 84 as a white solid.

yield: 47%

$^1$H NMR (DMSO-$d_6$) δ (ppm): 8.37 (1H, d, J = 2.2 Hz), 7.66 (1H, dd, J = 8.9, 2.2 Hz), 7.47 (1H, m), 6.67 (1H, d, J = 8.9 Hz), 6.60 (2H, s), 4.76 (1H, dd, J = 6.5, 4.6 Hz), 3.65-3.17 (6H, m), 2.25-1.71 (10H, m).

APCIMS (m/z): 339 $(M + H)^+$

Example 94: 1-[(5-Cyanopyridin-2-ylamino)methyl]-1-[(S)-2-cyanopyrrolidinylacetyl amino]cyclohexane fumarate (Compound 606)

(1) 1-[(5-Cyanopyridin-2-ylamino)methyl]cyclohexylamine (568 mg, 2.47 mmol) obtained in Reference example 85 and potassium fluoride (50 weight % on Celite, 1.43 g, 12.3 mmol) was dissolved in acetonitrile (25 mL), and a solution of (S)-1-bromoacetyl-2-pyrrolidinecarbonitrile(536 mg, 2.47 mmol) described in the U.S. Patent No. 6,011,155 in acetonitrile (4 mL) was added under ice-cooling, and the mixture was stirred at the same temperature overnight. The reaction mixture was filtered, and the filtrate was concentrated. The obtained residue was purified by silica gel column chromatography (chloroform/methanol=100/1) to obtain a free form of the title compound (638 mg, 1.74 mmol).

(2) To the free form (638 mg, 1.74 mmol) obtained in (1) was added fumaric acid (202 mg, 1.74 mmol), and the mixture was then dissolved in methanol. The methanol was evaporated to obtain the title compound (709 mg, 1.47 mmol) as a white solid.
yield: 60%
$^1H$ NMR (DMSO-$d_6$) δ (ppm): 8.32 (1H, d, J = 2.2 Hz), 7.63 (1H, dd, J = 8.9, 2.2 Hz), 7.39 (1H, m), 6.66 (1H, d, J = 8.9 Hz), 6.60 (2H, s), 4.71 (1H, t, J = 6.5 Hz), 3.63-3.39 (6H, m), 2.19-2.00 (4H, m), 1.50-1.39 (10H, m).

APCIMS (m/z): 367 $(M + H)^+$

Example 95: (S)-1-[2-Methyl- 1-(4-nitroanilino)-2-propylamino]acetyl-2-carbonitrile fumarate (Compound 701)

To a solution of 2-methyl-1-(4-nitroanilino)-2-propylamine (942 mg, 4.50 mmol) obtained by the method described in Reference example 86 in mixed solvent (THF : N,N-dimethylformamide = 5:1) (6 mL) was added (S)-1-bromoacetyl-2-pyrrolidinecarbonitrile (326 mg, 1.50 mmol) described in the U.S. Patent No. 6,011,155 at room temperature, and the mixture was stirred at the same temperature for 2 hours.

After chloroform was added to the reaction mixture, the organic layer was washed with water and dried over anhydrous magnesium sulfate. The solvent was evaporated under reduced pressure. The obtained residue was purified by silica gel column chromatography (chloroform/methanol=100/0 to 90/10) to obtain a free form of the title compound (450 mg, 1.31 mmol).

(2) To a solution of the free form obtained in (1) (450 mg, 1.31 mmol) in methanol (3 mL) was added fumaric acid (151 mg, 1.31 mmol). The methanol was evaporated under reduced pressure to obtain the title compound (466 mg, 1.00 mmol) as yellow crystals.
yield: 67%
$^1H$ NMR (DMSO-$d_6$) δ (ppm): 7.96 (2H, d, J = 11.2 Hz), 7.05 (1H, br s), 6.68 (2H, d, J = 11.2 Hz), 6.59 (2H, s), 4.72 (1H, dd, J = 6.0 Hz, 4.2 Hz), 3.65-3.05 (7H, m), 2.24-1.87 (4H, m), 1.10 (6H, s).
APCIMS (m/z): 346 $(M + H)^+$

Example 96: (S)-1-(1-Anilino-2-methyl-2-propylamino)acetyl-2-pyrrolidinecarbonitrile fumarate (Compound 702)

The title compound (292 mg, 0.701 mmol) was obtained in a similar manner to that of Example 95 by using 1-anilino-2-methyl-2-propylamine (739 mg, 4.50 mmol) obtained by the method described in Reference example 87 instead of 2-methyl-1-(4-nitroanilino)-2-propylamine.
yield: 47%
$^1H$ NMR (DMSO-$d_6$) δ (ppm): 7.04 (2H, dd, J = 8.6, 6.2 Hz), 6.63-6.47 (4H, m), 6.57 (2H, s), 4.73 (1H, dd, J = 6.6, 4.4 Hz), 3.64-3.16 (7H, m), 2.24-1.96 (4H, m), 1.11 (6H, s).
APCIMS (m/z): 301 $(M + H)^+$

Example 97: (S)- 1-[1-(4-Cyanoanilino)-2-methyl-2-propylamino]acetyl-2-carbonitrile fumarate (Compound 703)

The title compound (236 mg, 0.567 mmol) was obtained in a similar manner to that of Example 95 by using 1-(4-cyanoanilino)-2-methyl-2-propylamine (440 mg, 2.32 mmol) obtained by the method described in Reference example 88 instead of 2-methyl-1-(4-nitroanilino)-2-propylamine.
yield: 69%
$^1H$ NMR (DMSO-$d_6$) δ (ppm): 7.43 (2H, d, J = 8.4 Hz), 6.73 (2H, d, J = 8.4 Hz), 6.59 (2H, s), 6.49 (1H, t, J = 5.9 Hz), 4.74 (1H, dd, J = 6.8, 4.9 Hz), 3.58-3.30 (5H, m), 3.07 (2H, d, J = 5.9 Hz), 2.16-1.99 (4H, m), 1.10 (6H, s).
APCIMS (m/z): 326 $(M + H)^+$

Example 98: (S)-1-[1-(p-Anisidino)-2-methyl-2-propylamino]acetyl-2-carbonitrile fumarate (Compound 704)

(1) To a solution of 1-(p-anisidino)-2-methyl-2-propylamine (437 mg, 2.25 mmol) obtained by the method described in Reference example 89 and basic almina (326 mg) in N,N-dimethylformamide (15 mL) was added (S)-1-bromoacetyl-2-pyrrolidinecarbonitrile (326 mg, 1.50 mmol) described in the U.S. Patent No. 6,011,155 at 0°C, and the mixture was stirred at room temperature for 30 minutes. After the reaction mixture was filtered using Celite as a filtration aid, ethyl acetate was added to the filtrate. The organic layer was washed with water, and dried over anhydrous magnesium sulfate. After the solvent was evaporated under reduced pressure, the obtained residue was purified by silica gel column chromatography (chloroform/methanol -100/0 to 90/10) to obtain a free form of the title compound (33.0 mg, 0.0999 mmol).

(2) To a solution of the free form obtained in (1) (33 mg, 0.0999 mmol) in methanol (1 mL) was added fumaric acid (11.6 mg, 0.0999 mmol). The methanol was evaporated to obtain the title compound (30.0 mg, 0.0720 mmol) as colorless crystals.
yield: 5%
$^1$H NMR (DMSO-$d_6$) δ (ppm): 6.71 (2H, d, J = 8.9 Hz), 6.61 (2H, d, J = 8.9 Hz), 6.57 (2H, s), 6.50 (1H, br s), 4.76 (1H, dd, J = 6.5, 4.6 Hz), 3.81-3.35 (5H, m), 3.63 (3H, s), 3.01 (2H, s), 2.18-1.93 (4H, m), 1.16 (6H, s).
APCIMS (m/z): 331 $(M + H)^+$

Example 99: (S)-1-{2-Methyl-1-[4-(N,N-dimethylaminosulfonyl)anilino]-2-amino} acetyl-2-pyrrolidinecarbonitrile fumarate (Compound 705)

The title compound (220 mg, 0.420 mmol) was obtained in a similar manner to that of Example 98 by using 1-[4-(N,N-dimethylaminosulfonyl)anilino]-2-methyl-2-propylamine (611 mg, 2.25 mmol) obtained by the method described in Reference example 90 instead of 1-(p-anisidino)-2-methyl-2-propylamine.
yield: 28%
$^1$H NMR (DMSO-$d_6$) δ (ppm): 7.40 (2H, d, J = 8.9 Hz), 6.78 (2H, d, J = 8.9 Hz), 6.59 (2H, s), 6.43 (1H, t, J = 5.9 Hz), 4.75 (1H, dd, J = 6.5, 4.6 Hz), 3.67-3.37 (5H, m), 3.17 (6H, s), 3.08 (2H, d, J = 5.9 Hz), 2.17-1.93 (4H, m), 1.12 (6H, s).
APCIMS (m/z): 408 $(M + H)^+$

Example 100: (S)-1-[2-Methyl-1-(4-methylthioanilino)-2-propylamino]acetyl-2-pyrrolidinecarbonitrile fumarate (Compound 706)

The title compound (232 mg, 0.502 mmol) was obtained in a similar manner to that of Example 98 by using 2-methyl-1-(4-methylthioanilino)-2-propylamine (473 mg, 2.25 mmol) obtained by the method described in Reference example 91 instead of 1-(p-anisidino)-2-methyl-2-propylamine.
yield: 34%
$^1$H NMR (DMSO-$d_6$) δ (ppm): 7.10 (2H, d, J = 8.8 Hz), 6.64 (2H, d, J = 8.8 Hz), 6.57 (2H, s), 5.60 (1H, br s), 4.61 (1H, dd, J = 6.8, 4.6 Hz), 3.66-3.42 (5H, m), 3.00 (2H, s), 2.33 (3H, s), 2.22-1.88 (4H, m), 1.16 (6H, s).
APCIMS (m/z): 347 $(M + H)^+$

Example 101: (S)-1-[2-Methyl-1-(p-toluidyl)-2-propylamino]acetyl-2-carbonitrile fumarate (Compound 707)

The title compound (305 mg, 0.708 mmol) was obtained in a similar manner to that of Example 98 by using 2-methyl-1-(p-toluidino)-2-propylamine (401 mg, 2.25 mmol) obtained by the method described in Reference example 92 instead of 1-(p-anisidino)-2-methyl-2-propylamine.
yield: 47%
$^1$H NMR (DMSO-d6) δ (ppm) : 6.88 (2H, d, J = 8.1 Hz), 6.57 (2H, s), 6.55 (2H, d, J = 8.1 Hz), 5.05 (1H, br s), 4.75 (1H, dd, J = 6.5, 4.6 Hz), 3.62-3.88 (5H, m), 3.17 (2H, s), 2.58 (3H, s), 2.17-1.94 (4H, m), 1.16 (6H, s).
APCIMS (m/z): 315 $(M + H)^+$

Example 102: (S)-1-[1-(4-Methanesulfonylanilino)-2-methyl-2-propylamino]acetyl-2-pyrrolidinecarbonitrile fumarate (Compound 708)

To a solution of 1-(4-methanesulfonylanilino)-2-methyl-2-propylamine (161 mg, 0.664 mmol) obtained by the method described in Reference example 93 and potassium fluoride (50 weight % on Celite, 320 mg, 2.75 mmol) in N, N-dimethylformamide (3 mL) was added (S)-1-bromoacetyl-2-pyrrolidinecarbonitrile (120 mg, 0.553 mmol) described

in the U.S. Patent No. 6,011,155 at 0°C, and the mixture was stirred at the same temperature for 11 hours. After the reaction mixture was filtered using Celite as a filtration aid, ethyl acetate was added to the filtrate. The organic layer was washed with saturated aqueous sodium hydrogencarbonate solution, and dried over anhydrous magnesium sulfate. The solvent was evaporated under reduced pressure. The obtained residue was purified by liquid chromatography (chloroform/methanol= 100/0 to 90/10) to obtain a free form of the title compound (195 mg, 0.515 mmol).

(2) To a solution of the free form obtained in (1) (195 mg, 0.515 mmol) in methanol (2 mL) was added fumaric acid (59.8 mg, 0.515 mmol). The methanol was evaporated under reduced pressure to obtain the title compound (140 mg, 0.283 mmol) as colorless crystals.
yield: 52%
$^1$H NMR (DMSO-$d_6$) δ (ppm): 7.56 (2H, d, J = 8.6 Hz), 6.80 (2H, d, J = 8.6 Hz), 6.66 (1H, t, J = 4.9 Hz), 6.57 (2H, s), 4.77 (1H, dd, J = 6.2, 4.6 Hz), 3.73-3.30 (5H, m), 3.23 (2H, d, J = 4.9 Hz), 3.04 (3H, s), 2.26-1.85 (4H, m), 1.23 (6H, s).
APCIMS (m/z): 379 $(M + H)^+$

Example 103: (S)-1-[2-Methyl- 1-(4-pyrrolidinylsulfonylanilino)-2-propylamino]acetyl -2-pyrrolidinecarbonitrile fumarate (Compound 709)

The title compound (365 mg, 0.664 mmol) was obtained in a similar manner to that of Example 102 by using 2-methyl- 1-(4-pyrrolidinylsulfonylanilino)-2-propylamine (416 mg, 1.40 mmol) obtained by the method described in Reference example 94 instead of 1-(4-methanesulfonylanilino)-2-methyl-2-propylamine.
yield: 71%
$^1$H NMR (DMSO-$d_6$) δ (ppm): 7.46 (2H, d, J = 8.8 Hz), 6.75 (2H, d, J = 8.8 Hz), 6.60 (2H, s), 6.38 (1H, br s), 4.76 (1H, dd, J = 7.0, 4.3 Hz), 3.70-3.17 (7H, m), 3.10-2.98 (4H, m), 2.29-1.90 (4H, m), 1.65-1.60 (4H, m), 1.12 (6H, s).
FABMS (m/z): 434 $(M + H)^+$

Example 104: (S)-1-{1-[4-(N,N-Diethylaminosulfonyl)anilino]-2-methyl-2-propylamino} acetyl-2-pyrrolidinecarbonitrile fumarate (Compound 710)

The title compound (378 mg, 0.685 mmol) was obtained in a similar manner to that of Example 102 by using 1-[4-(N,N-diethylaminosulfonyl)anilino]-2-methyl-2-propylamine (674 mg, 2.25 mmol) obtained by the method described in Reference example 95 instead of 1-(4-methanesulfonylanilino)-2-methyl-2-propylamine.
yield: 46%
$^1$H NMR (DMSO-$d_6$) δ (ppm): 7.43 (2H, d, J = 8.8 Hz), 6.74 (2H, d, J = 8.8 Hz), 6.59 (2H, s), 6.35 (1H, br s), 4.75 (1H, dd, J = 6.5, 4.3 Hz), 3.73-3.35 (5H, m), 3.07-3.02 (6H, m), 2.24-1.91 (4H, m), 1.10 (6H, s), 1.01 (6H, t, J = 7.2 Hz).
FABMS (m/z): 436 $(M + H)^+$

Example 105: (S)-1 -[1-(4-Fluoroanilino)-2-methyl-2-propylamino]acetyl-2-carbonitrile fumarate (Compound 711)

To a solution of 1-(4-fuluoroanilino)-2-methyl-2-propylamine (273 mg, 1.50 mmol) obtained by the method described in Reference example 96 and potassium fluoride (spray dried, 174 mg, 3.00 mmol) in N,N-dimethylformamide (4 mL) was added (S)-1-bromoacetyl-2-pyrrolidinecarbonitrile (217 mg, 1.00 mmol) described in the U.S. Patent No. 6,011,155 at 0°C, and the mixture was stirred at the same temperature for 2 hours. After the reaction mixture was filtered using Celite as a filtration aid, ethyl acetate was added to the filtrate. The organic layer was washed with saturated aqueous sodium hydrogencarbonate solution, and dried over anhydrous magnesium sulfate. The solvent was evaporated under reduced pressure and the obtained residue was purified by silica gel column chromatography (chloroform/methanol = 100/0 to 90/10) to obtain a free form of the title compound (319 mg, 1.00 mmol).

(2) To a solution of the free form obtained in (1) (319 mg, 1.00 mmol) in methanol (3 mL) was added fumaric acid (116 mg, 1.00 mmol). The methanol was evaporated under reduced pressure to obtain the title compound (404 mg, 0.931 mmol) as colorless crystals.
yield: 93%
$^1$H NMR (DMSO-$d_6$) δ (ppm) : 6.92-6.86 (2H, m), 6.65-6.60 (2H, m), 6.55 (2H, s), 5.17 (1H, br s), 4.74 (1H, dd, J = 7.3, 6.5 Hz), 3.6,7-3.38 (5H, m), 3.01 (2H, br s), 2.24-1.90 (4H, m), 1.14 (6H, s).
APCIMS (m/z) : 319 $(M + H)^+$

Example 106: (S)-1-[1-(4-Chloro-1-phthalazinylamino)-2-methyl-2-propylamino]acetyl-2-pyrrolidinecarbonitrile fumarate (Compound 244)

The title compound (275 mg, 0.547 mmol) was obtained in a similar manner to that of Example 56 by using 2-amino-N-(4-chloro-1-phthalazinyl)-2-methylpropylamine (376 mg, 1.50 mmol) obtained by the method described in

Reference example 97 instead of 2-amino-N-(5-methoxycarbonyl-2-pyridyl)-2-methylpropylamine.
$^1$H NMR (DMSO-$d_6$) δ (ppm): 8.48-8.39 (1H, m), 8.09-7.95 (3H, m), 6.96 (1H, br s), 6.54 (2H, s), 4.72 (1H, dd, J = 6.9, 3.9 Hz), 3.85-3.43 (7H, m), 2.27-1.92 (4H, m), 1.23 (3H, s), 1.22 (3H, s).
APCIMS (m/z): 387 ($^{35}$ClM + H)+

Example 107: (S)-1-[2-Methyl-1-(1-phthalazinylamino)-2-propylamino]acetyl-2-pyrrolidinecarbonitrile fumarate (Compound 245)

The title compound (48.2 mg, 0.103 mmol) was obtained in a similar manner to that of Example 56 by using 2-amino-2-methyl-N-( 1-phthalazinyl)propylamine (324 mg, 1.50 mmol) obtained by the method described in Reference example 98 instead of 2-amino-N-(5-methoxycarbonyl-2-pyridyl)-2-methylpropylamine.
yield: 10%
$^1$H NMR (DMSO-$d_6$ ) δ (ppm): 8.88 (1H, s), 8.34-8.31 (1H, m), 7.94-7.84 (3H, m), 6.54 (2H, s), 4.72 (1H, dd, J = 6.8, 4.3 Hz), 3.76-3.43 (7H, m), 2.19-1.92 (4H, m), 1.20 (3H, s), 1.19 (3H, s).
APCIMS (m/z): 353 (M + H)$^+$

Example 108: (S)- 1-[2-Methyl-1-(3-pyridazinylamino)-2-propylamino]acetyl-2-pyrrolidinecarbonitrile fumarate (Compound 246)

(1) A free form of the title compound (719 mg, 2.38 mmol) was obtained in a similar manner to that of Example 37 (1) from 2-amino-2-methyl-N-(3-pyridazinyl)propylamine (948 mg, 5.71 mmol) obtained by the method described in Reference example 99.
(2) To a solution of the free form (719 mg, 2.38 mmol) obtained in (1) in methanol (10 mL) was added fumaric acid (442 mg, 3.81 mmol), and the mixture was stirred at room temperature for 10 minutes. The methanol was evaporated under reduced pressure. The obtained residue was crystallized from ethyl acetate to obtain the title compound (650 mg, 1.56 mmol) as yellow crystals.
yield: 28%
$^1$H NMR (DMSO-$d_6$) δ (ppm): 8.87 (1H, dd, J = 4.3, 1.1 Hz), 7.40 (1H, m), 7.18 (1H, dd, J = 8.9, 4.3 Hz), 6.88 (1H, dd, J = 8.9, 1.1 Hz), 6.54 (2H, s), 4.70 (1H, dd, J = 7.0, 4.1 Hz), 3.64-3.38 (7H, m), 2.12-1.98 (4H, m), 1.09 (6H, s).
FABMS (m/z): 303 (M + H)$^+$

Example 109: (S)-1-[2-Methyl-1-(4-pyrimidinylamino)-2-propylamino]acetyl-2-pyrrolidinecarbonitrile fumarate (Compound 247)

(1) A free form of the title compound (522 mg, 1.73 mmol) was obtained in a similar manner to that of Example 20 from 2-amino-2-methyl-N-(4-pyrimidinyl)-amine (2.03 g, 12.3 mmol) obtained by the method described in Reference example 100 and (S)-1-bromoacetyl-2-pyrrolidinecarbonitrile (434 mg, 2.00 mmol) described in the U.S. Patent No. 6,011,155.
(2) To a solution of the free form (522 mg, 1.73 mmol) obtained in (1) in methanol (10 mL) was added fumaric acid (442 mg, 3.81 mmol), and the mixture was stirred at room temperature for 10 minutes. The methanol was evaporated under reduced pressure. The obtained residue was crystallized from diethyl ether to obtain the title compound (459 mg, 1.10 mmol) as colorless crystals.
yield: 9%
$^1$H NMR (DMSO-$d_6$) δ (ppm): 8.36 (1H, s), 8.01 (1H, d, J = 5.9 Hz), 7.39 (1H, m), 6.58 (2H, s), 6.54 (1H, dd, J = 5.9, 1.6 Hz), 4.75 (1H, dd, J = 6.5, 4.6 Hz), 3.62 (2H, d, J = 5.1 Hz), 3.69-3.51 (3H, m), 3.59-3.16 (2H, m), 2.22-1.97 (4H, m), 1.12 (6H, s).
APCIMS (m/z): 303 (M + H)$^+$

Example 110: (S)-1-[1-(5-Methanesulfonyl-2-pyridylamino)-2-methyl-2-propylamino] acetyl-2-pyrrolidinecarbonitrile fumarate (Compound 248)

(1) A free form of the title compound (609 mg, 1.60 mmol) was obtained in a similar manner to that of Example 20

from 2-amino-N-(5-methanesulfonyl-2-pyridyl)-2-methylpropylamine (1.46 g, 6.00 mmol) obtained by the method described in Reference example 101.

(2) To a solution of the free form (609 mg, 1.60 mmol) obtained in (1) in methanol (10 mL) was added fumaric acid (186 mg, 1.60 mmol), and the mixture was stirred at room temperature for 10 minutes. The methanol was evaporated under reduced pressure. The obtained residue was crystallized from diethyl ether to obtain the title compound (697 mg, 1.41 mmol) as colorless crystals.

yield: 24%

$^1$H NMR (DMSO-$d_6$) δ (ppm): 8.35 (1H, d, J = 2.4 Hz), 7.73 (1H, dd, J = 8.9, 2.4 Hz), 7.54 (1H, m), 6.69 (1H, d, J = 8.9 Hz), 6.57 (2H, s), 4.72 (1H, dd, J = 6.5, 4.5 Hz), 3.65-3.55 (1H, m), 3.43 (2H, d, J = 4.9 Hz), 3.48-3.25 (4H, m), 3.11 (3H, s), 2.20-2.10 (2H, m), 2.05-1.90 (2H, m), 1.11 (6H, s).

APCIMS (m/z): 380 $(M + H)^+$

Example 111: (S)-1-[1-(5-Methanesulfonyl-2-pyridyl)-4-methyl-4-piperidylamino] acetyl-2-pyrrolidinecarbonitrile dihydrochloride (Compound 249)

(1) A free form of the title compound (608 mg, 1.50 mmol) was obtained in a similar manner to that of Example 52 from 4-amino-1-(5-methanesulfonyl-2-pyridyl)-4-methylpiperidine (1.28 g, 4.77 mmol) obtained in Reference example 102.

(2) To a solution of the free form obtained in (1) (608 mg, 1.50 mmol) in 1,4-dioxane (10 mL) was added a 4 mol/L solution of hydrogen chloride in 1,4-dioxane (8.0 mL) under ice-cooling, and the mixture was stirred at room temperature for 10 minutes. The solvent was evaporated under reduced pressure. The obtained residue was crystallized from diethyl ether to obtain the title compound (572 mg, 1.20 mmol) as colorless crystals.

yield: 23%

$^1$H NMR (DMSO-$d_6$) δ (ppm): 9.06 (2H, br s), 8.50 (1H, d, J = 2.7 Hz), 7.91 (1H, dd, J = 9.2, 2.7 Hz), 7.03 (1H, d, J = 9.2 Hz), 4.83 (1H, dd, J = 6.8, 4.3 Hz), 4.50-4.35 (2H, m), 4.15-3.90 (2H, m), 3.75-3.65 (1H, m), 3.60-3.49 (2H, m), 3.20-3.00 (2H, m), 3.15 (3H, s), 2.25-2.10 (2H, m), 2.10-1.90 (2H, m), 1.95-1.75 (4H, m), 1.46 (3H, s).

APCIMS (m/z): 406 $(M + H)^+$

Example 112: (S)-1-[4-Methyl-1-(5-methyl-2-pyridyl)-4-piperidylamino]acetyl-2-pyrrolidinecarbonitrile dihydrochloride (Compound 250)

The title compound was obtained in a similar manner to that of Example 51 by using 4-amino-4-methyl-1-(5-methyl-2-pyridyl)piperidine obtained in Reference example 103 instead of 4-amino-1-(5-chloro-2-pyridyl)-4-methylpiperidine.

yield: 54%

$^1$H NMR (DMSO-$d_6$) δ (ppm): 9.32 (1H, br s), 9.15 (1H, br s), 7.93-7.89 (2H, m), 7.38 (1H, d, J = 8.9 Hz), 4.83 (1H, dd, J = 7.3, 4.2 Hz), 4.40-4.25 (2H, m), 4.10-3.40 (5H, m), 3.40-3.20 (2H, m), 2.30-2.10 (2H, m), 2.22 (3H, s), 2.10-1.80 (6H, m), 1.45 (3H, s).

APCIMS (m/z): 342 $(M + H)^+$

Example 113: (S)-1-[4-Methyl-1-(3-pyridazinyl)-4-piperidylamino]acetyl-2-carbonitrile dihydrochloride (Compound 251)

The title compound was obtained in a similar manner to that of Example 111 by using 4-amino-4-methyl-1-(3-pyridazinyl)piperidine obtained in Reference example 104 instead of 4-amino-1-(5-methanesulfonyl-2-pyridyl)-4-methylpiperidine.

yield: 10%

$^1$H NMR (DMSO-$d_6$) δ (ppm): 9.21 (1H, br s), 9.12 (1H, br s), 8.72 (1H, d, J = 4.1 Hz), 7.92 (1H, d, J = 9.5 Hz), 7.86 (1H, dd, J = 9.5, 4.1 Hz), 4.84 (1H, dd, J = 7.0, 4.3 Hz), 4.35-4.29 (2H, m), 4.15-3.90 (1H, m), 3.69-3.60 (2H, m), 3.60-3.59 (2H, m), 3.29-3.21 (2H, m), 2.25-2.10 (2H, m), 2.10-1.80 (6H, m), 1.46 (3H, s).

APCIMS (m/z): 329 $(M + H)^+$

Example 114: (S)-1-[1-(5-Bromo-2-pyrimidinyl)-4-methyl-4-piperidylamino]acetyl-2-pyrrolidinecarbonitrile (Compound 252)

The title compound was obtained in a similar manner to that of Example 52 by using 4-amino-1-(5-bromo-2-pyrimidinyl)-4-methylpiperidine obtained in Reference example 105 instead of 4-amino-4-methyl-1-(5-phenyl-2-pyridyl)piperidine.
yield: 6%
$^1$H NMR ($CDCl_3$) δ (ppm): 8.26 (2H, s), 4.78 (1H, d, J = 7.6 Hz), 3.95-3.40 (6H, m), 3.38 (2H, d, J = 1.1 Hz), 2.40-2.10 (2H, m), 1.65-1.51 (7H, m), 1.14 (3H, s).
APCIMS (m/z): 409 ($^{81}$BrM + H)$^+$, 407 ($^{79}$BrM + H)$^+$

Example 115: (S)-1-[2-Methyl- 1-(N-methylanilino)-2-propylamino]acetyl-2-carbonitrile fumarate (Compound 712)

The title compound (360 mg, 0.836 mmol) was obtained in a similar manner to that of Example 105 by using 2-methyl-1-(N-methylanilino)-2-propylamine (267 mg, 1.50 mmol) obtained in Reference example 106 instead of 1-(4-fluoroanilino)-2-methyl-2-propylamine.
yield: 84%
$^1$H NMR (DMSO-$d_6$) δ (ppm): 7.13 (2H, dd, J = 7.8, 7.3 Hz), 6.82 (2H, d, J = 7.8 Hz), 6.59 (1H, t = 7.3 Hz), 6.56 (2H, s), 4.75 (1H, dd, J = 6.8, 4.3 Hz), 3.66-3.32 (7H, m), 2.94 (3H, s), 2.22-1.93 (4H, m), 1.12 (6H, s).
APCIMS (m/z): 315 (M + H)$^+$

Reference example 1: 2-(2-Quinolylamino)ethylamine

To a solution of commercially available 2-chloroquinoline (1.03 g, 6.30 mmol) in 1,4-dioxane (10 mL) were added potassium carbonate (1.31 g, 9.48 mmol) and ethylenediamine (1.26 mL, 18.8 mmol), and the mixture was refluxed for 7 hours. Ethylenediamine (2.52 mL, 37.7 mmol) was further added to the mixture, and the mixture was refluxed for 8 hours.

The reaction mixture was concentrated, and the residue was purified by silica gel column chromatography to obtain the title compound (1.79 g, 9.57 mmol) as brown crystals.
yield: quantitative
APCIMS (m/z): 188 (M + H)$^+$

In the following Reference example 2 to 12, the title compound was obtained in a similar manner to that of Reference example 1 by using the corresponding halide instead of 2-chloroquinoline.

Reference example 2: 2-(2-Pyrazinylamino)ethylamine

yield: 81%
$^1$H NMR (DMSO-d6) δ (ppm): 7.95-7.84 (2H, m), 7.25 (1H, d, J = 2.4 Hz), 7.19 (1H, br s), 3.28 (2H, dt, J = 5.9, 5.9 Hz), 2.78 (2H, t, J = 5.9 Hz).

Reference example 3: 2-(6-Chloro-3-pyridazinylamino)ethylamine

Reference example 4: 2-(2-Quinoxalinylamino)ethylamine

yield: 89%
$^1$H NMR (DMSO-d6) δ (ppm): 8.30 (1H, s), 7.74 (1H, d, J = 5.4 Hz), 7.65-7.48 (3H, m), 7.30 (1H, dd, J = 7.8, 3.2 Hz) , 3.39 (2H, dt, J = 6.1, 6.1 Hz), 2.78 (2H, t, J = 6.1 Hz).

Reference example 5: 2-(4-Chloro-1-phthalazinylamino)ethylamine

Reference example 6: 2-(6,7-Dimethoxy-4-quinazolinylamino)ethylamine

yield: 65%
$^1$H NMR (DMSO-$d_6$) δ (ppm): 8.31 (1H, s), 7.85 (1H, t, J = 6.2 Hz), 7.59 (1H, s), 7.01 (1H, s), 3.88 (3H, s), 3.88 (3H, s), 3.51 (2H, dt, J = 6.2, 6.2 Hz), 2.79 (2H, t, J = 6.2 Hz).

Reference example 7: 2- [2-(4-Pyridyl)-4-quinazolinylamino]ethylamine

Reference example 8: 2-(4-Methyl-2-quinolylamino)ethylamine

yield: 89%
APCIMS (m/z): 202 (M + H)$^+$

Reference example 9: 2-(4-Quinolylamino)ethylamine

yield: quantitative
APCIMS (m/z) : 188 $(M + H)^+$

Reference example 10: 2-(1-Isoquinolylamino)ethylamine

yield: 68%
APCIMS (m/z): 188 $(M + H)^+$

Reference example 11: 2-(2-Benzothiazolylamino)ethylamine

yield: 59%
APCIMS (m/z): 194 $(M + H)^+$

Reference example 12: 2-[5-(N,N-Dimethylaminosulfonyl)-2-pyridylamino]ethylamine

The title compound was obtained in a similar manner to the description in Reference example 1 by using pyridine instead of 1,4-dioxane and by using 2-chloro-5-(N,N-dimethylaminosulfonyl)pyridine and ethylenediamine (22 equivalence).
yield: 69%
$^1H$ NMR (DMSO-$d_6$) δ (ppm) : 8.26 (1H, d, J = 2.3 Hz), 7.60 (1H, dd, J = 8.9, 2.6 Hz), 7.51 (1H, m), 6.59 (1H, d, J = 8.9 Hz), 3.29 (3H, m), 2.70 (2H, d, J = 6.3 Hz), 2.56 (6H, s), 1.45 (2H, br s).
APCIMS (m/z): 245 $(M + H)^+$

Reference example 13: 2-Amino-N-(5-cyano-2-pyridyl)-2-methylpropylamine

To a solution of 6-chloronicotinonitrile (3.50 g, 25.3 mmol) in 1,4-dioxane (10 mL) were added potassium carbonate (5.24 g, 37.9 mmol) and 1,2-diamino-2-methylpropane (3.97 mL, 37.9 mmol), and the mixture was refluxed for 4 hours. The reaction mixture was, concentrated and crystals were allowed to precipitate. The deposited crude crystals were washed with toluene to obtain the title compound (4.03 g, 21.2 mmol) as white crystals.
yield: 84%
APCIMS (m/z): 191 $(M + H)^+$

Reference example 14: 2-(3-Cyano-2-pyridylamino)ethanol

To a solution of commercially available 2-chloro-3-cyanopyridine (1.07 g, 7.73 mmol) in 1,4-dioxane (20 mL) were added potassium carbonate (1.60 g, 11.6 mmol) and monoethanolamine (0.93 mL, 15.4 mmol), and the mixture was refluxed for 7 hours. After the reaction mixture was concentrated, the residue was purified by silica gel column chromatography to obtain the title compound (1.20 g, 7.36 mmol) as colorless crystals.
yield: 95%
APCIMS (m/z): 164 $(M + H)^+$

Reference example 15: 4-[(3-Cyano-2-pyridyl)aminomethyl]benzylamine

The title compound was obtained in a similar manner to that of Reference example 14 by using p-xylenediamine instead of monoethanolamine.
yield: 81%
APCIMS (m/z): 239 $(M + H)^+$

Reference example 16: 1-(3-Cyano-2-pyridyl)piperazine dihydrochloride

(1) 4-tert-Butoxycarbonyl-1-(3-cyano-2-pyridyl)piperazine was obtained in a similar manner to that of Reference example 14 by using N-tert-butoxycarbonyl piperazine instead of monoethanolamine.
yield: 88%
FABMS (m/z): 289 $(M + H)^+$

(2) To a solution of the compound obtained in (1) (800 mg, 2.78 mmol) in methanol (20 mL) was added a 4 mol/L solution of hydrogen chloride in ethyl acetate (6.94 mL, 27.8 mmol). After the mixture was stirred at room temperature for 4.5 hours, the reaction mixture was concentrated to obtain 1-(3-cyano-2-pyridyl)piperazine dihydrochloride (822 mg, 3.15 mmol).
yield: quantitative
APCIMS (m/z): 189 $(M + H)^+$

Reference example 17: 2-[(3-Cyano-2-pyridyl)-N-methylamino]ethyl-N-methylamine

The title compound was obtained in a similar manner to that of Reference example 14 by using N,N'-dimethylethylenediamine instead of monoethanolamine.
yield: quantitative
APCIMS (m/z): 191 $(M + H)^+$

Reference example 18: 2-Chloro-N-[2-(5-nitro-2-pyridylamino)ethyl]acetamide

To a solution of commercially available 2-(2-aminoethylamino)-5-nitropyridine (1.05g, 5.76 mmol) in methylene chloride (20 mL) and triethylamine (1.61 mL, 11.6 mmol) was added chloroacetyl chloride (0.51 mL, 6.40 mmol) under ice-cooling, and the mixture was stirred at room temperature overnight. A small amount of ethanol was added to the reaction mixture, and the solvent was evaporated. The obtained residue was purified by silica gel column chromatography to obtain the title compound.
yield: 57%
APCIMS (m/z): 257 ($^{35}$ClM - H), 259 ($^{37}$ClM - H)-

Reference example 19: (R)-3-[N-tert-butoxycarbonyl-1-(3-cyano-2-pyridyl)-4-piperidyl amino]acetyl-4-thiazolinecarbonitrile

(1) Commercially available 2-chloro-3-cyanopyridine (2.00 g, 14.4 mmol) and 1,4-dioxa-8-azaspiro[4,5]decane (3.10 g, 21.6 mmol) was mixed in pyridine (20 mL), and the mixture was heated with stirring at 100°C for 3.5 hours. The solvent was evaporated under reduced pressure. Water and chloroform was added to the obtained residue, and the solution was separated. The resulting organic layer was dried over anhydrous magnesium sulfate and concentrated. The obtained residue was purified by silica gel column chromatography (hexane/ethyl acetate=8/1 to 1/4) to obtain 8-(3-cyano-2-pyridyl)-1,4-dioxa-8-azaspiro[4,5]decane (1.30 g, 5.31 mmol). The obtained piperidine derivative (1.30 g, 5.31 mmol) was dissolved in acetone (20 mL), and concentrated hydrochloric acid (10 mL) was added to the solution, which was then stirred at room temperature for 2 hours. The solvent was evaporated under reduced pressure, and the residue was neutralized with saturated aqueous sodium hydrogencarbonate solution, and extracted with chloroform. The obtained organic layer was dried over anhydrous magnesium sulfate. The solvent was evaporated under reduced pressure to obtain 1-(3-cyano-2-pyridyl)-4-oxopiperidine (1.16 g, 5.77 mmol).
yield: 40%
$^1$H NMR ($CDCl_3$) $\delta$ (ppm): 8.38 (1H, dd, J = 4.8, 2.0 Hz), 7.83 (1H, dd, J = 7.7, 2.0 Hz), 6.84 (1H, dd, J = 7.7, 4.8 Hz), 4.02 (4H, t, J = 5.9 Hz), 2.63 (4H, t, J = 5.9 Hz).
(2) The compound obtained in (1) (984 mg, 4.89 mmol) and ammonium acetate (3.77g, 48.9mmol) was mixed in methanol (18 mL). Sodium cyanoborohydride (307 mg, 4.89 mmol) was added to the mixture, which was then stirred at room temperature for 2 hours. The solvent was evaporated under reduced pressure. The obtained residue was separated by water and chloroform. The obtained aqueous layer was adjusted to pH 10 with a 2 mol/L aqueous solution of sodium hydroxide, and extracted with methylene chloride. The obtained organic layer was washed with saturated brine, and dried over anhydrous magnesium sulfate. The solvent was evaporated under reduced pressure. The obtained residue was purified by silica gel column chromatography (chloroform/solvent A=100/0 to 60/40; solvent A was prepared by mixing chloroform and concentrated aqueous ammonia (in 10:1,v/v) and separating the layers, and adding to the resulting organic layer the same volume of methanol as that of the used aqueous ammonia) to obtain 4-amino-1-(3-cyano-2-pyridyl)piperidine (504 mg, 2.50 mmol).
yield: 51%
$^1$H NMR ($CDCl_3$) $\delta$ (ppm): 8.32 (1H, dd, J = 5.0, 2.0 Hz), 7.75 (1H, dd, J = 7.6, 2.0 Hz), 6.72 (1H, dd, J = 7.6, 5.0 Hz), 4.42-4.24 (2H, m), 3.21-3.06 (2H, m), 3.04-2.93 (1H, m), 2.13-2.00 (2H, m), 1.66-1.43 (2H, m).
APCIMS (m/z): 203 $(M + H)^+$

(3) The compound obtained in (2) (300 mg, 1.48 mmol) was dissolved in tetrahydrofuran (6 mL). To the solution was added benzyl bromoacetate (78 μL, 0.49 mmol) under ice-cooling, and the mixture was stirred for 5 hours. After triethylamine (68 μL, 0.49 mmol) was added to the mixture, the solvent was evaporated under reduced pressure. Ethyl acetate and water were added to the residue, and solution was separated. The obtained organic layer was washed with saturated brine, and dried over anhydrous magnesium sulfate. The solvent was evaporated, and the obtained residue was purified by silica gel column chromatography (hexane/ethyl acetate = 60/40 to 0/100) to obtain 4-(N-benzyloxycarbonylmethyl)amino-1-(3-cyano-2-pyridyl)piperidine (150 mg, 0.43 mmol).
yield: 29%
(4) The compound obtained in (3) (150 mg, 0.43 mmol) was dissolved in tetrahydrofuran (3 mL). To the solution was added di-tert-butyl dicarbonate (103 mg, 0.47 mmol) at room temperature, and the mixture was stirred at room temperature for 3 hours. Further, 4-dimethylaminopyridine (5.0 mg, 0.04 mmol) was added, and the mixture was stirred for 4 hours. The solvent was evaporated under reduced pressure, and the obtained residue was purified by silica gel column chromatography (hexane/ethyl acetate=90/10 to 70/30) to obtain 4-(N-benzyloxycarbonylmethyl-N-tert-butoxycarbonyl)amino-1-(3-cyano-2-pyridyl)piperidine (139 mg, 0.31 mmol).
yield: 72%
$^1$H NMR ($CDCl_3$) δ (ppm) : 8.32 (1H, dd, J = 4.8, 2.0 Hz), 7.77 (1H, dd, J = 7.6, 2.0 Hz), 7.35 (5H, s), 6.75 (1H, dd, J = 7.6, 4.8 Hz), 5.15 (2H, s), 4.44-4.38 (3H, m), 3.83 (2H, s), 3.09-3.00 (2H, m), 1.92-1.55 (4H, m), 1.36 (9H, s).
APCIMS (m/z): 451 $(M + H)^+$
(5) The compound obtained in (4) (127 mg, 0.28 mmol) was dissolved in ethanol (1 mL) and tetrahydrofuran (2 mL). To the solution was added 10% palladium on carbon (12 mg, containing 50% water), and the mixture was stirred at room temperature under hydrogen gas atmosphere for 20 hours. To the reaction mixture was added Celite. The mixture was stirred and filtered using Celite as filtration aid. The solvent was evaporated under reduced pressure to obtain oil (100 mg). The obtained oil (100 mg) was dissolved in tetrahydrofuran (3 mL). To the solution were added (R)-4-carbamoylthiazolidine (44 mg, 0.34 mmol), 1-(3-dimethylaminopropyl)-3-ethylcarbodiimide hydrochloride (91 mg, 0.47 mmol), N-hydroxybenzotriazole (56 mg, 0.42 mmol), and methylene chloride (2 mL), and the mixture was stirred at room temperature for 2 hours. The solvent was evaporated under reduced pressure and water was added to the obtained residue, which was then extracted with methylene chloride. The obtained organic layer was washed with saturated aqueous sodium hydrogencarbonate solution, and dried over anhydrous magnesium sulfate. The solvent was evaporated under reduced pressure, and the obtained residue was purified by silica gel column chromatography (chloroform/methanol=99/1 to 95/5) to obtain (R)-3-{N-tert-butoxycarbonyl-[1-(3-cyano-2-pyridyl)-4-piperidyl]amino}acetyl-4-carbam oylthiazolidine (89 mg, 0.19 mmol).
yield: 68%
$^1$H NMR ($CDCl_3$) δ (ppm): 8.33 (1H, dd, J = 5.0, 2.0 Hz), 7.76 (1H, dd, J = 7.6, 2.0 Hz), 6.76 (1H, dd, J = 7.6, 5.0 Hz), 5.8-4.9 (1H, m), 4.8-4.3 (5H, m), 4.2-3.2 (4H, m), 3.08-2.99 (2H, m), 2.1-1.6 (4H, m), 1.48 and 1.45 (9H, s).
APCIMS (m/z): 473 $(M - H)^-$
(6) Trifluoroacetic anhydride (41 μL, 0.29 mmol) was ice-cooled, to which were added the compound obtained in (5) (68 mg, 0.14 mmol) and a solution of pyridine (23 μL, 0.29 mmol) in methylene chloride (1 mL), and the mixture was stirred at the same temperature for 4 hours. Trifluoroacetic anhydride (41 μL) and pyridine (23 μ L) were further added to the mixture, and the mixture was stirred for 2 hours. Water was added to the reaction mixture, and the solution was extracted with methylene chloride. The solvent was evaporated under reduced pressure and the obtained residue was purified by silica gel column chromatography (hexane/ethyl acetate=90/10 to 60/40) to obtain (R)-3-{N-tert-butoxycarbonyl-[1-(3-cyano-2-pyridyl)-amino)acetyl-4-thiazolinecarbonitrile (21 mg, 0.05 mmol).
yield: 32%
$^1$H NMR ($CDCl_3$) δ (ppm): 8.33 (1H, dd, J = 5.0, 2.0 Hz), 7.77 (1H, dd, J = 7.6, 2.0 Hz), 6.76 (1H, dd, J = 7.6, 4.8 Hz), 5.33 (1H, br s), 4.8-4.3 (5H, m), 4.2-3.6 (2H, m), 3.28 (2H, br s), 3.08-2.99 (2H, m), 1.90-1.60 (4H, m), 1.46 (9H, s).
APCIMS (m/z): 357 $(M - CO(CH3)3 + H)^+$

Reference example 20: 2-(3-Cyano-2-pyridyl)aminoethylamine dihydrochloride

(1) To a solution of commercially available 2-chloro-3-cyanopyridine (1.12 g, 8.09 mmol) in 1,4-dioxane (10 mL) were added potassium carbonate (1.12 g, 8.09 mmol) and tert-butyl N-(2-aminoethyl)carbamate (1.53 mL, 9.70 mmol), and the mixture was refluxed for 6 hours. tert-Butyl N-(2-aminoethyl)carbamate (0.38 mL, 2.41 mmol) was further added to the mixture, and the mixture was refluxed for 3.5 hours. The residue was purified by silica gel column chromatography to obtain 3-cyano-2-(2-butoxycarbonylaminoethyl)aminopyridine (1.51 g, 5.76 mmol) as colorless crystals.

yield: 71%
APCIMS (m/z): 263 $(M + H)^+$
(2) To a solution of the compound obtained in (1) (0.92 g, 3.51 mmol) in ethyl acetate (20 mL) was added a 4 mol/L solution of hydrogen chloride in ethyl acetate (17.6 mL, 70.4 mmol), and the mixture was stirred at room temperature for 3 hours. The reaction mixture was concentrated to obtain the title compound (0.83 g, 3.52 mmol).
yield: quantitative
APCIMS (m/z): 163 $(M + H)^+$

Reference example 21: 3-(3-Cyano-2-pyridylamino)propylamine dihydrochloride

(1) 3-Cyano-2-(3-butoxycarbonylaminopropyl)aminopyridine was obtained in a similar manner to that of Reference example 20 (1) by using tert-butyl N-(3-aminopropyl)carbamate instead of tert-butyl N-(2-aminoethyl)carbamate.
yield: 96%
APCIMS (m/z) : 277 $(M + H)^+$
(2) The title compound was obtained in a similar manner to that of Reference example 20 (2) by using 3-cyano-2-(3-butoxycarbonylaminopropyl)aminopyridine instead of 3-cyano-2-(2-butoxycarbonylaminoethyl)aminopyridine.

Reference example 22: 4-Amino-1-(2-pyrazinyl)piperidine

To a solution of commercially available 2-chloropyrazine (824 μ L, 9.23 mmol) in 1,4-dioxane (8 mL) were added potassium carbonate (1.39 g, 10.1 mmol) and a known compound, 4-tert-butylcarbonylaminopiperidine (1.68 g, 8.39 mmol), and the mixture was refluxed for 6 hours. Chloroform was added to the reaction mixture, and the mixture was washed with water and the solvent was evaporated under reduced pressure. The obtained residue was dissolved in a 1,4-dioxane solution (10 mL). To the solution was added 4 mol/L solution of hydrogen chloride in 1,4-dioxane (20 mL) at room temperature, and the mixture was stirred at the same temperature for 15 hours. The deposited crystals were collected by filtration, and the obtained crystals were dissolved in methanol. The solution was neutralized with addition of BioRad AG ( registered trademark)1 X-8 ion-exchange resin. The solvent was evaporated under reduced pressure to obtain the title compound (1.40 g, 7.85 mmol).
yield: 93%
APCIMS (m/z): 179 $(M + H)^+$

The compounds of Reference example 23 to 26 were obtained in a similar manner to that of Reference example 22 by using the corresponding haloheteroaryl instead of 2-chloropyrazine.

Reference example 23: 4-Amino-1-(2-quinolyl)piperidine

yield: 52%
APCIMS (m/z): 228 $(M + H)^+$

Reference example 24: 4-Amino-1-(2-quinoxalinyl)piperidine

yield: 72%
APCIMS (m/z): 229 $(M + H)^+$

Reference example 25: 4-Amino-1-( 1-isoquinolyl)piperidine

yield: 50%
APCIMS (m/z): 228 $(M + H)^+$

Reference example 26: 4-Amino-1-(5-cyano-2-pyridyl)piperidine

yield: 54%
APCIMS (m/z): 203$(M + H)^+$

Reference example 27: 2-Amino-2-methyl-N-(2-quinoxalinyl)propylamine

To 2-chloroquinoxaline (3.27 g, 20.0 mmol) were added potassium carbonate (4.15 g, 30.0 mmol) and 1,2-diamino-2-methylpropane (3.14 mL, 30.0 mmol), and the mixture was refluxed at 120°C for 63 hours. The reaction mixture was filtered using Celite as a filtration aid, and the filtrate was concentrated under reduced pressure. The obtained residue was purified by silica gel column chromatography (Chromatorex (registered trademark) NH, Fuji Silysia, hexane/chloroform=3/1) to obtain the title compound (2.77 g, 12.8 mmol).
yield: 64%
$^1$H NMR (DMSO-$d_6$) δ (ppm): 8.41 (1H, s), 7.72 (1H, d, J = 7.8 Hz), 7.51-7.48 (2H, m), 7.43 (1H, t, J = 5.4 Hz), 7.30-7.24 (1H, m), 3.31 (2H, d, J = 5.4 Hz), 2.90 (2H, br s), 1.07 (6H, s)
APCIMS (m/z): 217 $(M + H)^+$

In the following Reference example 28, 29, 30, 33, 34, and 35, the title compounds were obtained in a similar manner to that of Reference example 27 by using the corresponding halide instead of 2-chloroquinoxaline.

In the following Reference example 31, 32, 36, and 37, the title compounds were obtained in a similar manner to that of Reference example 13 by using the corresponding halide instead of 6-chloronicotinonitrile.

Reference example 28: 2-Amino-2-methyl-N-(2-quinolyl)propylamine

yield: 20%
$^1$H NMR (DMSO-$d_6$) δ (ppm): 7.80 (1H, d, J = 8.9 Hz), 7.57 (1H, d, J = 7.8 Hz), 7.44-7.41 (2H, m), 7.13-7.07 (1H, m), 6.86 (1H, d, J = 8.9 Hz), 6.79 (1H, t, J = 5.6 Hz), 3.31 (2H, d, J = 5.6 Hz), 1.50 (2H, br s), 1.06 (6H, s).
APCIMS (m/z): 216 $(M + H)^+$

Reference example 29: 2-Amino-N-( 1 -isoquinolyl)-2-methylpropylamine

yield: 56%
$^1$H NMR (DMSO-d6) δ (ppm): 8.28 (1H, d, J = 6.9 Hz), 7.81 (1H, d, J = 5.8 Hz), 7.68-7.44 (3H, m), 7.03 (1H, t, J = 5.8 Hz), 6.84 (1H, d, J = 6.9 Hz), 3.45 (2H, d, J = 5.8 Hz), 3.14 (2H, br s), 1.06 (6H, s).
APCIMS (m/z): 216 $(M + H)^+$

Reference example 30: 2-Amino-2-methyl-N-(4-quinolyl)propylamine

yield: 35%
$^1$H NMR (DMSO-$d_6$) δ (ppm): 8.35 (1H, d, J = 5.4 Hz), 8.23 (1H, dd, J = 8.5, 1.6 Hz), 7.76 (1H, dd, J = 8.4, 1.3 Hz), 7.59 (1H, ddd, J = 8.4, 7.0, 1.6 Hz), 7.40 (1H, ddd, J = 8.5, 7.0, 1.3 Hz), 6.75 (1H, t, J = 3.9 Hz), 6.54 (1H, d, J = 5.4 Hz), 3.20 (2H, br s), 3.13 (2H, d, J = 3.9 Hz), 1.10 (6H, s).
APCIMS (m/z): 216 $(M + H)^+$

Reference example 31: 2-Amino-2-methyl-N-(2-pyrazinyl)propylamine

yield: 10%
$^1$H NMR (DMSO-$d_6$) δ (ppm): 7.80 (1H, s), 7.57 (1H, d, J = 2.8 Hz), 6.86 (1H, d, J = 2.8 Hz), 6.79 (1H, t, J = 5.6 Hz), 3.31 (2H, d, J = 5.6 Hz), 1.50 (2H, br s), 1.06 (6H, s).
APCIMS (m/z): 167 $(M + H)^+$

Reference example 32: 2-Amino-2-methyl-N-(5-nitro-2-pyridyl)propylamine

yield: quantitative
$^1$H NMR (DMSO-$d_6$) δ (ppm): 8.86 (1H, d, J = 2.4 Hz), 8.07 (1H, dd, J = 9.5 Hz, 2.4 Hz), 6.68 (1H, d, J = 9.5 Hz), 3.34 (2H, s), 3.24 (3H, br s), 1.01 (6H, s).
APCIMS (m/z): 211 $(M + H)^+$

Reference example 33: 2-Amino-2-methyl-N-(2-pyridyl )propylamine

yield: 49%
$^1$H NMR (DMSO-$d_6$) δ (ppm): 7.89 (2H, dd, J = 5.1, 1.5 Hz), 7.30 (1H, ddd, J = 8.2, 6.3, 1.5 Hz), 6.51 (2H, dd, J = 8.2, 1.1 Hz), 6.40 (1H, ddd, J = 6.3, 5.1, 1.1 Hz), 6.28 (1H, t, J = 5.9 Hz), 3.12 (2H, d, J = 5.9 Hz), 1.00 (6H, s).
APCIMS (m/z): 166 $(M + H)^+$

Reference example 34: 2-Amino-2-methyl-N-(5-trifluoromethyl-2-pyridyl)propylamine

yield: 44%
$^1$H NMR (DMSO-$d_6$) δ (ppm): 8.24 (1H, d, J = 2.6 Hz), 7.59 (1H, dd, J = 8.8, 2.6 Hz), 7.16 (1H, t, J = 6.1 Hz), 6.68 (1H, d, J = 8.8 Hz), 3.23 (2H, d, J = 6.1 Hz), 1.45 (2H, br s), 1.02 (6H, s).
APCIMS (m/z): 234 $(M + H)^+$

Reference example 35: 2-Amino-N-(3,5-dichloro-2-pyridyl)-2-methylpropylamine

yield: 52%
APCIMS (m/z): 234 ($^{35}Cl^{35}ClM$ + H)+, 236 ($^{35}Cl^{37}ClM + H)^+$, 238 ($3^7Cl3^7ClM + H)^+$

Reference example 36: 2-Amino-N-(5-carbamoyl-2-pyridyl)-2-methylpropylamine

yield: 54%
$^1$H NMR (DMSO-$d_6$) δ (ppm) : 8.47 (1H, d, J = 2.3 Hz), 7.78 (1H, dd, J = 8.3, 2.3 Hz), 6.88 (1H, m), 6.53 (1H, d, J = 8.3 Hz), 3.32 (2H, br s), 3.20 (2H, d, J = 6.0), 1.01 (6H, s). APCIMS (m/z): 209 $(M + H)^+$

Reference example 37: 2-Amino-N-(3-cyano-2-pyrazinyl)-2-methylpropylamine

yield: 83%
$^1$H NMR (DMSO-$d_6$) δ (ppm): 8.31 (1H, d, J = 2.4 Hz), 7.90 (1H, d, J = 2.4 Hz), 3.37 (5H, m), 1.06 (6H, s).
APCIMS (m/z): 192 $(M + H)^+$

Reference example 38: 2-Amino-N-[5-(N,N-dimethylaminocarbonyl)-2-pyridyl]-2-methylpropylamine

(1) 6-Chloro-N,N-dimethylnicotinamide
To a solution of commercially available 6-chloronicotinic acid (1.58 g, 10.0 mmol) in THF(40 mL) were added triethylamine (2.09 mL, 15.0 mmol), 50% dimethylamine (1.26 mL, 14.0 mmol), and 1-ethyl-3-[3-(N,N-dimethylamino)propyl]-carbodiimide hydrochloride (2.26 g, 11.0 mmol), and the mixture was stirred at room temperature for 15 hours. Chloroform was added to the reaction mixture. The organic layer was washed with water and saturated aqueous sodium hydrogencarbonate solution, and dried over anhydrous magnesium sulfate. The solvent was evaporated under reduced pressure, and the obtained residue was purified by silica gel column chromatography (chloroform) to obtain 6-chloro-N,N-dimethylnicotinamide (1.33 g, 7.20 mmol).
yield: 72%
(2) The title compound (934 mg, 3.95 mmol) was obtained in a similar manner to that of Reference example 27 by using 6-chloro-N,N-dimethylnicotinamide (1.33 g, 7.20 mmol) obtained in (1) instead of 2-chloroquinoxaline.
yield: 55%
$^1$H NMR ($CDCl_3$) δ (ppm): 8.20 (1H, d, J = 2.4 Hz), 7.54 (1H, dd, J = 8.6, 2.4 Hz), 6.43 (1H, d, J = 8.6 Hz), 5.41 (1H, t, J = 5.7 Hz), 3.25 (2H, d, J = 5.7 Hz), 3.08 (6H, s), 1.84 (2H, br s), 1.17 (6H, s).
APCIMS (m/z): 237 $(M + H)^+$

Reference example 39: 2-Amino-N-(5-chloro-2-pyridyl)-2-methylpropylamine

To 2,5-dichloropyridine (1.48 g, 10.0 mmol) were added diisopropylethylamine (1.92 mL, 11.0 mmol) and 1,2-diamino-2-methylpropane (3.14 mL, 30.0 mmol), and the mixture was heated with stirring at 170°C for 5.5 hours. The reaction mixture was concentrated under reduced pressure to evaporate excess 1,2-diamino-2-methylpropane. The obtained residue was purified by silica gel column chromatography (Chromatorex (registered trademark) NH, Fuji Silysia, hexane/ethyl acetate =2/1) to obtain the title compound (163 mg, 0.819 mmol).
yield: 8%
$^1$H NMR ($CDCl_3$) δ (ppm): 7.99 (1H, d, J = 2.2 Hz), 7.33 (1H, dd, J = 8.7, 2.2 Hz), 6.39 (1H, d, J = 8.7 Hz), 5.01 (1H, t, J = 5.9 Hz), 3.19 (2H, d, J = 5.9 Hz), 1.65 (2H, br s), 1.17 (6H, s).
FABMS (m/z): 200 ($^{35}ClM + H)^+$, 202 ($^{37}ClM + H)^+$

Reference example 40: 2-Amino-2-methyl-N-(2-pyrimidinyl)propylamine

The title compound (1.09 g, 3.34 mmol) was obtained in a similar manner to that of Reference example 39 by using 2-pyrimidine (2.00 g, 10.2 mmol) instead of 2,5-dichloropyridine.
yield: 33%
$^1$H NMR (DMSO-$d_6$) δ (ppm): 6.61 (2H, d, J = 7.4 Hz), 6.49 (1H, t, J = 7.4 Hz), 5.33 (1H, t, J = 5.9 Hz), 2.85 (2H, d, J = 5.9 Hz), 2.50 (2H, br s), 1.06 (6H, s).
Reference example 41: 2-Amino-2-methyl-N-(2-thiazolyl)propylamine

(1) Preparation of 2-methyl-2-nitro-N-(2-thiazolyl)propylamine
After 2-aminothiazole (5.00 g, 50.0 mmol), 2-nitropropane (4.45 g, 50.0 mmol), and triton B (0.500 mL) were dissolved in methanol (15 mL), 37% formalin (3.75 mL, 50.0 mmol) was added dropwise to the solution under heated reflux, and the solution was stirred as it was under heated reflux overnight. After cooling, the reaction mixture was concentrated, and the residue was purified by silica gel column chromatography (hexane/ethyl acetate=2/1 to 1/1) to obtain the title compound (7.31 g, 36.2 mmol).
yield: 72%
$^1$H NMR ($CDCl_3$) δ (ppm): 7.09 (1H, d, J = 3.8 Hz), 6.50 (1H, d, J = 3.8 Hz), 5.67 (1H, br s), 3.91 (2H, s), 1.65 (6H, s).
(2) 2-Methyl-2-nitro-N-(2-thiazolyl)propylamine (2.00 g, 10.0 mmol) obtained in (1) was dissolved in concentrated hydrochloric acid (20 mL) and methanol (20 mL) and zinc (4.00 g, 61.5 mmol) was added to the mixture at room temperature small portionwise. After the solution was stirred at the same temperature overnight, 28% aqueous ammonia was added to the solution until the solution became alkaline, and the solution was extracted four times with chloroform. The organic layer was dried over anhydrous magnesium sulfate, and concentrated to obtain the title compound (1.69 g, 9.83 mmol) as a white solid.
yield: 98%
$^1$H NMR ($CDCl_3$) δ (ppm): 7.41 (1H, m), 6.96 (1H, d, J = 3.8 Hz), 6.55 (1H, d, J = 3.8 Hz), 3.10 (2H, d, J = 5.7 Hz), 1.01 (6H, s).
APCIMS (m/z): 172 $(M + H)^+$

Reference example 42: 2-Amino-2-methyl-N-(1,3,4-thiadiazol-2-yl)propylamine

After 2-amino-1,3,4-thiadiazole (25.0 g, 248 mmol), 2-nitropropane (23.0 mL, 258 mmol), and triton B (2.00 m L) were dissolved in methanol (20 mL), 37% formalin (20.0 mL, 267 mmol) was added dropwise to the solution under heated reflux, and the solution was stirred as it was under heated reflux overnight. After cooling, water was added to the reaction mixture, and deposited solids were collected by filtration. The obtained solids were dissolved in saturated aqueous ammonium chloride solution (200 m L) and methanol (100 m L). To the solution was added small portionwise zinc-copper alloy (40.0 g) obtained according to the preparation method described in Org. Synth., 5, 855, with stirring at 60°C. After stirring at the same temperature for 4 hours, the reaction mixture was filtered using Celite as a filtration aid, and the filtrate was concentrated. An aqueous ammonia (28 % ) was added to the residue, and the solution was extracted four times with chloroform. The organic layer was dried over anhydrous magnesium sulfate and concentrated to obtain the title compound (16.8 g, 97.7 mmol) as a white solid.
yield: 39%
$^1$H NMR ($CDCl_3$) δ (ppm): 8.36 (1H, s), 3.26 (2H, s), 1.20 (6H, s).
APCIMS (m/z): 173 $(M + H)^+$

Reference example 43: 2-Amino-N-(5-cyano-2-thiazolyl)-2-methylpropylamine

(1) Preparation of 2-bromothiazole-5-carbonitrile
2-Aminothiazole-5-carbonitrile (875 mg, 7.00 mmol) obtained by the method described in Japanese Patent Unexamined Publication (KOKAI) No.9-169,748 was dissolved in 47% hydrobromic acid (14 mL) and water (14 mL). To the solution were added dropwise a solution of sodium nitrite (580 mg, 8.40 mmol) in water (7 mL) under ice-cooling. The mixture was stirred at the same temperature for 5 minutes, and further stirred at 50°C for 6 hours. Ethyl acetate was added to the reaction mixture, and the mixture was washed successively with water, saturated aqueous sodium hydrogencarbonate solution, and then with satutated brine. The organic layer was dried over anhydrous magnesium sulfate, and the solvent was evaporated to obtain the title compound (1.05 g, 5.56 mmol) as an orange solid.
yield: 79%

$^1H$ NMR (DMSO-$d_6$) δ (ppm): 8.57 (1H, s).
2) 2-Bmothiazole-5-carbonitrile (955 mg, 5.05 mmol) obtained in (1) and diisopropylethylamine (1.80 mL, 10.1 mmol) were dissolved in 1,4-dioxane (20 mL). To the solution was added 1,2-amino-2-methylpropane (1.10 mL, 10.1 mmol) under ice-cooling, and the mixture was allowed to react at room temperature overnight. After the reaction mixture was concentrated, the residue was purified by silica gel column chromatography (chloroform/methanol=10/1) to obtain the title compound (895 mg, 4.57 mmol).
yield: 90%
$^1H$ NMR ($CDCl_3$) δ (ppm): 7.64 (1H, s), 3.15 (2H, s), 1.21 (6H, s).
APCIMS (m/z): 197 $(M + H)^+$

Reference example 44: 2-Amino-2-methyl-N-(4-phenyl-2-thiazolyl)propylamine

1) 2-Methyl-2-nitro-N-(4-phenyl-2-thiazolyl)-2-propylamine (6.21 g, 22.4 mmol) was obtained in a similar manner to that of Reference example 38 (1) from 2-amino-4-phenylthiazole (13.8 g, 50.0 mmol).
yield: 45%
$^1H$ NMR (DMSO-$d_6$) δ (ppm): 7.88-7.81 (3H, m), 7.40-7.34 (2H, m), 7.26 (1H, m), 7.07 (1H, s), 3.91 (2H, d, J = 6.2 Hz), 1.61 (s, 6H).
(2) The title compound (827 mg, 3.35 mmol) was obtained in a similar manner to that of Reference example 38 (2) from 2-methyl-2-nitro-N-(4-phenyl-2-thiazolyl)-2-propylamine (2.00 g, 7.22 mmol).
yield: 46%
$^1H$ NMR (DMSO-$d_6$) δ (ppm) : 7.83 (2H, m), 7.55 (1H, m), 7.36 (2H, m), 7.27 (1H, s), 7.01 (1H, s), 3.18 (2H, d, J = 6.2 Hz), 1.02 (6H, s).
APCIMS (m/z): 248 $(M + H)^+$

Reference example 45: 2-Amino-N-[5-(N,N-dimethylaminosulfonyl)-4-methyl-2-thiazolyl)-2-methylpropylamine

(1) Preparation of 2-bromo-5-(N,N-dimethylaminosulfonyl)-4-methylthiazole
2-Acetamide-4-methylthiazole-5-sulfonylchloride (2.56 g, 10.1 mmol) was dissolved in THF (30 mL). To the solution was added dropwise a 2 mol/L solution of dimethylamine in THF (20 mL, 40 mmol) under ice-cooling. After the mixture was stirred at the same temperature for 30 minutes, saturated brine was added to the reaction mixture, and the mixture was extracted three times with chloroform. The organic layer was dried over anhydrous magnesium sulfate, and the solvent was evaporated. Concentrated hydrochloric acid (30 mL) and methanol (10 mL) were added to the residue, and the solution was stirred under heated reflux for 30 minutes. The reaction mixture was concentrated and aqueous sodium hydroxide solution was added to the residue so as to be alkaline. The solution was extracted three times with chloroform. The organic layer was dried over anhydrous magnesium sulfate, and the solvent was evaporated. 47% Hydrobromic acid (20 mL) and 2-propanol (20 mL) were added to the residue. To the solution was added dropwise a solution of sodium nitrite (723 mg, 10.5 mmol) and water (10 mL) under ice-cooling. The mixture was stirred at the same temperature for 5 minutes and at 60°C for 6 hours. Diethyl ether was added to the reaction mixture and washed successively with water, saturated aqueous sodium hydrogencarbonate solution, and then with saturated brine. The organic layer was dried over anhydrous magnesium sulfate, and the solvent was evaporated. The obtained residue was purified by silica gel column chromatography (hexane/ethyl acetate=4/1) to obtain the title compound (793 mg, 2.78 mmol).
yield: 28%
$^1H$ NMR ($CDCl_3$) δ (ppm): 2.84 (6H, s), 2.67 (3H, s).
(2) The title compound (706 mg, 2.42 mmol) was obtained in a similar manner to that of Reference example 43 (2) from 2-bromo-5-(N,N-dimethylaminosulfonyl)-4-methylthiazole (719 mg, 2.52 mmol).
yield: 96%
$^1H$ NMR ($CDCl_3$) δ (ppm) : 3.12 (2H, s), 2.79 (6H, s), 2.47 (3H, s), 1.21 (6H, s).
APCIMS (m/z): 293 $(M + H)^+$

Reference example 46: 2-Amino-2-methyl-N-(5-methyl-2-thiazolyl)propylamine

(1) 2-Methyl-N-(5-methyl-2-thiazolyl)-2-nitropropylamine (7.54 g, 35.1 mmol) was obtained in a similar manner to that of Reference example 38 (1) from 2-amino-5-methylthiazole (5.70 g, 50.0 mmol).
yield: 70%
$^1$H NMR ($CDCl_3$) δ(ppm): 6.71 (1H, q , J = 1.1 Hz), 3.86 (2H, s), 2.27 (3H, s), 1.62 (6H, s).
(2) The title compound (1.74 g, 9.41 mmol) was obtained in a similar manner to that of Reference example 38 (2) from 2-methyl-N-(5-methyl-2-thiazolyl)-2-nitropropyl amine (2.00 g, 9.30 mmol).
yield: 100%
$^1$H NMR ($CDCl_3$) δ (ppm): 6.70 (1H, q, J = 1.4 Hz), 3.16 (2H, s), 2.26 (3H, s), 1.18 (6H, s).
APCIMS (m/z): 186 $(M + H)^+$

Reference example 47: 4-Amino-4-methyl-1-(2-pyrazinyl)piperidine

(1) To a solution of 4-tert-butoxycarbonylamino-4-methyl-piperidine (856 mg, 4.00 mmol) described in European Patent 647,639 in 1,4-dioxane (16 mL) were added potassium carbonate (2.21 g, 16.0 mmol) and 2-chloropyrazine (0.764 mL, 8.00 mmol), and the mixture was refluxed for 4 days. After the reaction mixture was stand for cooling, the mixture was added with water and extracted three times with chloroform. The combined organic layer was washed with saturated brine, dried over anhydrous magnesium sulfate, and concentrated. The obtained residue was purified by silica gel column chromatography (hexane/ethyl acetate=1/1) to obtain 4-tert-butoxycarbonylamino-4-methyl-1-(2-pyrazinyl)piperidine (1.04 g, 3.55 mmol).
yield: 89 %
$^1$H NMR ($CDCl_3$) δ (ppm): 8.15 (1H, d, J = 1.4 Hz), 8.04 (1H, dd, J = 2.7, 1.4 Hz), 7.82 (1H, d, J = 2.7 Hz), 4.43 (1H, br s), 3.87 (2H, ddd, J = 13.5, 4.7, 4.7 Hz), 3.33 (2H, ddd, J = 13.5, 10.5, 3.0 Hz), 2.11 (2H, d, J = 13.8 Hz), 1.66 (2H, ddd, J = 13.8, 9.6, 4.2 Hz), 1.44 (9H, s), 1.40 (3H, s).
APCIMS (m/z): 293 $(M + H)^+$
(2) To a solution of 4-tert-butoxycarbonylamino-methyl-1-(2-pyrazinyl) piperidine (1.04 g, 3.55 mmol) in dichloromethane (50 mL) was added trifluoroacetic acid (50 mL) under ice-cooling. The reaction mixture was stirred at the same temperature for 2 hours. The reaction mixture was concentrated and the obtained residue was dissolved in ethanol. The solution was made alkaline with addition of BioRad AG (registered trademark) 1-X8 ion-exchange resin. After the reaction mixture was filtered, the filtrate was concentrated to obtain 4-amino-4-methyl-1-(2-pyrazinyl)piperidine (623 mg, 3.24 mmol).
yield: 91%
$^1$H NMR ($CDCl_3$) δ (ppm): 8.15 (1H, d, J = 1.6 Hz), 8.04 (1H, dd, J = 2.7, 1.6 Hz), 7.79 (1H, d, J = 2.7 Hz), 3.66-3.63 (4H, m), 3.09 (2H, br s), 1.71-1.51 (4H, m), 1.22 (3H, s).
APCIMS (m/z): 193 $(M + H)^+$

Reference example 48: 4-Amino-4-methyl-1-(2-pyrimidinyl)piperidine

(1) To a solution of 4-tert-butoxycarbonylamino-4-methylpiperidine (1.28 g, 6.0 mmol) described in European Patent No. 647,639 in 1,4-dioxane (25 mL) were added potassium carbonate (4.15 g, 30.0 mmol) and 2-chloropyrimidine (2.06 g, 18.0 mmol), and the mixture was refluxed for 2 days. After the reaction mixture was stand for cooling to room temperature and filtered, the filtrate was concentrated. The obtained residue was partially purified by silica gel column chromatography (hexane/ethyl acetate = 1/1) to obtain a mixture (1.97 g) of 4-tert-butoxycarbonylamino-4-methyl- 1-(2-pyrimidinyl)piperidine and unreacted 2-chloropyrimidine.
APCIMS (m/z): 293 (M + H)+
$^1$H NMR ($CDCl_3$) δ (ppm): 8.29 (2H, d, J = 4.6 Hz), 6.45 (1H, t, J = 4.6 Hz), 4.98 (1H, br s), 4.18 (2H, ddd, J = 13.5, 4.6, 4.6 Hz), 3.44 (2H, ddd, J = 13.5, 10.3, 3.2 Hz), 2.04 (2H, d, J = 14.0 Hz), 1.60 (2H, ddd, J = 14.0, 10.0, 4.1 Hz), 1.44 (9H, s), 1.40 (3H, s)
(2) To a solution of a mixture (1.97 g) of 4-tert-butoxycarbonylamino4-methyl- 1-(2-pyrimidinyl)piperidine and 2-chloropyrimidine in dichloromethane (10 mL) was added trifluoroacetic acid (10 mL) under ice-cooling. The reaction mixture was stirred at the same temperature for 2 hours. The reaction mixture was concentrated and the

obtained residue was dissolved in ethanol, and the solution was made alkaline with addition of BioRad AG (registered trademark) 1-X8 ion-exchange resin. The mixture was filtered, and the filtrate was concentrated to obtain 4-amino-4-methyl-1-(2-pyrimidinyl)piperidine (1.09 g, 5.68 mmol).
yield: 95%
$^1$H NMR ($CDCl_3$) δ (ppm): 8.29 (2H, d, J = 4.6 Hz), 6.44 (1H, t, J = 4.6 Hz), 3.96-3.87 (2H, m), 3.83-3.72 (2H, m), 1.65-1.55 (2H, m), 1.52-1.43 (2H, m), 1.36 (2H, br s), 1.20 (3H, s).
APCIMS (m/z): 193 $(M + H)^+$

Reference example 49: 4-Amino-4-methyl-1-(4-pyrimidinyl)piperidine

(1) To a solution of 4-tert-butoxycarbonylamino-4-methylpiperidine (1.28 g, 6.0 mmol) described in European Patent 647,639 in tetrahydrofuran (25 mL) were added triethylamine (2.78 mL, 20.0 mmol) and 4,6-dichloropyrimidine (1.79 g, 12.0 mmol) under ice-cooling, and the mixture was stirred at room temperature overnight. After the reaction mixture was filtered, the filtrate was concentrated. The obtained residue was purified by silica gel column chromatography (hexane/ethyl acetate = 3/1) to obtain 4-tert-butoxycarbonylamino-1-(6-chloro-4-pyrimidinyl)-4-methylpiperidine (1.96 g, 6.00 mmol).
$^1$H NMR ($CDCl_3$) δ (ppm) : 8.35 (1H, s), 6.51 (1H, s), 4.47 (1H, br s), 3.92 (2H, m), 3.36 (2H, ddd, J = 13.8, 10.5, 3.2 Hz), 2.12 (2H, d, J = 14.0 Hz), 1.59 (2H, ddd, J = 14.0, 10.5, 3.5 Hz), 1.44 (9H, s), 1.39 (3H, s).
APCIMS (m/z): 327 $(^{35}ClM + H)^+$, 329 $(^{37}ClM + H)^+$
(2) To a solution of 4-tert-butoxycarbonylamino-1-(6-chloro-4-pyrimidinyl)-4-methylpiperidine (1.96 g, 6 mmol) in ethanol (50 mL) were added 10% palladium on carbon (1.00 g, containing 50% water) and ammonium formate (2.78 g, 60.0 mmol), and the mixture was refluxed for two hours. After the reaction mixture was stand for cooling to room temperature and filtered, and then the filtrate was concentrated to obtain 4-amino-4-methyl-1-(4-pyrimidinyl)piperidine (1.75 g, 6.00 mmol).
yield: 100%
$^1$H NMR ($CDCl_3$) δ (ppm) : 8.58 (1H, d, J = 1.1 Hz), 8.18 (1H, d, J = 6.5 Hz), 6.55 (1H, dd, J = 6.5, 1.1 Hz), 4.48 (1H, br s), 3.97 (2H, m), 3.38 (2H, ddd, J = 13.8, 10.3, 3.5 Hz), 2.13 (2H, d, J = 14.0 Hz), 1.60 (2H, ddd, J = 14.0, 10.3, 4.3 Hz), 1.44 (9H, s), 1.39 (3H, s) APCIMS (m/z): 293 $(M + H)^+$
(3) To a solution of 4-tert-butoxycarbonylamino-methyl-1-(4-pyrimidinyl) piperidine (1.75 g, 6.00 mmol) in dichloromethane (10 mL) was added trifluoroacetic acid(10 mL) under ice-cooling. The reaction mixture was stirred at the same temperature for 3 hours. The reaction mixture was concentrated and the obtained residue was dissolved in ethanol, and the solution was made alkaline with addition of BioRad AG (registered trademark) 1-X8 ion-exchange resin. After the reaction mixture was filtered, the filtrate was concentrated to obtain 4-amino-4-methyl-1-(4-pyrimidinyl)piperidine (1.15 g, 6.00 mmol).
yield: 100%
$^1$H NMR ($CDCl_3$) δ (ppm): 8.57 (1H, d, J = 1.4 Hz), 8.16 (1H, d, J = 6.2 Hz), 6.51 (1H, dd, J = 6.2, 1.4 Hz), 3.83-3.70 (2H, m), 3.68-3.55 (2H, m), 1.65-1.55 (2H, m), 1.55-1.45 (2H, m), 1.20 (3H, s).
APCIMS (m/z): 193 $(M + H)^+$

Reference example 50: 4-Amino-4-methyl-1-(5-trifluoromethyl-2-pyridyl)piperidine

(1) To a solution of 4-tert-butoxycarbonylamino-4-methylpiperidine (1.28 g, 6.0 mmol) described in European Patent No. 647,639 in 1,4-dioxane (25 mL) were added potassium carbonate (4.15 g, 30.0 mmol) and 2-chloro-5-trifluoromethylpyridine (1.79 g, 18.0 mmol), and the mixture was refluxed overnight. After the reaction mixture was stand for cooling to room temperature and filtered, the filtrate was concentrated. The obtained residue was purified by silica gel column chromatography (hexane/ethyl acetate = 1/1) to obtain 4-tert-butoxycarbonylamino-4-methyl-1-(5-trifluoromethyl-2-pyridyl) piperidine (1.64 g, 4.57 mmol).
$^1$H NMR ($CDCl_3$) δ (ppm): 8.37 (1H, d, J = 2.7 Hz), 7.60 (1H, dd, J = 8.9, 2.7 Hz), 6.65 (1H, d, J = 8.9 Hz), 4.41 (1H, br s), 3.92 (2H, ddd, J = 13.5, 4.5, 4.5 Hz), 3.36 (2H, ddd, J = 13.5, 10.5, 3.0 Hz), 2.08 (2H, d, J = 13.5 Hz), 1.63 (2H, ddd, J = 13.5, 10.0, 3.5 Hz), 1.44 (9H, s), 1.39 (3H, s).
APCIMS (m/z): 360 $(M + H)^+$
(2) To a solution of 4-tert-butoxycarbonylamino-4-methyl-1-(5-trifluoromethyl-2-pyridyl)piperidine (1.64 g, 4.57 mmol) in dichloromethane (10 mL) was added trifluoroacetic acid (10 mL) under ice-cooling. The reaction mixture was stirred at the same temperature for 3 hours. The reaction mixture was concentrated and the obtained residue

was dissolved in ethanol, and the solution was made alkaline with addition of BioRad AG (registered trademark) 1-X8 ion-exchange resin. After the reaction mixture was filtered, the filtrate was concentrated to obtain 4-amino-4-methyl-1-(5-trifluoromethyl-2-pyridyl)piperidine (1.15 g, 4.44 mmol).
yield: 74%
$^1$H NMR ($CDCl_3$) δ (ppm): 8.37 (1H, d, J = 2.4 Hz), 7.59 (1H, dd, J = 9.2, 2.4 Hz), 6.64 (1H, d, J = 9.2 Hz), 3.80-3.58 (4H, m), 1.6,7-1.55 (2H, m), 1.55-1.45 (2H, m), 1.34 (2H, br s), 1.20 (3H, s).
APCIMS (m/z): 260 $(M + H)^+$

In the following Reference example 51, 52, the title compounds were obtained in a similar manner to that of Reference example 50 by using the corresponding halide instead of 2-chloro-5-trifluoromethylpyridine.

Reference example 51: 4-Amino-1-(5-chloro-2-pyridyl)-4-methylpiperidine

yield: 23%
$^1$H NMR (DMSO-$d_6$) δ (ppm): 8.09 (1H, d, J = 2.7 Hz), 7.39 (1H, dd, J = 9.2, 2.7 Hz), 6.60 (1H, d, J = 9.2 Hz), 3.65-3.45 (4H, m), 1.80-1.50 (4H, m), 1.19 (3H, s).
APCIMS (m/z): 226 $(^{35}ClM + H)^+$, 228 $(^{37}ClM + H)^+$

Reference example 52: 4-Amino-4-methyl-1-(2-pyridyl)piperidine

yield: 53%
$^1$H NMR (DMSO-$d_6$) δ (ppm): 8.17 (1H, ddd, J = 4.9, 1.9, 0.8 Hz), 7.45 (1H, ddd, J = 8.6, 7.3, 1.9 Hz), 6.66 (1H, d, J = 8.6 Hz), 6.56 (1H, ddd, J = 7.3, 4.9, 0.8 Hz), 3.64-3.51 (4H, m), 1.70-1.50 (4H, m), 1.49 (2H, br s), 1.19 (3H, s).
APCIMS (m/z): 192 $(M + H)^+$

Reference example 53: 4-Amino-4-methyl- 1-(5-phenyl-2-pyridyl)piperidine

(1) To a solution of 4-tert-butoxycarbonylamino-4-piperidine (1.71 g, 8.00 mmol) described in European Patent No. 647,639 in 1,4-dioxane (30 mL) were added potassium carbonate (11.1 g, 80.0 mmol) and 2,5-dibromopyridine (9.48 g, 40.0 mmol), and the mixture was refluxed for 5 days. After the reaction mixture was stand for cooling to room temperature and filtered, the filtrate was concentrated. The obtained residue was purified by silica gel column chromatography (hexane/ethyl acetate = 1/1) to obtain 1-(5-bromo-2-pyridyl)-4-tert-butoxycarbonylamino-4-methylpiperidine (2.79 g, 7.59 mmol).
yield: 94%
$^1$H NMR ($CDCl_3$) δ (ppm): 8.17 (1H, d, J = 2.7 Hz), 7.51 (1H, dd, J = 8.9, 2.7 Hz), 6.56 (1H, d, J = 8.9 Hz), 4.40 (1H, br s), 3.77 (2H, ddd, J = 13.8, 4.5, 4.5 Hz), 3.25 (2H, ddd, J = 13.8, 10.8, 3.0 Hz), 2.06 (2H, d, J = 13.5 Hz), 1.63 (2H, ddd, J = 13.5, 9.7, 3.8 Hz), 1.44 (9H, s), 1.38 (3H, s)
APCIMS (m/z): 370 $(79BrM + H)^+$, 372 $(^{81}BrM + H)^+$
(2) To a mixture of palladium acetate (13.4 mg, 0.060 mmol), di-tert-butylorthobiphenylphosphine (36.0 mg, 0.12 mmol) described in J. Am. Chem. Soc., 121, 9550 (1999), phenylboronic acid (1.10 g, 9.0 mmol), potassium fluoride (1.04 g, 18.0 mmol), and 1-(5-bromo-2-pyridyl)-4-tert-butoxycarbonylamino-4-methyl piperidine (1.92 g, 5.19 mmol) was added THF (6 mL) under an argon atmosphere, and the mixture was stirred at room temperature overnight. The reaction mixture was filtered, and the filtrate was concentrated. The obtained residue was purified by silica gel column chromatography [Chromatrex (registered trademark) NH, Fuji Silysia, ethyl acetate to ethyl acetate/methanol = 9/1] to obtain 4-tert-butoxycarbonylamino-4-methyl-1-(5-phenyl-2-pyridyl)piperidine (1.86 g, 5.07 mmol).
yield: 98%
$^1$H NMR ($CDCl_3$) δ (ppm) : 8.43 (1H, d, J = 2.4 Hz), 7.71 (1H, dd, J = 8.6, 2.4 Hz), 7.52 (2H, d, J = 7.3 Hz), 7.42 (2H, dd, J = 7.3, 7.3 Hz), 7.31 (1H, m), 6.74 (1H, d, J = 8.6 Hz), 4.44 (1H, br s), 3.87 (2H, ddd, J = 13.5, 4.6, 4.6 Hz), 3.31 (2H, ddd, J = 13.5, 10.8, 3.0 Hz), 2.09 (2H, d, J = 14.0 Hz), 1.68 (2H, ddd, J = 14.0, 9.7, 4.3 Hz), 1.44 (9H, s), 1.41 (3H, s).
APCIMS (m/z): 368 $(M + H)^+$
(3) To a solution of 4-tert-butoxycarbonylamino-4-methyl-1-(5-phenyl-2-pyridyl)piperidine (1.86 g, 5.07 mmol) in dichloromethane (10 mL) was added trifluoroacetic acid (10 mL) under ice-cooling. The reaction mixture was stirred at room temperature for 3 hours. The reaction mixture was concentrated, and the obtained residue was dissolved in ethanol. The solution was made alkaline with addition of BioRad AG (registered trademark) 1-X8 ion-exchange

resin. After the reaction mixture was filtered, the filtrate was concentrated to obtain 4-amino-4-methyl-1-(5-phenyl-2-pyridyl)piperidine (1.25 g, 4.69 mmol).
yield: 93%
$^1$H NMR ($CDCl_3$) δ (ppm) : 8.44 (1H, d, J = 2.7 Hz), 7.70 (1H, dd, J = 8.9, 2.7 Hz), 7.51 (2H, d, J = 7.3 Hz), 7.41 (2H, dd, J = 7.3, 7.3 Hz), 7.31 (1H, m), 6.74 (1H, d, J = 8.9 Hz), 3.72-3.48 (4H, m), 1.72-1.51 (4H, m), 1.48 (2H, br s), 1.20 (3H, s).
APCIMS (m/z): 268 $(M + H)^+$

Reference example 54: 4-Amino-1-(5-cyano-2-pyridyl)-4-ethylpiperidine

(1) To tetrahydrofuran (200 mL) was added ethylmagnesium bromide (1.0 mol/L solution in THF, 100 mL, 100 mmol) under an argon atmosphere. To the solution was added dropwise for 30 minutes a solution (100 mL) of 1-benzyl-4-piperidone (11.1 mL, 60.0 mmol) in THF under ice-cooling, and the mixture was stirred at room temperature for 1.5 hours. The reaction mixture was poured into an aqueous saturated ammonium chloride solution, and the mixture was extracted three times with ethyl acetate. The combined organic layer was washed with saturated aqueous sodium hydrogencarbonate solution and saturated brine, dried over anhydrous magnesium sulfate, and concentrated. The obtained residue was purified by silica gel column chromatography to obtain 1-benzyl-4-ethyl-4-hydroxypiperidine (12.8 g, 58.4 mmol).
yield: 94%
$^1$H NMR ($CDCl_3$) δ (ppm): 7.32-7.22 (5H, m), 3.52 (2H, s), 2.62 (2H, ddd, J = 11.9, 4.1, 4.1 Hz), 2.33 (2H, ddd, J = 11.3, 3.2, 3.2 Hz), 1.64 (2H, ddd, J = 12.4, 4.33, 4.33 Hz), 1.60-1.40 (2H, m), 1.49 (2H, q, J = 7.6 Hz), 0.91 (3H, t, J = 7.6 Hz).
APCIMS (m/z): 220 $(M + H)^+$
(2) To a solution of 1-benzyl-4-ethyl-4-hydroxypiperidine (12.8 g, 58.4 mmol) in acetonitrile (70 mL) was added dropwise for one hour concentrated sulfuric acid (60.0 mL) with the inner temperature kept under 30°C, and the mixture was stirred at room temperature overnight. The reaction mixture was poured into ice-water, and the mixture was adjusted to pH 10 with 50% aqueous potassium hydroxide solution while the mixture was being cooled. The mixture was extracted three times with chloroform. The combined organic layer was dried over sodium sulfate, and concentrated to obtain 4-acetylamino-1-benzyl-4-ethylpiperidine (14.3 g, 54.9 mmol).
yield: 94%
$^1$H NMR ($CDCl_3$) δ (ppm): 7.40-7.20 (5H, m), 5.00 (1H, brs), 3.49 (2H, s), 2.63 (2H, m), 2.20-1.90 (4H, m), 1.97 (3H, s), 1.81 (2H, q, J = 7.6 Hz), 1.58 (2H, ddd, J = 14.6, 10.4, 4.2 Hz), 0.79 (3H, t, J = 7.6 Hz).
APCIMS (m/z): 261 $(M + H)^+$
(3) 4-Acetylamino-1-benzyl-4-ethylpiperidine (14.3 g, 54.9 mmol) was dissolved in 6 mol/L hydrochloric acid (200mL), and the mixture was refluxed for 3 days. After cooling to room temperature, the mixture was adjusted to pH 12 with 50% aqueous sodium hydroxide solution and extracted three times with chloroform. The combined organic layer was dried over sodium sulfate, and concentrated to obtain 4-amino-1-benzyl-4-ethylpiperidine (10.3 g, 47.2 mmol).
yield: 94%
$^1$H NMR ($CDCl_3$) δ (ppm) : 7.40-7.20 (5H, m), 3.51 (2H, s), 2.56 (2H, ddd, J = 14.0, 4.3, 4.3 Hz), 2.32 (2H, ddd, J = 11.3, 11.3, 2.7 Hz), 1.60 (2H, ddd, J = 14.0, 9.7, 4.3 Hz), 1.44-1.36 (4H, m), 1.17 (2H, br s), 0.88 (3H, t, J = 7.4 Hz).
APCIMS (m/z): 219 $(M + H)^+$
(4) To a solution of 4-amino-1-benzyl-4-ethylpiperidine (10.3 g, 47.2 mmol) in chloroform (200 mL) was added di-tert-butyldicarbonate (13.0 mL, 56.6 mmol) under ice-cooling, and the mixture was stirred at room temperature overnight. After the solvent was concentrated, the obtained residue was purified by silica gel column chromatography to obtain 1-benzyl-4-tert-butoxycarbonylamino-4-ethylpiperidine (13.8 g, 43.3 mmol).
yield: 94%
$^1$H NMR ($CDCl_3$) δ (ppm) : 7.40-7.20 (5H, m), 4.20 (1H, br s), 3.49 (2H, s), 2.65-2.55 (2H, m), 2.25-2.15 (2H, m), 2.00-1.90 (2H, m), 1.71 (2H, q, J = 7.5 Hz), 1.55-1.45 (2H, m), 1.43 (9H, s), 0.81 (3H, t, J = 7.5 Hz).
APCIMS (m/z): 319 $(M + H)^+$
(5) To a solution of 1-benzyl-4-tert-butoxycarbonylamino-4-ethylpiperidine (13.8 g, 43.3 mmol) in ethanol (90 mL) was added 10% palladium on carbon (2.0 g, containing 50% water), and a hydrogen gas addition was conducted to the mixture in an ordinary atmosphere. After the mixture was stirred for 2 days, the catalyst was removed by filtration. The filtrate was evaporated under reduced pressure to obtain 4-tert-butoxycarbonylamino-4-ethylpiperidine (5.75 g, 25.2 mmol).
yield: 94%

$^1$H NMR ($CDCl_3$) δ (ppm): 4.30 (1H, br s), 2.95-2.75 (4H, m), 1.94 (2H, d, J = 13.2 Hz), 1.72 (2H, q, J = 7.4 Hz), 1.60-1.40 (2H, m), 1.43 (9H, s), 0.82 (3H, t, J = 7.4 Hz).
APCIMS (m/z): 229 $(M + H)^+$
(6) To a solution of 4-tert-butoxycarbonylamino-4-piperidine (912 mg, 4.00 mmol) in 1,4-dioxane (20 mL) were added potassium carbonate (2.21 g, 16.0 mmol) and 2-chloro-5-cyanopyridine (1.11 g, 8.00 mmol), and the mixture was refluxed overnight. After the reaction mixture was cooled to room temperature, water was added, and the solution was extracted three times with chloroform. The combined organic layer was washed with saturated brine, dried over anhydrous magnesium sulfate and concentrated. The obtained residue was purified by silica gel column chromatography to obtain 4-tert-butoxycarbonylamino-1-(5-cyano-2-pyridyl)-4-ethylpiperidine (970 mg, 2.93 mmol).
yield: 50 %
$^1$H NMR ($CDCl_3$) δ (ppm): 8.39 (1H, d, J = 2.4 Hz), 7.58 (1H, dd, J = 9.2, 2.4 Hz), 6.61 (1H, d, J = 9.2 Hz), 4.34 (1H, br s), 4.09 (2H, ddd, J = 13.8, 3.5, 3.5 Hz), 3.27 (2H, ddd, J = 13.8, 11.1, 2.7 Hz), 2.13 (2H, d, J = 13.5 Hz), 1.76 (2H, q, J = 7.6 Hz), 1.54 (2H, ddd, J = 13.5, 9.5, 2.2 Hz), 1.44 (9H, s), 0.86 (3H, t, J = 7.6 Hz).
APCIMS (m/z): 331 $(M + H)^+$
(7) To a solution of 4-tert-butoxycarbonylamino--2-pyridyl)-4-ethylpiperidine (970 mg, 2.93 mmol) in 1,4-dioxane (5 mL) was added a 4 mol/L solution of hydrogen chloride in 1,4-dioxane (20 mL) under ice-cooling, and the mixture was stirred at room temperature for 1 hour. The reaction mixture was concentrated and the obtained residue was dissolved in ethanol. The solution was made alkaline with addition of BioRad AG (registered trademark) 1-X8 ion-exchange resin. The reaction mixture was filtered, and the filtrate was concentrated to obtain 4-amino-1-(5-cyano-2-pyridyl)-4-ethylpiperidine (487 mg, 2.12 mmol).
yield: 72%
$^1$H NMR ($CDCl_3$) δ (ppm): 8.38 (1H, d, J = 2.2 Hz), 7.56 (1H, dd, J = 9.2, 2.2 Hz), 6.60 (1H, d, J = 9.2 Hz), 3.98 (2H, ddd, J = 13.5, 3.8, 3.8 Hz), 3.50 (2H, ddd, J = 13.5, 10.0, 3.5 Hz), 1.65-1.50 (4H, m), 1.44 (2H, q, J = 7.4 Hz), 1.26 (2H, br s), 0.91 (3H, t, J = 7.4 Hz).
APCIMS (m/z): 231 $(M + H)^+$

Reference example 55: 4-Amino-1-(5-cyano-2-pyridyl)-4-phenylpiperidine

(1) To a solution of 4-tert-butoxycarbonylamino-4-phenylpiperidine (1.11g, 4.0 mmol) described in WO 01/07050 in 1,4-dioxane (16 mL) were added potassium carbonate (2.21 g, 16.0 mmol) and 2-chloro-5-cyanopyridine (1.11 g, 8.00 mmol), and the mixture was refluxed for three days. After the reaction mixture was cooled to room temperature, water was added to the solution. The mixture was extracted with chloroform three times. The combined organic layer was washed with satutrted brine, dried over anhydrous magnesium sulfate, and concentrated. The obtained residue was partially purified by silica gel column chromatography to obtain a mixture of 4-tert-butoxycarbonylamino-1-(5-cyano-2-pyridyl)-4-phenylpiperidine and unreacted 2-chloro-5-cyanopyridine (1.90 g).
$^1$H NMR ($CDCl_3$) δ (ppm) : 8.41 (1H, d, J = 2.2 Hz), 7.61 (1H, dd, J = 9.2, 2.2 Hz), 7.30-7.10 (5H, m), 6.66 (1H, d, J = 9.2 Hz), 4.95 (1H, br s), 4.28 (2H, d, J = 13.5 Hz), 3.36 (2H, dd, J = 11.6, 11.6 Hz), 2.39 (2H, m), 2.07 (2H, m), 1.37 (9H, s).
APCIMS (m/z): 379 $(M + H)^+$
(2) To a solution of a mixture of 4-tert-butoxycarbonylamino-1-(5-cyano-2-pyridyl)-4-phenylpiperidine and 2-chloro-5-cyanopyridine (1.90 g) in 1,4-dioxane (5 mL) was added a 4 mol/L solution of hydrogen chloride in 1,4-dioxane (20 mL) under ice-cooling, and the mixture was stirred at room temperature for 1 hour. The reaction mixture was concentrated and the obtained residue was dissolved in ethanol, and the solution was made alkaline with addition of BioRad AG (registered trademark)1-X8 ion-exchange resin. The reaction mixture was filtered and the filtrate was concentrated. The obtained residue was purified by silica gel column chromatography to obtain 4-amino-1-(5-cyano-2-pyridyl)-4-phenylpiperidine (647 mg, 2.33 mmol).
yield: 58%
$^1$H NMR ($CDCl_3$) δ (ppm): 8.41 (1H, d, J = 2.2 Hz), 7.59 (1H, dd, J = 9.5, 2.2 Hz), 7.45 (2H, m), 7.36 (2H, m), 7.25 (1H, m), 6.64 (1H, d, J = 9.5 Hz), 4.13 (2H, ddd, J = 13.5, 3.8, 3.8 Hz), 3.64 (2H, ddd, J = 13.5, 10.8, 2.7 Hz), 2.14 (2H, ddd, J = 13.5, 9.5, 4.1 Hz), 1.80 (2H, d, J = 13.5 Hz), 1.53 (2H, br s).
APCIMS (m/z): 279 $(M + H)^+$

Reference example 56: 2-Amino-N-(5-methoxycarbonyl-2-pyridyl)-2-methyl-amine

To a solution of methyl 6-chloronicotinate (3.42 g, 20.0 mmol) in 2-propanol(20 mL) were added diisopropylethylamine (4.18 mL, 24.0 mmol) and 1,2-diamino-2-methylpropane (3.14 mL, 30.0 mmol), and the mixture was refluxed at 80°C for 48 hours. The reaction mixture was concentrated under reduced pressure, and chloroform and aqueous potassium carbonate solution were added to the residue. The aqueous layer was extracted with chloroform. The organic layer was dried over anhydrous magnesium sulfate, and the solvent was evaporated under reduced pressure. The obtained residue was purified by silica gel column chromatography (Chromatorex (registered trademark) NH, Fuji Silysia, hexane/ethyl acetate =5/1) to obtain the title compound (2.31 g, 10.3 mmol).
yield: 52%
$^1$H NMR ($CDCl_3$) δ (ppm): 8.71 (1H, d, J = 2.3 Hz), 7.95 (1H, dd, J = 8.9, 2.3 Hz), 6.40 (1H, d, J = 8.9 Hz), 5.53 (1H, t, J = 5.9 Hz), 3.86 (3H, s), 3.27 (2H, d, J = 5.9 Hz), 1.30 (2H, br s), 1.18 (6H, s).
APCIMS (m/z): 224 $(M + H)^+$

Reference example 57: 2-Amino-2-methyl-N-(5-methyl-2-pyridyl)propylamine

The title compound (678 mg, 3.78 mmol) was obtained in a similar manner to that of Reference example 13 by using 2-bromo-5-methylpyridine (1.72 g, 10.0 mmol) instead of 2,5-dichloropyridine.
yield: 38%
$^1$H NMR ($CDCl_3$) δ (ppm): 7.88 (1H, d, J = 2.3 Hz), 7.22 (1H, dd, J = 8.3, 2.3 Hz), 6.38 (1H, d, J = 8.3 Hz), 4.72 (1H, t, J = 6.2 Hz), 3.18 (2H, d, J = 6.2 Hz), 2.16 (3H, s), 1.40 (2H, br s), 1.17 (6H, s).
APCIMS (m/z): 180 $(M + H)^+$

Reference example 58: 2-Amino-N-(5-isopropyl-2-pyridyl)-2-methylpropylamine

(1) 2-(6-Chloro-3-pyridyl)-2-propanol
To a solution of methyl 6-chloronicotinate (1.72 g, 10.0 mmol) in THF (30 mL) was added a 3 mol/L solution of methyl magnesium bromide in THF (7.33 mL, 22.0 mmol) at -30°C, and the mixture was stirred at 0°C for 3 hours. Saturated aqueous ammonium chloride solution was added to the reaction mixture, which was then extracted with ethyl acetate. The organic layer was washed with saturated brine, and dried over anhydrous magnesium sulfate. The solvent was evaporated under reduced pressure. The obtained residue was purified by silica gel column chromatography (chloroform/methanol=40/1) to obtain 2-(6-chloro-3-pyridyl)-2-propanol (1.71 g, 10.0 mmol).
yield: quantitative
$^1$H NMR ($CDCl_3$) δ (ppm): 8.49 (1H, d, J = 2.6 Hz), 7.79 (1H, dd, J = 8.4, 2.6 Hz), 7.29 (1H, d, J = 8.4 Hz), 1.60 (6H, s).
APCIMS (m/z): 172 $(M + H)^+$

(2) 2-Amino-N-(5-isopropenyl-2-pyridyl)-2-methylpropylamine
To 2-(6-chloro-3-pyridyl)-2-propanol (1.71 g, 10.0 mmol) obtained in (1) was added 1,2-diamino-2-methylpropane (5.14 mL, 50.0 mmol), and the mixture was refluxed at 140°C for 72 hours. After the reaction mixture was concentrated under reduced pressure, dichloromethane and a 2 mol/L aqueous sodium hydroxide solution was added to the solution. The aqueous layer was extracted with dichloromethane. After the organic layer was dried over anhydrous magnesium sulfate, the solvent was evaporated under reduced pressure. The obtained residue was purified by silica gel column chromatography (Chromatorex (registered trademark) NH, Fuji Silysia, ethyl acetate) to obtain 2-amino-N-(5-isopropenyl-2-pyridyl)-2-methylpropylamine(102 mg, 0.456 mmol).
yield: 5%
$^1$H NMR ($CDCl_3$) δ (ppm): 8.19 (1H, d, J = 2.3 Hz), 7.55 (1H, dd, J = 8.8, 2.3 Hz), 6.41 (1H, d, J = 8.8 Hz), 5.22 (1H, s), 5.00-4.90 (2H, m), 3.23 (2H, d, J = 6.5 Hz), 3.07 (3H, s), 1.48 (2H, br s), 1.18 (6H, s).
APCIMS (m/z): 206 $(M + H)^+$

(3) To a solution of 2-amino-N-(5-isopropenyl-2-pyridyl)-2-methylpropylamine (102 mg, 0.456 mmol) obtained in (2) in methanol (4 mL) were added ammonium formate (631 mg, 10.0 mmol) and 10% palladium on carbon (36 mg, containing 50% water), and the mixture was stirred at 70°C for 3 hours. The reaction mixture was filtered using Celite as a filtration aid, and the methanol was evaporated under reduced pressure. To the obtained residue were added dichloromethane and a 2 mol/L aqueous sodium hydroxide solution. The aqueous layer was extracted with dichloromethane. After the organic layer was dried over anhydrous magnesium sulfate, the solvent was evaporated under reduced pressure to obtain the title compound (74 mg, 0.357 mmol).
yield: 71%
$^1$H NMR ($CDCl_3$) δ (ppm): 7.92 (1H, d, J = 2.4 Hz), 7.29 (1H, dd, J = 8.5, 2.4 Hz), 6.42 (1H, d, J = 8.5 Hz), 4.83 (1H, t, J = 6.2 Hz), 3.20 (2H, d, J = 6.2 Hz), 2.83-2.73 (1H, m), 1.94 (2H, br s), 1.20 (6H, d, J = 7.0 Hz), 1.18 (6H, s).
APCIMS (m/z): 208 $(M + H)^+$

Reference example 59: N-(2-Aminoethyl)-2-quinoxalinecarboxamide

To a solution of tert-butyl N-(2-aminoethyl)carbamate (770 mL, 4.85 mmol) and triethylamine (811 mL, 5.82 mmol) in chloroform (15 mL) was added a solution of 2-quinoxalinecarbonylchloride (1.03 g, 5.34 mmol) in chloroform (5 mL). After the mixture was refluxed at the same temperature for 2 hours, the organic layer was washed with water, and dried over anhydrous magnesium sulfate. The anhydrous magnesium sulfate was removed by filtration and the solvent was evaporated under reduced pressure. After the obtained residue was dissolved in chloroform (18 mL), trifluoroacetic acid (18 mL) was added to the solution, and the mixture was stirred at room temperature for 1 hour. The reaction mixture was concentrated under reduced pressure. A solution of the residue in methanol was neutralized by basic ion-exchange resin. The resin was removed by filtration, and the filtrate was concentrated under reduced pressure to obtain the title compound (867 mg, 4.01 mmol) as oil.
yield: 83%
APCIMS (m/z): 217 $(M + H)^+$
Compounds of Reference example 60 to 62 were obtained in a similar manner to that of Reference example 59 by using a corresponding acid chloride or sulfonylchloride instead of 2-quinoxalinecarbonylchloride.

Reference example 60: N-(2-Aminoethyl)-2-furanecarboxamide

yield: quantitative
APCIMS (m/z): 155 $(M + H)^+$

Reference example 61: N-(2-Aminoethyl)benzamide

yield: quantitative
APCIMS (m/z): 165 $(M + H)^+$

Reference example 62: N-(2-Aminoethyl)benzenesulfonamide

yield: quantitative
APCIMS (m/z): 201 $(M + H)^+$
Compounds of Reference example 63 to 65 were obtained in a similar manner to that of Reference example 59 by using tert-butyl piperidin-4-ylcarbamate instead of tert-butyl aminoethylcabamate from a corresponding acid chloride or sulfonylchloride.

Reference example 63: 4-Amino-1-benzoylpiperidine

yield: quantitative
APCIMS (m/z): 205 $(M + H)^+$

Reference example 64: 4-Amino-1-nicotinoylpiperidine

yield: quantitative
APCIMS (m/z): 206 $(M + H)^+$

Reference example 65: 4-Amino-1-benzenesulfonylpiperidine

yield: quantitative
APCIMS (m/z) : 241 $(M + H)^+$

Reference example 66: 2-Amino-N-[5-(N,N-dimethylaminosulfonyl)-2-pyridyl]-2-methylpropylamine

(1) 2-Chloropyridine-5-sulfonylchloride (1.00 g, 4.72 mmol) described in WO 98/40332 was dissolved in tetrahydrofuran (10 mL). To the solution were added triethylamine (657 μL) and dimethylamine (468 μL, 5.19 mmol), and the mixture was stirred at room temperature for two hours. The solvent was evaporated under reduced pressure. To the residue were added water and chloroform, and the solution were separated. The resulting organic layer was dried over anhydrous magnesium sulfate. The solvent was evaporated under reduced pressure to obtain

2-chloro-5-(N,N-dimethylaminosulfonyl)pyridine (1.04 g, quantitative).

(2) To a solution of the compound (900 mg, 4.09 mmol) obtained in (1) in 1,4-dioxane (4 mL) were added potassium carbonate (565 mg, 4.09 mmol) and 1,2-diamino-2-methylpropane (643 mL, 6.13 mmol), and the mixture was refluxed for 4.5 hours. The reaction mixture was filtered under reduced pressure, and the solids obtained by the filtration were washed with 1,4-dioxane (80 mL) and methanol(30 mL). The resulting filtrate was evaporated under reduced pressure, and the residue was purified by silica gel column chromatography (Chromatorex (registered trademark) NH Fuji Silysia, ethyl acetate/methanol = 24 /1) to obtain the title compound (625 mg, 2.39 mmol) as a yellow solid.

yield: 56 %

$^1$H NMR (DMSO-d6) δ (ppm) : 8.23 (1H, d, J = 2.3 Hz), 7.58 (1H, dd, J = 8.9, 2.3 Hz), 7.35 (1H, br s), 6.68 (1H, d, J = 8.9 Hz), 3.23 (1H, d, J = 4.6 Hz), 2.55 (6H, s), 1.02 (6H, s).

APCIMS (m/z): 273 (M + H)+

Reference example 67: 2-Amino-2-methyl-N-[5-(pyperidinosulfonyl)-2-pyridyl] amine

The title compound was obtained in a similar manner to that of Reference example 66 by using piperidine instead of dimethylamine.

yield: 65%

APCIMS (m/z): 313 $(M + H)^+$

Reference example 68: 2-Amino-2-methyl-N-[5-(1,2,3,4-tetrahydroisoquinoline-ylsulfonyl)-2-pyridyl]propylamine

The title compound was obtained in a similar manner to that of Reference example 66 by using 1,2,3,4-tetrahydroisoquinoline instead of dimethylamine.

yield: quantitative

$^1$H NMR (DMSO-$d_6$) δ(ppm) : 8.31 (1H, d, J = 2.3 Hz), 7.64 (1H, d, J = 8.9, 2.3 Hz), 7.52-(1H, br s), 7.11-7.14 (4H, m), 6.69 (1H, d, J = 8.9 Hz), 6.42 (1H, br s), 4.13 (2H, s), 3.26-3.22 (2H, m), 2.85 (2H, t, J = 5.6 Hz), 1.01 (6H, s), 0.93 (2H, s).

APCIMS (m/z): 361 $(M + H)^+$

Reference example 69-1: 2-Amino-2-methyl-N-(5-morpholinosulfonyl-2-pyridyl) amine

Reference example 69-2-Amino-2-methyl-N-[5-(1,3-thiazolidin-3-ylsulfonyl)-pyridylpropylamine

(1) To a suspension of 2-chloropyridine-5-chloride (254 mg, 1.20 mmol) described in WO 98/40332 in tetrahydrofuran (5 mL) was added morpholine (1.44 mmol) or 1,3-thiazolidine (1.44 mmol), and triethylamine (167 μL, 1.20 mmol), and the mixture was stirred for 2.5 hours. Chloroform (9.6 mL), polystyrenecarbonylchloride (2-3 mmol/g, 276 mg), and polyvinylpyridine (264 mg) were added to the obtained residue, and the mixture was stirred at room temperature overnight. After the resin was removed by filtration, the solvent was evaporated.

(2) To the obtained residue were added 1,4-dioxane (4.8 mL), 1,2-diamino-2-methylpropane (101 μL, 1.0 mmol), and potassium carbonate (164 mg, 1.2 mmol), and the mixture was heated with stirring at 100°C for 2 days. Chloroform (2.4 mL) and methanol (2.4 mL) was added to the reaction mixture, and the mixture was filtered. The solvent was evaporated, and to the obtained residue were added chloroform (3.6 mL) and methanol (3.6 mL). To the solution was added formylpolystyrene (1-2 mmol/g, 465 mg), and the mixture was stirred 2 overnights. The resin was removed by filtration and washed with chloroform (2.4 mL). The solvent was evaporated under reduced pressure to obtain each of titled amines. 2-Amino-2-methyl-N-(5-morpholinosulfonyl-2-pyridyl)propylamine

yield: 76%

$^1$H NMR ($CDCl_3$) δ (ppm) : 8.41 (1H, d, J = 2.3 Hz), 7.62 (1H, dd, J = 8.9, 2.3 Hz), 6.50 (1H, d, J = 8.9 Hz), 5.94 (1H, br t, J = 5.3 Hz), 3.75 (4H, t, J = 4.6 Hz), 3.31 (2H, d, J = 5.6 Hz), 2.99 (4H, t, J = 4.6 Hz), 1.89 (2H, br s), 1.19 (6H, s). 2-Amino-2-methyl-N-[5-(1,3-thiazolidin-3-ylsulfonyl)-2-pyridyl]propylamine

yield: 44%

$^1$H NMR ($CDCl_3$) δ (ppm) : 8.49 (1H, d, J = 2.3 Hz), 7.71 (1H, dd, J = 8.9, 2.3 Hz), 6.47 (1H, d, J = 8.9 Hz), 5.93 (1H, br s), 4.42 (2H, s), 3.61 (2H, t, J = 6.3 Hz), 3.30 (2H, d, J = 5.6 Hz), 2.80 (2H, t, J = 6.3 Hz), 1.92 (2H, br s), 1.19 (6H, s).

Reference example 70: 2-Amino-2-methyl-N-[5-(N-methyl-O-methyl-sulfonyl)-2-pyridyl]propylamine

To a suspension of 2-chloropyridine-5-sulfonylchloride (254 mg, 1.20 mmol) described in WO 98/40332 in tetrahydrofuran (5 mL) were added N-methyl-O-methylhydroxylamine hydrochloride (140mg, 1.44 mmol) and triethylamine (334 μL, 2.40 mmol), and the mixture was stirred at room temperature overnight. The reaction mixture was concentrated, and to the obtained residue were added water and chloroform. The solutions are separated and the organic layer was dried over anhydrous magnesium sulfate. The solvent was evaporated under reduced pressure, and the obtained residue was purified by silica gel column chromatography (hexane/ethyl acetate = 5/1) to obtain 2-chloro-5-(N-methyl-O-methylhydroxyamino)sulfonylpyridine (177mg).

In 1,4-dioxane (4 mL), to the pyridine derivative (175 mg, 0.740 mmol) obtained above were added 1,2-diamino-2-methylpropane (133 μL, 1.26 mmol) and potassium carbonate (152 mg, 1.10 mmol), and the mixture was stirred at 100°C for 16 hours. The reaction mixture was filtered and the filtrate was concentrated under reduced pressure. The obtained residue was purified by silica gel column chromatography (chloroform to chloroform/methanol = 93/7) to obtain the title compound (166 mg).
yield: 78%
$^1$H NMR ($CDCl_3$) δ (ppm) : 8.47 (1H, d, J = 2.3 Hz), 7.72 (1H, dd, J = 8.9, 2.3 Hz), 6.51 (1H, d, J = 8.9 Hz), 6.10 (1H, br t, J = 5.6 Hz), 3.79 (3H, s), 3.33 (2H, d, J = 5.9 Hz), 2.79 (3H, s), 2.20 (2H, br s), 1.19 (6H, s).
APCIMS (m/z): 289 $(M + H)^+$

Reference example 71: 2-Amino-N-[5-(N-cyclopropyl-N-methylaminosulfonyl)-2-pyridyl]-2-methylpropylamine

The title compound (275 mg) was obtained in a similar manner to that of Reference example 70 from 2-chloro-5-(N-cyclopropyl-N-methylaminosulfonyl)pyridine (230 mg, 0.933 mmol).
yield: 98%
$^1$H NMR ($CDCl_3$) δ (ppm) : 8.48 (1H, d, J = 2.3 Hz), 7.72 (1H, dd, J = 8.9, 2.3 Hz), 6.51 (1H, d, J = 8.9 Hz), 5.95 (1H, br s), 3.32 (2H, d, J = 5.6 Hz), 2.74 (3H, s), 2.16 (2H, br s), 1.87-1.81 (1H, m), 1.19 (6H, s), 0.88-0.83 (2H, m), 0.73-0.66 (2H, m).
APCIMS (m/z): 298 $(M + H)^+$

Reference example 72: 2-Amino-N-{5-[N-(2-hydroxyethyl)-N-methylaminosulfonyl]-2 - pyridyl}-2-methylpropylamine

The title compound (300 mg) was obtained in a similar manner to that of Reference example 70 from 2-chloro-5-[N-(2-hydroxyethyl)-N-methyl-pyridine (250 mg, 1.00 mmol).
yield: 99%
$^1$H NMR (DMSO-$d_6$) δ (ppm): 8.31 (1H, d, J = 2.6 Hz), 7.66 (1H, dd, J = 8.9, 2.3 Hz), 7.40 (1H, br t, J = 5.6 Hz), 6.73 (1H, d, J = 8.9 Hz), 4.81 (1H, br s), 3.56 (2H, t, J = 5.9 Hz), 3.30 (2H, d, J = 5.6 Hz), 3.22 (3H, s), 3.00 (2H, t, J = 5.9 Hz), 2.72 (3H, s), 1.09 (6H, s).
APCIMS (m/z): 303 $(M + H)^+$

Reference example 73: 2-Amino-N-5-(N-cyanomethyl-N-methylaminosulfonyl)-2-pyridyl-2-methylpropylamine

The title compound (70 mg) was obtained in a similar manner to that of Reference example 70 from 2-chloro-5-(N-cyanomethyl-N-methylaminosulfonyl) pyridine (169 mg, 0.688 mmol).
yield: 34%
$^1$H NMR ($CDCl_3$) δ (ppm): 8.49 (1H, d, J = 2.5 Hz), 7.69 (1H, dd, J = 8.9, 2.5 Hz), 6.50 (1H, d, J = 8.9 Hz), 5.88 (1H, br s), 4.18 (2H, s), 3.31 (2H, d, J = 5.6 Hz), 2.87 (3H, s), 1.79 (2H, br s), 1.19 (6H, s).
APCIMS (m/z): 298 (M + H)+

Reference example 74: 2-Amino-N-[5-(N-benzylaminosulfonyl)-2-pyridyl]-2-methyl propylamine

The title compound was obtained in a similar manner to that of Reference example 66 by using benzylamine instead of dimethylamine.
yield: 80%
$^1$H NMR (DMSO-$d_6$) δ (ppm) : 8.27 (1H, d, J = 2.6 Hz), 7.60 (1H, dd, J = 9.0, 2.6 Hz), 7.30-7.21 (5H, m), 6.62 (1H, d, J = 9.0 Hz), 3.94 (2H, s), 3.23 (2H, d, J = 5.6 Hz), 1.02 (6H, s).
APCIMS (m/z): 335 $(M + H)^+$

Reference example 75: 2-Amino-2-methyl-N-[5-(N-methylaminosulfonyl)-2-pyridyl] propylamine

The title compound was obtained in a similar manner to that of Reference example 66 by using methylamine hydrochloride instead of dimethylamine.
yield: 59%
$^1$H NMR (DMSO-$d_6$) δ (ppm) : 8.26 (1H, d, J = 2.3 Hz), 7.59 (1H, dd, J = 8.9, 2.3 Hz), 7.24 (1H, br s), 6.66 (1H, d, J = 8.9 Hz), 3.23 (2H, d, J = 5.6 Hz), 2.37 (3H, s), 1.01 (6H, s).
FABMS (m/z): 259 $(M + H)^+$

Reference example 76: 2-Amino-2-methyl-N-[5-(N-phenylaminosulfonyl)-2-pyridyl] propylamine

The title compound was obtained in a similar manner to that of Reference example 66 by using aniline instead of dimethylamine.
yield: 62%
$^1$H NMR (DMSO-$d_6$) δ (ppm) : 8.22 (1H, d, J = 2.3 Hz), 7.55 (1H, dd, J = 8.9, 2.6 Hz), 7.25-6.94 (5H, m), 6.57 (1H, d, J = 8.9 Hz), 3.18 (2H, d, J = 6.3 Hz), 0.99 (6H, s).
FABMS (m/z): 321$(M + H)^+$

Reference example 77: 2-Amino-N-{5-[N-(2-hydroxyethylamino)sulfonyl]-2-pyridyl}-2-methylpropylamine

The title compound was obtained in a similar manner to that of Reference example 66 by using ethanolamine instead of dimethylamine.
yield: 30%
$^1$H NMR (DMSO-$d_6$) δ (ppm) : 8.27 (1H, d, J = 2.3 Hz), 7.61 (1H, dd, J = 8.9, 2.3 Hz), 7.23 (1H, m), 6.65 (1H, d, J = 8.9 Hz), 4.67 (1H, br s), 3.20 (2H, d, J = 3.7 Hz), 2.89 (2H, s), 2.76 (4H, t, J = 6.3 Hz), 1.02 (6H, s).
APCIMS (m/z): 287 (M - H)-

Reference example 78: 2-Amino-N-(5-sulfamoyl-2-pyridyl)-2-methylpropylamine

The title compound was obtained in a similar manner to that of Reference example 66 by using 30% aqueous ammonia instead of dimethylamine.
yield: 77%
$^1$H NMR (DMSO-$d_6$) δ (ppm) : 8.30 (1H, d, J = 2.3 Hz), 7.65 (1H, dd, J = 8.9, 2.7 Hz), 7.16 (1H, m), 6.65 (1H, d, J = 8.9 Hz), 3.23 (2H, d, J = 5.9 Hz), 1.01 (6H, s).
APCIMS (m/z): 245 $(M + H)^+$

Reference example 79: 2-Amino-N-[5-(N-ethylaminosulfonyl)-2-pyridyl]-2-methyl propylamine

The title compound (313 mg) was obtained in a similar manner to that of Reference example 66 from 2-chloro-5-(N-ethylaminosulfonyl)pyridine (660 mg, 2.99 mmol).
yield: 38%
$^1$H NMR ($CDCl_3$) δ (ppm): 8.47 (1H, d, J = 2.5 Hz), 7.72 (1H, dd, J = 8.9, 2.5 Hz), 6.50 (1H, d, J = 8.9 Hz), 5.97 (1H, br t), 3.32 (2H, d, J = 5.9 Hz), 2.97 (2H, q, J = 7.3 Hz), 1.18 (6H, s), 1.11 (3H, t, J = 7.3 Hz).
APCIMS (m/z): 273 $(M + H)^+$

Reference example 80: 1-[(5-Cyanopyridin-2-ylamino)methyl]cyclopropylamine

1-(Aminomethyl)cyclopropylamine dihydrochloride (250 mg, 2.00 mmol) prepared according to the method described in literature [J. Org. Chem., 57, 6071 (1992)] was dissolved in methanol, and made into a free form by addition of BioRad AG (registered trademark) 1-X8 ion-exchange resin (6.0 g), and then the mixture was filtered. After the filtrate was concentrated, 2-chloro-5-cyanopyridine (139 mg, 1.00 mmol), potassium carbonate (276 mg, 2.00 mmol), and 1,4-dioxane (2 mL) was added to the filtrate, and the mixture was refluxed overnight. After the reaction mixture was concentrated, the residue was purified by silica gel column chromatography (chloroform/methanol=100/1 to 20/1) to obtain the title compound (70.0 mg, 0.372 mmol).
yield: 37%
$^1$H NMR ($CDCl_3$) δ (ppm) : 8.35 (1H, d, J = 1.9 Hz), 7.55 (1H, dd, J = 8.6, 1.9 Hz), 6.42 (1H, d, J = 8.6 Hz), 5.39 (1H, m), 3.37 (2H, d, J = 5.4 Hz), 0.70-0.59 (4H, m).
APCIMS (m/z): 189 $(M + H)^+$

Reference example 81: 1-[(5-Cyanopyridin-2-ylamino)methyl]cyclopentylamine

(1) 1-(Aminomethyl)cyclopentylamine dihydrochloride

To a mixture of benzyltriethylammonium chloride (500 mg, 2.20 mmol) and 50% aqueous sodium hydroxide solution (18.2 g, 22.7 mmol) was added dropwise a solution of N-(diphenylmethylene)aminoacetonitrile (5.00 g, 22.7 mmol) and 1,4-dibromobutane (5.88 g, 27.3 mmol) in toluene (10 mL) under ice-cooling. After stirring at room temperature overnight, the mixture was added with water, and extracted three times with chloroform. The organic layer was dried over anhydrous magnesium sulfate, and the reaction mixture was concentrated. The residue was purified by silica gel column chromatography (hexane/ethyl acetate = 1/1). 1-Cyano-1-[N-(diphenylmethylene)amino]cyclopentane was dissolved in THF (200 mL), and the solution was added with 2 mol/L hydrochloric acid (4 mL) and stirred at room temperature overnight. After the reaction mixture was washed twice with chloroform, the aqueous layer was concentrated to obtain a solid (1.58 g).

The obtained solid was dissolved in ethanol (200 mL), and platinum dioxide (270 mg) and concentrated hydrochloric acid (5.35 mL) were added to the solution. And the mixture was allowed to react under hydrogen (50 psi) at 36°C overnight. The reaction mixture was filtered using Celite as a filtration aid. The filtrate was concentrated, and the obtained product was recrystallized from 2-propanol to obtain the title compound (1.21 g, 6.44 mmol).

yield: 28%

$^1$H NMR ($D_2O$) δ (ppm): 3.28 (2H, s), 1.85-1.72 (8H, m).

APCIMS (m/z): 115 $(M + H)^+$

(2) The title compound (1.21 g, 5.58 mmol) was prepared in a similar manner to that of Reference example 80 from 1-(aminomethyl)cyclopentylamine dihydrochloride (2.07 g, 10.7 mmol) prepared in Reference example 81 (1).

yield: 52%

$^1$H NMR ($CDCl_3$) δ (ppm): 8.35 (1H, d, J = 2.2 Hz), 7.52 (1H, dd, J = 8.9, 2.2 Hz), 6.41 (1H, d, J = 8.9 Hz), 5.71 (1H, m), 3.33 (2H, d, J = 5.4 Hz), 1.83-1.41 (8H, m).

APCIMS (m/z): 217 $(M + H)^+$

Reference example 82: 2-[5-Cyanopyridin-2-ylamino]methyl]adamantan-2-ylamine

(1) 2-(Aminomethyl)-2-adamantanamine dihydrochloride

The title compound (3.23 g, 11.2 mmol) was prepared in a similar manner to that of Reference example 85 described below from 2-adamantanone (3.00 g, 20.0 mmol).

yield: 56%

$^1$H NMR ($D_2O$) δ (ppm) : 3.51 (2H, s), 1.96-1.65 (14H, m).

FABMS (m/z) : 181 $(M + H)^+$

(2) The title compound (928 mg, 3.29 mmol) was prepared in a similar manner to that of Reference example 80 from 2-aminomethyl-2-adamantanamine dihydrochloride (1.27 g, 5.00 mmol) prepared above.

yield: 66%

$^1$H NMR ($CDCl_3$) δ (ppm): 8.34 (1H, d, J = 1.9 Hz), 7.48 (1H, dd, J = 8.9, 1.9 Hz), 6.40 (1H, dd, J = 8.9, 0.54 Hz), 5.87 (1H, m), 3.56 (2H, d, J = 5.1 Hz), 2.03-1.66 (14H, m).

APCIMS (m/z): 283 $(M + H)^+$

Reference example 83: 1-[5-Cyanopyridin-2-ylamino]methyl]cyclooctylamine

(1) 1-(Aminomethyl)cyclooctylamine dihydrochloride

The title compound (3.01 g, 13.1 mmol) was prepared in a similar manner to that of Reference example 85 described below from cyclooctanone (10.1 g, 80.0 mmol).

yield: 16%

$^1$H NMR ($D_2O$) δ (ppm): 3.21 (2H, s), 1.79-1.46 (14H, m).

APCIMS (m/z): 157 $(M + H)^+$

(2) The title compound (1.01 g, 0.390 mmol) was prepared in a similar manner to that of Reference example 80 from 1-(aminomethyl)cyclooctylamine dihydrochloride (1.15 g, 5.00 mmol) prepared above.

yield: 78%

$^1H$ NMR ($CDCl_3$) δ (ppm): 8.33 (1H, d, J = 1.8 Hz), 7.48 (1H, dd, J = 8.8, 1.8 Hz), 6.56 (1H, d, J = 8.8 Hz), 6.31 (1H, t, J = 6.0 Hz), 3.64 (2H, d, J = 6.0 Hz), 1.80-1.26 (14H, m). APCIMS (m/z): 259 $(M + H)^+$

Reference example 84: 1-[(5-Cyanopyridin-2-ylamino)methyl]cyclobutylamine

(1) 1-(Aminomethyl)cyclobutylamine dihydrochloride

The title compound (5.14 g, 29.5 mmol) was prepared in a similar manner to that of Reference example 85 described below from cyclobutanone (5.00 g, 71.4 mmol) yield: 41%
$^1H$ NMR ($D_2O$) δ (ppm) : 3.38 (2H, s), 2.36-2.16 (4H, m), 2.03-1.83 (2H, m).
APCIMS (m/z): 101 $(M + H)^+$

(2) The title compound (497 mg, 2.45 mmol) was prepared in a similar manner to that of Reference example 85 (2) from 1-(aminomethyl)cyclobutylamine dihydrochloride (865mg, 5.00 mmol) prepared above.
yield: 49%
$^1H$ NMR (CDCls) δ (ppm) : 8.37 (1H, d, J = 2.4 Hz), 7.51 (1H, dd, J = 8.9, 2.4 Hz), 6.44 (1H, d, J = 8.9 Hz), 5.67 (1H, br s), 3.49 (2H, d, J = 5.4 Hz), 2.13-1.69 (6H, m).
APCIMS (m/z): 203 $(M + H)^+$

Reference example 85: 1-[(5-Cyanopyridin-2-ylamino)methyl]cyclohexylamine

(1) 1-(Aminomethyl)cyclohexylamine dihydrochloride

Cyclohexanone (9.80 g, 100 mmol), ammonium chloride (10.6 g, 200 mmol), sodium cyanide (4.90 g, 100 mmol), and 28% aqueous ammonia (20 mL) were dissolved in ethanol (180 mL) and water (100 mL), and the solution was allowed to react at 50°C for 3 hours. The reaction mixture was made alkaline with saturated aqueous sodium hydrogencarbonate solution, and the mixture was extracted twice with chloroform. The organic layer was dried over anhydrous magnesium sulfate and concentrated. To the residue were added acetonitrile and a 4 mol/L solution of hydrogen chloride in 1,4-dioxane (50 mL), and the deposited solid was collected by filtration. The solid was dissolved in ethanol (400 mL), and to the solution were added platinum dioxide (1.00 g, 4.41 mmol) and concentrated hydrochloric acid (20 mL). The mixture was allowed to react under hydrogen (50 psi) at 36°C overnight. The reaction mixture was filtered using Celite as a filtration aid, and the filtrate was concentrated. The obtained product was recrystallized from 2-propanol to obtain the title compound (7.28 g, 36.0 mmol).
yield: 36%
$^1H$ NMR ($D_2O$) δ (ppm): 3.27 (2H, s), 1.77-1.39 (10H, m).
APCIMS (m/z): 129 $(M + H)^+$

(2) 1-(Aminomethyl)cyclohexylamine dihydrochloride (1.00 g, 5.00 mmol) prepared above was dissolved in methanol, and made into a free form with addition of BioRad AG (registered trademark) 1-X8 ion-exchange resin (14 g), and the solution was filtered. The filtrate was concentrated, and to the residue were added 2-chloro-5-cyanopyridine (695 mg, 5.00 mmol), N,N-diisopropylethylamine (1 mL) and 1,4-dioxane (20 mL). The mixture was refluxed overnight. The reaction mixture was concentrated and the residue was purified by silica gel column chromatography (chloroform/methanol=100/1 to 6/1) to obtain the title compound (916 mg, 3.97 mmol).
yield: 79%
$^1H$ NMR ($CDCl_3$) δ (ppm): 8.34 (1H, d, J = 2.4 Hz), 7.49 (1H, dd, J = 8.6, 2.4 Hz), 6.61 (1H, d, J = 8.6 Hz), 6.54 (1H, t, J = 5.9 Hz), 3.85 (2H, d, J = 5.9 Hz), 1.79-1.46 (10H, m). APCIMS (m/z): 231 $(M + H)^+$

Reference example 86: 2-Methyl-1-(4-nitroanilino)-2-propylamine

To a solution of 4-fluoronitrobenzene (2.12 mL, 20.0 mmol) in 1,4-dioxane (20 mL) were added potassium carbonate (3.04 g, 22.0 mmol) and 1,2-diamino-2-methylpropane (4.19 mL, 40.0 mmol), and the mixture was refluxed for 3 hours. The reaction mixture was concentrated to allow crystals to precipitate. The resulting crude crystals were washed with toluene to obtain the title compound (4.42 g, 20.0 mmol) as white crystals.
yield: quantitative
$^1H$ NMR (DMSO-$d_6$) δ (ppm) : 7.95 (2H, d, J = 9.7 Hz), 7.12 (1H, br s), 6.72 (2H, d, J = 9.7 Hz), 3.02 (2H, br s), 1.50 (2H, br s), 1.04 (6H, s).
APCIMS (m/z): 208 $(M - H)^+$

Reference example 87: 1-Anilino-2-methyl-2-propylamine

(1) 1-Anilino-2-methyl-2-nitropropane

To a solution of aniline (9.11 mL, 100 mmol) in methanol (30 mL) were added 2-nitropropane (8.98 mL, 100 mmol) and triton B (0.500 mL). 37% Formalin (7.49 mL, 100 mmol) was added dropwise to the mixture while the mixture was refluxed. The mixture was further refluxed with stirring for 7 hours. The reaction mixture was stand for cooling at room temperature, and deposited crystals are collected by filtration. The crystals were washed with methanol cooled to 0°C and dried to obtain 1-anilino-2-methyl-2-nitropropane (13.0 g, 67.0 mmol).
yield: 67%

(2) To a solution of 1-anilino-2-methyl-2-nitropropane (2.00 g, 10.3 mmol) obtained in (1) in methanol (20 mL) and concentrated hydrochloric acid (20 mL) was added zinc powder (4.00 g, 61.5 mmol) at 0°C, and the mixture was stirred at room temperature for 2 hours. The solution was filtered using Celite as a filtration aid to remove excess zinc, and the solvent was evaporated under reduced pressure. To the obtained residue was added an aqueous ammonia, and the aqueous layer was extracted with chloroform. The organic layer was dried over anhydrous magnesium sulfate. The solvent was evaporated under reduced pressure to obtain the title compound (1.59 g, 9.68 mmol).
yield: 94%
$^1$H NMR (DMSO-$d_6$) δ (ppm): 7.07-7.00 (2H, m), 6.63-6.58 (2H, m), 6.50-6.44 (2H, m), 5.35 (1H, t, J = 5.8 Hz), 2.84 (2H, d, J = 5.8 Hz), 1.05 (6H, s).

Reference example 88: 1-(4-Cyanoanilino)-2-methyl-2-propylamine

The title compound (456 mg, 2.40 mmol) was obtained in a similar manner to that of Reference example 86, except 4-fluorobenzonitrile (2.42 g, 20.0 mmol) was used instead of 4-fluoronitrobenzene, and purification was conducted at a final step by silica gel column chromatography (Chromatorex (registered trademark) NH, Fuji Silysia, hexane/chloroform = 2/1)
yield: 12%
$^1$H NMR (DMSO-$d_6$) δ (ppm): 7.41 (2H, d, J = 8.9 Hz), 6.71 (2H, d, J = 8.9 Hz), 6.50 (1H, t, J = 5.7 Hz), 2.93 (2H, d, J = 5.7 Hz), 1.47 (2H, br s), 1.04 (6H, s).
APCIMS (m/z): 190 $(M + H)^+$

Reference example 89: 1-(p-Anisidino)-2-methyl-2-propylamine

The title compound (3.80 g, 28.4 mmol) was obtained in a similar manner to that of Reference example 87 by using p-anisidine (6.15 g, 50.0 mmol) instead of aniline.
yield: 28% for 2 steps
$^1$H NMR ($CDCl_3$) δ (ppm): 6.72 (2H, d, J = 9.2 Hz), 6.63 (2H, d, J = 9.2 Hz), 4.75 (3H, br s), 3.70 (3H, s), 3.08 (2H, s), 1.28 (6H, s).
APCIMS (m/z): 195 $(M + H)^+$

Reference example 90: 1-[4-(N,N-Dimethylaminosulfonyl)anilino]-2-methyl-2-propylamine

(1) 4-Fluoro-1-(N,N-dimethylaminosulfonyl)benzene

To a solution of 4-fluorobenzenesulfonyl chloride (1.95 g, 10.0 mmol) in THF (40 mL) were added triethylamine (2.09 mL, 15.0 mmol) and 50% aqueous dimethylamine solution (1.26 mL, 14.0 mmol) at room temperature, and the solution was stirred at the same temperature for 20 minutes. The reaction mixture was diluted with ethyl acetate, and washed with water. The organic layer was dried over anhydrous magnesium sulfate. The solvent was evaporated under reduced pressure to obtain 4-fluoro-1-(N,N-dimethylaminosulfonyl)benzene (2.03 g, 10.0 mmol).
yield: quantitative

(2) To 4-fluoro-1-(N,N-dimethylaminosulfonyl)benzene (2.03 g, 10.0 mmol) were added diisopropylethylamine (2.26 mL, 13.0 mmol) and 1,2-diamino-2-methylpropane (3.14 mL, 30.0 mmol), and the mixture was heated with stirring at 170°C for 10 hours. To the mixture was added a 2 mol/L aqueous sodium hydroxide solution, and the aqueous layer was extracted with chloroform. The organic layer was washed with saturated brine, and dried over anhydrous magnesium sulfate. The solvent was evaporated under reduced pressure. The obtained residue was

purified by silica gel column chromatography. (Chromatorex (registered trademark) NH, Fuji Silysia, hexane/chloroform = 1/1) to obtain the title compound (956 mg, 3.52 mmol).
yield: 35%
$^1$H NMR ($CDCl_3$) δ (ppm) : 7.55 (2H, d, J = 9.2 Hz), 6.64 (2H, d, J = 9.2 Hz), 4.83 (1H, t, J = 5.7 Hz), 3.00 (2H, d, J = 5.7 Hz), 2.66 (6H, s), 1.21 (6H, s).
APCIMS (m/z): 272 $(M + H)^+$

Reference example 91: 2-Methyl- 1-(4-methylthioanilino)-2-propylamine

(1) 2-Methyl-1-(4-methylthioanilino)-2-nitropropane
2-Methyl-1-(4-methylthioanilino)-2-nitropropane (1.44 g, 12.0 mmol) was obtained in a similar manner to that of Reference example 87 (1), except that 4-methylthioaniline(1.55 mL, 12.5 mmol) was used instead of aniline, and purification was conducted at a final stage by silica gel column chromatography (chloroform). yield: 48%
(2) The title compound (510 mg, 2.42 mmol) was obtained in a similar manner to that of Reference example 87 (2) except that 2-methyl-1-(4-methylthioanilino)-2-nitropropane (700 mg, 2.91 mmol) was used instead of 1-anilino-2-methyl-2-nitro propane, and purification was conducted at a final step by silica gel column chromatography (Chromatorex (registered trademark) NH, Fuji Silysia, hexane/chloroform = 2/1)
yield: 83%
$^1$H NMR ($CDCl_3$) δ (ppm): 7.20 (2H, d, J = 8.8 Hz), 6.59 (2H, d, J = 8.8 Hz), 4.18 (1H, t, J = 5.9 Hz), 2.94 (2H, d, J = 5.9 Hz), 2.34 (3H, s), 1.18 (6H, s).
APCIMS (m/z): 211 $(M + H)^+$

Reference example 92: 2-Methyl- 1-(p-toluidino)-2-propylamine

The title compound (500 mg, 2.80 mmol) was obtained in a similar manner to that of Reference example 87, except that p-toluidine (1.07 g, 10.0 mmol) was used instead of aniline, and purification was conducted at a final step by silica gel column chromatography (Chromatorex (registered trademark) NH, Fuji Silysia hexane/ethyl acetate =5/1)
yield: 2 steps 28%
$^1$H NMR ($CDCl_3$) δ (ppm): 6.96 (2H, d, J = 8.4 Hz), 6.56 (2H, d, J = 8.4 Hz), 3.93 (1H, t, J = 5.9 Hz), 2.93 (2H, d, J = 5.9 Hz), 2.22 (3H, s), 1.27 (2H, br s), 1.16 (6H, s).
APCIMS (m/z): 179 $(M + H)^+$

Reference example 93: 1-(4-Methanesulfonylanilino)-2-methyl-2-propylamine

(1) 1-(4-Methanesulfonylanilino)-2-methyl-2-nitropropane
To a solution of 2-methyl-1-(4-methylthioanilino)-2-propane (650 mg, 2.70 mmol) obtained in Reference example 91(1) in chloroform (25 mL) was added 70% m-chloroperbenzoic acid (1.53 g, 6.22 mmol) at 0°C, and the mixture was stirred at room temperature for 1 hour. The reaction mixture was washed with aqueous sodium hydrogencarbonate solution, and dried over anhydrous magnesium sulfate. The solvent was evaporated under reduced pressure.
(2) The title compound (161 mg, 0.664 mmol) was obtained in a similar manner to that of Reference example 87 (2) by using the obtained residue in (1) instead of 1-anilino-2-methyl-2-nitropropane.
yield: 2 steps 25%
$^1$H NMR ($CDCl_3$) δ (ppm): 7.68 (2H, d, J = 8.6 Hz), 6.65 (2H, d, J = 8.6 Hz), 4.96 (1H, br s), 3.02 (2H, s), 3.00 (3H, s), 1.50 (2H, br s), 1.21 (6H, s).
APCIMS (m/z): 243 (M + H)+

Reference example 94: 2-Methyl-1-(4-pyrrolidinylsulfonylanilino)-2-propylamine

The title compound (416 mg, 1.40 mmol) was obtained in a similar manner to that of Reference example 90 by using pyrrolidine (1.09 mL, 13.0 mmol) instead of dimethylamine.
yield: 2 steps 14%
$^1$H NMR ($CDCl_3$) δ (ppm): 7.60 (2H, d, J = 8.9 Hz), 6.63 (2H, d, J = 8.9 Hz), 4.82 (1H, t, J = 5.7 Hz), 3.22-3.17 (4H, m), 2.99 (2H, d, J = 5.7 Hz), 1.79-1.72 (4H, m), 1.46 (2H, br s), 1.21 (6H, s).

FABMS (m/z): 298 $(M + H)^+$

Reference example 95: 1-[4-(N,N-Diethylaminosulfonyl)anilino]-2-methyl-2-amine

The title compound (1.19 mg, 3.97 mmol) was obtained in a similar manner to that of Reference example 90 by using diethylamine (1.34 mL, 13.0 mmol) instead of dimethylamine.
yield: 2 steps 40%
$^1H$ NMR ($CDCl_3$) δ (ppm): 7.57 (2H, d, J = 8.8 Hz), 6.61 (2H, d, J = 8.8 Hz), 4.77 (1H, t, J = 5.7 Hz), 3.18 (4H, q, J = 7.3 Hz), 2.99 (2H, d, J = 5.7 Hz), 1.35 (2H, br s), 1.20 (6H, s), 1.12 (6H, t, J = 7.3 Hz).
FABMS (m/z): 300 $(M + H)^+$

Reference example 96: 1-(4-Fluoroanilino)-2-methyl-2-propylamine

(1) 1-(4-Fluoroanilino)-2-methyl-2-nitropropane
1-(4-Fluoroanilino)-2-methyl-2-nitropropane (4.04 g, 19.0 mmol) was obtained in a similar manner to that of Reference example 87 (1) by using 4-fluoroaniline(2.84 mL, 30.0 mmol) instead of aniline.
yield: 64%
$^1H$ NMR ($CDCl_3$) δ (ppm): 6.93-6.84 (2H, m), 6.60-6.53 (2H, m), 3.79 (1H, br s), 3.56 (2H, d, J = 7.3 Hz), 1.65 (6H, s).
APCIMS (m/z): 211 $(M - H)^-$
(2) To a solution of 1-(4-fluoroanilino)-2-methyl-2-nitropropane (2.12 g, 10.0 mmol) obtained in (1) in methanol (15 mL) and water (15 mL) were added concentrated hydrochloric acid (1.00 mL, 12.0 mmol) and ammonium chloride (1.34 g, 25.0 mmol).

To the solution was added zinc-copper alloy (3.28 g, 50.0 mmol) obtained according to the preparation method described in Org. Synth., 5, 855 and then the mixture was refluxed. At 1 hour and at 2 hours from the start of the reaction, concentrated hydrochloric acid (1.00 mL, 12.0 mmol) were further added, and refluxing was continued for 3 hours in total. The reaction mixture was filtered using Celite as a filtration aid to remove excess zinc, and the solvent was evaporated under reduced pressure. To the obtained residue was added aqueous ammonia, and the aqueous layer was extracted with chloroform. The organic layer was dried over anhydrous magnesium sulfate, and the solvent was evaporated under reduced pressure to obtain the title compound (1.20 g, 6.58 mmol).
yield: 66%
$^1H$ NMR ($CDCl_3$) δ (ppm): 6.92-6.82 (2H, m), 6.61-6.53 (2H, m), 4.01 (1H, br s), 2.91 (2H, d, J = 5.1 Hz), 1.23 (2H, br s), 1.19 (6H, s).
APCIMS (m/z): 183 $(M + H)^+$

Reference example 97: 2-Amino-N-(4-chloro- 1-phthalazinyl)-2-methylpropylamine

To a solution of 1,4-dichlorophthalazine (1.99 g, 10.0 mmol) in pyridine (10 mL) was added 1,2-diamino-2-methylpropane (3.14 mL, 30.0 mmol), and the mixture was refluxed for 7 hours. After the reaction mixture was concentrated, a 2 mol/L aqueous sodium hydroxide solution was added to the solution, and the aqueous layer was extracted with dichloromethane. The organic layer was dried over anhydrous magnesium sulfate, and the solvent was evaporated under reduced pressure. The obtained residue was purified by silica gel column chromatography (solvent A/methanol=15/1; solvent A was obtained as an organic layer by mixing chloroform and concentrated aqueous ammonia in 10: 1, v/v and then separating the layers) to obtain the title compound (2.22 g, 8.85 mmol).
yield: 89%
$^1H$ NMR ($CDCl_3$) d(ppm): 8.18-8.13 (1H, m), 7.91-7.81 (3H, m), 6.14 (1H, t, J = 5.1 Hz), 3.56 (2H, d, J = 5.1 Hz), 1.49 (2H, br s), 1.26 (6H, s).
APCIMS (m/z): 251 $(^{35}ClM + H)^+$, 253 $(^{37}ClM + H)^+$

Reference example 98: 2-Amino-2-methyl-N-( 1-phthalazinyl)propylamine

To a solution of 2-amino-N-(4-chloro-1-phthalazinyl)-2-methyl-propylamine (1.00 g, 3.99 mmol) obtained in Reference example 97 in methanol (20 mL) were added ammonium formate (5.03 g, 79.8 mmol) and 10% palladium on carbon (containing 50% water, 300 mg), and the mixture was refluxed with stirring for 4 hours.
After the reaction mixture was filtered using Celite as a filtration aid, the filtrate was concentrated under reduced pressure. To the obtained residue were added dichloromethane and a 2 mol/L aqueous sodium hydroxide solution, and the aqueous layer was extracted with dichloromethane. The organic layer was dried over anhydrous magnesium sulfate, and the solvent was evaporated under reduced pressure to obtain the title compound (830 mg, 3.84 mmol).
yield: 96%
$^1H$ NMR ($CDCl_3$) d(ppm): 8.90 (1H, s), 7.92-7.88 (1H, m), 7.79-7.75 (3H, m), 6.09 (1H, t, J = 4.3 Hz), 3.62 (2H, d, J = 4.3 Hz), 1.58 (2H, br s), 1.26 (6H, s).
APCIMS (m/z): 217 $(M + H)^+$

Reference example 99: 2-Amino-2-methyl-N-(3-pyridazinyl)propylamine

To 3-chloropyridazine (770 mg, 6.70 mmol) described in WO 97/24124 were added 1,2-diamino-2-methylpropane (1.39 mL, 13.5 mmol) and

N,N-diisopropylethylamine (3.48 mL, 20.0 mmol), and the mixture was stirred at 170°C overnight. The reaction mixture was concentrated, and the obtained residue was purified by silica gel column chromatography [Chromatrex (registered trademark)NH Fuji Silysia, ethyl acetate to ethyl acetate/methanol=9/1] to obtain the title compound (948 mg, 5.71 mmol).
yield: 85%
$^1H$ NMR ($CDCl_3$) δ (ppm): 8.50 (1H, dd, J = 4.3, 1.4 Hz), 7.13 (1H, dd, J = 8.9, 4.3 Hz), 6.71 (1H, dd, J = 8.9, 1.4 Hz), 5.43 (1H, m), 3.36 (2H, d, J = 5.9 Hz), 1.20 (6H, s).
APCIMS (m/z): 167 $(M + H)^+$

Reference example 100: 2-Amino-2-methyl-N-(4-pyrimidinyl)propylamine

(1) To a solution of 1,2-diamino-2-methylpropane (2.59 mL, 25 mmol) in THF (50 mL) was added potassium carbonate (4.14 g, 30.0 mmol) and 4,6-dichloropyrimidine (2.55 g, 17.0 mmol), and the mixture was stirred at room temperature overnight. After the reaction mixture was filtered, the filtrate was concentrated. The obtained residue was purified by silica gel column chromatography [Chromatrex (registered trademark)NH, Fuji Silysia, ethyl acetate to ethyl acetate/methanol = 9/1] to obtain 2-amino-N-(6-chloro-4-pyrimidinyl)-2-methylpropylamine (2.58 g, 12.8 mmol).
$^1H$ NMR ($CDCl_3$) δ (ppm) : 8.33 (1H, s), 6.38 (1H, s), 5.80 (1H, m), 3.20 (2H, m), 1.23 (2H, br s), 1.18 (6H, s).
APCIMS (m/z): 201 $(^{35}ClM + H)^+$, 203 $(^{37}ClM + H)^+$
(2) To a solution of
2-amino-N-(6-chloro-4-pyrimidinylamino)-2-methylpropylamine (2.58 g, 12.8 mmol) in ethanol (50 mL) were added 10% palladium-carbon (containing 50% water, 1.00 g) and ammonium formate (8.20 g, 130 mmol), and the mixture was refluxed for 4 hours. The reaction mixture was stand for cooling to room temperature, and filtered. The filtrate was concentrated and the obtained residue was purified by silica gel column chromatography [Chromatrex (registered trademark) NH, Fuji Silysia, ethyl acetate to ethyl acetate/methanol =9/1] to obtain the title compound (2.03 g, 12.3 mmol).
yield: 49%
$^1H$ NMR ($CDCl_3$) δ (ppm) : 8.54 (1H, s), 8.23 (1H, d, J = 5.9 Hz), 6.36 (1H, dd, J = 5.9, 1.1 Hz), 5.53 (1H, m), 3.23 (2H, d, J = 5.1 Hz), 1.41 (2H, br s), 1.18 (6H, s).
APCIMS (m/z): 167 $(M + H)^+$

Reference example 101: 2-Amino-N-(5-methanesulfonyl-2-pyridyl)-2-methyl-amine

To a solution of 1,2-diamino-2-methylpropane (1.66 mL, 16.0 mmol) in 1,4-dioxane (20 mL) were added potassium carbonate (2.76 g, 20.0 mmol) and 2-chloro-5-methanesulfonylpyridine (8.00 mmol) separately prepared, and the mixture was refluxed for 8 hours. The reaction mixture was stand for cooling to room temperature, and filtered, and the filtrate was concentrated. The obtained residue was purified by silica gel column chromatography [Chromatrex (registered trademark) NH, Fuji Silysia, ethyl acetate to ethyl acetate/methanol=9/1] to obtain the title compound (1.94 g, 8.00 mmol).
yield: 50%
$^1H$ NMR ($CDCl_3$) δ (ppm) : 8.57 (1H, d, J = 2.4 Hz), 7.78 (1H, dd, J = 8.9, 2.4 Hz), 6.46 (1H, d, J = 8.9 Hz), 5.75 (1H, m), 3.29 (2H, d, J = 5.7 Hz), 3.03 (3H, s), 1.29 (2H, br s), 1.19 (6H, s).
APCIMS (m/z): 244 $(M + H)^+$

Reference example 102: 4-Amino-1-(5-methanesulfonyl-2-pyridyl)-4-methylpiperidine

(1) To a solution of 2-chloro-5-methylthiopyridine (5.89 g, 36.9 mmol) described in literature [J. Med. Chem., 16, 319 (1973)] in acetonitrile (200 mL) and water (200mL) was added Oxone (registered trademark, 91.0 g, 148 mmol), and the mixture was stirred at 45°C for 10 hours. The reaction mixture was cooled to room temperature and filtered, and the filtrate was extracted 3 times with ethyl acetate. The combined organic layer was washed successively with a 5% aqueous solution of sodium thiosulfate, an aqueous saturated sodium hydrogencarbonate solution, and then with saturated brine, and further dried over anhydrous magnesium sulfate and concentrated. The obtained residue was purified from hexane to obtain 2-chloro-5-methanesulfonylpyridine (5.66 g, 29.5 mmol).
$^1$H NMR ($CDCl_3$) δ (ppm): 8.94 (1H, d, J = 2.4 Hz), 8.17 (1H, dd, J = 8.4, 2.4 Hz), 7.55 (1H, d, J = 8.4 Hz), 3.13 (3H, s).
FABMS (m/z): 192 ($^{35}$ClM + H)$^+$, 194 ($^{37}$ClM + H)$^+$
(2) To a solution of 4-tert-butoxycarbonylamino-4-methylpiperidine (1.93 g, 9.0 mmol) described in European Patent No. 647,639 in 1,4-dioxane (20 mL) were added potassium carbonate (2.79 g, 20.0 mmol) and 2-chloro-5-methanesulfonylpyridine (1.15 g, 6.00 mmol), and the mixture was refluxed overnight. The reaction mixture was stand for cooling to room temperature and filtered, and the filtrate was concentrated. The obtained residue was purified by silica gel column chromatography (hexane/ethyl acetate=1/1) to obtain
4-tert-butoxycarbonylamino- 1-(5 -methanesulfonyl-2-pyridyl)-4-methylpiperidine (2.12 g, 5.79 mmol).
$^1$H NMR ($CDCl_3$) δ (ppm) : 8.62 (1H, d, J = 2.7 Hz), 7.84 (1H, dd, J = 9.2, 2.7 Hz), 6.65 (1H, d, J = 9.2 Hz), 4.43 (1H, br s), 4.00 (2H, ddd, J = 13.5, 4.6, 4.6 Hz), 3.43 (2H, ddd, J = 13.8, 10.5, 3.2 Hz), 3.03 (3H, s), 2.12 (2H, d, J = 13.5 Hz), 1.68-1.55 (2H, m), 1.44 (9H, s), 1.40 (3H, s)
APCIMS (m/z): 370 (M + H)$^+$
(3) To a solution of
4-tert-butoxycarbonylamino-(5-methanesulfonyl-2-pyridyl)-4-methylpiperidine (2.12 g, 5.75 mmol) in dichloromethane (10 mL) was added trifluoroacetic acid (10 mL) under ice-cooling. The reaction mixture was stirred at the same temperature for 3 hours. The reaction mixture was concentrated, and the obtained residue was dissolved in ethanol. The solution was made alkaline with addition of BioRad AG (registered trademark) 1 X-8 ion-exchange resin. The reaction mixture was filtered and the filtrate was concentrated to obtain the title compound (1.28 g, 4.77 mmol).
yield: 64%
$^1$H NMR ($CDCl_3$) δ (ppm): 8.61 (1H, d, J = 2.7 Hz), 7.82 (1H, dd, J = 9.2, 2.7 Hz), 6.65 (1H, d, J = 9.2 Hz), 3.89-3.80 (2H, m), 3.73-3.63 (2H, m), 3.03 (3H, s), 1.69-1.45 (4H, m), 1.32 (2H, br s), 1.21 (3H, s).
APCIMS (m/z): 270 (M + H)$^+$

Reference example 103: 4-Amino-4-methyl-1-(5-methyl-2-pyridyl)piperidine

The title compound was obtained in a similar manner to that of Reference example 50 by using 2-bromo-5-methylpyridine instead of 2-chloro-5-trifluoromethyl pyridine.
yield: 33%
$^1$H NMR ($CDCl_3$) δ (ppm): 8.00 (1H, d, J = 2.7 Hz), 7.28 (1H, dd, J = 8.9, 2.7 Hz), 6.60 (1H, d, J = 8.9 Hz), 3.60-3.43 (4H, m), 2.18 (3H, s), 1.69-1.46 (4H, m), 1.34 (2H, br s), 1.17 (3H, s).
APCIMS (m/z): 206 (M + H)$^+$

Reference example 104: 4-Amino-4-methyl-1-(3-pyridazinyl)piperidine

The title compound was obtained in a similar manner to that of Reference example 50 by using 3-chloropyridazine described in WO 9724124 instead of 2-chloro-5-trifluoromethylpyridine.
yield: 36%
$^1$H NMR ($CDCl_3$) δ (ppm) : 8.54 (1H, dd, J = 4.3, 1.4 Hz), 7.17 (1H, dd, J = 9.3, 4.3 Hz), 6.91 (1H, dd, J = 9.3, 1.4 Hz), 3.80-3.61 (4H, m), 1.71-1.61 (2H, m), 1.58-1.49 (2H, m), 1.34 (2H, br s), 1.21 (3H, s).
APCIMS (m/z): 193 (M + H)$^+$

Reference example 105: 4-Amino- 1-(5-bromo-2-pyrimidinyl)-4-methylpiperidine

The title compound was obtained in a similar manner to that of Reference example 50 by using 5-bromo-2-chloropyrimidine instead of 2-chloro-5-trifluoromethyl pyridine.
yield: 58%

$^1H$ NMR ($CDCl_3$) δ (ppm) : 8.26 (2H, s), 3.92-3.69 (4H, m), 1.74 (2H, br s), 1.63-1.42 (4H, m), 1.19 (3H, s).
APCIMS (m/z) : 271 ($^{79}$ BrM + H)$^+$, 273 ($^{81}$BrM + H)$^+$

Reference example 106: 2-Methyl-1-(N-methylanilino)-2-propylamine

The title compound (2.04 g, 11.4 mmol) was obtained in a similar manner to that of Reference example 96 by using N-methylaniline (3.25 mL, 30.0 mmol) instead of 4-fluoroaniline.
yield: 38%
$^1H$ NMR ($CDCl_3$) δ (ppm): 7.22 (2H, dd, J = 8.6, 7.4 Hz), 6.84 (2H, d, J = 8.6 Hz), 6.69 (1H, t, J = 7.4 Hz), 3.24 (2H, s), 3.03 (3H, s), 2.17 (2H, br s), 1.18 (6H, s).
APCIMS (m/z): 179 (M + H)$^+$

Formulation Example 1: Tablet

A tablet according to the following formulation is prepared by an ordinary method.

| | |
|---|---|
| Compound 1 | 100 mg |
| Lactose | 60 mg |
| Potato starch | 30 mg |
| Polyvinyl alcohol | 2 mg |
| Magnesium stearate | 1 mg |
| Tar pigment | trace amount |

Formulation Example 2: Powder

A powder according to the following formulation is prepared by an ordinary method.

| | |
|---|---|
| Compound 1 | 150 mg |
| Lactose | 280 mg |

Formulation Example 3: Syrup

Syrup according to the following formulation is prepared by an ordinary method.

| | |
|---|---|
| Compound 1 | 100 mg |
| Purified Saccharose | 40 g |
| Ethyl p-hydroxybenzoate | 40 mg |
| Propyl p-hydroxybenzoate | 10 mg |
| Strawberry flavor | 0.1 cc |

Water was added to the above ingredients up to the total volume of 100 cc.

Test example 1: Test for inhibitory action against DPP-IV

This test was performed by the following method similar to that described in the literature (Villhauer et al. the U.S. Patent No. 6,011,155).

Under mild anesthesia with diethylether, a rat was incised at lower left side of the limb, and the crural aorta was cut and blood was collected. The collected blood was immediately ice-cooled. The coagulated blood was centrifuged at 3,000 rpm for 20 minutes to separate serum for use in the experiment. As reaction buffer, an aqueous solution containing HEPES (final concentration of 25 mmol/L) and sodium chloride (final concentration of 140 mmol/L) was prepared so as to give a final pH of 7.8, and used after addition of BSA (final concentration of 1%). An assay was conducted using a black, flat-bottomed 96 well plate. A test sample (2 μL) was added to each well, and the sample was added with rat serum (25 μL) and reaction buffer (25 μL) containing magnesium chloride (80 mmol/L), and the mixture was left stand at room temperature for 5 minutes. Then, a reaction was initiated by the addition of 50 μ L of a solution of Gly-Pro-AMC (AMC: 7-amino-4-methylcoumarin) (final concentration of 50 μmol/L) to each well, and the mixture was left stand in the dark at room temperature for 20 minutes. AMC, which was released by DPP-IV activity,

was quantified by measurement of fluorescence at 460 nm which was excited at 390 nm. DPP-IV activity was calculated according to the following equation: Inhibitory activity against

$$\text{DPP-IV} = 100 \times (1\text{-}\{Fs\text{-}F0)/(F100\text{-}F0)\}$$

F100 : Fluorescence intensity of AMC with addition of serum
F0 : Fluorescence intensity of AMC without addition of serum
Fs : Fluorescence intensity of AMC with addition of a sample and serum

The test results are indicated as concentrations at which the DPP-IV activity were 50% inhibited ($IC_{50}$). The results are shown in Table 2.

Table 2

| Compound number | Inhibitory activity against DPP-IV ($IC_{50}$, nmol/L) |
|---|---|
| 101 | 31 |
| 103 | 61 |
| 201 | 392 |
| 204 | 20 |
| 206 | 32 |
| 207 | 15 |
| 221 | 27 |
| 226 | 18 |
| 233 | 19 |
| 238 | 11 |
| 403 | 124 |
| 407 | 20 |
| 501 | 23 |
| 524 | 34 |
| 529 | 26 |
| 602 | 41 |
| 703 | 53 |

Test example 2: Suppressing effect on increase of blood sugar

Blood-sugar levels of Wistar male rats (9-week old, Charles river) fasted for 24 hours were measured. A test compound was orally administered to the rats in a dose of 30 mg/kg, and then immediately glucose (2 g/kg) was orally administered to the rats. After 30 minutes from the glucose loading, blood-sugar levels were further measured to evaluate suppressing effects of test compounds on increase of blood sugar. The blood-sugar levels were measured by a portable apparatus for measurement of blood sugar [Blood glucose meter, Dexter-Z (Bayer. Sankyo, Tokyo)]. The test compounds were dissolved in 0.5 % methylcellulose 400 for administration. The results are shown in Table 3 (** indicates $p<0.01$ in Student's t-test).

Table 3

| Effect of Compound 101 on supression of increase of blood sugar level | | | |
|---|---|---|---|
| Compound number | dose (mg/kg, po) | blood sugar level (mg/dL) | |
| | | 0 minute | 30 minites after glucose loading |
| Control group | - | 69 ± 2 | 127± 6 |
| 101 | 30 | 68 ± 2 | 104± 4** |

The above results indicate that the compound of the present invention significantly suppressed the increase of blood sugar after the loading of glucose.

Test example 3: Suppressing effect on increase of blood sugar

Blood-sugar levels of Wistar male rats (10-week old, Charles river) fasted for 24 hours were measured. A test compound was orally administered to the rats in a dose of 1 mg/kg, and then immediately glucose (2 g/kg) was orally administered to the rats. After 30 minutes from the glucose loading, blood-sugar levels were further measured to evaluate suppressing effects of test compounds on increase of blood sugar. The sugar levels were measured by the glucose oxidase method [Clin. Chem., 6, 466 (1960); J. Clin. Path., 22, 246 (1969); Clin. Chem. Acta, 40, 115 (1972); Clin. Chem., 20, 606 (1974) or the like]. The test compounds were dissolved in 0.5 % methylcellulose 400 for administration. The results are shown in Tables 4, 5, and 6.

Table 4

| Effect of Compound 501 on suppression of increase of blood sugar | | | |
|---|---|---|---|
| | | Blood-sugar level (mg/dL) | |
| Compound number | Dose (mg/kg, po) | 0 minute | 30 minutes after glucose loading |
| Control group | --- | 88 ±7 | 197 ±14 |
| 501 | 1 | 95±7 | 182±16 |

Table 5

| Effect of Compound 513 on suppression of increase of blood sugar | | | |
|---|---|---|---|
| | | Blood-sugar level (mg/dL) | |
| Compound number | Dose (mg/kg, po) | 0 minute | 30 minutes after glucose loading |
| Control group | --- | 120 ±10 | 310 ±23 |
| 513 | 1 | 109±6 | 252±22 |

Table 6

| Effect of Compound 233 on suppression of increase of blood sugar | | | |
|---|---|---|---|
| | | Blood-sugar level (mg/dL) | |
| Compound number | Dose (mg/kg, po) | 0 minute | 30 minutes after glucose loading |
| Control group | --- | 108 ±8 | 277 ±18 |
| 233 | 1 | 93±15 | 183±8 |

The above results indicate that the compounds of the present invention suppressed the increase of blood sugar after the loading of glucose.

Industrial Applicability

The compound of the present invention has an inhibitory action against dipeptidylpeptidase-IV (DPP-IV) and is useful as an active ingredient of a medicament for preventive and/or therapeutic treatment of Type II diabetes, and for preventive and/or therapeutic treatment of complications accompanying Type II diabetes.

## Claims

**1.** A compound represented by general formula (I):

A-B-D

<wherein

A represents a substituted or unsubstituted 1-pyrrolidinyl group, a substituted or unsubstituted 3-thiazolidinyl group, a substituted or unsubstituted 1-oxo-3-thiazolidinyl group, a substituted or unsubstituted 1,1-dioxo-3-thiazolidinyl group, a substituted or unsubstituted 3-oxazolidinyl group, a substituted or unsubstituted 2,5-dihydro-1-pyrrolyl group, a substituted or unsubstituted 1-pyrrolyl group, a substituted or unsubstituted piperidino group, a substituted or unsubstituted 1-indolinyl group, a substituted or unsubstituted 1-indolyl group, a substituted or unsubstituted 1-octahydroindolyl group, a substituted or unsubstituted 1-tetrahydroquinolyl group, or a substituted or unsubstituted 1-decahydroquinolyl group;
B represents

a) a group represented by $-(C(R^1)(R^2))_kCO-$ (wherein k represents an integer of 1 to 6, $R^1$ and $R^2$ may be the same or different and each represents a hydrogen atom, a hydroxyl group, a halogen atom, a cyano group, a nitro group, a substituted or unsubstituted alkyl group, a substituted or unsubstituted alicyclic alkyl group, a substituted or unsubstituted alicyclic heterocyclic group, a substituted or unsubstituted alkenyl group, a substituted or unsubstituted alkynyl group, a substituted or unsubstituted aryl group, a substituted or unsubstituted aralkyl group, a substituted or unsubstituted heteroaryl group, or a substituted or unsubstituted heteroarylalkyl group, or $R^1$ and $R^2$, which attach to the same carbon atom, together with said carbon atom, or two $R^1$s, which attach to adjacent carbon atoms, respectively, together with the two carbon atoms when k is two or more, may combine to form a substituted or unsubstituted alicyclic alkyl group or a substituted or unsubstituted alicyclic heterocyclic group),
b) a group represented by $-CO(C(R^3)(R^4))_{m}-$ (wherein $R^3$ and $R^4$ may be the same or different and each has the same meaning as that defined above for $R^1$ and $R^2$, respectively, and m represents an integer of 1 to 6),
c) a group represented by $-(C(R^5)(R^6))_n-$ (wherein $R^5$ and $R^6$ may be the same or different and each has the same meaning as that defined above for $R^1$ and $R^2$, respectively, and n represents an integer of 2 to 7)
d) -CO-, or
e) $-SO_2$,

D represents

a group represented by -U-V [wherein U represents a substituted or unsubstituted piperazinediyl group or a homopiperazinediyl group; V represents $-E-R^7$ (wherein E represents a single bond, -CO-, -C(=O)O-, or $-SO_2-$; $R^7$ represents a hydrogen atom, a substituted or unsubstituted alkyl group, a substituted or unsubstituted alicyclic alkyl group, a substituted or unsubstituted alicyclic heterocyclic group, a substituted or unsubstituted alkenyl group, a substituted or unsubstituted alkynyl group, a substituted or unsubstituted aryl group, a substituted or unsubstituted aralkyl group, a substituted or unsubstituted heteroaryl group, or a substituted or unsubstituted heteroarylalkyl group)]
or
a group represented by $-W^A-X^A-Y^A-Z^A$ {wherein

1) $W^A$ and $Y^A$ may be the same or different and each represents an oxygen atom, a sulfur atom, -SO-, $-SO_2-$, or $-N(R^{8A})-$ (wherein $R^{8A}$ has the same meaning as that defined above for $R^7$); $X^A$ represents a substituted or unsubstituted alicyclic alkylene group, a group formed by eliminating one hydrogen atom from a substituted or unsubstituted alicyclic heterocyclic group, a substituted or unsubstituted arylene group, a substituted or unsubstituted aralkylene group, a substituted or unsubstituted heteroarylene group, a substituted or unsubstituted heteroarylalkylene group, or $-(C(R^9)(R^{10}))q^-$ [wherein q represents an integer of 1 to 6, $R^9$ and $R^{10}$ may be the same or different and each represents a hydrogen atom, a substituted or unsubstituted alkyl group, a substituted or unsubstituted alicyclic alkyl group, a substituted or unsubstituted alicyclic heterocyclic group, a substituted or unsubstituted alkenyl group, a substituted or unsubstituted alkynyl group, a substituted or unsubstituted aryl group, a substituted or unsubstituted aralkyl group, a substituted or unsubstituted heteroaryl group, or a substituted or unsubstituted heteroarylalkyl group, or $R^9$ and $R^{10}$, which attach to the same carbon atom, may combine together with said carbon atom to form a substituted or unsubstituted alicyclic alkyl group (provided that not all of the substituents $R^9$ and $R^{10}$ in the chain consisting of $-(C(R^9)(R^{10}))_{q}-$ are hydrogen atoms)], or $X^A$ may combine together with one $-N(R^{8A})$ represented by $W^A$ or $Y^A$ to form a substituted or unsubstituted pyrrolidinediyl group, a substituted or unsubstituted piperidinediyl group, or a substituted or unsubstituted homopiperidinediyl group; $Z^A$ has the same meaning as that defined above for V (when $X^A$ combines with one $-N(R^{8A})-$ represented by $W^A$ or $Y^A$ to form a substituted or unsubstituted pyrrolidinediyl group or a substituted or unsubstituted piperidinediyl group,

V is not a hydrogen atom or an aralkyl group); or
2) $W^A$ and $Y^A$ may be the same or different and each represents an oxygen atom, a sulfur atom, -SO-, $-SO_2-$, or $N(R^{8B})-$ (wherein $R^{8B}$ has the same meaning as that defined above for $R^7$); $X^A$ represents $-(CH_2)_r-$ (wherein r represents an integer of 1 to 6); $Z^A$ represents a substituted or unsubstituted alicyclic alkyl group, a substituted or unsubstituted alicyclic heterocyclic group, a substituted or unsubstituted alkenyl group, a substituted or unsubstituted alkynyl group, a substituted or unsubstituted aralkyl group, a substituted or unsubstituted heteroarylalkyl group, a pyridyl group substituted with $-SO_2-N(R^{15})(R^{16})$ [wherein $R^{15}$ and $R^{16}$ may be the same or different and each represents a hydrogen atom, a substituted or unsubstituted alkyl group, a substituted or unsubstituted aryl group, a substituted or unsubstituted aralkyl group, a substituted or unsubstituted heteroaryl group, or a substituted or unsubstituted heteroarylalkyl group, or $R^{15}$ and R16, together with the adjacent nitrogen atom, may form a substituted or unsubstituted alicyclic heterocyclic group (said alicyclic heterocyclic group is selected from a pyrrolidinyl group, an oxazolidinyl group, a thiazolidinyl group, a piperidino group, a homopiperidino group, a piperazinyl group, a morpholino group, and a thiomorpholino group)], or a group selected from a trifluoromethylphenyl group, a methanesulfonylphenyl group, a nitrophenyl group, a cyanophenyl group, a naphthyl group, a pyrazinyl group, a pyridazinyl group, a triazinyl group, a thienyl group, a furyl group, a pyrrolyl group, an imidazolyl group, a pyrazolyl group, a triazolyl group, a tetrazolyl group, an oxazolyl group, a thiazolyl group, an isoxazolyl group, an isothiazolyl group, an oxadiazolyl group, a thiadiazolyl group, and a condensed heteroaryl group, each of which may be substituted or unsubstituted, or $G^A-R^{17}$ (wherein $G^A$ represents -CO-, -C(=O)O-, or $-SO_2-$, $R^{17}$ has the same meaning as that defined above for $R^7$)} (wherein when $W^A$ represents an oxygen atom, $Z^A$ may represent a pyridyl group substituted with a cyano group; when B represents -CO- or $-CH_2CO-$, $Z^A$ may represent a pyridyl group substituted with a nitro group or a cyano group)> or a pharmacologically acceptable salt thereof.

**2.** The compound according to claim 1, wherein D represents $-W^A-X^A-Y^A-Z^A$, or a pharmacologically acceptable salt thereof.

**3.** The compound according to claim 1 or claim 2, wherein B represents $CO(C(R^3)(R^4))_{m-}$ (wherein $R^3$ , $R^4$, and m have the same meanings as those defined above, respectively), or a pharmacologically acceptable salt thereof.

**4.** The compound according to claim 2, wherein A represents a substituted or unsubstituted 1-pyrrolidinyl group or a substituted or unsubstituted 3-thiazolidinyl group;

B represents $-CO(C(R^3)(R^4))_m-$ (wherein $R^3$, $R^4$, and m have the same meanings as those defined above, respectively);
D represents $-W^C-X^C-Y^C-Z^C$ {wherein $W^C$ and $Y^C$ represent $-N(R^{8C})-$ (wherein $R^{8C}$ has the same meaning as that defined above for $R^7$); $X^C$ represents a substituted or unsubstituted alicyclic alkylene group, a group formed by eliminating one hydrogen atom from a substituted or unsubstituted alicyclic heterocyclic group, a substituted or unsubstituted arylene group, a substituted or unsubstituted aralkylene group, a substituted or unsubstituted heteroarylene group, a substituted or unsubstituted heteroarylalkylene group, or $-(C(R^9)(R^{10}))_{q-}$ [wherein q represents an integer of 1 to 6, $R^9$ and $R^{10}$ may be the same or different and each represents a hydrogen atom, a substituted or unsubstituted alkyl group, a substituted or unsubstituted alicyclic alkyl group, a substituted or unsubstituted alicyclic heterocyclic group, a substituted or unsubstituted alkenyl group, a substituted or unsubstituted alkynyl group, a substituted or unsubstituted aryl group, a substituted or unsubstituted aralkyl group, a substituted or unsubstituted heteroaryl group, or a substituted or unsubstituted heteroarylalkyl group, or $R^9$ and $R^{10}$, which attach to the same carbon atom, may combine together with said carbon atom to form a substituted or unsubstituted alicyclic alkyl group (provided that not all of the substituents $R^9$ and $R^{10}$ in the chain consisting of $(C(R^9)(R^{10}))_q-$ are hydrogen atoms)], or $X^c$ combines with one $-N(R^{8C})$-represented by $W^C$ or $Y^C$ to form a substituted or unsubstituted pyrrolidinediyl group, a substituted or unsubstituted piperidinediyl group, or a substituted or unsubstituted homopiperidinediyl group; and $Z^C$ has the same meaning as that defined above for V}, or a pharmacologically acceptable salt thereof.

**5.** The compound according to claim 2, wherein A represents a substituted or unsubstituted 1-pyrrolidinyl group or a substituted or unsubstituted 3-thiazolidinyl group;

B represents $-CO(C(R^3)(R^4))_{m-}$ (wherein $R^3$ , $R^4$, and m have the same meanings as those defined above, respectively);

D represents $-W^D-X^D-Y^D-Z^D$ {wherein $X^D$ represents $-(CH_2)_r-$ (wherein r represents an integer of 1 to 6), $W^D$ and $Y^D$ represents $-N(R^{8D})-$ (wherein $R^{8D}$ has the same meaning as that defined above for $R^7$), $Z^D$ represents a substituted or unsubstituted alicyclic alkyl group, a substituted or unsubstituted alicyclic heterocyclic group, a substituted or unsubstituted alkenyl group, a substituted or unsubstituted alkynyl group, a substituted or unsubstituted aralkyl group, a substituted or unsubstituted heteroarylalkyl group, a pyridyl group substituted with $-SO_2-N(R^{15})(R^{16})$ [wherein $R^{15}$ and $R^{16}$ may be the same or different and each represents a hydrogen atom, a substituted or unsubstituted alkyl group, a substituted or unsubstituted aryl group, a substituted or unsubstituted aralkyl group, a substituted or unsubstituted heteroaryl group, or a substituted or unsubstituted heteroarylalkyl group, or $R^{15}$ and $R^{16}$ combine together with the adjacent nitrogen atom to form a substituted or unsubstituted alicyclic heterocyclic group (said alicyclic heterocyclic group is selected from a pyrrolidinyl group, an oxazolidinyl group, a thiazolidinyl group, a piperidino group, a homopiperidino group, a piperazinyl group, a morpholino group, and a thiomorpholino group)], or a group selected from a trifluoromethylphenyl group, a methanesulfonylphenyl group, a nitrophenyl group, a cyanophenyl group, a naphthyl group, a pyrazinyl group, a pyridazinyl group, a triazinyl group, a thienyl group, a furyl group, a pyrrolyl group, an imidazolyl group, a pyrazolyl group, a triazolyl group, a tetrazolyl group, an oxazolyl group, a thiazolyl group, an isoxazolyl group, an isothiazolyl group, an oxadiazolyl group, a thiadiazolyl group, and a condensed heteroaryl group, each of which may be substituted or unsubstituted, or $G^A-R^{17}$ (wherein $G^A$ represents -CO-, -C(=O)O-, or $-SO_2-$, and $R^{17}$ has the same meaning as that defined above for $R^7$), or a pharmacologically acceptable salt thereof.

**6.** The compound according to claim 4 or claim 5, wherein $Z^C$ or $Z^D$ is a substituted or unsubstituted heteroaryl group, or a pharmacologically acceptable salt thereof.

**7.** The compound according to claim 6, wherein the heteroaryl group is a 6-membered heteroaryl ring, or a 10-membered condensed heteroaryl ring wherein a benzene ring is fused, or a pharmacologically acceptable salt thereof.

**8.** The compound according to claim 6 or claim 7, wherein the substituent of the substituted heteroaryl group is a cyano group, a halogen atom, an alkoxy group, a heteroaryl group, or $-SO_2-R^{18}$ (wherein $R^{18}$ represents an alkyl group; a trifluoromethyl group; an alicyclic alkyl group; an alicyclic heterocyclic group; an alkenyl group; an alkynyl group; an aryl group; an aralkyl group; a heteroaryl group; a heteroarylalkyl group; an alkoxy group; an alicyclic alkoxy group; an O-(alicyclic heterocycle)-substituted hydroxyl group; an alkenyloxy group; an alkynyloxy group; an aryloxy group; an aralkyloxy group; a heteroaryloxy group; a heteroarylalkoxy group; an amino group; an alkylamino group; a dialkylamino group; an alicyclic alkylamino group; an N-(alicyclic heterocycle)-substituted amino group; an alkenylamino group; an alkynylamino group; an arylamino group; an aralkylamino group; a heteroarylamino group; or a heteroarylalkylamino group), or a pharmacologically acceptable salt thereof.

**9.** The compound according to any one of claims 2 to 8, wherein $-W^A-X^A-Y^A-$, $-W^C-X^C-Y^C-$, or $-W^D-X^D-Y^D-$ has two nitrogen atoms, and said two nitrogen atoms are separated by 2 to 6 carbon atoms linked to each other, or a pharmacologically acceptable salt thereof.

**10.** The compound according to claim 9, wherein the 2 to 6 carbon atoms linked to each other has 1 to 3 alkyl groups as substituents, or a pharmacologically acceptable salt thereof.

**11.** The compound according to claim 9, wherein the 2 to 6 carbon atoms linked to each other has an alicyclic alkyl group formed together with one of said carbon atoms as a substituent, or a pharmacologically acceptable salt thereof.

**12.** The compound according to claim 8, wherein the substituent of the substituted heteroaryl group is $SO_2-R^{18}$ (wherein $R^{18}$ has the same meaning as that defined above), or a pharmacologically acceptable salt thereof.

**13.** The compound according to claim 6 or claim 7, wherein $X^A$ combines with one $-N(R^{8A})-$ represented by $W^A$ or $Y^A$ to form a substituted or unsubstituted piperidinediyl group, or a pharmacologically acceptable salt thereof.

**14.** The compound according to any one of claims 1 to 13, wherein A is a 2-cyano-1-pyrrolidinyl group, or a pharmacologically acceptable salt thereof.

**15.** A medicament which comprises as an active ingredient the compound according to any one of claims 1 to 14 or a pharmacologically acceptable salt thereof.

**16.** The medicament according to claim 15 used for therapeutic treatment of a pathological condition in which dipeptidylpeptidase-IV is involved.

**17.** The medicament according to claim 15 used for preventive and/or therapeutic treatment of Type II diabetes.

**18.** The medicament according to claim 15 used for preventive and/or therapeutic treatment of a complication accompanying Type II diabetes.

**19.** A medicament for therapeutic treatment of Type II diabetes which comprises as an active ingredient the compound according to any one of claims 1 to 14 or a pharmacologically acceptable salt thereof.

**20.** A medicament for therapeutic treatment of a complication accompanying Type II diabetes which comprises as an active ingredient the compound according to any one of claims 1 to 14 or a pharmacologically acceptable salt thereof.

**21.** A dipeptidylpeptidase-IV inhibitor which comprises as an active ingredient the compound according to any one of claims 1 to 14 or a pharmacologically acceptable salt thereof.

**22.** A combination use for preventive and/or therapeutic treatment of Type II diabetes of the dipeptidylpeptidase-IV inhibitor according to any one of claims 1 to 14 and a medicament for therapeutic treatment of diabetes other than the dipeptidylpeptidase-IV inhibitor.

**23.** A combination use for preventive and/or therapeutic treatment of Type II diabetes of the dipeptidylpeptidase-IV inhibitor according to any one of claims 1 to 14 and one to three medicaments for therapeutic treatment of diabetes selected from a biguanide agent, a sulfonylurea agent, an $\alpha$-glucosidase inhibitor, a PPAR $\gamma$ agonist, a PPAR $\alpha$/ $\gamma$ dual agonist, a SGLT2 inhibitor, an aP2 inhibitor, a glycogen phosphorylase inhibitor, an insulin sensitivity potentiator, a glucagon-like peptide-1 (GLP-1) or the analogues thereof, Insulin, and Meglinitide.

**24.** A combination use for preventive and/or therapeutic treatment of Type II diabetes of the dipeptidylpeptidase- IV inhibitor according to any one of claims 1 to 14 and one to three medicaments for therapeutic treatment of diabetes selected from Metformin, Tolbutamide, Glibenclamide, Glyburide, Glimepiride, Glipiride, Glipizide, Chloropropamide, Gliclazid, Acarbose, Voglibose, Miglitol, Pioglitazone, Troglitazone, Rosiglitazone, Insulin, Gl-262570, Isaglitazone, JTT-501, NN-2344, L895645, YM-440, R-119702, AJ9677, Repaglinide, Nateglinide, KAD1229, AR-HO39242, GW-409544, KRP297, AC2993, T-1095, Exendin-4, LY307161, NN2211, and LY315902.

**25.** A method for therapeutic treatment of a disease selected from the group consisting of Type II diabetes, hyperlipemia, syndrome X, diabetes complications, hyperglycemia, hyperinsulinism, arteriosclerosis, impaired glucose tolerance, infertility, polycystic ovary syndrome, a growth defect, arthritis, rejection against allotransplantation, autoimmune disease, acquired immunodeficiency disease (AIDS), enterocolitis, anorexia, and osteoporosis, comprising administration of a therapeutically effective amount of the compound according to any one of claims 1 to 14 to a human.

## INTERNATIONAL SEARCH REPORT

International application No.
PCT/JP01/11578

A. CLASSIFICATION OF SUBJECT MATTER
(See extra sheet.)

According to International Patent Classification (IPC) or to both national classification and IPC

B. FIELDS SEARCHED

Minimum documentation searched (classification system followed by classification symbols)
(See extra sheet.)

Documentation searched other than minimum documentation to the extent that such documents are included in the fields searched

Electronic data base consulted during the international search (name of data base and, where practicable, search terms used)
CA(STN), REGISTRY(STN), WPIDS(STN)

C. DOCUMENTS CONSIDERED TO BE RELEVANT

| Category* | Citation of document, with indication, where appropriate, of the relevant passages | Relevant to claim No. |
|---|---|---|
| P,X | WO, 01/96295, A (Novartis A.-G.), 20 December, 2001 (20.12.01), (Family: none) | 1-21 |
| X | WO, 00/34241, A (Novartis A.-G.), 15 June, 2000 (15.06.00), Claims, etc & EP 1137635 A | 1-21 |
| X | US, 6011155, A (Novartis A.-G.), 04 January, 2000 (04.01.00), Claims, etc & US 6124305 A | 1-21 |
| X | JP, 2000-511559, A (Novartis A.-G.), 05 September, 2000 (05.09.00), Claims, etc & WO 98/19998 A | 1-21 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 March, 2002 (08.03.02) | 19 March, 2002 (19.03.02) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP01/11578

C (Continuation). DOCUMENTS CONSIDERED TO BE RELEVANT

| Category* | Citation of document, with indication, where appropriate, of the relevant passages | Relevant to claim No. |
|---|---|---|
| X | Hughes, Thomas E.; Mone, Manisha D.; Russell, Mary E.; Weldon, Stephen C.; Villhauer, Edwin B., NVP-DPP728 (1-[[[2-[(5-Cyanopyridin-2-yl)amino]ethyl]amino] acetyl]-2-cyano-(S)-pyrrolidine), a Slow-Binding Inhibitor of Dipeptidyl Peptidase IV, Biochemistry (1999), 38(36), 11597-11603, particularly, Scheme 2. | 1-21 |
| X | Toll, Lawrence; Almquist, Ronald G., Inhibition of [3H]captopril binding by peptide analog angiotensin converting enzyme inhibitors, Biochem. Biophys. Res. Commun. (1986), 135(3), 770-7, particularly, Compound 7 | 1-21 |
| X | Almquist, Ronald G.; Christie, Pamela H.; Chao, Wanru; Johnson, Howard L., Synthesis and biological activity of an amino analog of a tripeptide inhibitor of angiotensin-converting enzyme, J. Pharm. Sci. (1983), 72(1), 63-7, particularly, Compound III | 1-21 |
| X | US, 6107317, A (Novartis A.-G.), 22 August, 2000 (22.08.00), Claims, etc (Family: none) | 1-21 |
| X | WO, 95/31442, A (Nisshin Flour Milling Co., Ltd.), 23 November, 1995 (23.11.95), Example 51 & EP 760368 A | 1,3,15 |
| X | FR, 2152441, A (Delalande.S.A.), 27 April, 1973 (27.04.73), Claims, etc (Family: none) | 1,3,15 |
| X | WO, 99/06397, A (Abbott Laboratories), 11 February, 1999 (11.02.99), Claim 95 & EP 1003740 A | 1,2,9,15 |
| X | Valenta, Vladimir; Sindelar, Karel; Holubek, Jiri; Ryska, Miroslav; Krejci, Ivan; Dlabac, Antonin; Protiva, Miroslav, Potential nootropic agents: synthesis of a series of (2-oxo-1-pyrrolidinyl)acetic acid piperazides, Collect. Czech. Chem. Commun. (1990), 55(6), 1613-29, particularly, 1615 | 1,15 |
| X | Gandolfi, Carmelo A.; Di Domenico, Roberto; Spinelli, Silvano; Gallico, Licia; Fiocchi, Luigi; Lotto, Andrea; Menta, Ernesto; Borghi, Alessandra; Rosa, Carla Dalla; Tognella, Sergio, N-Acyl-2-substituted-1, 3-thiazolidines, a new class of non-narcotic antitussive agents: studies leading to the discovery of ethyl 2-[(2-methoxyphenoxy)methyl]-beta.-oxothiazolidine-3-propanoate, J. Med. Chem. (1995), 38(3), 508-25, compound 22c, 225 | 1,3,15 |

Form PCT/ISA/210 (continuation of second sheet) (July 1998)

**INTERNATIONAL SEARCH REPORT**

International application No.
PCT/JP01/11578

C (Continuation). DOCUMENTS CONSIDERED TO BE RELEVANT

| Category* | Citation of document, with indication, where appropriate, of the relevant passages | Relevant to claim No. |
|---|---|---|
| X | De Julian-Ortiz, Jesus V.; Galvez, Jorge; Munoz-Collado, Carlos; Garcia-Domenech, Ramon; Gimeno-Cardona, Concepcion, Virtual Combinatorial Syntheses and Computational Screening of New Potential Anti-Herpes Compounds, J. Med. Chem. (1999), 42(17), 3308-3314, 7th compound from the top of Table 2. | 1,3 |
| X | OTAKA, Hiroshi; YOSHIDA, Kenji; SUZUKI, Kenji; SHIMOHARA, Koichi; TAJIMA, Shigeru; ITO, Keizo, Benzylpiperazine derivatives. VIII. Syntheses, antiulcer and cytoprotective activities of 1-(aminocarbonylalkyl)-4-benzylpiperazine derivatives and related compounds, Chem. Pharm. Bull. (1988), 36(10), 3948-54, compounds of 1, 3a, 3b, 3c, 3d, 4h, 4J-4v, 4w-4cc | 1,3,15 |
| X | JP, 11-106375, A (Pfizer Pharmaceuticals Inc.), 20 April, 1999 (20.04.99), Choseirei 7 & EP 899261 A | 1,15 |
| X | WO, 92/02498, A (Pulitzer Italiana S.r.l.), 20 February, 1992 (20.02.92), Claims, etc & AU 9181834 A | 1,15 |
| X | JP, 2-45464, A (Meiji Seika Kaisha, Ltd.), 15 February, 1990 (15.02.90), Claims, etc; compound (V) (Family: none) | 1,3,15 |
| X | JP, 62-185068, A (Ciba-Geigy AG.), 13 August, 1987 (13.08.87), Claims, etc & EP 236263 A | 1,15 |
| X | JP, 61-63669, A (Boehringer Biochemia Robbin S.p.A.), 01 April, 1986 (01.04.86), Claims, etc & EP 169581 A | 1,3,15 |
| X | JP, 9-71564, A (Boehringer Biochemia Robbin S.p.A.), 18 March, 1997 (18.03.97), Claims, etc & WO 97/02245 A | 1,3,15 |
| X | JP, 2-229162, A (Nisshin Flour Milling Co., Ltd.), 11 September, 1990 (11.09.90), Compound 12 & EP 385351 A | 1,3,15 |

Form PCT/ISA/210 (continuation of second sheet) (July 1998)

## INTERNATIONAL SEARCH REPORT

International application No.
PCT/JP01/11578

**Box I Observations where certain claims were found unsearchable (Continuation of item 1 of first sheet)**

This international search report has not been established in respect of certain claims under Article 17(2)(a) for the following reasons:

1. [X] Claims Nos.: 22-25
because they relate to subject matter not required to be searched by this Authority, namely:
Claims 22 to 25 pertain to a method for treatment of the human body by therapy.

2. [X] Claims Nos.: 1-21
because they relate to parts of the international application that do not comply with the prescribed requirements to such an extent that no meaningful international search can be carried out, specifically:
(See extra sheet)

3. [ ] Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

Additionally, this international search was made within the range of a reasonable burden.

1. [ ] As all required additional search fees were timely paid by the applicant, this international search report covers all searchable claims.

2. [X] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)

**INTERNATIONAL SEARCH REPORT**

International application No.
PCT/JP01/11578

Continuation of A. CLASSIFICATION OF SUBJECT MATTER
(International Patent Classification (IPC))

Int.Cl$^7$ C07D401/12, 417/12, 401/14, 403/12, 405/12, 207/16, 417/14, A61K31/4709, 31/4725, 31/428, 31/4439, 31/496, 31/4545, 31/497, 31/498, 31/506, 31/433, 31/502, 31/501, 31/5377, 31/517, 31/4025, 31/454, A61P43/00, 3/10, 3/06, 9/10, 15/08, 15/00, 19/02, 37/06, 31/18, 1/04, 1/14
(According to International Patent Classification (IPC) or to both national classification and IPC)

Continuation of B. FIELDS SEARCHED
Minimum Documentation Searched(International Patent Classification (IPC))

Int.Cl$^7$ C07D401/12, 417/12, 401/14, 403/12, 405/12, 207/16, 417/14, A61K31/4709, 31/4725, 31/428, 31/4439, 31/496, 31/4545, 31/497, 31/498, 31/506, 31/433, 31/502, 31/501, 31/5377, 31/517, 31/4025, 31/454
Minimum documentation searched (classification system followed by classification symbols)

Continuation of Box No. I (2) of Continuation of first sheet (1)

The subject matters of claims 1 to 21 are compounds represented by the general formula (I) or medicines each containing any of the compounds as the active ingredient. However, there is no chemical structure common to all of the compounds. Consequently, it cannot be said that the chemical structures of the compounds have a common novel basic skeleton. One invention cannot be clearly grasped from the claims, which describe such compounds.

On the other hand, a search was made through prior-art documents in view of the contents of the description of this application. In part of the search, many compounds falling under the category of the compounds of the invention were found. In addition, there are many compounds which may fall under the category of the compounds of the invention. It is virtually impossible to thoroughly investigate and show all the documents in which those compounds are disclosed.

Therefore, it cannot be said that the claims comply with the given requirements in such a degree that a meaningful international search can be made.

Form PCT/ISA/210 (extra sheet) (July 1998)